# EUROPEAN PATENT SPECIFICATION

(11) **EP 1 539 221 B1**
(45) Date of publication and mention of the grant of the patent: **23.12.2009**
(21) Application number: 03751903.0
(22) Date of filing: 28.08.2003
(51) Int. Cl.: A61K 38/40, A61K 38/16, A61K 38/41, C07K 14/79

(54) **TRANSFERRIN FUSION PROTEIN LIBRARIES**
TRANSFERRIN-FUSIONSPROTEINBIBLIOTHEKEN
BANQUES DE PROTEINES DE FUSION DE LA TRANSFERINE

(30) Priority: 30.08.2002 US 406977 P; 10.03.2003 US 384060; 09.07.2003 US 485404 P
(43) Date of publication of application: 15.06.2005
(73) Proprietor: Biorexis Pharmaceutical Corporation, New York, NY 10017-5755 (US)
(72) Inventor: PRIOR, Christopher, P., New York, NY 10017-5755 (US); TURNER, Andrew, J., Durham NG 27713 (US); SADEGHI, Homayoun, Hillsborough, NC 27278 (US)
(74) Representative: Cripps, Joanna Elizabeth
(86) International application number: PCT/US2003/026779
(87) International publication number: WO 2004/020588

(56) References cited:
- WO-A-03/020746
- US-A- 5 026 651
- US-A- 5 672 683
- US-A- 5 986 067
- US-A1- 2003 221 201
- PARK E ET AL: "Production and characterization of fusion proteins containing transferrin and nerve growth factor" JOURNAL OF DRUG TARGETING, HARWOOD ACADEMIC PUBLISHERS GMBH, DE, vol. 6, no. 1, 1998, pages 53-64, XP002960815 ISSN: 1061-186X
- PARISE F ET AL: "Construction and in vitro functional evaluation of a low-density lipoprotein receptor/transferrin fusion protein as a therapeutic tool for familial hypercholesterolemia" HUMAN GENE THERAPY, MARY ANN LIEBERT, NEW YORK ,NY, US, vol. 10, no. 7, 1 May 1999 (1999-05-01), pages 1219-1228, XP009057500 ISSN: 1043-0342
- SHIN ET AL.: 'Transferin-antibody fusion proteins are effective in brain targeting' PROC. NATL. ACAD. SCI. USA vol. 92, March 1995, pages 2820 - 2824, XP001126925
- ALI ET AL.: 'Trasnferin trojan horses as a rational approach for the biological delivery of therapeutic peptide domains' J. BIOL. CHEM. vol. 274, no. 24, 20 August 1999, pages 24066 - 24073, XP002985527
- GALLOP ET AL.: 'Applications of combinatorial technologies to drug discovery. 1. Background and peptide combinatorial libraries' J. MED. CHEM. vol. 37, no. 9, 29 April 1994, pages 1234 - 1251, XP002911078
- MACGILLIVRAY ET AL.: 'The primary structure of human serum transferin' J. BIOL. CHEM. vol. 258, no. 6, 25 March 1983, pages 3543 - 3553, XP002985528

### SEQUENCE LISTING

<110> Prior, Christopher P.
   Turner, Andrew J. Sadeghi, Homayoun
<120> Transferrin Fusion Protein Libraries
<130> 054710-5007-WO
<150> US 60/406,977
   <151> 2002-08-30
<150> US 10/384,060
   <151> 2003-03-10
<150> US 60/485,404
   <151> 2003-07-09
<160> 75
<170> PatentIn Ver. 2.1
<210> 1
   <211> 2318
   <212> DNA
   <213> Homo sapiens
<220>
   <221> CDS
   <222> (51)..(2147)
   <223> GenBank No. NM_001063, transferrin gene and protein
<220>
   <221> sig_peptide
   <222> (51)..(107)
<400> 1
   gcacagaagc gagtccgact gtgctcgctg ctcagcgccg cacccggaag atg agg     56
                                                           Met Arg
                                                             1
   ctc gcc gtg gga gcc ctg ctg gtc tgc gcc gtc ctg ggg ctg tgt ctg   104
   Leu Ala Val Gly Ala Leu Leu Val Cys Ala Val Leu Gly Leu Cys Leu
           5                   10                  15
   gct gtc cct gat aaa act gtg aga tgg tgt gca gtg tcg gag cat gag   152
   Ala Val Pro Asp Lys Thr Val Arg Trp Cys Ala Val Ser Glu His Glu
        20                  25                  30
   gcc act aag tgc cag agt ttc cgc gac cat atg aaa agc gtc att cca   200
   Ala Thr Lys Cys Gln Ser Phe Arg Asp His Met Lys Ser Val Ile Pro
    35                  40                  45                  50
   tcc gat ggt ccc agt gtt gct tgt gtg aag aaa gcc tcc tac ctt gat   248
   Ser Asp Gly Pro Ser Val Ala Cys Val Lys Lys Ala Ser Tyr Leu Asp
                    55                  60                  65
   tgc atc agg gcc att gcg gca aac gaa gcg gat gct gtg aca ctg gat   296
   Cys Ile Arg Ala Ile Ala Ala Asn Glu Ala Asp Ala Val Thr Leu Asp
                   70                  75                  80
   gca ggt ttg gtg tat gat gct tac ctg gct ccc aat aac ctg aag cct    344
   Ala Gly Leu Val Tyr Asp Ala Tyr Leu Ala Pro Asn Asn Leu Lys Pro
           85                  90                  95
   gtg gtg gca gag ttc tat ggg tca aaa gag gat cca cag act ttc tat    392
   Val Val Ala Glu Phe Tyr Gly Ser Lys Glu Asp Pro Gln Thr Phe Tyr
       100                 105                 110
   tat gct gtt gct gtg gtg aag aag gat agt ggc ttc cag atg aac cag    440
   Tyr Ala Val Ala Val Val Lys Lys Asp Ser Gly Phe Gln Met Asn Gln
   115                 120                 125                 130
   ctt cga ggc aag aag tcc tgc cac acg ggt cta ggc agg tcc gct ggg    488
   Leu Arg Gly Lys Lys Ser Cys His Thr Gly Leu Gly Arg Ser Ala Gly
                   135                 140                 145
   tgg aac atc ccc ata ggc tta ctt tac tgt gac tta cct gag cca cgt    536
   Trp Asn Ile Pro Ile Gly Leu Leu Tyr Cys Asp Leu Pro Glu Pro Arg
               150                 155                 160
   aaa cct ctt gag aaa gca gtg gcc aat ttc ttc tcg ggc agc tgt gcc    584
   Lys Pro Leu Glu Lys Ala Val Ala Asn Phe Phe Ser Gly Ser Cys Ala
           165                 170                 175
   cct tgt gcg gat ggg acg gac ttc ccc cag ctg tgt caa ctg tgt cca    632
   Pro Cys Ala Asp Gly Thr Asp Phe Pro Gln Leu Cys Gln Leu Cys Pro
       180                 185                 190
   ggg tgt ggc tgc tcc acc ctt aac caa tac ttc ggc tac tcg gga gcc    680
   Gly Cys Gly Cys Ser Thr Leu Asn Gln Tyr Phe Gly Tyr Ser Gly Ala
   195                 200                 205                 210
   ttc aag tgt ctg aag gat ggt gct ggg gat gtg gcc ttt gtc aag cac    728
   Phe Lys Cys Leu Lys Asp Gly Ala Gly Asp Val Ala Phe Val Lys His
                   215                 220                 225
   tcg act ata ttt gag aac ttg gca aac aag gct gac agg gac cag tat    776
   Ser Thr Ile Phe Glu Asn Leu Ala Asn Lys Ala Asp Arg Asp Gln Tyr
               230                 235                 240
   gag ctg ctt tgc ctg gac aac acc cgg aag ccg gta gat gaa tac aag    824
   Glu Leu Leu Cys Leu Asp Asn Thr Arg Lys Pro Val Asp Glu Tyr Lys
           245                 250                 255
   gac tgc cac ttg gcc cag gtc cct tct cat acc gtc gtg gcc cga agt    872
   Asp Cys His Leu Ala Gln Val Pro Ser His Thr Val Val Ala Arg Ser
       260                 265                 270
   atg ggc ggc aag gag gac ttg atc tgg gag ctt ctc aac cag gcc cag    920
   Met Gly Gly Lys Glu Asp Leu Ile Trp Glu Leu Leu Asn Gln Ala Gln
   275                 280                 285                 290
   gaa cat ttt ggc aaa gac aaa tca aaa gaa ttc caa cta ttc agc tct    968
   Glu His Phe Gly Lys Asp Lys Ser Lys Glu Phe Gln Leu Phe Ser Ser
                   295                 300                 305
   cct cat ggg aag gac ctg ctg ttt aag gac tct gcc cac ggg ttt tta    1016
   Pro His Gly Lys Asp Leu Leu Phe Lys Asp Ser Ala His Gly Phe Leu
               310                 315                 320
   aaa gtc ccc ccc agg atg gat gcc aag atg tac ctg ggc tat gag tat    1064
   Lys Val Pro Pro Arg Met Asp Ala Lys Met Tyr Leu Gly Tyr Glu Tyr
           325                 330                 335
   gtc act gcc atc cgg aat cta cgg gaa ggc aca tgc cca gaa gcc cca    1112
   Val Thr Ala Ile Arg Asn Leu Arg Glu Gly Thr Cys Pro Glu Ala Pro
       340                 345                 350
   aca gat gaa tgc aag cct gtg aag tgg tgt gcg ctg agc cac cac gag    1160
   Thr Asp Glu Cys Lys Pro Val Lys Trp Cys Ala Leu Ser His His Glu
   355                 360                 365                 370
   agg ctc aag tgt gat gag tgg agt gtt aac agt gta ggg aaa ata gag    1208
   Arg Leu Lys Cys Asp Glu Trp Ser Val Asn Ser Val Gly Lys Ile Glu
                   375                 380                 385
   tgt gta tca gca gag acc acc gaa gac tgc atc gcc aag atc atg aat    1256
   Cys Val Ser Ala Glu Thr Thr Glu Asp Cys Ile Ala Lys Ile Met Asn
               390                 395                 400
   gga gaa gct gat gcc atg agc ttg gat gga ggg ttt gtc tac ata gcg    1304
   Gly Glu Ala Asp Ala Met Ser Leu Asp Gly Gly Phe Val Tyr Ile Ala
           405                 410                 415
   ggc aag tgt ggt ctg gtg cct gtc ttg gca gaa aac tac aat aag agc    1352
   Gly Lys Cys Gly Leu Val Pro Val Leu Ala Glu Asn Tyr Asn Lys Ser
       420                 425                 430
   gat aat tgt gag gat aca cca gag gca ggg tat ttt gct gta gca gtg    1400
   Asp Asn Cys Glu Asp Thr Pro Glu Ala Gly Tyr Phe Ala Val Ala Val
   435                 440                 445                 450
   gtg aag aaa tca gct tct gac ctc acc tgg gac aat ctg aaa ggc aag    1448
   Val Lys Lys Ser Ala Ser Asp Leu Thr Trp Asp Asn Leu Lys Gly Lys
                   455                 460                 465
   aag tcc tgc cat acg gca gtt ggc aga acc gct ggc tgg aac atc ccc    1496
   Lys Ser Cys His Thr Ala Val Gly Arg Thr Ala Gly Trp Asn Ile Pro
               470                 475                 480
   atg ggc ctg ctc tac aat aag atc aac cac tgc aga ttt gat gaa ttt    1544
   Met Gly Leu Leu Tyr Asn Lys Ile Asn His Cys Arg Phe Asp Glu Phe
           485                 490                 495
   ttc agt gaa ggt tgt gcc cct ggg tct aag aaa gac tcc agt ctc tgt    1592
   Phe Ser Glu Gly Cys Ala Pro Gly Ser Lys Lys Asp Ser Ser Leu Cys
       500                 505                 510
   aag ctg tgt atg ggc tca ggc cta aac ctg tgt gaa ccc aac aac aaa    1640
   Lys Leu Cys Met Gly Ser Gly Leu Asn Leu Cys Glu Pro Asn Asn Lys
   515                 520                 525                 530
   gag gga tac tac ggc tac aca ggc gct ttc agg tgt ctg gtt gag aag    1688
   Glu Gly Tyr Tyr Gly Tyr Thr Gly Ala Phe Arg Cys Leu Val Glu Lys
                   535                 540                 545
   gga gat gtg gcc ttt gtg aaa cac cag act gtc cca cag aac act ggg    1736
   Gly Asp Val Ala Phe Val Lys His Gln Thr Val Pro Gln Asn Thr Gly
               550                 555                 560
   gga aaa aac cct gat cca tgg gct aag aat ctg aat gaa aaa gac tat    1784
   Gly Lys Asn Pro Asp Pro Trp Ala Lys Asn Leu Asn Glu Lys Asp Tyr
           565                 570                 575
   gag ttg ctg tgc ctt gat ggt acc agg aaa cct gtg gag gag tat gcg    1832
   Glu Leu Leu Cys Leu Asp Gly Thr Arg Lys Pro Val Glu Glu Tyr Ala
       580                 585                 590
   aac tgc cac ctg gcc aga gcc ccg aat cac gct gtg gtc aca cgg aaa    1880
   Asn Cys His Leu Ala Arg Ala Pro Asn His Ala Val Val Thr Arg Lys
   595                 600                 605                 610
   gat aag gaa gct tgc gtc cac aag ata tta cgt caa cag cag cac cta    1928
   Asp Lys Glu Ala Cys Val His Lys Ile Leu Arg Gln Gln Gln His Leu
                   615                 620                 625
   ttt gga agc aac gta act gac tgc tcg ggc aac ttt tgt ttg ttc cgg    1976
   Phe Gly Ser Asn Val Thr Asp Cys Ser Gly Asn Phe Cys Leu Phe Arg
               630                 635                 640
   tcg gaa acc aag gac ctt ctg ttc aga gat gac aca gta tgt ttg gcc    2024
   Ser Glu Thr Lys Asp Leu Leu Phe Arg Asp Asp Thr Val Cys Leu Ala
           645                 650                 655
   aaa ctt cat gac aga aac aca tat gaa aaa tac tta gga gaa gaa tat    2072
   Lys Leu His Asp Arg Asn Thr Tyr Glu Lys Tyr Leu Gly Glu Glu Tyr
       660                 665                 670
   gtc aag gct gtt ggt aac ctg aga aaa tgc tcc acc tca tca ctc ctg    2120
   Val Lys Ala Val Gly Asn Leu Arg Lys Cys Ser Thr Ser Ser Leu Leu
   675                 680                 685                 690
   gaa gcc tgc act ttc cgt aga cct taa aatctcagag gtagggctgc          2167
   Glu Ala Cys Thr Phe Arg Arg Pro
                   695
   caccaaggtg aagatgggaa cgcagatgat ccatgagttt gccctggttt cactggccca 2227
   agtggtttgt gctaaccacg tctgtcttca cagctctgtg ttgccatgtg tgctgaacaa 2287
   aaaataaaaa ttattattga ttttatattt c                                 2318
<210> 2
   <211> 698
   <212> PRT
   <213> Homo sapiens
<400> 2
   Met Arg Leu Ala Val Gly Ala Leu Leu Val Cys Ala Val Leu Gly Leu
    1               5                  10                  15
   Cys Leu Ala Val Pro Asp Lys Thr Val Arg Trp Cys Ala Val Ser Glu
               20                  25                  30
   His Glu Ala Thr Lys Cys Gln Ser Phe Arg Asp His Met Lys Ser Val
           35                  40                  45
   Ile Pro Ser Asp Gly Pro Ser Val Ala Cys Val Lys Lys Ala Ser Tyr
       50                  55                  60
   Leu Asp Cys Ile Arg Ala Ile Ala Ala Asn Glu Ala Asp Ala Val Thr
   65 70 75 80
   Leu Asp Ala Gly Leu Val Tyr Asp Ala Tyr Leu Ala Pro Asn Asn Leu
   85 90 95
   Lys Pro Val Val Ala Glu Phe Tyr Gly Ser Lys Glu Asp Pro Gln Thr
   100 105 110
   Phe Tyr Tyr Ala Val Ala Val Val Lys Lys Asp Ser Gly Phe Gln Met
   115 120 125
   Asn Gln Leu Arg Gly Lys Lys Ser Cys His Thr Gly Leu Gly Arg Ser
   130 135 140
   Ala Gly Trp Asn Ile Pro Ile Gly Leu Leu Tyr Cys Asp Leu Pro Glu
   145 150 155 160
   Pro Arg Lys Pro Leu Glu Lys Ala Val Ala Asn Phe Phe Ser Gly Ser
   165 170 175
   Cys Ala Pro Cys Ala Asp Gly Thr Asp Phe Pro Gln Leu Cys Gln Leu
   180 185 190
   Cys Pro Gly Cys Gly Cys Ser Thr Leu Asn Gln Tyr Phe Gly Tyr Ser
   195 200 205
   Gly Ala Phe Lys Cys Leu Lys Asp Gly Ala Gly Asp Val Ala Phe Val
   210 215 220
   Lys His Ser Thr Ile Phe Glu Asn Leu Ala Asn Lys Ala Asp Arg Asp
   225 230 235 240
   Gln Tyr Glu Leu Leu Cys Leu Asp Asn Thr Arg Lys Pro Val Asp Glu
   245 250 255
   Tyr Lys Asp Cys His Leu Ala Gln Val Pro Ser His Thr Val Val Ala
   260 265 270
   Arg Ser Met Gly Gly Lys Glu Asp Leu Ile Trp Glu Leu Leu Asn Gln
   275 280 285
   Ala Gln Glu His Phe Gly Lys Asp Lys Ser Lys Glu Phe Gln Leu Phe
   290 295 300
   Ser Ser Pro His Gly Lys Asp Leu Leu Phe Lys Asp Ser Ala His Gly
   305 310 315 320
   Phe Leu Lys Val Pro Pro Arg Met Asp Ala Lys Met Tyr Leu Gly Tyr
   325 330 335
   Glu Tyr Val Thr Ala Ile Arg Asn Leu Arg Glu Gly Thr Cys Pro Glu
   340 345 350
   Ala Pro Thr Asp Glu Cys Lys Pro Val Lys Trp Cys Ala Leu Ser His
   355 360 365
   His Glu Arg Leu Lys Cys Asp Glu Trp Ser Val Asn Ser Val Gly Lys
   370 375 380
   Ile Glu Cys Val Ser Ala Glu Thr Thr Glu Asp Cys Ile Ala Lys Ile
   385                 390                 395                 400
   Met Asn Gly Glu Ala Asp Ala Met Ser Leu Asp Gly Gly Phe Val Tyr
                   405                 410                 415
   Ile Ala Gly Lys Cys Gly Leu Val Pro Val Leu Ala Glu Asn Tyr Asn
               420                 425                 430
   Lys Ser Asp Asn Cys Glu Asp Thr Pro Glu Ala Gly Tyr Phe Ala Val
           435                 440                 445
   Ala Val Val Lys Lys Ser Ala Ser Asp Leu Thr Trp Asp Asn Leu Lys
       450                 455                 460
   Gly Lys Lys Ser Cys His Thr Ala Val Gly Arg Thr Ala Gly Trp Asn
   465                 470                 475                 480
   Ile Pro Met Gly Leu Leu Tyr Asn Lys Ile Asn His Cys Arg Phe Asp
                   485                 490                 495
   Glu Phe Phe Ser Glu Gly Cys Ala Pro Gly Ser Lys Lys Asp Ser Ser
               500                 505                 510
   Leu Cys Lys Leu Cys Met Gly Ser Gly Leu Asn Leu Cys Glu Pro Asn
           515                 520                 525
   Asn Lys Glu Gly Tyr Tyr Gly Tyr Thr Gly Ala Phe Arg Cys Leu Val
       530                 535                 540
   Glu Lys Gly Asp Val Ala Phe Val Lys His Gln Thr Val Pro Gln Asn
   545                 550                 555                 560
   Thr Gly Gly Lys Asn Pro Asp Pro Trp Ala Lys Asn Leu Asn Glu Lys
                   565                 570                 575
   Asp Tyr Glu Leu Leu Cys Leu Asp Gly Thr Arg Lys Pro Val Glu Glu
               580                 585                 590
   Tyr Ala Asn Cys His Leu Ala Arg Ala Pro Asn His Ala Val Val Thr
           595                 600                 605
   Arg Lys Asp Lys Glu Ala Cys Val His Lys Ile Leu Arg Gln Gln Gln
       610                 615                 620
   His Leu Phe Gly Ser Asn Val Thr Asp Cys Ser Gly Asn Phe Cys Leu
   625                 630                 635                 640
   Phe Arg Ser Glu Thr Lys Asp Leu Leu Phe Arg Asp Asp Thr Val Cys
                   645                 650                 655
   Leu Ala Lys Leu His Asp Arg Asn Thr Tyr Glu Lys Tyr Leu Gly Glu
               660                 665                 670
   Glu Tyr Val Lys Ala Val Gly Asn Leu Arg Lys Cys Ser Thr Ser Ser
           675                 680                 685
   Leu Leu Glu Ala Cys Thr Phe Arg Arg Pro
       690                 695
<210> 3
   <211> 679
   <212> PRT
   <213> Homo sapiens
<220>
   <223> Mature transferrin protein
<400> 3
   Val Pro Asp Lys Thr Val Arg Trp Cys Ala Val Ser Glu His Glu Ala
   1 5 10 15
   Thr Lys Cys Gln Ser Phe Arg Asp His Met Lys Ser Val Ile Pro Ser
   20 25 30
   Asp Gly Pro Ser Val Ala Cys Val Lys Lys Ala Ser Tyr Leu Asp Cys
   35 40 45
   Ile Arg Ala Ile Ala Ala Asn Glu Ala Asp Ala Val Thr Leu Asp Ala
   50 55 60
   Gly Leu Val Tyr Asp Ala Tyr Leu Ala Pro Asn Asn Leu Lys Pro Val
   65 70 75 80
   Val Ala Glu Phe Tyr Gly Ser Lys Glu Asp Pro Gln Thr Phe Tyr Tyr
   85 90 95
   Ala Val Ala Val Val Lys Lys Asp Ser Gly Phe Gln Met Asn Gln Leu
   100 105 110
   Arg Gly Lys Lys Ser Cys His Thr Gly Leu Gly Arg Ser Ala Gly Trp
   115 120 125
   Asn Ile Pro Ile Gly Leu Leu Tyr Cys Asp Leu Pro Glu Pro Arg Lys
   130 135 140
   Pro Leu Glu Lys Ala Val Ala Asn Phe Phe Ser Gly Ser Cys Ala Pro
   145 150 155 160
   Cys Ala Asp Gly Thr Asp Phe Pro Gln Leu Cys Gln Leu Cys Pro Gly
   165 170 175
   Cys Gly Cys Ser Thr Leu Asn Gln Tyr Phe Gly Tyr Ser Gly Ala Phe
   180 185 190
   Lys Cys Leu Lys Asp Gly Ala Gly Asp Val Ala Phe Val Lys His Ser
   195 200 205
   Thr Ile Phe Glu Asn Leu Ala Asn Lys Ala Asp Arg Asp Gln Tyr Glu
   210 215 220
   Leu Leu Cys Leu Asp Asn Thr Arg Lys Pro Val Asp Glu Tyr Lys Asp
   225 230 235 240
   Cys His Leu Ala Gln Val Pro Ser His Thr Val Val Ala Arg Ser Met
   245 250 255
   Gly Gly Lys Glu Asp Leu Ile Trp Glu Leu Leu Asn Gln Ala Gln Glu
   260 265 270
   His Phe Gly Lys Asp Lys Ser Lys Glu Phe Gln Leu Phe Ser Ser Pro
   275 280 285
   His Gly Lys Asp Leu Leu Phe Lys Asp Ser Ala His Gly Phe Leu Lys
   290 295 300
   Val Pro Pro Arg Met Asp Ala Lys Met Tyr Leu Gly Tyr Glu Tyr Val
   305 310 315 320
   Thr Ala Ile Arg Asn Leu Arg Glu Gly Thr Cys Pro Glu Ala Pro Thr
   325 330 335
   Asp Glu Cys Lys Pro Val Lys Trp Cys Ala Leu Ser His His Glu Arg
   340 345 350
   Leu Lys Cys Asp Glu Trp Ser Val Asn Ser Val Gly Lys Ile Glu Cys
   355 360 365
   Val Ser Ala Glu Thr Thr Glu Asp Cys Ile Ala Lys Ile Met Asn Gly
   370 375 380
   Glu Ala Asp Ala Met Ser Leu Asp Gly Gly Phe Val Tyr Ile Ala Gly
   385 390 395 400
   Lys Cys Gly Leu Val Pro Val Leu Ala Glu Asn Tyr Asn Lys Ser Asp
   405 410 415
   Asn Cys Glu Asp Thr Pro Glu Ala Gly Tyr Phe Ala Val Ala Val Val
   420 425 430
   Lys Lys Ser Ala Ser Asp Leu Thr Trp Asp Asn Leu Lys Gly Lys Lys
   435 440 445
   Ser Cys His Thr Ala Val Gly Arg Thr Ala Gly Trp Asn Ile Pro Met
   450 455 460
   Gly Leu Leu Tyr Asn Lys Ile Asn His Cys Arg Phe Asp Glu Phe Phe
   465 470 475 480
   Ser Glu Gly Cys Ala Pro Gly Ser Lys Lys Asp Ser Ser Leu Cys Lys
   485 490 495
   Leu Cys Met Gly Ser Gly Leu Asn Leu Cys Glu Pro Asn Asn Lys Glu
   500 505 510
   Gly Tyr Tyr Gly Tyr Thr Gly Ala Phe Arg Cys Leu Val Glu Lys Gly
   515 520 525
   Asp Val Ala Phe Val Lys His Gln Thr Val Pro Gln Asn Thr Gly Gly
   530 535 540
   Lys Asn Pro Asp Pro Trp Ala Lys Asn Leu Asn Glu Lys Asp Tyr Glu
   545 550 555 560
   Leu Leu Cys Leu Asp Gly Thr Arg Lys Pro Val Glu Glu Tyr Ala Asn
   565 570 575
   Cys His Leu Ala Arg Ala Pro Asn His Ala Val Val Thr Arg Lys Asp
   580 585 590
   Lys Glu Ala Cys Val His Lys Ile Leu Arg Gln Gln Gln His Leu Phe
   595 600 605
   Gly Ser Asn Val Thr Asp Cys Ser Gly Asn Phe Cys Leu Phe Arg Ser
   610 615 620
   Glu Thr Lys Asp Leu Leu Phe Arg Asp Asp Thr Val Cys Leu Ala Lys
   625 630 635 640
   Leu His Asp Arg Asn Thr Tyr Glu Lys Tyr Leu Gly Glu Glu Tyr Val
   645 650 655
   Lys Ala Val Gly Asn Leu Arg Lys Cys Ser Thr Ser Ser Leu Leu Glu
   660 665 670
   Ala Cys Thr Phe Arg Arg Pro
   675
<210> 4
   <211> 36
   <212> PRT
   <213> Human immunodeficiency virus
<220>
   <223> Antifusogenic peptide
<400> 4
   Phe Trp Asn Trp Leu Ser Ala Trp Lys Asp Leu Glu Leu Leu Glu Gln
   1 5 10 15
   Glu Asn Lys Glu Gln Gln Asn Gln Ser Glu Glu Ile Leu Ser His Ile
   20 25 30
   Leu Ser Thr Tyr
   35
<210> 5
   <211> 35
   <212> PRT
   <213> Human respiratory syncytial virus
<220>
   <223> Antifusogenic peptide
<400> 5
   Val Tyr Pro Ser Asp Glu Tyr Asp Ala Ser Ile Ser Gln Val Asn Glu
   1 5 10 15
   Glu Ile Asn Gln Ala Leu Ala Tyr Ile Arg Lys Ala Asp Glu Leu Leu
   20 25 30
   Glu Asn Val
   35
<210> 6
   <211> 51
   <212> PRT
   <213> Human respiratory syncytial virus
<220>
   <223> Antifusogenic peptide
<400> 6
   Ala Val Ser Lys Val Leu His Leu Glu Gly Glu Val Asn Lys Ile Lys
   1               5                   10                  15
   Ser Ala Leu Leu Ser Thr Asn Lys Ala Val Val Ser Leu Ser Asn Gly
               20                  25                  30
   Val Ser Val Leu Thr Ser Lys Val Leu Asp Leu Lys Asn Tyr Ile Asp
           35                  40                  45
   Lys Gln Leu
       50
<210> 7
   <211> 35
   <212> PRT
   <213> Human respiratory syncytial virus
<220>
   <223> Antifusogenic peptide
<400> 7
   Val Phe Pro Ser Asp Glu Phe Asp Ala Ser Ile Ser Gln Val Asn Glu
   1               5                   10                  15
   Lys Ile Asn Gln Ser Leu Ala Phe Ile Arg Glu Ser Asp Glu Leu Leu
               20                  25                  30
   His Asn Val
           35
<210> 8
   <211> 12
   <212> PRT
   <213> Homo sapiens
<220>
   <223> Lactoferrin splice variant sequence
<400> 8
   Glu Asp Cys Ile Ala Leu Lys Gly Glu Ala Asp Ala
   1               5                   10
<210> 9
   <211> 27
   <212> DNA
   <213> Artificial Sequence
<220>
   <223> Description of Artificial Sequence: Oligonucleotide for mutagenesis
<400> 9
   gcagaaaact acgataagag cgataat 27
<210> 10
   <211> 27
   <212> DNA
   <213> Artificial Sequence
<220>
<223> Description of Artificial Sequence: Oligonucleotide for mutagenesis
<400> 10
   ctatttggaa gcgacgtaac tgactgc 27
<210> 11
   <211> 20
   <212> PRT
   <213> Artificial Sequence
<220>
   <223> Description of Artificial Sequence: EPO mimetic peptide
<400> 11
   Gly Gly Thr Tyr Ser Cys His Phe Gly Pro Leu Thr Trp Val Cys Lys
   1               5                   10                  15
   Pro Gln Gly Gly
               20
<210> 12
   <211> 18
   <212> PRT
   <213> Artificial Sequence
<220>
   <223> Description of Artificial Sequence: EPO mimetic peptide
<400> 12
   Asp Arg Glu Gly Cys Arg Arg Gly Trp Val Gly Gln Cys Lys Ala Trp
   1               5                   10                  15
   Phe Asn
<210> 13
   <211> 108
   <212> DNA
   <213> Artificial Sequence
<220>
   <223> Description of Artificial Sequence: HIV antifusogenic sequences
<220>
   <221> CDS
   <222> (1)..(108)
<400> 13
   tac aca agc tta ata cac tcc tta att gaa gaa tcg caa aac cag caa    48
   Tyr Thr Ser Leu Ile His Ser Leu Ile Glu Glu Ser Gln Asn Gln Gln
   1               5                   10                  15
   gaa aag aat gaa caa gaa tta ttg gaa tta gat aaa tgg gca agt ttg    96
   Glu Lys Asn Glu Gln Glu Leu Leu Glu Leu Asp Lys Trp Ala Ser Leu
               20                  25                  30
   tgg aat tgg ttt                                                    108
   Trp Asn Trp Phe
           35
<210> 14
   <211> 36
   <212> PRT
   <213> Artificial Sequence
<220>
   <223> Description of Artificial Sequence: HIV antifusogenic sequences
<400> 14
   Tyr Thr Ser Leu Ile His Ser Leu Ile Glu Glu Ser Gln Asn Gln Gln
   1               5                   10                  15
   Glu Lys Asn Glu Gln Glu Leu Leu Glu Leu Asp Lys Trp Ala Ser Leu
               20                  25                  30
   Trp Asn Trp Phe
           35
<210> 15
   <211> 124
   <212> DNA
   <213> Artificial Sequence
<220>
   <223> Description of Artificial Sequence: HIV antifusogenic sequences for fusion proteins
<220>
   <221> CDS
   <222> (1)..(123)
<400> 15
   cta gag aaa agg tac act agc tta ata cac tcc tta att gaa gaa tcg    48
   Leu Glu Lys Arg Tyr Thr Ser Leu Ile His Ser Leu Ile Glu Glu Ser
     1               5                  10                  15
   caa aac cag caa gaa aag aat gaa caa gaa tta ttg gaa tta gat aaa    96
   Gln Asn Gln Gln Glu Lys Asn Glu Gln Glu Leu Leu Glu Leu Asp Lys
                20                  25                  30
   tgg gca agt ttg tgg aat tgg ttt gta c                              124
   Trp Ala Ser Leu Trp Asn Trp Phe Val
            35                  40
<210> 16
   <211> 41
   <212> PRT
   <213> Artificial Sequence
<220>
   <223> Description of Artificial Sequence: HIV antifusogenic sequences for fusion proteins
<400> 16
   Leu Glu Lys Arg Tyr Thr Ser Leu Ile His Ser Leu Ile Glu Glu Ser
     1               5                  10                  15
   Gln Asn Gln Gln Glu Lys Asn Glu Gln Glu Leu Leu Glu Leu Asp Lys
                20                  25                  30
   Trp Ala Ser Leu Trp Asn Trp Phe Val
            35                  40
<210> 17
   <211> 174
   <212> PRT
   <213> Homo sapiens
<220>
   <223> INGAP protein
<400> 17
   Met Met Leu Pro Met Thr Leu Cys Arg Met Ser Trp Met Leu Leu Ser
     1               5                  10                  15
   Cys Leu Met Phe Leu Ser Trp Val Glu Gly Glu Glu Ser Gln Lys Lys
               20                  25                  30
   Leu Pro Ser Ser Arg Ile Thr Cys Pro Gln Gly Ser Val Ala Tyr Gly
           35                  40                  45
   Ser Tyr Cys Tyr Ser Leu Ile Leu Ile Pro Gln Thr Trp Ser Asn Ala
       50                  55                  60
   Glu Leu Ser Cys Gln Met His Phe Ser Gly His Leu Ala Phe Leu Leu
    65                  70                  75                  80
   Ser Thr Gly Glu Ile Thr Phe Val Ser Ser Leu Val Lys Asn Ser Leu
                   85                  90                  95
   Thr Ala Tyr Gln Tyr Ile Trp Ile Gly Leu His Asp Pro Ser His Gly
               100                 105                 110
   Thr Leu Pro Asn Gly Gly Trp Lys Trp Ser Ser Ser Asn Val Leu Thr
           115                 120                 125
   Phe Tyr Asn Trp Glu Arg Asn Pro Ser Ile Ala Ala Asp Arg Gly Tyr
       130                 135                 140
   Cys Ala Val Leu Ser Gln Lys Ser Gly Phe Gln Lys Trp Arg Asp Phe
   145                 150                 155                 160
   Asn Cys Glu Asn Glu Leu Pro Tyr Ile Cys Lys Phe Lys Val
                   165                 170
<210> 18
   <211> 525
   <212> DNA
   <213> Artificial Sequence
<220>
   <223> Description of Artificial Sequence: INGAP sequences
<220>
   <221> CDS
   <222> (1)..(525)
<400> 18
   atg atg ttg cca atg act ttg tgt aga atg tct tgg atg ttg ttg tct    48
   Met Met Leu Pro Met Thr Leu Cys Arg Met Ser Trp Met Leu Leu Ser
    1               5                  10                  15
   tgt ttg atg ttt ttg tct tgg gtt gaa ggt gaa gaa tct caa aaa aaa    96
   Cys Leu Met Phe Leu Ser Trp Val Glu Gly Glu Glu Ser Gln Lys Lys
               20                  25                  30
   ttg cca tct tct aga att act tgt cca caa ggt tct gtt gct tat ggt    144
   Leu Pro Ser Ser Arg Ile Thr Cys Pro Gln Gly Ser Val Ala Tyr Gly
           35                  40                  45
   tct tat tgt tat tct ttg att ttg att cca caa act tgg tct aat gct    192
   Ser Tyr Cys Tyr Ser Leu Ile Leu Ile Pro Gln Thr Trp Ser Asn Ala
       50                  55                  60
   gaa ttg tct tgt caa atg cat ttt tct ggt cat ttg gct ttt ttg ttg    240
   Glu Leu Ser Cys Gln Met His Phe Ser Gly His Leu Ala Phe Leu Leu
   65                  70                  75                  80
   tct act ggt gaa att act ttt gtt tct tct ttg gtt aaa aat tct ttg    288
   Ser Thr Gly Glu Ile Thr Phe Val Ser Ser Leu Val Lys Asn Ser Leu
                   85                  90                  95
   act gct tat caa tat att tgg att ggt ttg cat gat cca tct cat ggt    336
   Thr Ala Tyr Gln Tyr Ile Trp Ile Gly Leu His Asp Pro Ser His Gly
               100                 105                 110
   act ttg cca aat ggt tct ggt tgg aaa tgg tct tct tct aat gtt ttg    384
   Thr Leu Pro Asn Gly Ser Gly Trp Lys Trp Ser Ser Ser Asn Val Leu
           115                 120                 125
   act ttt tat aat tgg gaa aga aat cca tct att gct gct gat aga ggt    432
   Thr Phe Tyr Asn Trp Glu Arg Asn Pro Ser Ile Ala Ala Asp Arg Gly
       130                 135                 140
   tat tgt gct gtt ttg tct caa aaa tct ggt ttt caa aaa tgg aga gat    480
   Tyr Cys Ala Val Leu Ser Gln Lys Ser Gly Phe Gln Lys Trp Arg Asp
   145                 150                 155                 160
   ttt aat tgt gaa aat gaa ttg cca tat att tgt aaa ttt aaa gtt        525
   Phe Asn Cys Glu Asn Glu Leu Pro Tyr Ile Cys Lys Phe Lys Val
                   165                 170                 175
<210> 19
   <211> 175
   <212> PRT
   <213> Artificial Sequence
<220>
   <223> Description of Artificial Sequence: INGAP sequences
<400> 19
   Met Met Leu Pro Met Thr Leu Cys Arg Met Ser Trp Met Leu Leu Ser
   1               5                   10                  15
   Cys Leu Met Phe Leu Ser Trp Val Glu Gly Glu Glu Ser Gln Lys Lys
               20                  25                  30
   Leu Pro Ser Ser Arg Ile Thr Cys Pro Gln Gly Ser Val Ala Tyr Gly
           35                  40                  45
   Ser Tyr Cys Tyr Ser Leu Ile Leu Ile Pro Gln Thr Trp Ser Asn Ala
       50                  55                  60
   Glu Leu Ser Cys Gln Met His Phe Ser Gly His Leu Ala Phe Leu Leu
   65                  70                  75                  80
   Ser Thr Gly Glu Ile Thr Phe Val Ser Ser Leu Val Lys Asn Ser Leu
                   85                  90                  95
   Thr Ala Tyr Gln Tyr Ile Trp Ile Gly Leu His Asp Pro Ser His Gly
               100                 105                 110
   Thr Leu Pro Asn Gly Ser Gly Trp Lys Trp Ser Ser Ser Asn Val Leu
           115                 120                 125
   Thr Phe Tyr Asn Trp Glu Arg Asn Pro Ser Ile Ala Ala Asp Arg Gly
       130                 135                 140
   Tyr Cys Ala Val Leu Ser Gln Lys Ser Gly Phe Gln Lys Trp Arg Asp
   145                 150                 155                 160
   Phe Asn Cys Glu Asn Glu Leu Pro Tyr Ile Cys Lys Phe Lys Val
                   165                 170                 175
<210> 20
   <211> 445
   <212> DNA
   <213> Artificial Sequence
<220>
   <223> Description of Artificial Sequence: INGAP sequences for fusion proteins
<220>
   <221> CDS
   <222> (1)..(444)
<400> 20
   cta gag aaa agg ttg cca tct tcc aga att act tgt cca caa ggt tct      48
   Leu Glu Lys Arg Leu Pro Ser Ser Arg Ile Thr Cys Pro Gln Gly Ser
   1               5                   10                  15
   gtt gct tat ggt tct tat tgt tat tct ttg att ttg att cca caa act      96
   Val Ala Tyr Gly Ser Tyr Cys Tyr Ser Leu Ile Leu Ile Pro Gln Thr
               20                  25                  30
   tgg tct aat gct gaa ttg tct tgt caa atg cat ttt tct ggt cat ttg      144
   Trp Ser Asn Ala Glu Leu Ser Cys Gln Met His Phe Ser Gly His Leu
           35                  40                  45
   gct ttt ttg ttg tct act ggt gaa att act ttt gtt tct tct ttg gtt      192
   Ala Phe Leu Leu Ser Thr Gly Glu Ile Thr Phe Val Ser Ser Leu Val
       50                  55                  60
   aaa aat tct ttg act gct tat caa tat att tgg att ggt ttg cat gat      240
   Lys Asn Ser Leu Thr Ala Tyr Gln Tyr Ile Trp Ile Gly Leu His Asp
   65                  70                  75                  80
   cca tct cat ggt act ttg cca aat ggt tct ggt tgg aaa tgg tct tct      288
   Pro Ser His Gly Thr Leu Pro Asn Gly Ser Gly Trp Lys Trp Ser Ser
                   85                  90                  95
   tct aat gtt ttg act ttt tac aat tgg gaa aga aat cca tct att gct      336
   Ser Asn Val Leu Thr Phe Tyr Asn Trp Glu Arg Asn Pro Ser Ile Ala
               100                 105                 110
   gct gat aga ggt tat tgt gct gtt ttg tct caa aaa tct ggt ttt caa      384
   Ala Asp Arg Gly Tyr Cys Ala Val Leu Ser Gln Lys Ser Gly Phe Gln
           115                 120                 125
   aaa tgg aga gat ttt aat tgt gaa aat gaa ttg cca tat att tgt aaa      432
   Lys Trp Arg Asp Phe Asn Cys Glu Asn Glu Leu Pro Tyr Ile Cys Lys
       130                 135                 140
   ttt aaa gtt gta c                                                    445
   Phe Lys Val Val
   145
<210> 21
   <211> 148
   <212> PRT
   <213> Artificial Sequence
<220>
   <223> Description of Artificial Sequence: INGAP sequences for fusion proteins
<400> 21
   Leu Glu Lys Arg Leu Pro Ser Ser Arg Ile Thr Cys Pro Gln Gly Ser
   1 5 10 15
   Val Ala Tyr Gly Ser Tyr Cys Tyr Ser Leu Ile Leu Ile Pro Gln Thr
   20 25 30
   Trp Ser Asn Ala Glu Leu Ser Cys Gln Met His Phe Ser Gly His Leu
   35 40 45
   Ala Phe Leu Leu Ser Thr Gly Glu Ile Thr Phe Val Ser Ser Leu Val
   50 55 60
   Lys Asn Ser Leu Thr Ala Tyr Gln Tyr Ile Trp Ile Gly Leu His Asp
   65 70 75 80
   Pro Ser His Gly Thr Leu Pro Asn Gly Ser Gly Trp Lys Trp Ser Ser
   85 90 95
   Ser Asn Val Leu Thr Phe Tyr Asn Trp Glu Arg Asn Pro Ser Ile Ala
   100 105 110
   Ala Asp Arg Gly Tyr Cys Ala Val Leu Ser Gln Lys Ser Gly Phe Gln
   115 120 125
   Lys Trp Arg Asp Phe Asn Cys Glu Asn Glu Leu Pro Tyr Ile Cys Lys
   130 135 140
   Phe Lys Val Val
   145
<210> 22
   <211> 445
   <212> DNA
   <213> Artificial Sequence
<220>
   <223> Description of Artificial Sequence: INGAP sequences for fusion proteins
<220>
   <221> CDS
   <222> (2)..(436)
<400> 22
   t cga cct ttg cca tct tcc aga att act tgt cca caa ggt tct gtt gct 49
     Arg Pro Leu Pro Ser Ser Arg Ile Thr Cys Pro Gln Gly Ser Val Ala
       1               5                  10                  15
   tat ggt tct tat tgt tat tct ttg att ttg att cca caa act tgg tct   97
   Tyr Gly Ser Tyr Cys Tyr Ser Leu Ile Leu Ile Pro Gln Thr Trp Ser
               20                  25                  30
   aat gct gaa ttg tct tgt caa atg cat ttt tct ggt cat ttg gct ttt   145
   Asn Ala Glu Leu Ser Cys Gln Met His Phe Ser Gly His Leu Ala Phe
           35                  40                  45
   ttg ttg tct act ggt gaa att act ttt gtt tct tct ttg gtt aaa aat   193
   Leu Leu Ser Thr Gly Glu Ile Thr Phe Val Ser Ser Leu Val Lys Asn
       50                  55                  60
   tct ttg act gct tat caa tat att tgg att ggt ttg cat gat cca tct   241
   Ser Leu Thr Ala Tyr Gln Tyr Ile Trp Ile Gly Leu His Asp Pro Ser
   65                  70                  75                  80
   cat ggt act ttg cca aat ggt tct ggt tgg aaa tgg tct tct tct aat   289
   His Gly Thr Leu Pro Asn Gly Ser Gly Trp Lys Trp Ser Ser Ser Asn
                   85                  90                  95
   gtt ttg act ttt tac aat tgg gaa aga aat cca tct att gct gct gat   337
   Val Leu Thr Phe Tyr Asn Trp Glu Arg Asn Pro Ser Ile Ala Ala Asp
               100                 105                 110
   aga ggt tat tgt gct gtt ttg tct caa aaa tct ggt ttt caa aaa tgg   385
   Arg Gly Tyr Cys Ala Val Leu Ser Gln Lys Ser Gly Phe Gln Lys Trp
           115                 120                 125
   aga gat ttt aat tgt gaa aat gaa ttg cca tat att tgt aaa ttt aaa   433
   Arg Asp Phe Asn Cys Glu Asn Glu Leu Pro Tyr Ile Cys Lys Phe Lys
       130                 135                 140
   gtt taataagct                                                     445
   Val
   145
<210> 23
   <211> 145
   <212> PRT
   <213> Artificial Sequence
<220>
   <223> Description of Artificial Sequence: INGAP sequences for fusion proteins
<400> 23
   Arg Pro Leu Pro Ser Ser Arg Ile Thr Cys Pro Gln Gly Ser Val Ala
    1               5                  10                  15
   Tyr Gly Ser Tyr Cys Tyr Ser Leu Ile Leu Ile Pro Gln Thr Trp Ser
               20                  25                  30
   Asn Ala Glu Leu Ser Cys Gln Met His Phe Ser Gly His Leu Ala Phe
           35                  40                  45
   Leu Leu Ser Thr Gly Glu Ile Thr Phe Val Ser Ser Leu Val Lys Asn
       50                  55                  60
   Ser Leu Thr Ala Tyr Gln Tyr Ile Trp Ile Gly Leu His Asp Pro Ser
   65                  70                  75                  80
   His Gly Thr Leu Pro Asn Gly Ser Gly Trp Lys Trp Ser Ser Ser Asn
                   85                  90                  95
   Val Leu Thr Phe Tyr Asn Trp Glu Arg Asn Pro Ser Ile Ala Ala Asp
               100                 105                 110
   Arg Gly Tyr Cys Ala Val Leu Ser Gln Lys Ser Gly Phe Gln Lys Trp
           115                 120                 125
   Arg Asp Phe Asn Cys Glu Asn Glu Leu Pro Tyr Ile Cys Lys Phe Lys
       130                 135                 140
   Val
   145
<210> 24
   <211> 60
   <212> DNA
   <213> Artificial Sequence
<220>
   <223> Description of Artificial Sequence: EPO mimetic sequences
<220>
   <221> CDS
   <222> (1)..(60)
<400> 24
   ggt ggt act tac tct tgt cat ttt ggt cca ttg act tgg gtt tgt aag      48
   Gly Gly Thr Tyr Ser Cys His Phe Gly Pro Leu Thr Trp Val Cys Lys
    1               5                  10                  15
   cca caa ggt ggt                                                      60
   Pro Gln Gly Gly
               20
<210> 25
   <211> 20
   <212> PRT
   <213> Artificial Sequence
<220>
   <223> Description of Artificial Sequence: EPO mimetic sequences
<400> 25
   Gly Gly Thr Tyr Ser Cys His Phe Gly Pro Leu Thr Trp Val Cys Lys
   1               5                   10                  15
   Pro Gln Gly Gly
               20
<210> 26
   <211> 47
   <212> PRT
   <213> Artificial Sequence
<220>
   <223> Description of Artificial Sequence: Transferrin peptide insertion region
<400> 26
   Asp Lys Ser Lys Glu Phe Gln Leu Phe Ser Ser Pro His Gly Lys Asp
   1               5                   10                  15
   Leu Leu Phe Lys Asp Ser Ala His Gly Phe Leu Lys Val Pro Pro Arg
               20                  25                  30
   Met Asp Ala Lys Met Tyr Leu Gly Tyr Glu Tyr Val Thr Ala Ile
           35                  40                  45
<210> 27
   <211> 49
   <212> PRT
   <213> Artificial Sequence
<220>
   <223> Description of Artificial Sequence: Transferrin peptide insertion region
<400> 27
   Asn Val Thr Asp Cys Ser Gly Asn Phe Cys Leu Phe Arg Ser Glu Thr
   1               5                   10                  15
   Lys Asp Leu Leu Phe Arg Asp Asp Thr Val Cys Leu Ala Lys Leu His
               20                  25                  30
   Asp Arg Asn Thr Tyr Glu Lys Tyr Leu Gly Glu Glu Tyr Val Lys Ala
           35                  40                  45
   Val
<210> 28
   <211> 140
   <212> DNA
   <213> Artificial Sequence
<220>
   <223> Description of Artificial Sequence: Transferrin DNA sequence for peptide insertion region
<400> 28
   agacaaatca aaagaatttc aactattcag ctctcctcat ggtggtactt actcttgtca 60
   ttttggtcca ttgacttggg tttgtaagcc acaaggtggt gggaaggacc tgctgtttaa 120
   ggactctgcc cacgggtttt                                              140
<210> 29
   <211> 210
   <212> DNA
   <213> Artificial Sequence
<220>
   <223> Description of Artificial Sequence: Transferrin DNA sequence for peptide insertion region
<400> 29
   cctatttgga agcaacgtaa ctgactgctc gggcaacttt tgtttgttcc ggtcggaagg 60
   tggtacttac tcttgtcatt ttggtccatt gacttgggtt tgtaagccac aaggtggtac 120
   caaggacctt ctgttcagag atgacacagt atgtttggcc aaacttcatg acagaaacac 180
   atatgaaaaa tacttaggag aagaatatgt                                  210
<210> 30
   <211> 23
   <212> PRT
   <213> Artificial Sequence
<220>
   <223> Description of Artificial Sequence: EPO mimetic peptide
<400> 30
   Gln Arg Val Glu Ile Leu Glu Gly Arg Thr Glu Cys Val Leu Ser Asn
    1               5                  10                  15
   Leu Arg Gly Arg Thr Arg Tyr
               20
<210> 31
   <211> 30
   <212> DNA
   <213> Artificial
<220>
   <223> motif for generating peptide library
<220>
   <221> misc_feature
   <222> (1)..(30)
   <223> n = a or c or g or t; k = g or t.
<400> 31
   nnknnknnkn nknnknnknn knnknnknnk 30
<210> 32
   <211> 30
   <212> DNA
   <213> Artificial
<220>
   <223> motif for generating peptide library
<220>
   <221> misc_feature
   <222> (1)..(30)
   <223> n = a or c or g or t; m = a or c.
<400> 32
   nnmnnmnnmn nmnnmnnmnn mnnmnnmnnm 30
<210> 33
   <211> 5
   <212> PRT
   <213> Artificial
<220>
   <223> motif for spacer region of single-chain antibody
<400> 33
   Gly Gly Gly Gly Ser
   1               5
<210> 34
   <211> 31
   <212> PRT
   <213> Homo sapiens
<220>
   <221> MISC_FEATURE
   <222> (1)..(31)
   <223> Xaa = Gly or -NH2, amino acids 7-36/37 of GLP-1
<400> 34
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Xaa
               20                  25                  30
<210> 35
   <211> 4
   <212> PRT
   <213> Artificial
<220>
   <223> C-terminal motif for amidation
<220>
   <221> MISC_FEATURE
   <222> (1)..(4)
   <223> Xaa = any amino acid
<400> 35
   Gly Lys Arg Xaa
   1
<210> 36
   <211> 15
   <212> PRT
   <213> Artificial
<220>
   <223> linker peptide for Fv fragments
<400> 36
   Gly Gly Gly Gly Ser Gly Gly Gly Gly Ser Gly Gly Gly Gly Ser
   1               5                   10                  15
<210> 37
   <211> 676
   <212> PRT
   <213> Oryctolagus cuniculus
<400> 37
   Val Thr Glu Lys Thr Val Arg Trp Cys Ala Val Asn Asp His Glu Ala
   1               5                   10                  15
   Ser Lys Cys Ala Asn Phe Arg Asp Ser Met Lys Lys Val Leu Pro Glu
               20                  25                  30
   Asp Gly Pro Arg Ile Ile Cys Val Lys Lys Ala Ser Tyr Leu Asp Cys
           35                  40                  45
   Ile Lys Ala Ile Ala Ala His Glu Ala Asp Ala Val Thr Leu Asp Ala
       50                  55                  60
   Gly Leu Val His Glu Ala Gly Leu Thr Pro Asn Asn Leu Lys Pro Val
   65                  70                  75                  80
   Val Ala Glu Phe Tyr Gly Ser Lys Glu Asn Pro Lys Thr Phe Tyr Tyr
   85 90 95
   Ala Val Ala Leu Val Lys Lys Gly Ser Asn Phe Gln Leu Asn Glu Leu
   100 105 110
   Gln Gly Lys Lys Ser Cys His Thr Gly Leu Gly Arg Ser Ala Gly Trp
   115 120 125
   Asn Ile Pro Ile Gly Leu Leu Tyr Cys Asp Leu Pro Glu Pro Arg Lys
   130 135 140
   Pro Leu Glu Lys Ala Val Ala Ser Phe Phe Ser Gly Ser Cys Val Pro
   145 150 155 160
   Cys Ala Asp Gly Ala Asp Phe Pro Gln Leu Cys Gln Leu Cys Pro Gly
   165 170 175
   Cys Gly Cys Ser Ser Val Gln Pro Tyr Phe Gly Tyr Ser Gly Ala Phe
   180 185 190
   Lys Cys Leu Lys Asp Gly Leu Gly Asp Val Ala Phe Val Lys Gln Glu
   195 200 205
   Thr Ile Phe Glu Asn Leu Pro Ser Lys Asp Glu Arg Asp Gln Tyr Glu
   210 215 220
   Leu Leu Cys Leu Asp Asn Thr Arg Lys Pro Val Asp Glu Tyr Glu Gln
   225 230 235 240
   Cys His Leu Ala Arg Val Pro Ser His Ala Val Val Ala Arg Ser Val
   245 250 255
   Asp Gly Lys Glu Asp Leu Ile Trp Glu Leu Leu Asn Gln Ala Gln Glu
   260 265 270
   His Phe Gly Lys Asp Lys Ser Gly Asp Phe Gln Leu Phe Ser Ser Pro
   275 280 285
   His Gly Lys Asn Leu Leu Phe Lys Asp Ser Ala Tyr Gly Phe Phe Lys
   290 295 300
   Val Pro Pro Arg Met Asp Ala Asn Leu Tyr Leu Gly Tyr Glu Tyr Val
   305 310 315 320
   Thr Ala Val Arg Asn Leu Arg Glu Gly Ile Cys Pro Asp Pro Leu Gln
   325 330 335
   Asp Glu Cys Lys Ala Val Lys Trp Cys Ala Leu Ser His His Glu Arg
   340 345 350
   Leu Lys Cys Asp Glu Trp Ser Val Thr Ser Gly Gly Leu Ile Glu Cys
   355 360 365
   Glu Ser Ala Glu Thr Pro Glu Asp Cys Ile Ala Lys Ile Met Asn Gly
   370 375 380
   Glu Ala Asp Ala Met Ser Leu Asp Gly Gly Tyr Val Tyr Ile Ala Gly
   385 390 395 400
   Gln Cys Gly Leu Val Pro Val Leu Ala Glu Asn Tyr Glu Ser Thr Asp
   405 410 415
   Cys Lys Lys Ala Pro Glu Glu Gly Tyr Leu Ser Val Ala Val Val Lys
   420 425 430
   Lys Ser Asn Pro Asp Ile Asn Trp Asn Asn Leu Glu Gly Lys Lys Ser
   435 440 445
   Cys His Thr Ala Val Asp Arg Thr Ala Gly Trp Asn Ile Pro Met Gly
   450 455 460
   Leu Leu Tyr Asn Arg Ile Asn His Cys Arg Phe Asp Glu Phe Phe Arg
   465 470 475 480
   Gln Gly Cys Ala Pro Gly Ser Gln Lys Asn Ser Ser Leu Cys Glu Leu
   485 490 495
   Cys Ile Gly Pro Ser Val Cys Ala Pro Asn Asn Arg Glu Gly Tyr Tyr
   500 505 510
   Gly Tyr Thr Gly Ala Phe Arg Cys Leu Val Glu Lys Gly Asp Val Ala
   515 520 525
   Phe Val Lys Ser Gln Thr Val Leu Gln Asn Thr Gly Gly Arg Asn Ser
   530 535 540
   Glu Pro Trp Ala Lys Asp Leu Lys Glu Glu Asp Phe Glu Leu Leu Cys
   545 550 555 560
   Leu Asp Gly Thr Arg Lys Pro Val Ser Glu Ala His Asn Cys His Leu
   565 570 575
   Ala Lys Ala Pro Asn His Ala Val Val Ser Arg Lys Asp Lys Ala Ala
   580 585 590
   Cys Val Lys Gln Lys Leu Leu Asp Leu Gln Val Glu Tyr Gly Asn Thr
   595 600 605
   Val Ala Asp Cys Ser Ser Lys Phe Cys Met Phe His Ser Lys Thr Lys
   610 615 620
   Asp Leu Leu Phe Arg Asp Asp Thr Lys Cys Leu Val Asp Leu Arg Gly
   625 630 635 640
   Lys Asn Thr Tyr Glu Lys Tyr Leu Gly Ala Asp Tyr Ile Lys Ala Val
   645 650 655
   Ser Asn Leu Arg Lys Cys Ser Thr Ser Arg Leu Leu Glu Ala Cys Thr
   660 665 670
   Phe His Lys His
   675
<210> 38
   <211> 676
   <212> PRT
   <213> Rattus norvegicus
<400> 38
   Val Pro Asp Lys Thr Val Lys Trp Cys Ala Val Ser Glu His Glu Asn
   1               5                   10                  15
   Thr Lys Cys Ile Ser Phe Arg Asp His Met Lys Thr Val Leu Pro Ala
               20                  25                  30
   Asp Gly Pro Arg Leu Pro Cys Val Lys Lys Thr Ser Tyr Gln Asp Cys
           35                  40                  45
   Ile Lys Ala Ile Ser Gly Gly Glu Ala Asp Ala Ile Thr Leu Asp Gly
       50                  55                  60
   Gly Trp Val Tyr Asp Ala Gly Leu Thr Pro Asn Asn Leu Lys Pro Val
   65                  70                  75                  80
   Ala Ala Glu Phe Tyr Gly Ser Leu Glu His Arg Gln Thr His Tyr Leu
                   85                  90                  95
   Ala Val Ala Val Val Lys Lys Gly Thr Asp Phe Gln Leu Asn Gln Leu
               100                 105                 110
   Gln Gly Lys Lys Ser Cys His Thr Gly Leu Gly Arg Ser Ala Gly Trp
           115                 120                 125
   Ile Ile Pro Ile Gly Leu Leu Phe Cys Asn Leu Pro Glu Pro Arg Lys
       130                 135                 140
   Pro Leu Glu Lys Ala Val Ala Ser Phe Phe Ser Gly Ser Cys Val Pro
   145                 150                 155                 160
   Cys Ala Asp Pro Val Ala Phe Pro Gln Leu Cys Gln Leu Cys Pro Gly
                   165                 170                 175
   Cys Gly Cys Ser Pro Thr Gln Pro Phe Phe Gly Tyr Val Gly Ala Phe
               180                 185                 190
   Lys Cys Leu Arg Asp Gly Gly Gly Asp Val Ala Phe Val Lys His Thr
           195                 200                 205
   Thr Ile Phe Glu Val Leu Pro Gln Lys Ala Asp Arg Asp Gln Tyr Glu
       210                 215                 220
   Leu Leu Cys Leu Asp Asn Thr Arg Lys Pro Val Asp Gln Tyr Glu Asp
   225                 230                 235                 240
   Cys Tyr Leu Ala Arg Ile Pro Ser His Ala Val Val Ala Arg Asn Gly
                   245                 250                 255
   Asp Gly Lys Glu Asp Leu Ile Trp Glu Ile Leu Lys Val Ala Gln Glu
               260                 265                 270
   His Phe Gly Lys Gly Lys Ser Lys Asp Phe Gln Leu Phe Gly Ser Pro
           275                 280                 285
   Leu Gly Lys Asp Leu Leu Phe Lys Asp Ser Arg Phe Gly Leu Leu Arg
       290                 295                 300
   Ala Pro Lys Asp Gly Leu Gln Ala Val Pro Arg Pro Gln Leu Cys His
   305                 310                 315                 320
   Cys His Ser Lys Ser Ala Gly Ser Cys Pro Asp Ala Ile Asp Ser Ala
                   325                 330                 335
   Pro Val Lys Trp Cys Ala Leu Ser His Gln Glu Arg Ala Lys Cys Asp
               340                 345                 350
   Glu Trp Ser Val Thr Gly Asn Gly Gln Ile Glu Cys Glu Ser Ala Glu
           355                 360                 365
   Ser Thr Glu Asp Cys Ile Asp Lys Ile Val Asn Gly Glu Ala Asp Ala
       370                 375                 380
   Met Ser Leu Asp Gly Gly His Ala Tyr Ile Ala Gly Gln Cys Gly Leu
   385                 390                 395                 400
   Val Pro Val Met Ala Glu Asn Tyr Asp Ile Ser Ser Cys Thr Asn Pro
                   405                 410                 415
   Gln Ser Asp Val Phe Pro Lys Gly Tyr Tyr Ala Val Ala Val Val Lys
               420                 425                 430
   Ala Ser Asp Ser Ser Ile Asn Trp Asn Asn Leu Lys Gly Lys Lys Ser
           435                 440                 445
   Cys His Thr Gly Val Asp Arg Thr Ala Gly Trp Asn Ile Pro Met Gly
       450                 455                 460
   Leu Leu Phe Ser Arg Ile Asn His Cys Lys Phe Asp Glu Phe Phe Ser
   465                 470                 475                 480
   Gln Gly Cys Ala Pro Gly Tyr Lys Lys Asn Ser Thr Leu Cys Asp Leu
                   485                 490                 495
   Cys Ile Gly Pro Ala Lys Cys Ala Pro Asn Asn Arg Glu Gly Tyr Asn
               500                 505                 510
   Gly Tyr Thr Gly Ala Phe Gln Cys Leu Val Glu Lys Gly Asp Val Ala
           515                 520                 525
   Phe Val Lys His Gln Thr Val Leu Glu Asn Thr Asn Gly Lys Asn Thr
       530                 535                 540
   Ala Ala Trp Ala Lys Asp Leu Lys Gln Glu Asp Phe Gln Leu Leu Cys
   545                 550                 555                 560
   Pro Asp Gly Thr Lys Lys Pro Val Thr Glu Phe Ala Thr Cys His Leu
                   565                 570                 575
   Ala Gln Ala Pro Asn His Val Val Val Ser Arg Lys Glu Lys Ala Ala
               580                 585                 590
   Arg Val Ser Thr Val Leu Thr Ala Gln Lys Asp Leu Phe Trp Lys Gly
           595                 600                 605
   Asp Lys Asp Cys Thr Gly Asn Phe Cys Leu Phe Arg Ser Ser Thr Lys
       610                 615                 620
   Asp Leu Leu Phe Arg Asp Asp Thr Lys Cys Leu Thr Lys Leu Pro Glu
   625                 630                 635                 640
   Gly Thr Thr Tyr Glu Glu Tyr Leu Gly Ala Glu Tyr Leu Gln Ala Val
                   645                 650                 655
   Gly Asn Ile Arg Lys Cys Ser Thr Ser Arg Leu Leu Glu Ala Cys Thr
               660                 665                 670
   Phe His Lys Ser
           675
<210> 39
   <211> 677
   <212> PRT
   <213> Mus musculus
<400> 39
   Val Pro Asp Lys Thr Val Lys Trp Cys Ala Val Ser Glu His Glu Asn
   1               5                   10                  15
   Thr Lys Cys Ile Ser Phe Arg Asp His Met Lys Thr Val Leu Pro Pro
               20                  25                  30
   Asp Gly Pro Arg Leu Ala Cys Val Lys Lys Thr Ser Tyr Pro Asp Cys
           35                  40                  45
   Ile Lys Ala Ile Ser Ala Ser Glu Ala Asp Ala Met Thr Leu Asp Gly
       50                  55                  60
   Gly Trp Val Tyr Asp Ala Gly Leu Thr Pro Asn Asn Leu Lys Pro Val
   65                  70                  75                  80
   Ala Ala Glu Phe Tyr Gly Ser Val Glu His Pro Gln Thr Tyr Tyr Tyr
                   85                  90                  95
   Ala Val Ala Val Val Lys Lys Gly Thr Asp Phe Gln Leu Asn Gln Leu
               100                 105                 110
   Glu Gly Lys Lys Ser Cys His Thr Gly Leu Gly Arg Ser Ala Gly Trp
           115                 120                 125
   Val Ile Pro Ile Gly Leu Leu Phe Cys Lys Leu Ser Glu Pro Arg Ser
       130                 135                 140
   Pro Leu Glu Lys Ala Val Ser Ser Phe Phe Ser Gly Ser Cys Val Pro
   145                 150                 155                 160
   Cys Ala Asp Pro Val Ala Phe Pro Lys Leu Cys Gln Leu Cys Pro Gly
                   165                 170                 175
   Cys Gly Cys Ser Ser Thr Gln Pro Phe Phe Gly Tyr Val Gly Ala Phe
               180                 185                 190
   Lys Cys Leu Lys Asp Gly Gly Gly Asp Val Ala Phe Val Lys His Thr
           195                 200                 205
   Thr Ile Phe Glu Val Leu Pro Glu Lys Ala Asp Arg Asp Gln Tyr Glu
       210                 215                 220
   Leu Leu Cys Leu Asp Asn Thr Arg Lys Pro Val Asp Gln Tyr Glu Asp
   225                 230                 235                 240
   Cys Tyr Leu Ala Arg Ile Pro Ser His Ala Val Val Ala Arg Lys Asn
                   245                 250                 255
   Asn Gly Lys Glu Asp Leu Ile Trp Glu Ile Leu Lys Val Ala Gln Glu
               260                 265                 270
   His Phe Gly Lys Gly Lys Ser Lys Asp Phe Gln Leu Phe Ser Ser Pro
           275                 280                 285
   Leu Gly Lys Asp Leu Leu Phe Lys Asp Ser Ala Phe Gly Leu Leu Arg
       290                 295                 300
   Val Pro Pro Arg Met Asp Tyr Arg Leu Tyr Leu Gly His Asn Tyr Val
   305                 310                 315                 320
   Thr Ala Ile Arg Asn Gln Gln Glu Gly Val Cys Pro Glu Gly Ser Ile
                   325                 330                 335
   Asp Asn Ser Pro Val Lys Trp Cys Ala Leu Ser His Leu Glu Arg Thr
               340                 345                 350
   Lys Cys Asp Glu Trp Ser Ile Ile Ser Glu Gly Lys Ile Glu Cys Glu
           355                 360                 365
   Ser Ala Glu Thr Thr Glu Asp Cys Ile Glu Lys Ile Val Asn Gly Glu
       370                 375                 380
   Ala Asp Ala Met Thr Leu Asp Gly Gly His Ala Tyr Ile Ala Gly Gln
   385                 390                 395                 400
   Cys Gly Leu Val Pro Val Met Ala Glu Tyr Tyr Glu Ser Ser Asn Cys
                   405                 410                 415
   Ala Ile Pro Ser Gln Gln Gly Ile Phe Pro Lys Gly Tyr Tyr Ala Val
               420                 425                 430
   Ala Val Val Lys Ala Ser Asp Thr Ser Ile Thr Trp Asn Asn Leu Lys
           435                 440                 445
   Gly Lys Lys Ser Cys His Thr Gly Val Asp Arg Thr Ala Gly Trp Asn
       450                 455                 460
   Ile Pro Met Gly Met Leu Tyr Asn Arg Ile Asn His Cys Lys Phe Asp
   465                 470                 475                 480
   Glu Phe Phe Ser Gln Gly Cys Ala Pro Gly Tyr Glu Lys Asn Ser Thr
                   485                 490                 495
   Leu Cys Asp Leu Cys Ile Gly Pro Leu Lys Cys Ala Pro Asn Asn Lys
               500                 505                 510
   Glu Glu Tyr Asn Gly Tyr Thr Gly Ala Phe Arg Cys Leu Val Glu Lys
           515                 520                 525
   Gly Asp Val Ala Phe Val Lys His Gln Thr Val Leu Asp Asn Thr Glu
       530                 535                 540
   Gly Lys Asn Pro Ala Glu Trp Ala Lys Asn Leu Lys Gln Glu Asp Phe
   545                 550                 555                 560
   Glu Leu Leu Cys Pro Asp Gly Thr Arg Lys Pro Val Lys Asp Phe Ala
                   565                 570                 575
   Ser Cys His Leu Ala Gln Ala Pro Asn His Val Val Val Ser Arg Lys
               580                 585                 590
   Glu Lys Ala Ala Arg Val Lys Ala Val Leu Thr Ser Gln Glu Thr Leu
           595                 600                 605
   Phe Gly Gly Ser Asp Cys Thr Gly Asn Phe Cys Leu Phe Lys Ser Thr
       610                 615                 620
   Thr Lys Asp Leu Leu Phe Arg Asp Asp Thr Lys Cys Phe Val Lys Leu
   625                 630                 635                 640
   Pro Glu Gly Thr Thr Pro Glu Lys Tyr Leu Gly Ala Glu Tyr Met Gln
                   645                 650                 655
   Ser Val Gly Asn Met Arg Lys Cys Ser Thr Ser Arg Leu Leu Glu Ala
               660                 665                 670
   Cys Thr Phe His Lys
           675
<210> 40
   <211> 688
   <212> PRT
   <213> Equus caballus
<400> 40
   Ala Glu Gln Thr Val Arg Trp Cys Thr Val Ser Asn His Glu Val Ser
   1               5                   10                  15
   Lys Cys Ala Ser Phe Arg Asp Ser Met Lys Ser Ile Val Pro Ala Pro
               20                  25                  30
   Pro Leu Val Ala Cys Val Lys Arg Thr Ser Tyr Leu Glu Cys Ile Lys
           35                  40                  45
   Ala Ile Ala Asp Asn Glu Ala Asp Ala Val Thr Leu Asp Ala Gly Leu
       50                  55                  60
   Val Phe Glu Ala Gly Leu Ser Pro Tyr Asn Leu Lys Pro Val Val Ala
   65                  70                  75                  80
   Glu Phe Tyr Gly Ser Lys Thr Glu Pro Gln Thr His Tyr Tyr Ala Val
                   85                  90                  95
   Ala Val Val Lys Lys Asn Ser Asn Phe Gln Leu Asn Gln Leu Gln Gly
               100                 105                 110
   Lys Lys Ser Cys His Thr Gly Leu Gly Arg Ser Ala Gly Trp Asn Ile
           115                 120                 125
   Pro Ile Gly Leu Leu Tyr Trp Gln Leu Pro Glu Pro Arg Glu Ser Leu
       130                 135                 140
   Gln Lys Ala Val Ser Asn Phe Phe Ala Gly Ser Cys Val Pro Cys Ala
   145                 150                 155                 160
   Asp Arg Thr Ala Val Pro Asn Leu Cys Gln Leu Cys Val Gly Lys Gly
                   165                 170                 175
   Thr Asp Lys Cys Ala Cys Ser Asn His Glu Pro Tyr Phe Gly Tyr Ser
               180                 185                 190
   Gly Ala Phe Lys Cys Leu Ala Asp Gly Ala Gly Asp Val Ala Phe Val
           195                 200                 205
   Lys His Ser Thr Val Leu Glu Asn Leu Pro Gln Glu Ala Asp Arg Asp
       210                 215                 220
   Glu Tyr Gln Leu Leu Cys Arg Asp Asn Thr Arg Lys Ser Val Asp Glu
   225                 230                 235                 240
   Tyr Lys Asp Cys Tyr Leu Ala Ser Ile Pro Ser His Ala Val Val Ala
                   245                 250                 255
   Arg Ser Val Asp Gly Lys Glu Asp Leu Ile Trp Gly Leu Leu Asn Gln
               260                 265                 270
   Ala Gln Glu His Phe Gly Thr Glu Lys Ser Lys Asp Phe His Leu Phe
           275                 280                 285
   Ser Ser Pro His Gly Lys Asp Leu Leu Phe Lys Asp Ser Ala Leu Gly
       290                 295                 300
   Phe Leu Arg Ile Pro Pro Ala Met Asp Thr Trp Leu Tyr Leu Gly Tyr
   305                 310                 315                 320
   Glu Tyr Val Thr Ala Ile Arg Asn Leu Arg Glu Asp Ile Arg Pro Glu
                   325                 330                 335
   Val Pro Lys Asp Glu Cys Lys Lys Val Lys Trp Cys Ala Ile Gly His
               340                 345                 350
   His Glu Lys Val Lys Cys Asp Glu Trp Ser Val Asn Ser Gly Gly Asn
           355                 360                 365
   Ile Glu Cys Glu Ser Ala Gln Ser Thr Glu Asp Cys Ile Ala Lys Ile
       370                 375                 380
   Val Lys Gly Glu Ala Asp Ala Met Ser Leu Asp Gly Gly Phe Ile Tyr
   385                 390                 395                 400
   Ile Ala Gly Lys Cys Gly Leu Val Pro Val Leu Ala Glu Asn Tyr Glu
                   405                 410                 415
   Thr Arg Ser Gly Ser Ala Cys Val Asp Thr Pro Glu Glu Gly Tyr His
               420                 425                 430
   Ala Val Ala Val Val Lys Ser Ser Ser Asp Pro Asp Leu Thr Trp Asn
           435                 440                 445
   Ser Leu Lys Gly Lys Lys Ser Cys His Thr Gly Val Asp Arg Thr Ala
       450                 455                 460
   Gly Trp Asn Ile Pro Met Gly Leu Leu Tyr Ser Glu Ile Lys His Cys
   465                 470                 475                 480
   Glu Phe Asp Lys Phe Phe Arg Glu Gly Cys Ala Pro Gly Tyr Arg Arg
                   485                 490                 495
   Asn Ser Thr Leu Cys Asn Leu Cys Ile Gly Ser Ala Ser Gly Pro Gly
               500                 505                 510
   Arg Glu Cys Glu Pro Asn Asn His Glu Arg Tyr Tyr Gly Tyr Thr Gly
           515                 520                 525
   Ala Phe Arg Cys Leu Val Glu Lys Gly Asp Val Ala Phe Val Lys His
       530                 535                 540
   Gln Thr Val Glu Gln Asn Thr Asp Gly Arg Asn Pro Asp Asp Trp Ala
   545                 550                 555                 560
   Lys Asp Leu Lys Ser Glu Asn Phe Lys Leu Leu Cys Pro Asp Gly Thr
                   565                 570                 575
   Arg Lys Ser Val Thr Glu Phe Lys Ser Cys Tyr Leu Ala Arg Ala Pro
               580                 585                 590
   Asn His Ala Val Val Ser Arg Lys Glu Lys Ala Ala Cys Val Cys Gln
           595                 600                 605
   Glu Leu His Asn Gln Gln Ala Ser Tyr Gly Lys Asn Gly Ser His Cys
       610                 615                 620
   Pro Asp Lys Phe Cys Leu Phe Gln Ser Ala Thr Lys Asp Leu Leu Phe
   625                 630                 635                 640
   Arg Asp Asp Thr Gln Cys Leu Ala Asn Leu Gln Pro Thr Thr Thr Tyr
                   645                 650                 655
   Lys Thr Tyr Leu Gly Glu Lys Tyr Leu Thr Ala Val Ala Asn Leu Arg
               660                 665                 670
   Gln Cys Ser Thr Ser Arg Leu Leu Glu Ala Cys Thr Phe His Arg Val
           675                 680                 685
<210> 41
   <211> 685
   <212> PRT
   <213> Bos taurus
<400> 41
   Asp Pro Glu Arg Thr Val Arg Trp Cys Thr Ile Ser Thr His Glu Ala
   1 5 10 15
   Asn Lys Cys Ala Ser Phe Arg Glu Asn Val Leu Arg Ile Leu Glu Ser
   20 25 30
   Gly Pro Phe Val Ser Cys Val Lys Lys Thr Ser His Met Asp Cys Ile
   35 40 45
   Lys Ala Ile Ser Asn Asn Glu Ala Asp Ala Val Thr Leu Asp Gly Gly
   50 55 60
   Leu Val Tyr Glu Ala Gly Leu Lys Pro Asn Asn Leu Lys Pro Val Val
   65 70 75 80
   Ala Glu Phe His Gly Thr Lys Asp Asn Pro Gln Thr His Tyr Tyr Ala
   85 90 95
   Val Ala Val Val Lys Lys Asp Thr Asp Phe Lys Leu Asn Glu Leu Arg
   100 105 110
   Gly Lys Lys Ser Cys His Thr Gly Leu Gly Arg Ser Ala Gly Trp Asn
   115 120 125
   Ile Pro Met Ala Lys Leu Tyr Lys Glu Leu Pro Asp Pro Gln Glu Ser
   130 135 140
   Ile Gln Arg Ala Ala Ala Asn Phe Phe Ser Ala Ser Cys Val Pro Cys
   145 150 155 160
   Ala Asp Gln Ser Ser Phe Pro Lys Leu Cys Gln Leu Cys Ala Gly Lys
   165 170 175
   Gly Thr Asp Lys Cys Ala Cys Ser Asn His Glu Pro Tyr Phe Gly Tyr
   180 185 190
   Ser Gly Ala Phe Lys Cys Leu Met Glu Gly Ala Gly Asp Val Ala Phe
   195 200 205
   Val Lys His Ser Thr Val Phe Asp Asn Leu Pro Asn Pro Glu Asp Arg
   210 215 220
   Lys Asn Tyr Glu Leu Leu Cys Gly Asp Asn Thr Arg Lys Ser Val Asp
   225 230 235 240
   Asp Tyr Gln Glu Cys Tyr Leu Ala Met Val Pro Ser His Ala Val Val
   245 250 255
   Ala Arg Thr Val Gly Gly Lys Glu Asp Val Ile Trp Glu Leu Leu Asn
   260 265 270
   His Ala Gln Glu His Phe Gly Lys Asp Lys Pro Asp Asn Phe Gln Leu
   275 280 285
   Phe Gln Ser Pro His Gly Lys Asp Leu Leu Phe Lys Asp Ser Ala Asp
   290 295 300
   Gly Phe Leu Lys Ile Pro Ser Lys Met Asp Phe Glu Leu Tyr Leu Gly
   305 310 315 320
   Tyr Glu Tyr Val Thr Ala Leu Gln Asn Leu Arg Glu Ser Lys Pro Pro
   325 330 335
   Asp Ser Ser Lys Asp Glu Cys Met Val Lys Trp Cys Ala Ile Gly His
   340 345 350
   Gln Glu Arg Thr Lys Cys Asp Arg Trp Ser Gly Phe Ser Gly Gly Ala
   355 360 365
   Ile Glu Cys Glu Thr Ala Glu Asn Thr Glu Glu Cys Ile Ala Lys Ile
   370 375 380
   Met Lys Gly Glu Ala Asp Ala Met Ser Leu Asp Gly Gly Tyr Leu Tyr
   385 390 395 400
   Ile Ala Gly Lys Cys Gly Leu Val Pro Val Leu Ala Glu Asn Tyr Lys
   405 410 415
   Thr Glu Gly Glu Ser Cys Lys Asn Thr Pro Glu Lys Gly Tyr Leu Ala
   420 425 430
   Val Ala Val Val Lys Thr Ser Asp Ala Asn Ile Asn Trp Asn Asn Leu
   435 440 445
   Lys Asp Lys Lys Ser Cys His Thr Ala Val Asp Arg Thr Ala Gly Trp
   450 455 460
   Asn Ile Pro Met Gly Leu Leu Tyr Ser Lys Ile Asn Asn Cys Lys Phe
   465 470 475 480
   Asp Glu Phe Phe Ser Ala Gly Cys Ala Pro Gly Ser Pro Arg Asn Ser
   485 490 495
   Ser Leu Cys Ala Leu Cys Ile Gly Ser Glu Lys Gly Thr Gly Lys Glu
   500 505 510
   Cys Val Pro Asn Ser Asn Glu Arg Tyr Tyr Gly Tyr Thr Gly Ala Phe
   515 520 525
   Arg Cys Leu Val Glu Lys Gly Asp Val Ala Phe Val Lys Asp Gln Thr
   530 535 540
   Val Ile Gln Asn Thr Asp Gly Asn Asn Asn Glu Ala Trp Ala Lys Asn
   545 550 555 560
   Leu Lys Lys Glu Asn Phe Glu Val Leu Cys Lys Asp Gly Thr Arg Lys
   565 570 575
   Pro Val Thr Asp Ala Glu Asn Cys His Leu Ala Arg Gly Pro Asn His
   580 585 590
   Ala Val Val Ser Arg Lys Asp Lys Ala Thr Cys Val Glu Lys Ile Leu
   595 600 605
   Asn Lys Gln Gln Asp Asp Phe Gly Lys Ser Val Thr Asp Cys Thr Ser
   610 615 620
   Asn Phe Cys Leu Phe Gln Ser Asn Ser Lys Asp Leu Leu Phe Arg Asp
   625 630 635 640
   Asp Thr Lys Cys Leu Ala Ser Ile Ala Lys Lys Thr Tyr Asp Ser Tyr
   645 650 655
   Leu Gly Asp Asp Tyr Val Arg Ala Met Thr Asn Leu Arg Gln Cys Ser
   660 665 670
   Thr Ser Lys Leu Leu Glu Ala Cys Thr Phe His Lys Pro
   675 680 685
<210> 42
   <211> 696
   <212> PRT
   <213> Sus scrofa
<220>
   <221> misc_feature
   <222> (308)..(308)
   <223> Xaa can be any naturally occurring amino acid
<400> 42
   Val Ala Gln Lys Thr Val Arg Trp Cys Thr Ile Ser Asn Gln Glu Ala
   1               5                   10                  15
   Asn Lys Cys Ser Ser Phe Arg Glu Asn Met Ser Lys Ala Val Lys Asn
               20                  25                  30
   Gly Pro Leu Val Ser Cys Val Lys Lys Ser Ser Tyr Leu Asp Cys Ile
           35                  40                  45
   Lys Ala Ile Arg Asp Lys Glu Ala Asp Ala Val Thr Leu Asp Ala Gly
       50                  55                  60
   Leu Val Phe Glu Ala Gly Leu Ala Pro Tyr Asn Leu Lys Pro Val Val
   65                  70                  75                  80
   Ala Glu Phe Tyr Gly Gln Lys Asp Asn Pro Gln Thr His Tyr Tyr Ala
                   85                  90                  95
   Val Ala Val Val Lys Lys Gly Ser Asn Phe Gln Trp Asn Gln Leu Gln
               100                 105                 110
   Gly Lys Arg Ser Cys His Thr Gly Leu Gly Arg Ser Ala Gly Trp Ile
           115                 120                 125
   Ile Pro Met Gly Leu Leu Tyr Asp Gln Leu Pro Glu Pro Arg Lys Pro
       130                 135                 140
   Ile Glu Lys Ala Val Ala Ser Phe Phe Ser Ser Ser Cys Val Pro Cys
   145                 150                 155                 160
   Ala Asp Pro Val Asn Phe Pro Lys Leu Cys Gln Gln Cys Ala Gly Lys
                   165                 170                 175
   Gly Ala Glu Lys Cys Ala Cys Ser Asn His Glu Pro Tyr Phe Gly Tyr
               180                 185                 190
   Ala Gly Ala Phe Asn Cys Leu Lys Glu Asp Ala Gly Asp Val Ala Phe
           195                 200                 205
   Val Lys His Ser Thr Val Leu Glu Asn Leu Pro Asp Lys Ala Asp Arg
       210                 215                 220
   Asp Gln Tyr Glu Leu Leu Cys Arg Asp Asn Thr Arg Arg Pro Val Asp
   225                 230                 235                 240
   Asp Tyr Glu Asn Cys Tyr Leu Ala Gln Val Pro Ser His Ala Val Val
                   245                 250                 255
   Ala Arg Ser Val Asp Gly Gln Glu Asp Ser Ile Trp Glu Leu Leu Asn
               260                 265                 270
   Gln Ala Gln Glu His Phe Gly Arg Asp Lys Ser Pro Asp Phe Gln Leu
           275                 280                 285
   Phe Ser Ser Ser His Gly Lys Asp Leu Leu Phe Lys Asp Ser Ala Asn
       290                 295                 300
   Gly Phe Leu Xaa Ile Pro Ser Lys Met Asp Ser Ser Leu Tyr Leu Gly
   305 310 315 320
   Tyr Gln Tyr Val Thr Ala Leu Arg Asn Leu Arg Glu Glu Ile Ser Pro
   325 330 335
   Asp Ser Ser Lys Asn Glu Cys Lys Lys Val Arg Trp Cys Ala Ile Gly
   340 345 350
   His Glu Glu Thr Gln Lys Cys Asp Ala Trp Ser Ile Asn Ser Gly Gly
   355 360 365
   Lys Ile Glu Cys Val Ser Ala Glu Asn Thr Glu Asp Cys Ile Ala Lys
   370 375 380
   Ile Val Lys Gly Glu Ala Asp Ala Met Ser Leu Asp Gly Gly Tyr Ile
   385 390 395 400
   Tyr Ile Ala Gly Lys Cys Gly Leu Val Pro Val Leu Ala Glu Asn Tyr
   405 410 415
   Lys Thr Glu Gly Glu Asn Cys Val Asn Thr Pro Glu Lys Gly Tyr Leu
   420 425 430
   Ala Val Ala Val Val Lys Lys Ser Ser Gly Pro Asp Leu Asn Trp Asn
   435 440 445
   Asn Leu Lys Gly Lys Lys Ser Cys His Thr Ala Val Asp Arg Thr Ala
   450 455 460
   Gly Trp Asn Ile Pro Met Gly Leu Leu Tyr Asn Lys Ile Asn Ser Cys
   465 470 475 480
   Lys Phe Asp Gln Phe Phe Gly Glu Gly Cys Ala Pro Gly Ser Gln Arg
   485 490 495
   Asn Ser Ser Leu Cys Ala Leu Cys Ile Gly Ser Glu Arg Ala Pro Gly
   500 505 510
   Arg Glu Cys Leu Ala Asn Asn His Glu Arg Tyr Tyr Gly Tyr Thr Gly
   515 520 525
   Ala Phe Arg Cys Leu Val Glu Lys Gly Asp Val Ala Phe Val Lys Asp
   530 535 540
   Gln Val Val Gln Gln Asn Thr Asp Gly Lys Asn Lys Asp Asp Trp Ala
   545 550 555 560
   Lys Asp Leu Lys Gln Met Asp Phe Glu Leu Leu Cys Gln Asn Gly Ala
   565 570 575
   Arg Glu Pro Val Asp Asn Ala Glu Asn Cys His Leu Ala Arg Ala Pro
   580 585 590
   Asn His Ala Val Val Ala Arg Asp Asp Lys Val Thr Cys Val Ala Glu
   595 600 605
   Glu Leu Leu Lys Gln Gln Ala Gln Phe Gly Arg His Val Thr Asp Cys
   610 615 620
   Ser Ser Ser Phe Cys Met Phe Lys Ser Asn Thr Lys Asp Leu Leu Phe
   625                 630                 635                 640
   Arg Asp Asp Thr Gln Cys Leu Ala Arg Val Gly Lys Thr Thr Tyr Glu
                   645                 650                 655
   Ser Tyr Leu Gly Ala Asp Tyr Ile Thr Ala Val Ala Asn Leu Arg Lys
               660                 665                 670
   Cys Ser Thr Ser Lys Leu Leu Glu Ala Cys Thr Phe His Ser Ala Lys
           675                 680                 685
   Asn Pro Arg Val Glu Thr Thr Thr
       690                 695
<210> 43
   <211> 686
   <212> PRT
   <213> Gallus gallus
<400> 43
   Ala Pro Pro Lys Ser Val Ile Arg Trp Cys Thr Ile Ser Ser Pro Glu
   1               5                   10                  15
   Glu Lys Lys Cys Asn Asn Leu Arg Asp Leu Thr Gln Gln Glu Arg Ile
               20                  25                  30
   Ser Leu Thr Cys Val Gln Lys Ala Thr Tyr Leu Asp Cys Ile Lys Ala
           35                  40                  45
   Ile Ala Asn Asn Glu Ala Asp Ala Ile Ser Leu Asp Gly Gly Gln Ala
       50                  55                  60
   Phe Glu Ala Gly Leu Ala Pro Tyr Lys Leu Lys Pro Ile Ala Ala Glu
   65                  70                  75                  80
   Val Tyr Glu His Thr Glu Gly Ser Thr Thr Ser Tyr Tyr Ala Val Ala
                   85                  90                  95
   Val Val Lys Lys Gly Thr Glu Phe Thr Val Asn Asp Leu Gln Gly Lys
               100                 105                 110
   Thr Ser Cys His Thr Gly Leu Gly Arg Ser Ala Gly Trp Asn Ile Pro
           115                 120                 125
   Ile Gly Thr Leu Leu His Arg Gly Ala Ile Glu Trp Glu Gly Ile Glu
       130                 135                 140
   Ser Gly Ser Val Glu Gln Ala Val Ala Lys Phe Phe Ser Ala Ser Cys
   145                 150                 155                 160
   Val Pro Gly Ala Thr Ile Glu Gln Lys Leu Cys Arg Gln Cys Lys Gly
                   165                 170                 175
   Asp Pro Lys Thr Lys Cys Ala Arg Asn Ala Pro Tyr Ser Gly Tyr Ser
               180                 185                 190
   Gly Ala Phe His Cys Leu Lys Asp Gly Lys Gly Asp Val Ala Phe Val
           195                 200                 205
   Lys His Thr Thr Val Asn Glu Asn Ala Pro Asp Gln Lys Asp Glu Tyr
       210                 215                 220
   Glu Leu Leu Cys Leu Asp Gly Ser Arg Gln Pro Val Asp Asn Tyr Lys
   225                 230                 235                 240
   Thr Cys Asn Trp Ala Arg Val Ala Ala His Ala Val Val Ala Arg Asp
                   245                 250                 255
   Asp Asn Lys Val Glu Asp Ile Trp Ser Phe Leu Ser Lys Ala Gln Ser
               260                 265                 270
   Asp Phe Gly Val Asp Thr Lys Ser Asp Phe His Leu Phe Gly Pro Pro
           275                 280                 285
   Gly Lys Lys Asp Pro Val Leu Lys Asp Leu Leu Phe Lys Asp Ser Ala
       290                 295                 300
   Ile Met Leu Lys Arg Val Pro Ser Leu Met Asp Ser Gln Leu Tyr Leu
   305                 310                 315                 320
   Gly Phe Glu Tyr Tyr Ser Ala Ile Gln Ser Met Arg Lys Asp Gln Leu
                   325                 330                 335
   Thr Pro Ser Pro Arg Glu Asn Arg Ile Gln Trp Cys Ala Val Gly Lys
               340                 345                 350
   Asp Glu Lys Ser Lys Cys Asp Arg Trp Ser Val Val Ser Asn Gly Asp
           355                 360                 365
   Val Glu Cys Thr Val Val Asp Glu Thr Lys Asp Cys Ile Ile Lys Ile
       370                 375                 380
   Met Lys Gly Glu Ala Asp Ala Val Ala Leu Asp Gly Gly Leu Val Tyr
   385                 390                 395                 400
   Thr Ala Gly Val Cys Gly Leu Val Pro Val Met Ala Glu Arg Tyr Asp
                   405                 410                 415
   Asp Glu Ser Gln Cys Ser Lys Thr Asp Glu Arg Pro Ala Ser Tyr Phe
               420                 425                 430
   Ala Val Ala Val Ala Arg Lys Asp Ser Asn Val Asn Trp Asn Asn Leu
           435                 440                 445
   Lys Gly Lys Lys Ser Cys His Thr Ala Val Gly Arg Thr Ala Gly Trp
       450                 455                 460
   Val Ile Pro Met Gly Leu Ile His Asn Arg Thr Gly Thr Cys Asn Phe
   465                 470                 475                 480
   Asp Glu Tyr Phe Ser Glu Gly Cys Ala Pro Gly Ser Pro Pro Asn Ser
                   485                 490                 495
   Arg Leu Cys Gln Leu Cys Gln Gly Ser Gly Gly Ile Pro Pro Glu Lys
               500                 505                 510
   Cys Val Ala Ser Ser His Glu Lys Tyr Phe Gly Tyr Thr Gly Ala Leu
           515                 520                 525
   Arg Cys Leu Val Glu Lys Gly Asp Val Ala Phe Ile Gln His Ser Thr
   530 535 540
   Val Glu Glu Asn Thr Gly Gly Lys Asn Lys Ala Asp Trp Ala Lys Asn
   545 550 555 560
   Leu Gln Met Asp Asp Phe Glu Leu Leu Cys Thr Asp Gly Arg Arg Ala
   565 570 575
   Asn Val Met Asp Tyr Arg Glu Cys Asn Leu Ala Glu Val Pro Thr His
   580 585 590
   Ala Val Val Val Arg Pro Glu Lys Ala Asn Lys Ile Arg Asp Leu Leu
   595 600 605
   Glu Arg Gln Glu Lys Arg Phe Gly Val Asn Gly Ser Glu Lys Ser Lys
   610 615 620
   Phe Met Met Phe Glu Ser Gln Asn Lys Asp Leu Leu Phe Lys Asp Leu
   625 630 635 640
   Thr Lys Cys Leu Phe Lys Val Arg Glu Gly Thr Thr Tyr Lys Glu Phe
   645 650 655
   Leu Gly Asp Lys Phe Tyr Thr Val Ile Ser Ser Leu Lys Thr Cys Asn
   660 665 670
   Pro Ser Asp Ile Leu Gln Met Cys Ser Phe Leu Glu Gly Lys
   675 680 685
<210> 44
   <211> 117
   <212> PRT
   <213> artificial
<220>
   <223> VH region of anti-TNF-alpha/ScFv antibody
<400> 44
   Gln Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg His Gly Trp Gly Met Asp Val Trp Gly Gln Gly Thr Leu Val
               100                 105                 110
   Thr Val Phe Ile Gly
           115
<210> 45
   <211> 119
   <212> PRT
   <213> artificial
<220>
   <223> VH region, SEQ ID NO: 5 of US 5,698,195
<400> 45
   Glu Val Lys Leu Glu Glu Ser Gly Gly Gly Leu Val Gln Pro Gly Gly
   1 5 10 15
   Ser Met Lys Leu Ser Cys Val Ala Ser Gly Phe Ile Phe Ser Asn His
   20 25 30
   Trp Met Asn Trp Val Arg Gln Ser Pro Glu Lys Gly Leu Glu Trp Val
   35 40 45
   Ala Glu Ile Arg Ser Lys Ser Ile Asn Ser Ala Thr His Tyr Ala Glu
   50 55 60
   Ser Val Lys Gly Arg Phe Thr Ile Ser Arg Asp Asp Ser Lys Ser Ala
   65 70 75 80
   Val Tyr Leu Gln Met Thr Asp Leu Arg Thr Glu Asp Thr Gly Val Tyr
   85 90 95
   Tyr Cys Ser Arg Asn Tyr Tyr Gly Ser Thr Tyr Asp Tyr Trp Gly Gln
   100 105 110
   Gly Thr Thr Leu Thr Val Ser
   115
<210> 46
   <211> 223
   <212> PRT
   <213> Homo sapiens
<220>
   <221> misc_feature
   <223> VH region of anti-TNF-alpha antibody, Gen Bank No. BAB18250
<400> 46
   Gln Val Gln Leu Leu Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Asp Ser Gly Asp Leu Ala Phe Asp Ile Trp Gly Gln Gly Thr
   100 105 110
   Met Val Thr Val Ser Ser Gly Ser Ala Ser Ala Pro Thr Leu Phe Pro
   115 120 125
   Leu Val Ser Cys Glu Asn Ser Pro Ser Asp Thr Ser Ser Val Ala Val
   130 135 140
   Gly Cys Leu Ala Gln Asp Phe Leu Pro Asp Ser Ile Ile Phe Ser Trp
   145 150 155 160
   Lys Tyr Lys Asn Asn Ser Asp Ile Ser Ser Thr Arg Gly Phe Pro Ser
   165 170 175
   Val Leu Arg Gly Gly Lys Tyr Ala Ala Thr Ser Gln Val Leu Leu Pro
   180 185 190
   Ser Lys Asp Val Met Gln Gly Thr Asp Glu His Val Val Cys Lys Val
   195 200 205
   Gln His Pro Asn Gly Asn Lys Glu Lys Asn Val Pro Leu Pro Val
   210 215 220
<210> 47
   <211> 222
   <212> PRT
   <213> Homo sapiens
<220>
   <221> misc_feature
   <223> VH region of anti-TNF-alpha antibody, Gen Bank No. BAB18252
<400> 47
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Asn Ser Phe
               20                  25                  30
   Pro Ile Asn Trp Val Arg Gln Ala Pro Gly Gln Gly Leu Glu Trp Met
           35                  40                  45
   Gly Arg Ile Ile Pro Ile Ile Gly Ile Ala Asp Tyr Ala Gln Glu Phe
       50                  55                  60
   Gln Gly Arg Val Thr Ile Thr Ala Asp Arg Ser Thr Ser Thr Ala Tyr
   65                  70                  75                  80
   Met Glu Leu Arg Ser Leu Ile Ser Glu Asp Thr Ala Val Tyr Phe Cys
                   85                  90                  95
   Ala Arg Pro Glu Ala Val Thr Val Pro Ala Pro Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Thr Val Glu Arg Lys
       210                 215                 220
<210> 48
   <211> 221
   <212> PRT
   <213> Homo sapiens
<220>
   <221> misc_feature
   <223> VH region of anti-TNF-alpha antibody, Gen Bank No. BAB18254
<400> 48
   Gln Val Gln Leu Val Gln Ser Gly Ala Glu Val Lys Lys Pro Gly Ser
   1               5                   10                  15
   Ser Val Lys Val Ser Cys Met Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Thr Ser Asn Tyr Ala Gln Lys Phe Gln Gly Arg Val Thr Ile
       50                  55                  60
   Thr Ala Asp Glu Ser Thr Ser Thr Ala Tyr Met Glu Leu Ser Ser Leu
   65                  70                  75                  80
   Arg Ser Glu Asp Thr Ala Val Tyr Tyr Cys Ala Arg Glu Val Gln Phe
                   85                  90                  95
   Tyr His Asp Ser Ser Gly Tyr Leu Asp Ala Leu Asp Ile Trp Gly Gln
               100                 105                 110
   Gly Thr Met Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Thr Val Glu Arg Lys
       210                 215                 220
<210> 49
   <211> 228
   <212> PRT
   <213> Homo sapiens
<220>
   <221> misc_feature Gen Bank No. BAB18256
   <223> VH region of anti-TNF-alpha antibody, Gen Bank No. BAB18256
<400> 49
   Gln Val Gln Leu Val Gln Ser Gly Gly Gly Leu Val Gln Pro Gly Gly
   1 5 10 15
   Ser Leu Arg Leu Ser Cys Ala Ala Ser Gly Phe Thr Phe Ser Thr Tyr
   20 25 30
   Val Met Asn Trp Val Arg Gln Ala Pro Gly Lys Gly Leu Glu Trp Val
   35 40 45
   Ser Gly Ile Ser Gly Gly Gly Gly Ser Thr Tyr Tyr Ala Asp Ser Val
   50 55 60
   Lys Gly Arg Phe Thr Ile Ser Arg Asp Asn Ser Met Asn Thr Leu Tyr
   65 70 75 80
   Leu Gln Met Asn Ser Leu Arg Ala Glu Asp Thr Ala Val Tyr Tyr Cys
   85 90 95
   Ala Lys Asp Leu Ser Asn Arg Leu Ser Gly Gly Gly Thr Phe Asp Ile
   100 105 110
   Trp Gly Gln Gly Thr Met Val Thr Val Ser Ser Gly Ser Ala Ser Ala
   115 120 125
   Pro Thr Leu Phe Pro Leu Val Ser Cys Glu Asn Ser Pro Ser Asp Thr
   130 135 140
   Ser Ser Val Ala Val Gly Cys Leu Ala Gln Asp Phe Leu Pro Asp Ser
   145 150 155 160
   Ile Thr Phe Ser Trp Lys Tyr Lys Asn Asn Ser Asp Ile Ser Ser Thr
   165 170 175
   Arg Gly Phe Pro Ser Val Leu Arg Gly Gly Lys Tyr Ala Ala Thr Ser
   180 185 190
   Gln Val Leu Leu Pro Ser Lys Asp Val Met Gln Gly Thr Asp Glu His
   195 200 205
   Val Val Cys Lys Val Gln His Pro Asn Gly Asn Lys Glu Lys Asn Val
   210 215 220
   Pro Leu Pro Val
   225
<210> 50
<211> 140
<212> DNA
   <213> artificial
<220>
   <223> NcoI site region of pUC18
<220>
   <221> CDS
   <222> (48)..(140)
<400> 50
   gttgtgtgga attgtgagcg gataacaatt tcacacagga aacagct atg acc atg        56
                                                       Met Thr Met
                                                       1
   att acg aat tcg agc tcg gta ccc ggg gat cct cta gag tcg acc tgc      104
   Ile Thr Asn Ser Ser Ser Val Pro Gly Asp Pro Leu Glu Ser Thr Cys
       5                   10                  15
   agg cat gca agc ttg gca ctg gcc gtc gtt tta caa                      140
   Arg His Ala Ser Leu Ala Leu Ala Val Val Leu Gln
   20                  25                  30
<210> 51
   <211> 31
   <212> PRT
   <213> artificial
<220>
   <223> NcoI site region of pUC18
<400> 51
   Met Thr Met Ile Thr Asn Ser Ser Ser Val Pro Gly Asp Pro Leu Glu
   1               5                   10                  15
   Ser Thr Cys Arg His Ala Ser Leu Ala Leu Ala Val Val Leu Gln
               20                  25                  30
<210> 52
   <211> 27
   <212> DNA
   <213> artificial
<220>
   <223> mutagenesis primer
<400> 52
   caggaaacag ccatggccat gattacg 27
<210> 53
   <211> 27
   <212> DNA
   <213> artificial
<220>
   <223> mutagenesis primer
<400> 53
   cgtaatcatg gccatggctg tttcctg 27
<210> 54
   <211> 1400
   <212> DNA
   <213> artificial
<220>
   <223> M13 pIII coat protein for insertion into pUC18 vectors
<220>
   <221> CDS
   <222> (48)..(1349)
<400> 54
   gttgtgtgga attgtgagcg gataacaatt tcacacagga aacagcc atg gtg aaa        56
                                                       Met Val Lys
                                                       1
   aaa tta tta ttc gca att cct tta gtt gtt cct ttc tat tct cac tcc       104
   Lys Leu Leu Phe Ala Ile Pro Leu Val Val Pro Phe Tyr Ser His Ser
       5                   10                  15
   gcc gac ggg gct ggg gcc gct ggg gcc gaa act gtt gaa agt tgt tta       152
   Ala Asp Gly Ala Gly Ala Ala Gly Ala Glu Thr Val Glu Ser Cys Leu
   20                  25                  30                  35
   gca aaa cct cat aca gaa aat tca ttt act aac gtc tgg aaa gac gac       200
   Ala Lys Pro His Thr Glu Asn Ser Phe Thr Asn Val Trp Lys Asp Asp
                   40                  45                  50
   aaa act tta gat cgt tac gct aac tat gag ggc tgt ctg tgg aat gct       248
   Lys Thr Leu Asp Arg Tyr Ala Asn Tyr Glu Gly Cys Leu Trp Asn Ala
               55                  60                  65
   aca ggc gtt gtg gtt tgt act ggt gac gaa act cag tgt tac ggt aca       296
   Thr Gly Val Val Val Cys Thr Gly Asp Glu Thr Gln Cys Tyr Gly Thr
           70                  75                  80
   tgg gtt cct att ggg ctt gct atc cct gaa aat gag ggt ggt ggc tct       344
   Trp Val Pro Ile Gly Leu Ala Ile Pro Glu Asn Glu Gly Gly Gly Ser
               85                  90                  95
   gag ggt ggc ggt tct gag ggt ggc ggt tct gag ggt ggc ggt act aaa      392
   Glu Gly Gly Gly Ser Glu Gly Gly Gly Ser Glu Gly Gly Gly Thr Lys
   100                 105                 110                 115
   cct cct gag tac ggt gat aca cct att ccg ggc tat act tat atc aac      440
   Pro Pro Glu Tyr Gly Asp Thr Pro Ile Pro Gly Tyr Thr Tyr Ile Asn
                   120                 125                 130
   cct ctc gac ggc act tat ccg cct ggt act gag caa aac ccc gct aat      488
   Pro Leu Asp Gly Thr Tyr Pro Pro Gly Thr Glu Gln Asn Pro Ala Asn
               135                 140                 145
   cct aat cct tct ctt gag gag tct cag cct ctt aat act ttc atg ttt      536
   Pro Asn Pro Ser Leu Glu Glu Ser Gln Pro Leu Asn Thr Phe Met Phe
           150                 155                 160
   cag aat aat agg ttc cga aat agg cag ggt gca tta act gtt tat acg      584
   Gln Asn Asn Arg Phe Arg Asn Arg Gln Gly Ala Leu Thr Val Tyr Thr
       165                 170                 175
   ggc act gtt act caa ggc act gac ccc gtt aaa act tat tac cag tac      632
   Gly Thr Val Thr Gln Gly Thr Asp Pro Val Lys Thr Tyr Tyr Gln Tyr
   180                 185                 190                 195
   act cct gta tca tca aaa gcc atg tat gac gct tac tgg aac ggt aaa      680
   Thr Pro Val Ser Ser Lys Ala Met Tyr Asp Ala Tyr Trp Asn Gly Lys
                   200                 205                 210
   ttc aga gac tgc gct ttc cat tct ggc ttt aat gag gat cca ttc gtt      728
   Phe Arg Asp Cys Ala Phe His Ser Gly Phe Asn Glu Asp Pro Phe Val
               215                 220                 225
   tgt gaa tat caa ggc caa tcg tct gac ctg cct caa cct cct gtc aat      776
   Cys Glu Tyr Gln Gly Gln Ser Ser Asp Leu Pro Gln Pro Pro Val Asn
           230                 235                 240
   gct ggc ggc ggc tct ggt ggt ggt tct ggt ggc ggc tct gag ggt ggc      824
   Ala Gly Gly Gly Ser Gly Gly Gly Ser Gly Gly Gly Ser Glu Gly Gly
       245                 250                 255
   ggc tct gag ggt ggc ggt tct gag ggt ggc ggc tct gag ggt ggc ggt      872
   Gly Ser Glu Gly Gly Gly Ser Glu Gly Gly Gly Ser Glu Gly Gly Gly
   260                 265                 270                 275
   tcc ggt ggc ggc tcc ggt tcc ggt gat ttt gat tat gaa aaa atg gca      920
   Ser Gly Gly Gly Ser Gly Ser Gly Asp Phe Asp Tyr Glu Lys Met Ala
                   280                 285                 290
   aac gct aat aag ggg gct atg acc gaa aat gcc gat gaa aac gcg cta      968
   Asn Ala Asn Lys Gly Ala Met Thr Glu Asn Ala Asp Glu Asn Ala Leu
               295                 300                 305
   cag tct gac gct aaa ggc aaa ctt gat tct gtc gct act gat tac ggt     1016
   Gln Ser Asp Ala Lys Gly Lys Leu Asp Ser Val Ala Thr Asp Tyr Gly
           310                 315                 320
   gct gct atc gat ggt ttc att ggt gac gtt tcc ggc ctt gct aat ggt     1064
   Ala Ala Ile Asp Gly Phe Ile Gly Asp Val Ser Gly Leu Ala Asn Gly
       325                 330                 335
   aat ggt gct act ggt gat ttt gct ggc tct aat tcc caa atg gct caa      1112
   Asn Gly Ala Thr Gly Asp Phe Ala Gly Ser Asn Ser Gln Met Ala Gln
   340                 345                 350                 355
   gtc ggt gac ggt gat aat tca cct tta atg aat aat ttc cgt caa tat      1160
   Val Gly Asp Gly Asp Asn Ser Pro Leu Met Asn Asn Phe Arg Gln Tyr
                   360                 365                 370
   tta cct tct ttg cct cag tcg gtt gaa tgt cgc cct tat gtc ttt ggc      1208
   Leu Pro Ser Leu Pro Gln Ser Val Glu Cys Arg Pro Tyr Val Phe Gly
               375                 380                 385
   gct ggt aaa cca tat gaa ttt tct att gat tgt gac aaa ata aac tta      1256
   Ala Gly Lys Pro Tyr Glu Phe Ser Ile Asp Cys Asp Lys Ile Asn Leu
           390                 395                 400
   ttc cgt ggt gtc ttt gcg ttt ctt tta tat gtt gcc acc ttt atg tat      1304
   Phe Arg Gly Val Phe Ala Phe Leu Leu Tyr Val Ala Thr Phe Met Tyr
       405                 410                 415
   gta ttt tcg acg ttt gct aac ata ctg cgt aat aag gag tct taa          1349
   Val Phe Ser Thr Phe Ala Asn Ile Leu Arg Asn Lys Glu Ser
   420                 425                 430
   taagcttggc actggccgtc gttttacaac gtcgtgactg ggaaaaccct g             1400
<210> 55
   <211> 433
   <212> PRT
   <213> artificial
<220>
   <223> M13 pIII coat protein for insertion into pUC18 vectors
<400> 55
   Met Val Lys Lys Leu Leu Phe Ala Ile Pro Leu Val Val Pro Phe Tyr
   1               5                   10                  15
   Ser His Ser Ala Asp Gly Ala Gly Ala Ala Gly Ala Glu Thr Val Glu
               20                  25                  30
   Ser Cys Leu Ala Lys Pro His Thr Glu Asn Ser Phe Thr Asn Val Trp
           35                  40                  45
   Lys Asp Asp Lys Thr Leu Asp Arg Tyr Ala Asn Tyr Glu Gly Cys Leu
       50                  55                  60
   Trp Asn Ala Thr Gly Val Val Val Cys Thr Gly Asp Glu Thr Gln Cys
   65                  70                  75                  80
   Tyr Gly Thr Trp Val Pro Ile Gly Leu Ala Ile Pro Glu Asn Glu Gly
                   85                  90                  95
   Gly Gly Ser Glu Gly Gly Gly Ser Glu Gly Gly Gly Ser Glu Gly Gly
               100                 105                 110
   Gly Thr Lys Pro Pro Glu Tyr Gly Asp Thr Pro Ile Pro Gly Tyr Thr
           115                 120                 125
   Tyr Ile Asn Pro Leu Asp Gly Thr Tyr Pro Pro Gly Thr Glu Gln Asn
       130                 135                 140
   Pro Ala Asn Pro Asn Pro Ser Leu Glu Glu Ser Gln Pro Leu Asn Thr
   145                 150                 155                 160
   Phe Met Phe Gln Asn Asn Arg Phe Arg Asn Arg Gln Gly Ala Leu Thr
                   165                 170                 175
   Val Tyr Thr Gly Thr Val Thr Gln Gly Thr Asp Pro Val Lys Thr Tyr
               180                 185                 190
   Tyr Gln Tyr Thr Pro Val Ser Ser Lys Ala Met Tyr Asp Ala Tyr Trp
           195                 200                 205
   Asn Gly Lys Phe Arg Asp Cys Ala Phe His Ser Gly Phe Asn Glu Asp
       210                 215                 220
   Pro Phe Val Cys Glu Tyr Gln Gly Gln Ser Ser Asp Leu Pro Gln Pro
   225                 230                 235                 240
   Pro Val Asn Ala Gly Gly Gly Ser Gly Gly Gly Ser Gly Gly Gly Ser
                   245                 250                 255
   Glu Gly Gly Gly Ser Glu Gly Gly Gly Ser Glu Gly Gly Gly Ser Glu
               260                 265                 270
   Gly Gly Gly Ser Gly Gly Gly Ser Gly Ser Gly Asp Phe Asp Tyr Glu
           275                 280                 285
   Lys Met Ala Asn Ala Asn Lys Gly Ala Met Thr Glu Asn Ala Asp Glu
       290                 295                 300
   Asn Ala Leu Gln Ser Asp Ala Lys Gly Lys Leu Asp Ser Val Ala Thr
   305                 310                 315                 320
   Asp Tyr Gly Ala Ala Ile Asp Gly Phe Ile Gly Asp Val Ser Gly Leu
                   325                 330                 335
   Ala Asn Gly Asn Gly Ala Thr Gly Asp Phe Ala Gly Ser Asn Ser Gln
               340                 345                 350
   Met Ala Gln Val Gly Asp Gly Asp Asn Ser Pro Leu Met Asn Asn Phe
           355                 360                 365
   Arg Gln Tyr Leu Pro Ser Leu Pro Gln Ser Val Glu Cys Arg Pro Tyr
       370                 375                 380
   Val Phe Gly Ala Gly Lys Pro Tyr Glu Phe Ser Ile Asp Cys Asp Lys
   385                 390                 395                 400
   Ile Asn Leu Phe Arg Gly Val Phe Ala Phe Leu Leu Tyr Val Ala Thr
                   405                 410                 415
   Phe Met Tyr Val Phe Ser Thr Phe Ala Asn Ile Leu Arg Asn Lys Glu
               420                 425                 430
   Ser
<210> 56
   <211> 44
   <212> DNA
   <213> artificial
<220>
   <223> cloning primer
<400> 56
   ggaaacagcc atggtgaaaa aattattatt cgcaattcct ttag 44
<210> 57
   <211> 51
   <212> DNA
   <213> artificial
<220>
   <223> cloning primer
<400> 57
   cgacggccag tgccaagctt attaagactc cttattacgc agtatgttag c 51
<210> 58
   <211> 140
   <212> DNA
   <213> artificial
<220>
   <223> transferrin insertion region in M13-pUC18 vectors
<220>
   <221> CDS
   <222> (48)..(140)
<400> 58
   gttgtgtgga attgtgagcg gataacaatt tcacacagga aacagcc atg gtg aaa        56
                                                       Met Val Lys
                                                       1
   aaa tta tta ttc gca att cct tta gtt gtt cct ttc tat tct cac tcc       104
   Lys Leu Leu Phe Ala Ile Pro Leu Val Val Pro Phe Tyr Ser His Ser
       5                   10                  15
   gcc gac ggg gct ggg gcc gct ggg gcc gaa act gtt                       140
   Ala Asp Gly Ala Gly Ala Ala Gly Ala Glu Thr Val
   20                  25                  30
<210> 59
   <211> 31
   <212> PRT
   <213> artificial
<220>
   <223> transferrin insertion region in M13-pUC18 vectors
<400> 59
   Met Val Lys Lys Leu Leu Phe Ala Ile Pro Leu Val Val Pro Phe Tyr
   1 5 10 15
   Ser His Ser Ala Asp Gly Ala Gly Ala Ala Gly Ala Glu Thr Val
   20 25 30
<210> 60
   <211> 27
   <212> DNA
   <213> artificial
<220>
   <223> mutagenesis primer
<400> 60
   ctattctcac tccgcggacg gggctgg 27
<210> 61
   <211> 27
   <212> DNA
   <213> artificial
<220>
   <223> mutagenesis primer
<400> 61
   ccagccccgt ccgcggagtg agaatag 27
<210> 62
   <211> 1120
   <212> DNA
   <213> artificial
<220>
   <223> N-domain of transferrin modified for cloning into M13-pUC18 vectors
<220>
   <221> CDS
   <222> (6)..(1118)
<400> 62
   cagcc atg gtg aaa aaa tta tta ttc gca att cct tta gtt gtt cct ttc      50
         Met Val Lys Lys Leu Leu Phe Ala Ile Pro Leu Val Val Pro Phe
         1               5                   10                  15
   tat tct cac tcc gcg gac ggg gcg gta cct gat aaa act gtg aga tgg        98
   Tyr Ser His Ser Ala Asp Gly Ala Val Pro Asp Lys Thr Val Arg Trp
                   20                  25                  30
   tgt gca gtg tcg gag cat gag gcc act aag tgc cag agt ttc cgc gac       146
   Cys Ala Val Ser Glu His Glu Ala Thr Lys Cys Gln Ser Phe Arg Asp
               35                  40                  45
   cat atg aaa agc gtc att cca tcc gat ggt ccc agt gtt gct tgt gtg       194
   His Met Lys Ser Val Ile Pro Ser Asp Gly Pro Ser Val Ala Cys Val
           50                  55                  60
   aag aaa gcc tcc tac ctt gat tgc atc agg gcc att gcg gca aac gaa       242
   Lys Lys Ala Ser Tyr Leu Asp Cys Ile Arg Ala Ile Ala Ala Asn Glu
       65                  70                  75
   gcg gat gct gtg aca ctg gat gca ggt ttg gtg tat gat gct tac ctg      290
   Ala Asp Ala Val Thr Leu Asp Ala Gly Leu Val Tyr Asp Ala Tyr Leu
   80                  85                  90                  95
   gct ccc aat aac ctg aag cct gtg gtg gca gag ttc tat ggg tca aaa      338
   Ala Pro Asn Asn Leu Lys Pro Val Val Ala Glu Phe Tyr Gly Ser Lys
                   100                 105                 110
   gag gat cca cag act ttc tat tat gct gtt gct gtg gtg aag aag gat      386
   Glu Asp Pro Gln Thr Phe Tyr Tyr Ala Val Ala Val Val Lys Lys Asp
               115                 120                 125
   agt ggc ttc cag atg aac cag ctt cga ggc aag aag tcc tgc cac acg      434
   Ser Gly Phe Gln Met Asn Gln Leu Arg Gly Lys Lys Ser Cys His Thr
           130                 135                 140
   ggt cta ggc agg tcc gct ggg tgg aac atc ccc ata ggc tta ctt tac      482
   Gly Leu Gly Arg Ser Ala Gly Trp Asn Ile Pro Ile Gly Leu Leu Tyr
       145                 150                 155
   tgt gac tta cct gag cca cgt aaa cct ctt gag aaa gca gtg gcc aat      530
   Cys Asp Leu Pro Glu Pro Arg Lys Pro Leu Glu Lys Ala Val Ala Asn
   160                 165                 170                 175
   ttc ttc tcg ggc agc tgt gcc cct tgt gcg gat ggg acg gac ttc ccc      578
   Phe Phe Ser Gly Ser Cys Ala Pro Cys Ala Asp Gly Thr Asp Phe Pro
                   180                 185                 190
   cag ctg tgt caa ctg tgt cca ggg tgt ggc tgc tcc acc ctt aac caa      626
   Gln Leu Cys Gln Leu Cys Pro Gly Cys Gly Cys Ser Thr Leu Asn Gln
               195                 200                 205
   tac ttc ggc tac tcg gga gcc ttc aag tgt ctg aag gat ggt gct ggg      674
   Tyr Phe Gly Tyr Ser Gly Ala Phe Lys Cys Leu Lys Asp Gly Ala Gly
           210                 215                 220
   gat gtg gcc ttt gtc aag cac tcg act ata ttt gag aac ttg gca aac      722
   Asp Val Ala Phe Val Lys His Ser Thr Ile Phe Glu Asn Leu Ala Asn
       225                 230                 235
   aag gct gac agg gac cag tat gag ctg ctt tgc ctg gac aac acc cgg      770
   Lys Ala Asp Arg Asp Gln Tyr Glu Leu Leu Cys Leu Asp Asn Thr Arg
   240                 245                 250                 255
   aag ccg gta gat gaa tac aag gac tgc cac ttg gcc cag gtc cct tct      818
   Lys Pro Val Asp Glu Tyr Lys Asp Cys His Leu Ala Gln Val Pro Ser
                   260                 265                 270
   cat acc gtc gtg gcc cga agt atg ggc ggc aag gag gac ttg atc tgg      866
   His Thr Val Val Ala Arg Ser Met Gly Gly Lys Glu Asp Leu Ile Trp
               275                 280                 285
   gag ctt ctc aac cag gcc cag gaa cat ttt ggc aaa gac aaa tca aaa      914
   Glu Leu Leu Asn Gln Ala Gln Glu His Phe Gly Lys Asp Lys Ser Lys
           290                 295                 300
   gaa ttc caa cta ttc agc tct cct cat ggg aag gac ctg ctg ttt aag      962
   Glu Phe Gln Leu Phe Ser Ser Pro His Gly Lys Asp Leu Leu Phe Lys
       305                 310                 315
   gac tct gcc cac ggg ttt tta aaa gtc ccc ccc agg atg gat gcc aag      1010
   Asp Ser Ala His Gly Phe Leu Lys Val Pro Pro Arg Met Asp Ala Lys
   320                 325                 330                 335
   atg tac ctg ggc tat gag tat gtc act gcc atc cgg aat cta cgg gaa      1058
   Met Tyr Leu Gly Tyr Glu Tyr Val Thr Ala Ile Arg Asn Leu Arg Glu
                   340                 345                 350
   ggc aca tgc cca gaa gcc cca aca gat gaa ggg gcc gct ggg gcc gaa      1106
   Gly Thr Cys Pro Glu Ala Pro Thr Asp Glu Gly Ala Ala Gly Ala Glu
               355                 360                 365
   act gtt gaa agt tg                                                   1120
   Thr Val Glu Ser
           370
<210> 63
   <211> 371
   <212> PRT
   <213> artificial
<220>
   <223> N-domain of transferrin modified for cloning into M13-pUC18 vectors
<400> 63
   Met Val Lys Lys Leu Leu Phe Ala Ile Pro Leu Val Val Pro Phe Tyr
   1 5 10 15
   Ser His Ser Ala Asp Gly Ala Val Pro Asp Lys Thr Val Arg Trp Cys
   20 25 30
   Ala Val Ser Glu His Glu Ala Thr Lys Cys Gln Ser Phe Arg Asp His
   35 40 45
   Met Lys Ser Val Ile Pro Ser Asp Gly Pro Ser Val Ala Cys Val Lys
   50 55 60
   Lys Ala Ser Tyr Leu Asp Cys Ile Arg Ala Ile Ala Ala Asn Glu Ala
   65 70 75 80
   Asp Ala Val Thr Leu Asp Ala Gly Leu Val Tyr Asp Ala Tyr Leu Ala
   85 90 95
   Pro Asn Asn Leu Lys Pro Val Val Ala Glu Phe Tyr Gly Ser Lys Glu
   100 105 110
   Asp Pro Gln Thr Phe Tyr Tyr Ala Val Ala Val Val Lys Lys Asp Ser
   115 120 125
   Gly Phe Gln Met Asn Gln Leu Arg Gly Lys Lys Ser Cys His Thr Gly
   130 135 140
   Leu Gly Arg Ser Ala Gly Trp Asn Ile Pro Ile Gly Leu Leu Tyr Cys
   145 150 155 160
   Asp Leu Pro Glu Pro Arg Lys Pro Leu Glu Lys Ala Val Ala Asn Phe
                   165                 170                 175
   Phe Ser Gly Ser Cys Ala Pro Cys Ala Asp Gly Thr Asp Phe Pro Gln
               180                 185                 190
   Leu Cys Gln Leu Cys Pro Gly Cys Gly Cys Ser Thr Leu Asn Gln Tyr
           195                 200                 205
   Phe Gly Tyr Ser Gly Ala Phe Lys Cys Leu Lys Asp Gly Ala Gly Asp
       210                 215                 220
   Val Ala Phe Val Lys His Ser Thr Ile Phe Glu Asn Leu Ala Asn Lys
   225                 230                 235                 240
   Ala Asp Arg Asp Gln Tyr Glu Leu Leu Cys Leu Asp Asn Thr Arg Lys
                   245                 250                 255
   Pro Val Asp Glu Tyr Lys Asp Cys His Leu Ala Gln Val Pro Ser His
               260                 265                 270
   Thr Val Val Ala Arg Ser Met Gly Gly Lys Glu Asp Leu Ile Trp Glu
           275                 280                 285
   Leu Leu Asn Gln Ala Gln Glu His Phe Gly Lys Asp Lys Ser Lys Glu
       290                 295                 300
   Phe Gln Leu Phe Ser Ser Pro His Gly Lys Asp Leu Leu Phe Lys Asp
   305                 310                 315                 320
   Ser Ala His Gly Phe Leu Lys Val Pro Pro Arg Met Asp Ala Lys Met
                   325                 330                 335
   Tyr Leu Gly Tyr Glu Tyr Val Thr Ala Ile Arg Asn Leu Arg Glu Gly
               340                 345                 350
   Thr Cys Pro Glu Ala Pro Thr Asp Glu Gly Ala Ala Gly Ala Glu Thr
           355                 360                 365
   Val Glu Ser
       370
<210> 64
   <211> 44
   <212> DNA
   <213> artificial
<220>
   <223> cloning primer
<400> 64
   ctcactccgc ggacggggcg gtacctgata aaactgtgag atgg 44
<210> 65
   <211> 49
   <212> DNA
   <213> artificial
<220>
   <223> cloning primer
<400> 65
   ctttcaacag tttcggcccc agcggcccct tcatctgttg gggcttctg 49
<210> 66
   <211> 282
   <212> DNA
   <213> artificial
<220>
   <223> variable region for generating library in M13-pUC18 vectors
<220>
   <221> misc feature
   <222> (1)..(282)
   <223> n = a or c or g or t
<220>
   <221> CDS
   <222> (1)..(282)
<400> 66
   tca aaa gaa ttc caa cta ttc agc tct cct nnn nnn nnn nnn nnn nnn      48
   Ser Lys Glu Phe Gln Leu Phe Ser Ser Pro Xaa Xaa Xaa Xaa Xaa Xaa
   1               5                   10                  15
   nnn nnn cat ggg aag gac ctg ctg ttt aag gac tct gcc cac ggg ttt      96
   Xaa Xaa His Gly Lys Asp Leu Leu Phe Lys Asp Ser Ala His Gly Phe
               20                  25                  30
   tta aaa gtc ccc ccc agg atg gat gcc aag atg tac ctg ggc tat gag     144
   Leu Lys Val Pro Pro Arg Met Asp Ala Lys Met Tyr Leu Gly Tyr Glu
           35                  40                  45
   tat gtc act gcc atc cgg aat cta cgg gaa ggc aca tgc cca gaa gcc     192
   Tyr Val Thr Ala Ile Arg Asn Leu Arg Glu Gly Thr Cys Pro Glu Ala
       50                  55                  60
   cca aca gat gaa ggg gcc gct ggg gcc gaa act gtt gaa agt tgt tta     240
   Pro Thr Asp Glu Gly Ala Ala Gly Ala Glu Thr Val Glu Ser Cys Leu
   65                  70                  75                  80
   gca aaa cct cat aca gaa aat tca ttt act aac gtc tgg aaa             282
   Ala Lys Pro His Thr Glu Asn Ser Phe Thr Asn Val Trp Lys
                   85                  90
<210> 67
   <211> 94
   <212> PRT
   <213> artificial
<220>
   <221> misc_feature
   <222> (11)..(11)
   <223> The 'Xaa' at location 11 stands for Lys, Asn, Arg, Ser, Thr, Ile, Met, Glu, Asp, Gly, Ala, Val, Gln, His, Pro, Leu, Tyr, Trp, Cys, or Phe.
<220>
   <221> misc_feature
   <222> (12)..(12)
   <223> The 'Xaa' at location 12 stands for Lys, Asn, Arg, Ser, Thr, Ile, Met, Glu, Asp, Gly, Ala, Val, Gln, His, Pro, Leu, Tyr, Trp, Cys, or Phe.
<220>
   <221> misc_feature
   <222> (13)..(13)
   <223> The 'Xaa' at location 13 stands for Lys, Asn, Arg, Ser, Thr, Ile, Met, Glu, Asp, Gly, Ala, Val, Gln, His, Pro, Leu, Tyr, Trp, Cys, or Phe.
<220>
   <221> misc_feature
   <222> (14)..(14)
   <223> The 'Xaa' at location 14 stands for Lys, Asn, Arg, Ser, Thr, Ile, Met, Glu, Asp, Gly, Ala, Val, Gln, His, Pro, Leu, Tyr, Trp, Cys, or Phe.
<220>
   <221> misc_feature
   <222> (15)..(15)
   <223> The 'Xaa' at location 15 stands for Lys, Asn, Arg, Ser, Thr, Ile, Met, Glu, Asp, Gly, Ala, Val, Gln, His, Pro, Leu, Tyr, Trp, Cys, or Phe.
<220>
   <221> misc_feature
   <222> (16)..(16)
   <223> The 'Xaa' at location 16 stands for Lys, Asn, Arg, Ser, Thr, Ile, Met, Glu, Asp, Gly, Ala, Val, Gln, His, Pro, Leu, Tyr, Trp, Cys, or Phe.
<220>
   <221> misc_feature
   <222> (17)..(17)
   <223> The 'Xaa' at location 17 stands for Lys, Asn, Arg, Ser, Thr, Ile, Met, Glu, Asp, Gly, Ala, Val, Gln, His, Pro, Leu, Tyr, Trp, Cys, or Phe.
<220>
   <221> misc_feature
   <222> (18)..(18)
   <223> The 'Xaa' at location 18 stands for Lys, Asn, Arg, Ser, Thr, Ile, Met, Glu, Asp, Gly, Ala, Val, Gln, His, Pro, Leu, Tyr, Trp, Cys, or Phe.
<220>
   <223> variable region for generating library in M13-pUC18 vectors
<400> 67
   Ser Lys Glu Phe Gln Leu Phe Ser Ser Pro Xaa Xaa Xaa Xaa Xaa Xaa
   1               5                   10                  15
   Xaa Xaa His Gly Lys Asp Leu Leu Phe Lys Asp Ser Ala His Gly Phe
               20                  25                  30
   Leu Lys Val Pro Pro Arg Met Asp Ala Lys Met Tyr Leu Gly Tyr Glu
           35                  40                  45
   Tyr Val Thr Ala Ile Arg Asn Leu Arg Glu Gly Thr Cys Pro Glu Ala
       50                  55                  60
   Pro Thr Asp Glu Gly Ala Ala Gly Ala Glu Thr Val Glu Ser Cys Leu
   65                  70                  75                  80
   Ala Lys Pro His Thr Glu Asn Ser Phe Thr Asn Val Trp Lys
                   85                  90
<210> 68
   <211> 61
   <212> DNA
   <213> artificial
<220>
   <223> cloning primer
<220>
   <221> misc_feature
   <222> (1)..(61)
   <223> n = a or c or g or t
<400> 68
   ccaactattc agctctcctn nnnnnnnnnn nnnnnnnnnn nnncatggga aggacctgct      60
   g                                                                      61
<210> 69
   <211> 61
   <212> DNA
   <213> artificial
<220>
   <223> cloning primer
<220>
   <221> misc_feature
   <222> (1)..(61)
   <223> n = a or c or g or t
<400> 69
   ggttgataag tcgagaggan nnnnnnnnnn nnnnnnnnnn nnngtaccct tcctggacga      60
   c                                                                      61
<210> 70
   <211> 111
   <212> PRT
   <213> artificial
<220>
   <223> VL region of anti-TNF-alpha/ScFv antibody
<400> 70
   Gln Ala Val Leu Thr Gln Pro Ser Ser Val Ser Gly Ala Pro Gly Gln
   1 5 10 15
   Arg Val Thr Ile Ser Cys Thr Gly Ser Ser Ser Asn Ile Gly Ala Gly
   20 25 30
   Tyr Asp Val His Trp Tyr Gln Gln Leu Pro Gly Thr Ala Pro Lys Leu
   35 40 45
   Leu Ile Tyr Gly Asn Ser Asn Arg Pro Ser Gly Val Pro Asp Arg Phe
   50 55 60
   Ser Gly Ser Lys Ser Gly Thr Ser Ala Ser Leu Ala Ile Thr Gly Leu
   65 70 75 80
   Gln Ala Glu Asp Glu Ala Asp Tyr Tyr Cys Gln Ser Tyr Asp Ser Ser
   85 90 95
   Leu Ser Gly Ser Val Phe Gly Gly Gly Thr Lys Leu Thr Val Leu
   100 105 110
<210> 71
   <211> 107
   <212> PRT
   <213> artificial
<220>
   <223> VL region, SEQ ID NO: 3 of US 5,698,195
<400> 71
   Asp Ile Leu Leu Thr Gln Ser Pro Ala Ile Leu Ser Val Ser Pro Gly
   1               5                   10                  15
   Glu Arg Val Ser Phe Ser Cys Arg Ala Ser Gln Phe Val Gly Ser Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Met Ser Gly Ile Pro Ser Arg Phe Ser Gly
       50                  55                  60
   Ser Gly Ser Gly Thr Asp Phe Thr Leu Ser Ile Asn Thr Val Glu Ser
   65                  70                  75                  80
   Glu Asp Ile Ala Asp Tyr Tyr Cys Gln Gln Ser His Ser Trp Pro Phe
                   85                  90                  95
   Thr Phe Gly Ser Gly Thr Asn Leu Glu Val Lys
               100                 105
<210> 72
   <211> 214
   <212> PRT
   <213> Homo sapiens
<220>
   <221> MISC_FEATURE
   <223> VL region of anti-TNF-alpha antibody, Gen Bank No. BAB18251
<400> 72
   Glu Ile Val Met Thr Gln Ser Pro Ala Thr Leu Ser Leu Ser Pro Gly
   1 5 10 15
   Glu Arg Ala Thr Pro Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Val Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Leu Gln Arg Asp Asn Trp Pro Trp
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 73
   <211> 213
   <212> PRT
   <213> Homo sapiens
<220>
   <221> MISC_FEATURE
   <223> VL region of anti-TNF-alpha antibody, Gen Bank No. BAB18253
<400> 73
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Gly Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Trp Thr
                   85                  90                  95
   Phe Gly Gln Gly Thr Lys Val Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Leu Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 74
   <211> 213
   <212> PRT
   <213> Homo sapiens
<220>
   <221> MISC_FEATURE
   <223> VL region of anti-TNF-alpha antibody, Gen Bank No. BAB18255
<400> 74
   Asp Ile Glu Leu Thr Gln Ser Pro Ser Thr Leu Ser Ala Ser Val Gly
   1               5                   10                  15
   Asp Arg Val Thr Ile Thr Cys Arg Ala Ser Gln Ser Leu Asn Asn Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Ala Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Pro Trp Thr
                   85                  90                  95
   Phe Gly Gln Gly Thr Lys Val Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Leu Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 75
<211> 214
   <212> PRT
   <213> Homo sapiens
<220>
   <221> MISC_FEATURE
   <223> VL region of anti-TNF-alpha antibody, Gen Bank No. BAB18257
<400> 75
   Asp Ile Glu Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Asn Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Val Val Tyr Tyr Cys Gln Gln Arg Ser Asn Trp Pro Leu
   85 90 95
   Thr Phe Gly Gly Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210

## Description

### RELATED APPLICATIONS

This application claims priority to U.S. Provisional Application 60/485,404, filed July 9, 2003, U.S. Patent Application 10/384,060, filed March 10, 2003, and U.S. Provisional Application 60/406,997, filed August 30, 2002.

### FIELD OF THE INVENTION

The present invention relates to phage libraries containing proteins or peptides with extended serum stability and/or *in vivo* circulatory half-life, particularly to proteins or peptides fused to or inserted in a transferrin molecule modified to reduce or inhibit transferrin receptor binding.

### BACKGROUND OF THE INVENTION

Therapeutic proteins or peptides in their native state or when recombinantly produced are typically labile molecules exhibiting short periods of serum stability or short *in vivo* circulatory half-lives. In addition, these molecules are often extremely labile when formulated, particularly when formulated in aqueous solutions for diagnostic and therapeutic purposes.

Few practical solutions exist to extend or promote the stability in *vivo* or *in vitro* of proteinaceous therapeutic molecules. Polyethylene glycol (PEG) is a substance that can be attached to a protein, resulting in longer-acting, sustained activity of the protein. If the activity of a protein is prolonged by the attachment to PEG, the frequency that the protein needs to be administered may be decreased. PEG attachment, however, often decreases or destroys the protein's therapeutic activity. While in some instance PEG attachment can reduce immunogenicity of the protein, in other instances it may increase immunogenicity.

Therapeutic proteins or peptides have also been stabilized by fusion to a protein capable of extending the *in vivo* circulatory half-life of the therapeutic protein. For instance, therapeutic proteins fused to albumin or to antibody fragments may exhibit extended in *vivo* circulatory half-life when compared to the therapeutic protein in the unfused state. See U.S. Patents 5,876,969 and 5,766,883.

Another serum protein, glycosylated human transferrin (Tf) has also been used to make fusions with therapeutic proteins to target delivery to the interior of cells or to carry agents across the blood-brain barrier. These fusion proteins comprising glycosylated human Tf have been used to target nerve growth factor (NGF) or ciliary neurotrophic factor (CNTF) across the blood-brain barrier by fusing full-length Tf to the agent. See U.S. Patents 5,672,683 and 5977,307. In these fusion proteins, the Tf portion of the molecule is glycosylated and binds to two atoms of iron, which is required for Tf binding to its receptor on a cell and, according to the inventors of these patents, to target delivery of the NGF or CNTF moiety across the blood-brain barrier. Park et al (Journal of Drug Targeting Vol 6, No.1 pp 53- 64) explores the ability to use genetic fusions of transferrin as carrier for brain targeting and delivery. Transferrin fusion proteins have also been produced by inserting an HIV-1 protease target sequence into surface exposed loops of glycosylated transferrin to investigate the ability to produce another form of Tf fusion for targeted delivery to the inside of a cell via the Tf receptor (Ali et al. (1999) J. Biol. Chem. 274(34):24066-24073). See also Shin et al (Proc. Natl. Acad. Sci. USA Vol. 92, pp2820-2824).

Serum transferrin (Tf) is a monomeric glycoprotein with a molecular weight of 80,000 daltons that binds iron in the circulation and transports it to various tissues via the transferrin receptor (TfR) (Aisen et al. (1980) Ann. Rev. Biochem. 49: 357-393; MacGillivray et al. (1981) J. Biol. Chem. 258: 3543-3553, U.S. Patent 5,026,651). Tf is one of the most common serum molecules, comprising up to about 5-10% of total serum proteins. Carbohydrate deficient transferrin occurs in elevated levels in the blood of alcoholic individuals and exhibits a longer half life (approximately 14-17 days) than that of glycosylated transferrin (approximately 7-10 days). See van Eijk et al. (1983) Clin. Chim. Acta 132:167-171, Stibler (1991) Clin. Chem. 37:2029-2037 (1991), Arndt (2001) Clin. Chem. 47(1):13-27 and Stibler et al. in "Carbohydrate-deficient consumption", Advances in the Biosciences, (Ed Nordmann et al.), Pergamon, 1988, Vol. 71, pages 353-357).

The structure of Tf has been well characterized and the mechanism of receptor binding, iron binding and release and carbonate ion binding have been elucidated (U.S. Patents 5,026,651, 5,986,067 and MacGillivray et al. (1983) J. Biol. Chem. 258(6):3543-3546).

Transferrin and antibodies that bind the transferrin receptor have also been used to deliver or carry toxic agents to tumor cells as cancer therapy (Baselga and Mendelsohn, 1994), and transferrin has been used as a non-viral gene therapy vector to deliver DNA to cells (Frank *et al*., 1994; Wagner *et al*., 1992). The ability to deliver proteins to the central nervous system (CNS) using the transferrin receptor as the entry point has been demonstrated with several proteins and peptides including CD4 (Walus *et al*., 1996), brain derived neurotrophic factor (Pardridge *et al*., 1994), glial derived neurotrophic factor (Albeck *et al*.), a vasointestinal peptide analogue (Bickel *et al*., 1993), a beta-amyloid peptide (Saito *et al*., 1995), and an antisense oligonucleotide (Pardridge *et al*., 1995).

Transferrin fusion proteins have not, however, been modified or engineered to extend the *in vivo circulatory* half-life of a therapeutic protein nor peptide or to increase bioavailability by reducing or inhibiting glycosylation of the Tf moiety nor to reduce or prevent iron and/or Tf receptor binding.

### SUMMARY OF THE INVENTION

As described in more detail below, the present invention includes a phage library containing a plurality of fusion proteins, each comprising a first transferrin (Tf) polypeptide fused to at least one second polypeptide, wherein the Tf peptide has reduced affinity for a transferrin receptor (TfR).

In a preferred embodiment, the modified Tf fusion proteins comprise a human transferrin Tf moiety that has been modified to reduce or prevent iron and receptor binding and optionally glycosylation.

### BRIEF DESCRIPTION OF THE DRAWINGS

**Figure 1** shows an alignment of the N and C Domains of Human (Hu) transferrin (Tf) (amino acids I-331 and 332-679 of SEQ ID NO: 3, respectively) with similarities and identities highlighted.
**Figure 2A-2B** show an alignment of transferrin sequences from different animal species. Light shading: Similarity; Dark shading: Identity. The species are as follows: rabbit (SEQ ID NO: 37), rat (SEQ ID NO: 38); mouse (SEQ ID NO: 39), horse (SEQ ID NO: 40), bovine (SEQ ID NO: 41), pig (SEQ ID NO: 42) and chicken (SEQ ID NO: 43).
**Figure 3** shows the location of a number of Tf surface exposed insertion sites for therapeutic proteins, polypeptides or peptides.

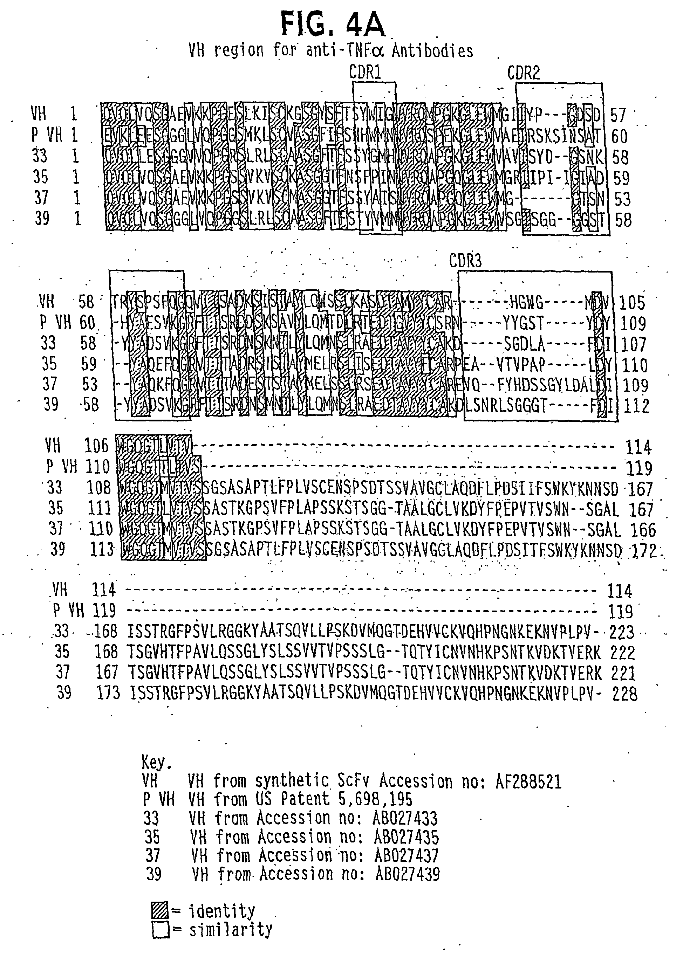

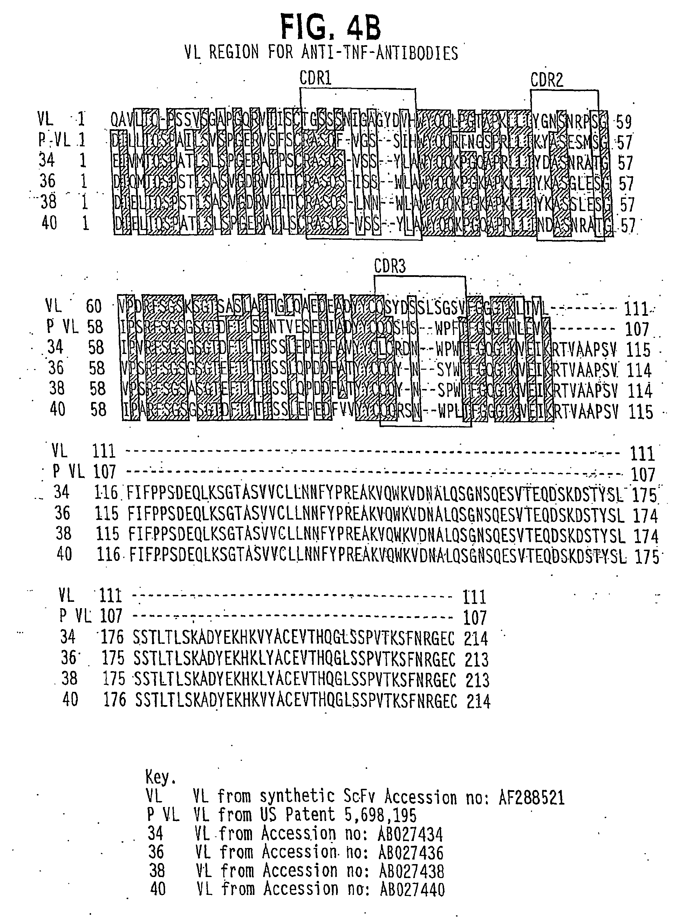

**Figures 4A****-4B** show the VH and VL regions for a number of preferred anti-TNFα antibodies used to produce modified Tf fusion proteins. The VH regions are as follows: VH from synthetic ScFv, GenBank Accession No. AAK83057 (SEQ ID NO: 44), VH from SEQ ID NO: 5 of U.S. Patent No. 5,698,195 (SEQ ID NO: 45), VH from GenBank Accession No. BAB 18250 (SEQ ID NO: 46), VH from GenBank Accession No. BAB 18252 (SEQ ID NO: 47), VH from GenBank Accession No. BAB 18254 (SEQ ID NO: 48), VH from GenBank Accession No. BAB 18256 (SEQ ID NO: 49), VL from synthetic ScFv, GenBank Accession No. AAK83057 (SEQ ID NO: 70), VL from SEQ ID NO: 3 of U.S. Patent No. 5,698,195 (SEQ ID NO: 71), VL from GenBank Accession No. BAB18251 (SEQ ID NO: 72), VL from GenBank Accession No. BAB 18253 (SEQ ID NO: 73), VL from GenBank Accession No. BAB 18255 (SEQ ID NO: 74) and VL from GenBank Accession No. BAB 18257 (SEQ ID NO: 75).
**Figure 5** shows mutation of the pUC18 sequence for insertion of a *Nco*I site (SEQ ID NOS: 50 and 51, DNA and protein sequences, respectively). The mutagenesis primers used (P 180 and P181, SEQ ID NOS: 52 and 53, respectively) are also shown.
**Figures 6A****-6B** show the sequence of M13 pIII generated by PCR for insertion into pUC18/*Nco*I (SEQ ID NOS: 54 and 55, DNA and protein sequences, respectively). Also shown are the cloning primers used, P182 and P183 (SEQ ID NOS: 56 and 57, respectively).
**Figure 7** shows the insertion site for the N-domain of transferrrin (SEQ ID NOS: 58 and 59, DNA and protein sequences, respectively). The primers used to generate the Sac II recognition sequence are also shown, P184 and P185 (SEQ ID NOS: 60 and 61, respectively).
**Figures 8A****-8B** show the result of PCR mutagenesis of the N-domain for insertion into pUC18pIIISacII (SEQ ID NOS: 62 and 63, DNA and protein sequences, respectively). The cloning primers used are also shown (P186 and P187, SEQ ID NOS: 64 and 65, respectively).
**Figure 9** shows mutagenic PCR (two rounds).
**Figure 10** shows mutagenic primers for insertion of a variable eight amino acid region, (X)₈, primers P0234 and P0235 (SEQ ID NOS: 68 and 69, respectively). Also shown is the region of M13-pUC18 vectors generated by reaction with these primers (SEQ ID NOS: 66 and 67, DNA and protein sequences, respectively).

### DETAILED DESCRIPTION

### General Description

It has been discovered that a therapeutic protein (*e*.*g*., a polypeptide, antibody, or peptide, or fragments and variants thereof) can be stabilized to extend the serum half-life and/or retain the therapeutic protein's activity for extended periods of time in *vivo* by genetically fusing or chemically conjugating the therapeutic protein, polypeptide or peptide to all or a portion of modified transferrin sufficient to extend its half life in serum. The modified transferrin fusion proteins include a transferrin protein or domain covalently linked to a therapeutic protein or peptide, wherein the transferrin portion is modified to contain one or more amino acid substitutions, insertions or deletions compared to a wild-type transferrin sequence. The Tf fusion proteins may be engineered to reduce or prevent glycosylation within the Tf or a Tf domain, or the Tf protein or Tf domain(s) may be modified to exhibit reduced or no binding to iron or carbonate ion, or to have a reduced affinity or not bind to a Tf receptor (TfR).

The present invention includes a phage library containing a plurality of fusion proteins, each comprising a first transferrin (Tf) polypeptide fused to at least one second polypeptide, wherein the Tf peptide has reduced affinity for a transferrin receptor (TfR). The transferrin fusion protein may include at least a fragment or variant of a therapeutic protein and at least a fragment or variant of modified transferrin, which are associated with one another, by genetic fusion (*i*.*e*., the transferrin fusion protein is generated by translation of a nucleic acid in which a polynucleotide encoding all or a portion of a therapeutic protein is joined in-frame with a polynucleotide encoding all or a portion of modified transferrin). The therapeutic protein and transferrin protein, once part of the transferrin fusion protein, may be referred to as a "portion", "region" or "moiety" of the.transferrin fusion protein (*e*.*g*., a "therapeutic protein portion' or a "transferrin protein portion").

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

### Definitions

As used herein, the term "biological activity" refers to a function or set of activities performed by a therapeutic molecule, protein or peptide in a biological context (*i*.*e*., in an organism or an *in vitro* facsimile thereof). Biological activities may include but are not limited to the functions of the therapeutic molecule portion of the claimed fusion proteins, such as, but not limited to, the induction of extracellular matrix secretion from responsive cell lines, the induction of hormone secretion, the induction of chemotaxis, the induction of mitogenesis, the induction of differentiation, or the inhibition of cell division of responsive cells. A fusion protein or peptide of the invention is considered to be biologically active if it exhibits one or more biological activities of its therapeutic protein's native counterpart.

As used herein, an "amino acid corresponding to" or an "equivalent amino acid" in a transferrin sequence is identified by alignment to maximize the identity or similarity between a first transferrin sequence and at least a second transferrin sequence. The number used to identify an equivalent amino acid in a second transferrin sequence is based on the number used to identify the corresponding amino acid in the first transferrin sequence. In certain cases, these phrases may be used to describe the amino acid residues in human transferrin compared to certain residues in rabbit serum transferrin.

As used herein, the terms "fragment of a Tf protein" or "Tf protein," or "portion of a Tf protein" refer to an amino acid sequence comprising at least about 5%, 10%, 20%, 30%, 40%, 50%, 60%, 70%, 80%, 90%, 95%, 96%, 97%, 98%, 99% or 100% of a naturally occurring Tf protein or mutant thereof.

As used herein, the term "gene" refers to any segment of DNA associated with a biological function. Thus, genes include, but are not limited to, coding sequences and/or the regulatory sequences required for their expression. Genes can also include nonexpressed DNA segments that, for example, form recognition sequences for other proteins. Genes can be obtained from a variety of sources, including cloning from a source of interest or synthesizing from known or predicted sequence information, and may include sequences designed to have desired parameters.

As used herein, a "heterologous polynucleotide" or a "heterologous nucleic acid" or a "heterologous gene" or a "heterologous sequence" or an "exogenous DNA segment" refers to a polynucleotide, nucleic acid or DNA segment that originates from a source foreign to the particular host cell, or, if from the same source, is modified from its original form. A heterologous gene in a host cell includes a gene that is endogenous to the particular host cell, but has been modified. Thus, the terms refer to a DNA segment which is foreign or heterologous to the cell, or homologous to the cell but in a position within the host cell nucleic acid in which the element is not ordinarily found. As an example, a signal sequence native to a yeast cell but attached to a human Tf sequence is heterologous.

As used herein, an "isolated" nucleic acid sequence refers to a nucleic acid sequence which is essentially free of other nucleic acid sequences, *e.g.*, at least about 20% pure, preferably at least about 40% pure, more preferably about 60% pure, even more preferably about 80% pure, most preferably about 90% pure, and even most preferably about 95% pure, as determined by agarose gel electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

As used herein, two or more DNA coding sequences are said to be "joined" or "fused" when, as a result of in-frame fusions between the DNA coding sequences, the DNA coding sequences are translated into a fusion polypeptide.

As used herein, the term "fusion" in reference to Tf fusions includes, but is not limited to, attachment of at least one therapeutic protein, polypeptide or peptide, preferably an antibody variable region, to the N-terminal end of Tf, attachment to the C-terminal end of Tf, insertion between any two amino acids within Tf, and/or replacement of a portion of Tf sequence such as the Tf loop.

"Modified transferrin" as used herein refers to a transferrin molecule that exhibits at least one modification of its amino acid sequence, compared to wildtype transferrin. "Modified transferrin fusion protein" as used herein refers to a protein formed by the fusion of at least one molecule of modified transferrin (or a fragment or variant thereof) to at least one molecule of a therapeutic protein (or fragment or variant thereof).

As used herein, the terms "nucleic acid" or "polynucleotide" refer to deoxyribonucleotides or ribonucleotides and polymers thereof in either single- or doublestranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.* degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al. (1991) Nucleic Acid Res. 19:5081; Ohtsuka et al. (1985) J. Biol. Chem. 260:2605-2608; Cassol *et al*. (1992); Rossolini et al. (1994) Mol. Cell. Probes 8:91-98). The term nucleic acid is used interchangeably with gene, cDNA, and mRNA encoded by a gene.

As used herein, a DNA segment is referred to as "operably linked" when it is placed into a functional relationship with another DNA segment. For example, DNA for a signal sequence is operably linked to DNA encoding a fusion protein of the invention if it is expressed as a preprotein that participates in the secretion of the fusion protein; a promoter or enhancer is operably linked to a coding sequence if it stimulates the transcription of the sequence. Generally, DNA sequences that are operably linked are contiguous, and in the case of a signal sequence or fusion protein both contiguous and in reading phase. However, enhancers need not be contiguous with the coding sequences whose transcription they control. Linking, in this context, is accomplished by ligation at convenient restriction sites or at adapters or linkers inserted in lieu thereof.

As used herein, the term "promoter" refers to a region of DNA involved in binding RNA polymerase to initiate transcription.

As used herein, the term "recombinant" refers to a cell, tissue or organism that has undergone transformation with a new combination of genes or DNA.

As used herein, a targeting entity, protein, polypeptide or peptide refers to a molecule that binds specifically to a particular cell type [normal (*e.g.*, lymphocytes) or abnormal (*e*.*g*., cancer cell)] and therefore may be used to target a Tf fusion protein or compound (drug, or cytotoxic agent) to that cell type specifically.

As used herein, "therapeutic protein" refers to proteins, polypeptides, peptides or fragments or variants thereof, having one or more therapeutic and/or biological activities. Therapeutic proteins encompassed by the invention include but are not limited to proteins, polypeptides, peptides, antibodies, and biologics. The terms peptides, proteins, and polypeptides are used interchangeably herein. Additionally, the term "therapeutic protein" may refer to the endogenous or naturally occurring correlate of a therapeutic protein. By a polypeptide displaying a "therapeutic activity" or a protein that is "therapeutically active" is meant a polypeptide that possesses one or more known biological and/or therapeutic activities associated with a therapeutic protein such as one or more of the therapeutic proteins described herein or otherwise known in the art. As a non-limiting example, a "therapeutic protein" is a protein that is useful to treat, prevent or ameliorate a disease, condition or disorder. Such a disease, condition or disorder may be in humans or in a non-human animal, *e*.*g*., veterinary use.

As used herein, the term "transformation" refers to the transfer of nucleic acid (*i*.*e*., a nucleotide polymer) into a cell. As used herein, the term "genetic transformation" refers to the transfer and incorporation of DNA, especially recombinant DNA, into a cell.

As used herein, the term "transformant" refers to a cell, tissue or organism that has undergone transformation.

As used herein, the term "transgene" refers to a nucleic acid that is inserted into an organism, host cell or vector in a manner that ensures its function.

As used herein, the term "transgenic" refers to cells, cell cultures, organisms, bacteria, fungi, animals, plants, and progeny of any of the preceding, which have received a foreign or modified gene and in particular a gene encoding a modified Tf fusion protein by one of the various methods of transformation, wherein the foreign or modified gene is from the same or different species than the species of the organism receiving the foreign or modified gene.

"Variants or variant" refers to a polynucleotide or nucleic acid differing from a reference nucleic acid or polypeptide, but retaining essential properties thereof. Generally, variants are overall closely similar, and, in many regions, identical to the reference nucleic acid or polypeptide. As used herein, "variant" refers to a therapeutic protein portion of a transferrin fusion protein of the invention, differing in sequence from a native therapeutic protein but retaining at least one functional and/or therapeutic property thereof as described elsewhere herein or otherwise known in the art.

As used herein, the term "vector" refers broadly to any plasmid, phagemid or virus encoding an exogenous nucleic acid. The term is also be construed to include non-plasmid, non-phagemid and non-viral compounds which facilitate the transfer of nucleic acid into virions or cells, such as, for example, polylysine compounds and the like. The vector may be a viral vector that is suitable as a delivery vehicle for delivery of the nucleic acid, or mutant thereof, to a cell, or the vector may be a non-viral vector which is suitable for the same purpose. Examples of viral and non-viral vectors for delivery of DNA to cells and tissues are well known in the art and are described, for example, in Ma et al. (1997, Proc. Natl. Acad. Sci. U.S.A. 94:12744-12746). Examples of viral vectors include, but are not limited to, a recombinant vaccinia virus, a recombinant adenovirus, a recombinant retrovirus, a recombinant adeno-associated virus, a recombinant avian pox virus, and the like (Cranage et al., 1986, EMBO J. 5:3057-3063; International Patent Application No. WO94/17810, published August 18, 1994; International Patent Application No. WO94/23744, published October 27, 1994). Examples of non-viral vectors include, but are not limited to, liposomes, polyamine derivatives of DNA, and the like.

As used herein, the term "wild type" refers to a polynucleotide or polypeptide sequence that is naturally occurring.

As used herein, "scaffold protein", "scaffold polypeptide", or "scaffold" refers to a protein to which amino acid sequences such as random peptides, can be fused. The peptides are exogenous to the scaffold.

As used herein, "random peptide sequence" refers to an amino acid sequence composed of two or more amino acid monomers and constructed by a stochastic or random process. A random peptide can include framework or scaffolding motifs, which may comprise invariant sequences.

As used herein "random peptide library" refers to a set of polynucleotide sequences that encodes a set of random peptides, and to the set of random peptides encoded by those polynucleotide sequences, as well as the fusion proteins containing those random peptides.

As used herein, the term "pseudorandom" refers to a set of sequences that have limited variability, so that for example, the degree of residue variability at one position is different than the degree of residue variability at another position, but any pseudorandom position is allowed some degree of residue variation, however circumscribed.

As used herein, the term "defined sequence framework" refers to a set of defined sequences that are selected on a nonrandom basis, generally on the basis of experimental data or structural data; for example, a defined sequence framework may comprise a set of amino acid sequences that are predicted to form a β-sheet structure or may comprise a leucine zipper heptad repeat motif, a zinc-finger domain, among other variations. A "defined sequence kemal" is a set of sequences which encompass a limited scope of variability. Whereas (1) a completely random 10-mer sequence of the 20 conventional amino acids can be any of (20)¹⁰ sequences, and (2) a pseudorandom 10-mer sequence of the 20 conventional amino acids can be any of (20)¹⁰ sequences but will exhibit a bias for certain residues at certain positions and/or overall, (3) a defined sequence kemal is a subset of sequences which is less that the maximum number of potential sequences if each residue position was allowed to be any of the allowable 20 conventional amino acids (and/or allowable unconventional amino/imino acids). A defined sequence kemal generally comprises variant and invariant residue positions and/or comprises variant residue positions which can comprise a residue selected from a defmed subset of amino acid residues), and the like, either segmentally or over the entire length of the individual selected library member sequence. Defined sequence kernals can refer to either amino acid sequences or polynucleotide sequences. For illustration and not limitation, the sequences (NNK)₁₀ (SEQ ID NO: 31) and (NNM)₁₀ (SEQ ID NO: 32), where N represents A, T, G, or C; K represents G or T; and M represents A or C, are defined sequence kemals.

As used herein, "linker" or "spacer" refers to a molecule or group of molecules that connects two molecules, such as a DNA binding protein and a random peptide, and serves to place the two molecules in a preferred configuration, *e.g.*, so that the random peptide can bind to a receptor with minimal steric hindrance from the DNA binding protein.

As used herein, the term "variable segment" refers to a portion of a nascent peptide which comprises a random, pseudorandom, or defined kemal sequence. A variable segment can comprise both variant and invariant residue positions, and the degree of residue variation at a variant residue position may be limited; both options are selected at the discretion of the practitioner. Typically, variable segments are about 5 to 20 amino acid residues in length (*e*.*g*., 8 to 10), although variable segments may be longer and may comprise antibody portions or receptor proteins, such as an antibody fragment, a nucleic acid binding protein, a receptor protein, and the like.

As used herein, the term "epitope" refers to that portion of an antigen or other macromolecule capable of forming a binding interaction that interacts with the variable region binding pocket of an antibody. Typically, such binding interaction is manifested as an intermolecular contact with one or more amino acid residues of a CDR.

As used herein, the term "receptor" refers to a molecule that has an affinity for a given ligand. Receptors can be naturally occurring or synthetic molecules. Receptors can be employed in an unaltered state or as aggregates with other species. Receptors can be attached, covalently or noncovalently, to a binding member, either directly or via a specific binding substance. Examples of receptors include, but are not limited to, antibodies, including monoclonal antibodies and antisera reactive with specific antigenic determinants (such as on viruses, cells, or other materials), cell membrane receptors, complex carbohydrates and glycoproteins, enzymes, and hormone receptors.

As used herein, the term "ligand" refers to a molecule, such as a random peptide or variable segment sequence, that is recognized by a particular receptor. As one of skill in the art will recognize, a molecule (or macromolecular complex) can be both a receptor and a ligand. In general, the binding partner having a smaller molecular weight is referred to as the ligand and the binding partner having a greater molecular weight is referred to as a receptor. As used herein, "fused" or "operably linked" is meant that the random peptide and the scaffold protein are linked together, in such a manner as to minimize the disruption to the stability of the scaffold structure.

As used herein, the term "single-chain antibody" refers to a polypeptide comprising a V_{H} domain and a V_{L} domain in polypeptide linkage, generally linked via a spacer peptide (*e*.*g*., [Gly-Gly-Gly-Gly-Ser]ₓ) (SEQ ID NO: 33), and which may comprise additional amino acid sequences at the amino- and/or carboxy-termini. For example, a single-chain antibody may comprise a tether segment for linking to the encoding polynucleotide. As an example, a scFv is a single-chain antibody. Single-chain antibodies are generally proteins consisting of one or more polypeptide segments of at least 10 contiguous amino acids substantially encoded by genes of the immunoglobulin superfamily (*e*.*g*., see The Immunoglobulin Gene Superfamily, A. F. Williams and A. N. Barclay, in Immunoglobulin Genes, T. Honjo, F. W. Alt, and T. H. Rabbitts, eds., (1989) Academic Press: San Diego, Calif., pp. 361-387, which is incorporated herein by reference), most frequently encoded by a rodent, non-human primate, avian, porcine, bovine, ovine, goat, or human heavy chain or light chain gene sequence. A functional single-chain antibody generally contains a sufficient portion of an immunoglobulin superfamily gene product so as to retain the property of binding to a specific target molecule, typically a receptor or antigen (epitope).

As used herein, the term "complementarity-determining region" and "CDR" refer to the art-recognized term as exemplified by the Kabat and Chothia CDR definitions also generally known as hypervariable regions or hypervariable loops (Chothia and Lesk (1987) J. Mol. Biol. 196: 901; Chothia et al. (1989) Nature 342: 877; E. A. Kabat et al., Sequences of Proteins of Immunological Interest (National Institutes of Health, Bethesda, Md.) (1987); and Tramontano et al. (1990) J. Mol. Biol. 215: 175). Variable region domains typically comprise the amino-terminal approximately 105-115 amino acids of a naturally-occurring immunoglobulin chain (*e*.*g*., amino acids 1-110), although variable domains somewhat shorter or longer are also suitable for forming single-chain antibodies.

An immunoglobulin light or heavy chain variable region consists of a "framework" region interrupted by three hypervariable regions, also called CDR's. The extent of the framework region and CDR's have been precisely defined (see, "Sequences of Proteins of Immunological Interest," E. Kabat et al., 4th Ed., U.S. Department of Health and Human Services, Bethesda, Md. (1987)). The sequences of the framework regions of different light or heavy chains are relatively conserved within a species. As used herein, a "human framework region" is a framework region that is substantially identical (about 85% or more, usually 90-95% or more) to the framework region of a naturally occurring human immunoglobulin. The framework region of an antibody, that is the combined framework regions of the constituent light and heavy chains, serves to position and align the CDR's. The CDR's are primarily responsible for binding to an epitope of an antigen.

### Transferrin and Transferrin Modifications

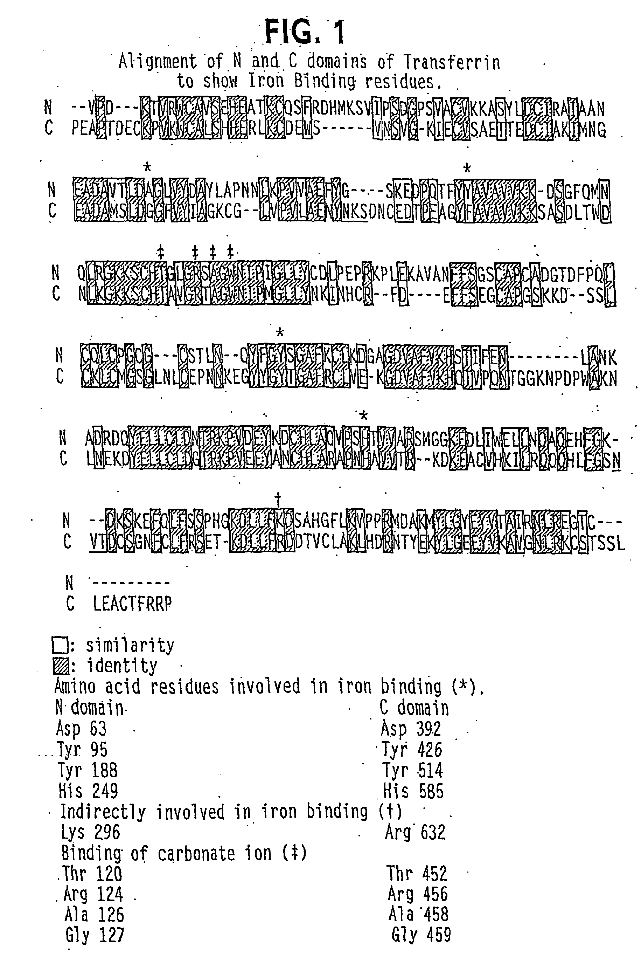

Any transferrin may be used to make modified Tf fusion proteins. As an example, wild-type human Tf (Tf) is a 679 amino acid protein, of approximately 75 KDa (not accounting for glycosylation), with two main domains, N (about 330 amino acids) and C (about 340 amino acids), which appear to originate from a gene duplication. See GenBank accession numbers NM001063, XM002793, M12530, XM039845, XM 039847 and S95936 (www.ncbi.nlm.nih.gov/) as well as SEQ ID NOS: 1, 2 and 3. The two domains have diverged over time but retain a large degree of identity/similarity (Fig. 1).

Each of the N and C domains is further divided into two subdomains, N1 and N2, C1 and C2. The function of Tf is to transport iron to the cells of the body. This process is mediated by the Tf receptor (TfR), which is expressed on all cells, particularly actively growing cells. TfR recognizes the iron bound form of Tf (two molecules of which are bound per receptor), endocytosis then occurs whereby the TfR/Tf complex is transported to the endosome, at which point the localized drop in pH results in release of bound iron and the recycling of the TfR/Tf complex to the cell surface and release of Tf (known as apoTf in its un-iron bound form). Receptor binding is through the C domain of Tf. The two glycosylation sites in the C domain do not appear to be involved in receptor binding as unglycosylated iron bound Tf does bind the receptor.

Each Tf molecule can carry two iron ions (Fe³⁺). These are complexed in the space between the N1 and N2, C1 and C2 sub domains resulting in a conformational change in the molecule. Tf crosses the blood brain barrier (BBB) via the Tf receptor.

In human transferrin, the iron binding sites comprise at least amino acids Asp 63 (Asp 82 of SEQ ID NO: 2 which includes the native Tf signal sequence), Asp 392 (Asp 411 of SEQ ID NO: 2), Tyr 95 (Tyr 114 of SEQ ID NO: 2), Tyr 426 (Tyr 445 of SEQ ID NO: 2), Tyr 188 (Tyr 207 of SEQ ID NO: 2), Tyr 514 or 517 (Tyr 533 or Tyr 536 SEQ ID NO: 2), His 249 (His 268 of SEQ ID NO: 2), and His 585 (His 604 of SEQ ID NO: 2) of SEQ ID NO: 3. The hinge regions comprise at least N domain amino acid residues 94-96, 245- 247 and/or 316-318 as well as C domain amino acid residues 425-427, 581-582 and/or 652-658 of SEQ ID NO: 3. The carbonate binding sites comprise at least amino acids Thr 120 (Thr 139 of SEQ ID NO: 2), Thr 452 (Thr 471 of SEQ ID NO: 2), Arg 124 (Arg 143 of SEQ ID NO: 2), Arg 456 (Arg 475 of SEQ ID NO: 2), Ala 126 (Ala 145 of SEQ ID NO: 2), Ala 458 (Ala 477 of SEQ ID NO: 2), Gly 127 (Gly 146 of SEQ ID NO: 2), and Gly 459 (Gly 478 of SEQ ID NO: 2) of SEQ ID NO: 3.

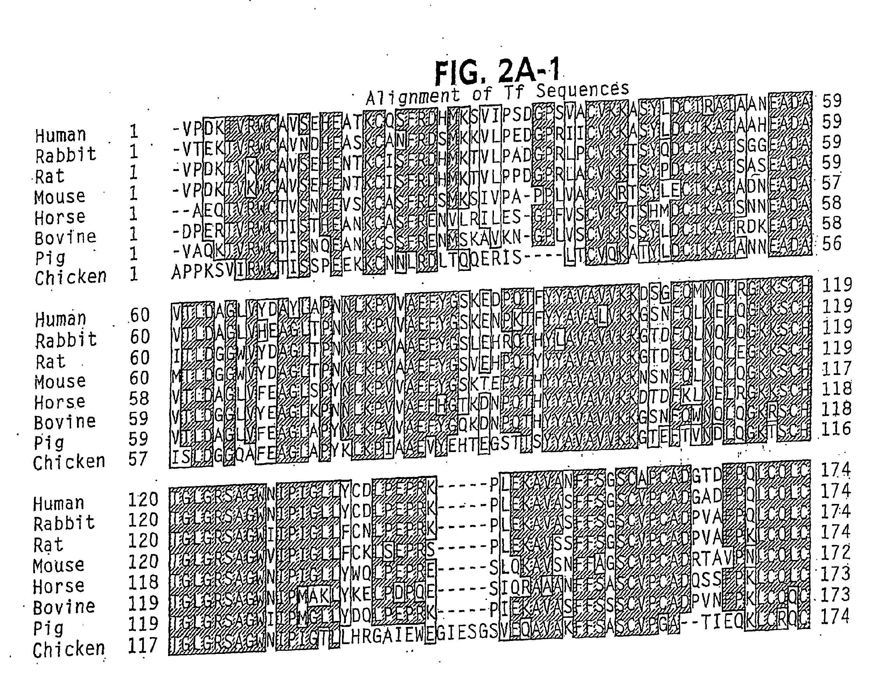

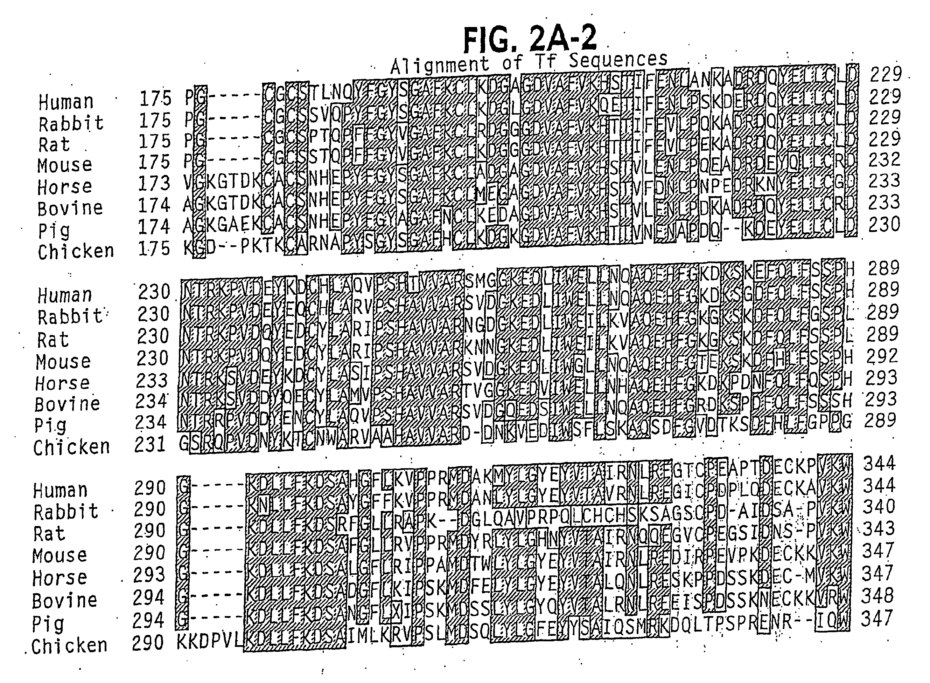

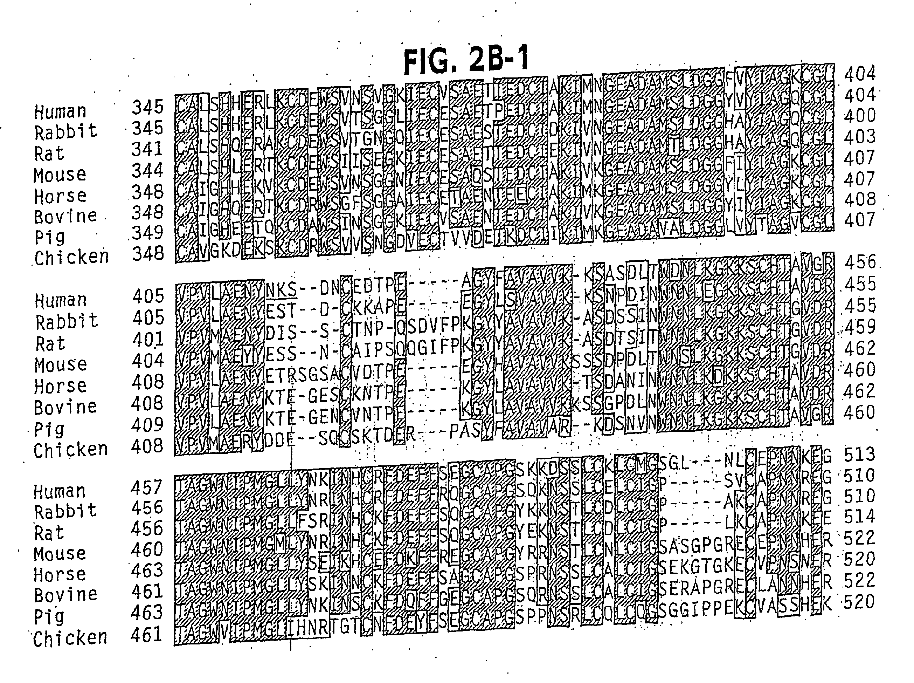

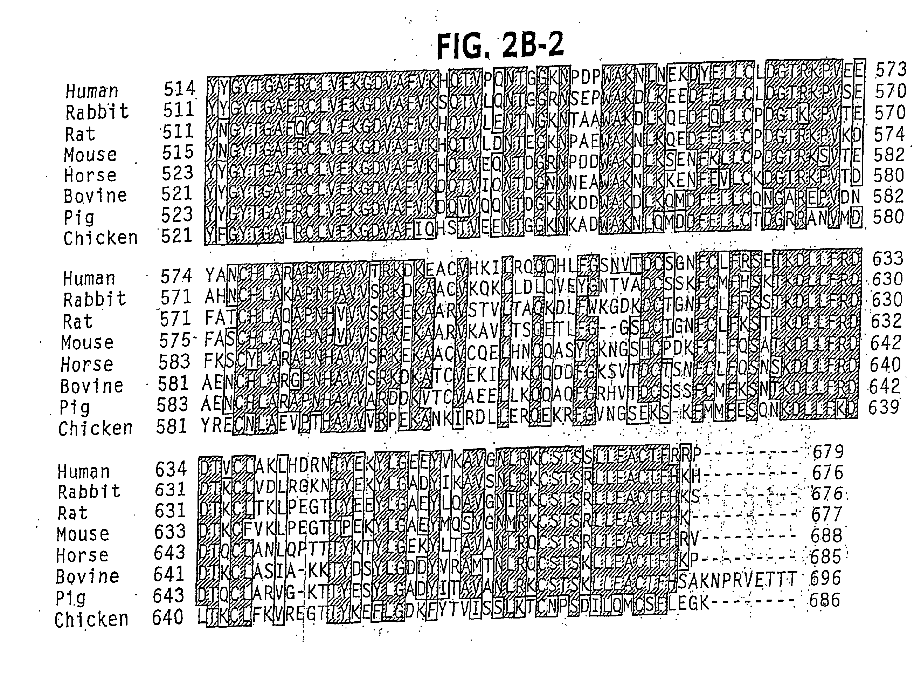

In one embodiment of the invention, the modified transferrin fusion protein includes a modified human transferrin, although any animal Tf molecule may be used to produce the fusion proteins of the invention, including human Tf variants, cow, pig, sheep, dog, rabbit, rat, mouse, hamster, echnida, platypus, chicken, frog, hornworm, monkey, as well as other bovine, canine and avian species (see Fig. 2 for a representative set of Tf sequences). All of these Tf sequences are readily available in GenBank and other public databases. The human Tf nucleotide sequence is available (see SEQ ID NOS: 1, 2 and 3 and the accession numbers described above and available at www.ncbi.nlm.nih.gov) and can be used to make genetic fusions between Tf or a domain of Tf and the therapeutic molecule of choice. Fusions may also be made from related molecules such as lacto transferrin (lactoferrin) GenBank Acc: NM_002343) or melanotransferrin (GenBank Acc. NM_013900, murine melanotransferrin).

Melanotransferrin is a glycosylated protein found at high levels in malignant melanoma cells and was originally named human melanoma antigen p97 (Brown et al., 1982, Nature, 296: 171-173). It possesses high sequence homology with human serum transferrin, human lactoferrin, and chicken transferrin (Brown et al., 1982, Nature, 296: 171-173; Rose et al., Proc. Natl. Acad. Sci. USA, 1986, 83: 1261-1265). However, unlike these receptors, no cellular receptor has been identified for melanotransferrin. Melanotransferrin reversibly binds iron and it exists in two forms, one of which is bound to cell membranes by a glycosyl phosphatidylinositol anchor while the other form is both soluble and actively secreted (Baker et al., 1992, FEBS Lett, 298: 215-218; Alemany et al., 1993, J. Cell Sci., 104: 1155-1162; Food et al., 1994, J. Biol. Chem. 274: 7011-7017).

Lactoferrin (Lf), a natural defense iron-binding protein, has been found to possess antibacterial, antimycotic, antiviral, antineoplastic and anti-inflammatory activity. The protein is present in exocrine secretions that are commonly exposed to normal flora: milk, tears, nasal exudate, saliva, bronchial mucus, gastrointestinal fluids, cervico-vaginal mucus and seminal fluid. Additionally, Lf is a major constituent of the secondary specific granules of circulating polymorphonuclear neutrophils (PMNs). The apoprotein is released on degranulation of the PMNs in septic areas. A principal function of Lf is that of scavenging free iron in fluids and inflamed areas so as to suppress free radical-mediated damage and decrease the availability of the metal to invading microbial and neoplastic cells. In a study that examined the turnover rate of ¹²⁵I Lf in adults, it was shown that Lf is rapidly taken up by the liver and spleen, and the radioactivity persisted for several weeks in the liver and spleen (Bennett et al. (1979), Clin. Sci. (Lond.) 57: 453-460).

In one embodiment, the transferrin portion of the transferrin fusion protein in the library of the invention includes a transferrin splice variant. In one example, a transferrin splice variant can be a splice variant of human transferrin. In one specific embodiment, the human transferrin splice variant can be that of Genbank Accession AAA61140.

In another embodiment, the transferrin portion of the transferrin fusion protein in the library of the invention includes a lactoferrin splice variant. In one example, a human serum lactoferrin splice variant can be a novel splice variant of a neutrophil lactoferrin. In one specific embodiment, the neutrophil lactoferrin splice variant can be that of Genbank Accession AAA59479. In another specific embodiment, the neutrophil lactoferrin splice variant can comprise the following amino acid sequence EDCIALKGEADA (SEQ ID NO: 8), which includes the novel region of splice-variance.

In another embodiment, the transferrin portion of the transferrin fusion protein in the library of the invention includes a melanotransferrin variant.

Modified Tf fusions may be made with any Tf protein, fragment, domain, or engineered domain. For instance, fusion proteins may be produced using the full-length Tf sequence, with or without the native Tf signal sequence. Tf fusion proteins may also be made using a single Tf domain, such as an individual N or C domain or a modified form of Tf comprising 2N or 2C domains (see U.S. Provisional Application 60/406,977, filed August 30, 2002). In some embodiments, fusions of a therapeutic protein to a single C domain may be produced, wherein the C domain is altered to reduce, inhibit or prevent glycosylation. In other embodiments, the use of a single N domain is advantageous as the Tf glycosylation sites reside in the C domain and the N domain, on its own. A preferred embodiment is the Tf fusion protein having a single N domain which is expressed at a high level.

As used herein, a C terminal domain or lobe modified to function as an N-like domain is modified to exhibit glycosylation patterns or iron binding properties substantially like that of a native or wild-type N domain or lobe. In a preferred embodiment, the C domain or lobe is modified so that it is not glycosylated and does not bind iron by substitution of the relevant C domain regions or amino acids to those present in the corresponding regions or sites of a native or wild-type N domain.

As used herein, a Tf moiety comprising "two N domains or lobes" includes a Tf molecule that is modified to replace the native C domain or lobe with a second native or wild-type N domain or lobe or a modified N domain or lobe or contains a C domain that has been modified to function substantially like a wild-type or modified N domain. See U.S. provisional application 60/406,977. Analysis of the two domains by overlay of the 3-dimensional structure of the two domains (Swiss PDB Viewer 3.7b2, Iterative Magic Fit) and by direct amino acid alignment (ClustalW multiple alignment) reveals that the two domains have diverged over time. Amino acid alignment shows 42% identity and 59% similarity between the two domains. However, approximately 80% of the N domain matches the C domain for structural equivalence. The C domain also has several extra disulfide bonds compared to the N domain.

Alignment of molecular models for the N and C domain reveals the following structural equivalents:

| | | | | | | | | | | | | | | |
|---|---|---|---|---|---|---|---|---|---|---|---|---|---|---|
| **N domain (1-330)** | 4-24 | 36-72 75-88 | 94-136 | 138-139 | 149-164 | 168-173 | 178-198 200-214 | 219-255 | 259-260 | 263-268 | 271-275 | 279-280 | 283-288 290-304 | 309-327 |
| **C domain (340-679)** | 340-361 | 365-415 | 425-437 439-468 | 470-471 | 475-490 | 492-497 | 507-542 | 555-591 | 593-594 | 597-602 | 605-609 | 614-615 | 620-640 | 645-663 |

The disulfide bonds for the two domains align as follows:

| **N** | **C** | | |
|---|---|---|---|
| | *C339-C596* | | |
| **C9-C48** | **C345-C377** | | |
| **C19-C39** | **C355-C368** | | **Bold** aligned disulfide bonds |
| | C402-C674 | | *Italics* bridging peptide |
| | C418-C637 | | |
| **C118-C194** | **C450-C523** | | |
| *C137-C331* | | | |
| | C474-C665 | | |
| **C158-C174** | **C484-C498** | | |
| C161-C179 | | | |
| **C171-C177** | **C495-C506** | | |
| **C227-C241** | **C563-C577** | | |
| | C615-C620 | | |

In one embodiment, the transferrin portion of the Tf fusion protein includes at least two N terminal lobes of transferrin. In further embodiments, the transferrin portion of the Tf fusion protein includes at least two N terminal lobes of transferrin derived from human serum transferrin.

In another embodiment, the transferrin portion of the fusion protein includes, comprises, or consists of at least two N terminal lobes of transferrin having a mutation in at least one amino acid residue selected from the group consisting of Asp63, Gly65, Tyr95, Tyr188, and His249 of SEQ ID NO: 3.

In another embodiment, the transferrin portion of the modified fusion protein includes a recombinant human serum transferrin N-terminal lobe mutant having a mutation at Lys206 or His207 of SEQ ID NO: 3.

In another embodiment, the transferrin portion of the fusion protein includes, comprises, or consists of at least two C terminal lobes of transferrin. In further embodiments, the transferrin portion of the fusion protein includes at least two C terminal lobes of transferrin derived from human serum transferrin.

In a further embodiment, the C terminal lobe mutant further includes a mutation of at least one of Asn413 and Asn611 of SEQ ID NO: 3 which does not allow glycosylation. In another embodiment, the transferrin portion of the fusion protein includes at least two C terminal lobes of transferrin having a mutation in at least one amino acid residue selected from the group consisting of Asp392, Tyr426, Tyr514, Tyr517 and His585 of SEQ ID NO: 3, wherein the mutant retains the ability to bind metal ions. In an alternate embodiment, the transferrin portion of the fusion protein includes at least two C terminal lobes of transferrin having a mutation in at least one amino acid residue selected from the group consisting of Tyr426, Tyr514, Tyr517 and His585 of SEQ ID NO: 3, wherein the mutant has a reduced ability to bind metal ions. In another embodiment, the transferrin portion of the fusion protein includes at least two C terminal lobes of transferrin having a mutation in at least one amino acid residue selected from the group consisting of Asp392, Tyr426, Tyr517 and His585 of SEQ ID NO:3, wherein the mutant does not retain the ability to bind metal ions and functions substantially like an N domain.

In some embodiments, the Tf or Tf portion will be of sufficient length to increase the *in vivo* circulatory half-life, serum stability, *in vitro* solution stability or bioavailability of the antibody variable region compared to the *in vivo* circulatory half-life, serum stability (half-life), *in vitro* stability or bioavailability of antibody variable region in an unfused state. Such an increase in stability, *in vivo* circulatory half-life or bioavailability may be about a 30%, 50%, 70%, 80%, 90% or more increase over the unfused antibody variable region. In some cases, the trans-bodies comprising modified transferrin exhibit a serum half-life of about 10-20 or more days, about 12-18 days or about 14-17 days.

When the C domain of Tf is part of the fusion protein, the two N-linked glycosylation sites, amino acid residues corresponding to N413 and N611 of SEQ ID NO: 3 may be mutated for expression in a yeast system to prevent glycosylation or hypermannosylationn and extend the *in vivo* circulatory half-life of the fusion protein and/or therapeutic protein ( to produce asialo-, or in some instances, monosialo-Tf or disialo-Tf). In addition to Tf amino acids corresponding to N413 and N611, mutations may be to the adjacent residues within the N-X-S/T glycosylation site to prevent or substantially reduce glycosylation. See U.S. Patent 5,986,067 of Funk et al. It has also been reported that the N domain of Tf expressed in *Pichia pastoris* becomes O-linked glycosylated with a single hexose at S32 which also may be mutated or modified to prevent such glycosylation. Moreover, O-linked glycosylation may be reduced or eliminated in a yeast host cell with mutations in the PMT genes.

Accordingly, in one embodiment of the invention, the transferrin fusion protein includes a modified transferrin molecule wherein the transferrin exhibits reduced glycosylation, including but not limited to asialo- monosialo- and disialo- forms of Tf. In another embodiment, the transferrin portion of the transferrin fusion protein includes a recombinant transferrin mutant that is mutated to prevent glycosylation. In another embodiment, the transferrin portion of the transferrin fusion protein includes a recombinant transferrin mutant that is fully glycosylated. In a further embodiment, the transferrin portion of the transferrin fusion protein includes a recombinant human serum transferrin mutant that is mutated to prevent glycosylation, wherein at least one of Asn413 and Asn611 of SEQ ID NO:3 are mutated to an amino acid which does not allow glycosylation. In another embodiment, the transferrin portion of the transferrin fusion protein includes a recombinant human serum transferrin mutant that is mutated to prevent or substantially reduce glycosylation, wherein mutations may be to the adjacent residues within the N-X-S/T glycosylation site. Moreover, glycosylation may be reduced or prevented by mutating the serine or threonine residue. Further, changing the X to proline is known to inhibit glycosylation.

As discussed below in more detail, modified Tf fusion proteins may also be engineered to not bind iron and/or not bind the Tf receptor. When the iron binding is retained the iron binding ability of Tf may be used in two ways, one to deliver a therapeutic protein or peptide(s) to the inside of a cell and/or across the BBB. These embodiments that bind iron and/or the Tf receptor will often be engineered to reduce or prevent glycosylation to extend the serum half-life of the therapeutic protein. The N domain alone will not bind to TfR when loaded with iron, and the iron bound C domain will bind TfR but not with the same affinity as the whole molecule.

In libraries of the invention, the Tf peptide has a reduced affinity for a transferrin receptor.

In another embodiment, the transferrin portion of the transferrin fusion protein includes a recombinant transferrin mutant having a mutation wherein the mutant does not retain the ability to bind metal ions. In an alternate embodiment, the transferrin portion of the transferrin fusion protein includes a recombinant transferrin mutant having a mutation wherein the mutant has a weaker binding avidity for metal ions than wild-type serum transferrin. In an alternate embodiment, the transferrin portion of the transferrin fusion protein includes a recombinant transferrin mutant having a mutation wherein the mutant has a stronger binding avidity for metal ions than wild-type serum transferrin.

In another embodiment, the transferrin portion of the transferrin fusion protein includes a recombinant transferrin mutant having a mutation wherein the mutant does not retain the ability to bind to the transferrin receptor. In an alternate embodiment, the transferrin portion of the transferrin fusion protein includes a recombinant transferrin mutant having a mutation wherein the mutant has a weaker binding avidity for the transferrin receptor than wild-type serum transferrin.

In another embodiment, the transferrin portion of the transferrin fusion protein includes a recombinant transferrin mutant having a mutation wherein the mutant does not retain the ability to bind to carbonate ions. In an alternate embodiment, the transferrin portion of the transferrin fusion protein includes a recombinant transferrin mutant having a mutation wherein the mutant has a weaker binding avidity for carbonate ions than wild-type serum transferrin. In an alternate embodiment, the transferrin portion of the transferrin fusion protein includes a recombinant transferrin mutant having a mutation wherein the mutant has a stronger binding avidity for carbonate ions than wild-type serum transferrin.

In another embodiment, the transferrin portion of the transferrin fusion protein includes a recombinant human serum transferrin mutant having a mutation in at least one amino acid residue selected from the group consisting of Asp63, Gly65, Tyr95, Tyr188, His249, Asp392, Tyr426, Tyr514, Tyr517 and Hits585 of SEQ ID NO:3, wherein the mutant retains the ability to bind metal ions. In an alternate embodiment, a recombinant human serum transferrin mutant having a mutation in at least one amino acid residue selected from the group consisting of Asp63, Gly65, Tyr95, Tyr188, His249, Asp392, Tyr426, Tyr514, Tyr517 and His585 of SEQ ID NO:3, wherein the mutant has a reduced ability to bind metal ions. In another embodiment, a recombinant human serum transferrin mutant having a mutation in at least one amino acid residue selected from the group consisting of Asp63, Gly65, Tyr95, Tyr188, His249, Asp392, Tyr426, Tyr517 and His585 of SEQ ID NO:3, wherein the mutant does not retain the ability to bind metal ions.

In another embodiment, the transferrin portion of the transferrin fusion protein includes a recombinant human serum transferrin mutant having a mutation at Lys206 or His207 of SEQ ID NO:3, wherein the mutant has a stronger binding avidity for metal ions than wild-type human serum transferrin (see U.S. Patent 5,986,067, which is herein incorporated by reference in its entirety). In an alternate embodiment, the transferrin portion of the transferrin fusion protein includes a recombinant human serum transferrin mutant having a mutation at Lys206 or His207 of SEQ ID NO:3, wherein the mutant has a weaker binding avidity for metal ions than wild-type human serum transferrin. In a further embodiment, the transferrin portion of the transferrin fusion protein includes a recombinant human serum transferrin mutant having a mutation at Lys206 or His207 of SEQ ID NO:3, wherein the mutant does not bind metal ions.

Any available technique may be used to make the fusion proteins of the invention, including but not limited to molecular techniques commonly available, for instance, those disclosed in Sambrook et al. Molecular Cloning: A Laboratory Manual, 2nd Ed., Cold Spring Harbor Laboratory Press, 1989. When carrying out nucleotide substitutions using techniques for accomplishing site-specific mutagenesis that are well known in the art, the encoded amino acid changes are preferably of a minor nature, that is, conservative amino acid substitutions, although other, non-conservative, substitutions are contemplated as well, particularly when producing a modified transferrin portion of a Tf fusion protein, *e.g.*, a modified Tf fusion protein exhibiting reduced glycosylation, reduced iron binding and the like. Specifically contemplated are amino acid substitutions, small deletions or insertions, typically of one to about 30 amino acids; insertions between transferrin domains; small amino- or carboxyl-terminal extensions, such as an amino-terminal methionine residue, or small linker peptides of less than 50, 40, 30, 20 or 10 residues between transferrin domains or linking a transferrin protein and a therapeutic protein or peptide; or a small extension that facilitates purification, such as a poly-histidine tract, an antigenic epitope or a binding domain.

Examples of conservative amino acid substitutions are substitutions made within the same group such as within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, valine), aromatic amino acids (such as phenylalanine, tryptophan, tyrosine) and small amino acids (such as glycine, alanine, serine, threonine, methionine).

Non-conservative substitutions encompass substitutions of amino acids in one group by amino acids in another group. For example, a non-conservative substitution would include the substitution of a polar amino acid for a hydrophobic amino acid. For a general description of nucleotide substitution, see *e*.*g*. Ford et al. (1991), Prot. Exp. Pur. 2: 95-107. Non-conservative substitutions, deletions and insertions are particularly useful to produce TF fusion proteins of the invention that exhibit no or reduced binding of iron, no or reduced binding of the fusion protein to the Tf receptor.

In the polypeptide and proteins of the invention, the following system is followed for designating amino acids in accordance with the following conventional list:

**TABLE OF AMINO ACIDS**

| **AMINO ACID** | **ONE-LETTER SYMBOL** | **THREE-LETTER SYMBOL** |
|---|---|---|
| Alanine | A | Ala |
| Arginine | R | Arg |
| Asparagine | N | Asn |
| Aspartic Acid | D | Asp |
| Cysteine | C | Cys |
| Glutamine | Q | Gln |
| Glutamic Acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

Iron binding and/or receptor binding may be reduced or disrupted by mutation, including deletion, substitution or insertion into, amino acid residues corresponding to one or more of Tf N domain residues Asp63, Tyr95, Tyr188, His249 and/or C domain residues Asp 392, Tyr 426, Tyr 514 and/or His 585 of SEQ ID NO: 3. Iron binding may also be affected by mutation to amino acids Lys206, His207 or Arg632 of SEQ ID NO: 3. Carbonate binding may be reduced or disrupted by mutation, including deletion, substitution or insertion into, amino acid residues corresponding to one or more of Tf N domain residues Thr120, Arg124, Ala126, Gly 127 and/or C domain residues Thr 452, Arg 456, Ala 458 and/or Gly 459 of SEQ ID NO: 3. A reduction or disruption of carbonate binding may adversely affect iron and/or receptor binding.

Binding to the Tf receptor may be reduced or disrupted by mutation, including deletion, substitution or insertion into, amino acid residues corresponding to one or more of TfN domain residues described above for iron binding.

As discussed above, glycosylation may be reduced or prevented by mutation, including deletion, substitution or insertion into, amino acid residues corresponding to one or more of Tf C domain residues around the N-X-S/T sites corresponding to C domain residues N413 and/or N611 (See U.S. Patent No. 5,986,067). For instance, the N413 and/or N611 may be mutated to Glu residues.

In instances where the Tf fusion proteins of the invention are not modified to prevent glycosylation, iron binding, carbonate binding and/or receptor binding, glycosylation, iron and/or carbonate ions may be stripped from or cleaved off of the fusion protein. For instance, available deglycosylases may be used to cleave glycosylation residues from the fusion protein, in particular the sugar residues attached to the Tf portion, yeast deficient in glycosylation enzymes may be used to prevent glycosylation and/or recombinant cells may be grown in the presence of an agent that prevents glycosylation, *e.g.*, tunicamycin.

The carbohydrates on the fusion protein may also be reduced or completely removed enzymatically by treating the fusion protein with deglycosylases. Deglycosylases are well known in the art. Examples of deglycosylases include but are not limited to galactosidase, PNGase A, PNGase F, glucosidase, mannosidase, fucosidase, and Endo H deglycosylase.

Additional mutations may be made with Tf to alter the three dimensional structure of Tf, such as modifications to the hinge region to prevent the conformational change needed for iron biding and Tf receptor recognition. For instance, mutations may be made in or around N domain amino acid residues 94-96, 245-247 and/or 316-318 as well as C domain amino acid residues 425-427, 581-582 and/or 652-658. In addition, mutations may be made in or around the flanking regions of these sites to alter Tf structure and function.

In one aspect of the invention, the transferrin fusion protein can function as a carrier protein to extend the half life or bioavailability of the therapeutic protein as well as, in some instances, delivering the therapeutic protein inside a cell and/or across the blood brain barrier. In an alternate embodiment, the transferrin fusion protein includes a modified transferrin molecule wherein the transferrin does not retain the ability to cross the blood brain barrier.

In another embodiment, the transferrin fusion protein includes a modified transferrin molecule wherein the transferrin molecule retains the ability to bind to the transferrin receptor and transport the therapeutic peptide inside cells. In an alternate embodiment, the transferrin fusion protein includes a modified transferrin molecule wherein the transferrin molecule does not retain the ability to bind to the transferrin receptor and transport the therapeutic peptide inside cells.

In further embodiments, the transferrin fusion protein includes a modified transferrin molecule wherein the transferrin molecule retains the ability to bind to the transferrin receptor and transport the therapeutic peptide inside cells and retains the ability to cross the blood brain barrier. In an alternate embodiment, the transferrin fusion protein includes a modified transferrin molecule wherein the transferrin molecule retains the ability to cross the blood brain barrier, but does not retain the ability to bind to the transferrin receptor and transport the therapeutic peptide inside cells.

### Modified Transferrin Fusion Proteins

The fusion proteins in the libraries of the invention may contain one or more copies of the therapeutic protein or polypeptide attached to the N-terminus and/or the C-terminus of the Tf protein. In some embodiments, the therapeutic protein or polypeptide is attached to both the N- and C-terminus of the Tf protein and the fusion protein may contain one or more equivalents of the therapeutic protein or polypeptide on either or both ends of Tf. In other embodiments, the therapeutic protein or polypeptide is inserted into known domains of the Tf protein, for instance, into one or more of the loops of Tf (see Ali et al. (1999) J. Biol. Chem. 274(34):24066-24073). In other embodiments, the therapeutic protein or therapeutic peptide is inserted between the N and C domains of Tf.

Generally, the transferrin fusion protein may have one modified transferrin-derived region and one therapeutic protein-derived region. Multiple regions of each protein, however, may be used to make a transferrin fusion protein Similarly, more than one therapeutic protein may be used to make a transferrin fusion protein thereby producing a multi-functional modified Tf fusion protein.

The present invention provides phage libraries containing transferrin fusion protein containing a protein or polypeptide or portion thereof fused to a transferrin molecule or portion thereof, having reduced affinity for a transferrin receptor. In one embodiment, the transferrin fusion protein of the invention contains a therapeutic protein or polypeptide fused to the N terminus of a transferrin molecule. In an alternate embodiment, the transferrin fusion protein contains a therapeutic protein fused to the C terminus of a transferrin molecule. In another embodiments, the transferrin fusion protein may contain a therapeutic protein or polypeptide or portion thereof fused to a modified transferrin molecule or portion thererof.

In other embodiments, the transferrin fusion protein contains a therapeutic protein fused to both the N-terminus and the C-terminus of modified transferrin. In another embodiment, the therapeutic proteins fused at the N- and C- termini are the same therapeutic proteins. In an alternate embodiment, the therapeutic proteins fused at the N-and C- termini are different therapeutic proteins. In another alternate embodiment, the therapeutic proteins fused to the N- and C- termini are different therapeutic proteins which may be used to treat or prevent the same disease, disorder, or condition. In another embodiment, the therapeutic proteins fused at the N- and C- termini are different therapeutic proteins which may be used to treat or prevent diseases or disorders which are known in the art to commonly occur in patients simultaneously.

In addition to modified transferrin fusion protein in which the modified transferrin portion is fused to the N terminal and/or C-terminal of the therapeutic protein portion, transferrin fusion protein may also be produced by inserting the therapeutic protein or peptide of interest (*e*.*g*., a therapeutic protein or peptide as disclosed herein, or, for instance, a single chain antibody that binds a therapeutic protein or a fragment or variant thereof) into an internal region of the modified transferrin. Internal regions of modified transferrin include, but are not limited to, the iron binding sites, the hinge regions, the bicarbonate binding sites, or the receptor binding domain.

Within the protein sequence of the modified transferrin molecule a number of loops or turns exist, which are stabilized by disulfide bonds. These loops are useful for the insertion, or internal fusion, of therapeutically active peptides, particularly those requiring a secondary structure to be functional, or therapeutic proteins to generate a modified transferrin molecule with specific biological activity.

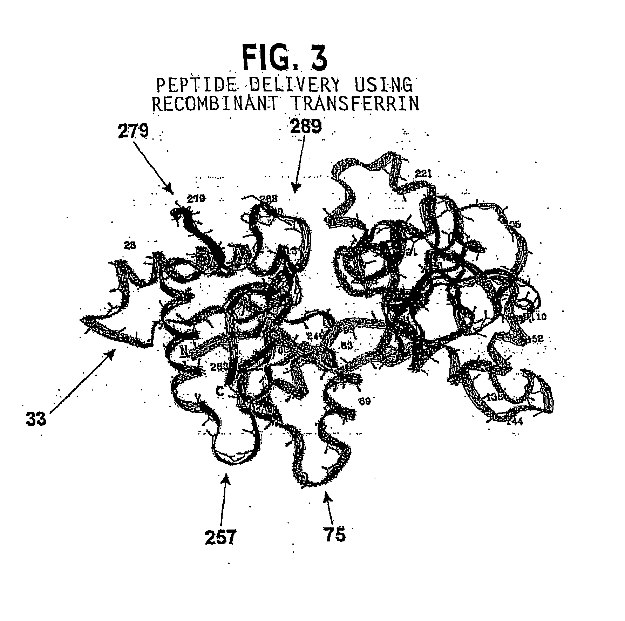

When therapeutic proteins or peptides are inserted into or replace at least one loop of a Tf molecule, insertions may be made within any of the surface exposed loop regions, in addition to other areas of Tf. For instance, insertions may be made within the loops comprising Tf amino acids 32-33, 74-75, 256-257, 279-280 and 288-289 (Ali *et al*., *supra*) (See Fig. 3). As previously described, insertions may also be made within other regions of Tf such as the sites for iron and bicarbonate binding, hinge regions, and the receptor binding domain as described in more detail below. The loops in the Tf protein sequence that are amenable to modification/replacement for the insertion of proteins or peptides may also be used for the development of a screenable library of random peptide inserts. Any procedures may be used to produce nucleic acid inserts for the generation of peptide libraries, including available phage and bacterial display systems, prior to cloning into a Tf domain and/or fusion to the ends of Tf. In other embodiments, the library is made directly in or on the ends of a Tf peptide as described below.

The N-terminus of Tf is free and points away from the body of the molecule. Fusions of proteins or peptides on the N-tenninus may therefore be a preferred embodiment. Such fusions may include a linker region, such as but not limited to a poly-glycine stretch, to separate the therapeutic protein or peptide from Tf. Attention to the junction between the leader sequence, the choice of leader sequence, and the structure of the mRNA by codon manipulation/optimization (no major stem loops to inhibit ribosome progress) will increase secretion and can be readily accomplished using standard recombinant protein techniques.

The C-terminus of Tf appears to be more buried and secured by a disulfide bond 6 amino acids from the C-terminus. In human Tf, the C-terminal amino acid is a proline which, depending on the way that it is orientated, will either point a fusion away or into the body of the molecule. A linker or spacer moiety at the C-terminus may be used in some embodiments of the invention. There is also a proline near the N-terminus. In one aspect of the invention, the proline at the N- and/or the C- termini may be changed out. In another aspect of the invention, the C-terminal disulfide bond may be eliminated to untether the C-terminus.

In yet other embodiments, small molecule therapeutics may be complexed with iron and loaded on a modified Tf protein fusion for delivery to the inside of cells and across the BBB. The addition of a targeting peptide or, for example, a single chain antibody (SCA) can be used to target the payload to a particular cell type, *e*.*g*., a cancer cell.

### Nucleic Acids

Libraries of Nucleic acid molecules are also provided by the present invention. These encode a Tf fusion protein comprising a transferrin protein or a portion of a transferrin protein having reduced affinity for a transferrin receptor covalently linked or joined to a at least one second peptide. As discussed in more detail below, any therapeutic protein may be used. The fusion protein may further comprise a linker region, for instance a linker less than about 50, 40, 30, 20, or 10 amino acid residues. The linker can be covalently linked to and between the transferrin protein or portion thereof and the therapeutic protein. Nucleic acid molecules of the invention may be purified or not.

Host cells and vectors for replicating the nucleic acid molecules and for expressing the encoded fusion proteins are also provided. Any vectors or host cells may be used, whether prokaryotic or eukaryotic, but eukaryotic expression systems, in particular yeast expression systems, may be preferred. Many vectors and host cells are known in the art for such purposes. It is well within the skill of the art to select an appropriate set for the desired application.

DNA sequences encoding transferrin, portions of transferrin and therapeutic proteins of interest may be cloned from a variety of genomic or cDNA libraries known in the art. The techniques for isolating such DNA sequences using probe-based methods are conventional techniques and are well known to those skilled in the art. Probes for isolating such DNA sequences may be based on published DNA or protein sequences (see, for example, Baldwin, G.S. (1993) Comparison of Transferrin Sequences from Different Species. Comp. Biochem. Physiol. 106B/1:203-218 and all references cited therein). Alternatively, the polymerase chain reaction (PCR) method disclosed by Mullis et al. (U.S. Pat. No. 4,683,195) and Mullis (U.S. Pat. No. 4,683,202) may be used. The choice of library and selection of probes for the isolation of such DNA sequences is within the level of ordinary skill in the art.

As known in the art, "similarity" between two polynucleotides or polypeptides is determined by comparing the nucleotide or amino acid sequence and its conserved nucleotide or amino acid substitutes of one polynucleotide or polypeptide to the sequence of a second polynucleotide or polypeptide. Also known in the art is "identity" which means the degree of sequence relatedness between two polypeptide or two polynucleotide sequences as determined by the identity of the match between two strings of such sequences. Both identity and similarity can be readily calculated (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991).

While there exist a number of methods to measure identity and similarity between two polynucleotide or polypeptide sequences, the terms "identity" and "similarity" are well known to skilled artisans (Sequence Analysis in Molecular Biology, von Heinje, G., Academic Press, 1987; Sequence Analysis Primer, Gribskov, M. and Devereux, J., eds., M Stockton Press, New York, 1991; and Carillo, H., and Lipman, D., SIAM J. Applied Math., 48: 1073 (1988). Methods commonly employed to determine identity or similarity between two sequences include, but are not limited to those disclosed in Guide to Huge Computers, Martin J. Bishop, ed., Academic Press, San Diego, 1994, and Carillo, H., and Lipman, D., SIAM J. Applied Math. 48:1073 (1988).

Preferred methods to determine identity are designed to give the largest match between the two sequences tested. Methods to determine identity and similarity are codified in computer programs. Preferred computer program methods to determine identity and similarity between two sequences include, but are not limited to, GCG program package (Devereux, et al., Nucl. Acid Res. 12(1):387 (1984)), BLASTP, BLASTN, FASTA (Atschul, et al., J. Mol. Biol. 215:403 (1990)). The degree of similarity or identity referred to above is determined as the degree of identity between the two sequences, often indicating a derivation of the first sequence from the second. The degree of identity between two nucleic acid sequences may be determined by means of computer programs known in the art such as GAP provided in the GCG program package (Needleman and Wunsch J. Mol. Biol. 48:443-453 (1970)). For purposes of determining the degree of identity between two nucleic acid sequences for the present invention, GAP is used with the following settings: GAP creation penalty of 5.0 and GAP extension penalty of 0.3.

### Codon Optimization

The degeneracy of the genetic code permits variations of the nucleotide sequence of a transferrin protein and/or therapeutic protein of interest, while still producing a polypeptide having the identical amino acid sequence as the polypeptide encoded by the native DNA sequence. The procedure, known as "codon optimization" (described in U.S. Patent 5,547,871) provides one with a means of designing such an altered DNA sequence. The design of codon optimized genes should take into account a variety of factors, including the frequency of codon usage in an organism, nearest neighbor frequencies, RNA stability, the potential for secondary structure formation, the route of synthesis and the intended future DNA manipulations of that gene. In particular, available methods may be used to alter the codons encoding a given fusion protein with those most readily recognized by yeast when yeast expression systems are used.

The degeneracy of the genetic code permits the same amino acid sequence to be encoded and translated in many different ways. For example, leucine, serine and arginine are each encoded by six different codons, while valine, proline, threonine, alanine and glycine are each encoded by four different codons. However, the frequency of use of such synonymous codons varies from genome to genome among eukaryotes and prokaryotes. For example, synonymous codon-choice patterns among mammals are very similar, while evolutionarily distant organisms such as yeast (such as *S. cerevisiae*), bacteria (such as *E. coil*) and insects (such as *D. melanogaster*) reveal a clearly different pattern of genomic codon use frequencies (Grantham, R, et al., Nucl. Acid Res., 8, 49-62 (1980); Grantham, R., et al., Nucl. Acid Res., 9, 43-74 (1981); Maroyarna, T., et al., Nucl. Acid Res., 14, 151-197 (1986); Aota, S., et al., Nucl. Acid Res., 16, 315-402 (1988); Wada, K., et al., Nucl. Acid Res., 19 Supp., 1981-1985 (1991); Kurland, C. G., FEBS Lett., 285, 165-169 (1991)). These differences in codon-choice patterns appear to contribute to the overall expression levels of individual genes by modulating peptide elongation rates. (Kurland, C. G., FEBS Lett., 285, 165-169 (1991); Pedersen, S., EMBO J., 3, 2895-2898 (1984); Sorensen, M. A., J. Mol. Biol., 207, 365-377 (1989); Randall, L. L., et al., Eur. J. Biochem., 107, 375-379 (1980); Curran, J. F., and Yarus, M., J. Mol. Biol., 209, 65-77 (1989); Varenne, S., et al., J. Mol. Biol., 180, 549-576 (1984), Varenne, S., et al., J. Mol, Biol., 180, 549-576 (1984); Garel, J.-P., J. Theor. Biol., 43, 211-225 (1974); Ikemura, T., J. Mol. Biol., 146, 1-21 (1981); Ikemura, T., J. Mol. Biol., 151, 389-409 (1981)).

The preferred codon usage frequencies for a synthetic gene should reflect the codon usages of nuclear genes derived from the exact (or as closely related as possible) genome of the cell/organism that is intended to be used for recombinant protein expression, particularly that of yeast species. As discussed above, in one preferred embodiment the human Tf sequence is codon optimized, before or after modification as herein described for yeast expression as may be the therapeutic protein nucleotide sequence(s).

### Vectors

Expression units for use in the present invention will generally comprise the following elements, operably linked in a 5' to 3' orientation: a transcriptional promoter, a secretory signal sequence, a DNA sequence encoding a modified Tf fusion protein comprising transferrin protein or a portion of a transferrin protein joined to a DNA sequence encoding a therapeutic protein or peptide of interest and a transcriptional terminator. As discussed above, any arrangement of the therapeutic protein or peptide fused to or within the Tf portion may be used in the vectors of the invention. The selection of suitable promoters, signal sequences and terminators will be determined by the selected host cell and will be evident to one skilled in the art and are discussed more specifically below.

Suitable yeast vectors for use in the present invention are described in U.S. Patent 6,291,212 and include YRp7 (Struhl et al., Proc. Natl. Acad. Sci. USA 76: 1035-1039, 1978), YEp13 (Broach et al., Gene 8: 121-133, 1979), pJDB249 and pJDB219 (Beggs, Nature 275:104-108, 1978), pPPC0005, pSeCHSA, pScNHSA, pC4 and derivatives thereof. Useful yeast plasmid vectors also include pRS403-406, pRS413-416 and the *Pichia* vectors available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3.* PlasmidspRS413∼41.6 are Yeast Centromere plasmids (YCps).

Such vectors will generally include a selectable marker, which may be one of any number of genes that exhibit a dominant phenotype for which a phenotypic assay exists to enable transformants to be selected. Preferred selectable markers are those that complement host cell auxotrophy, provide antibiotic resistance or enable a cell to utilize specific carbon sources, and include *LEU2* (Broach *et al.* ibid.), URA3 (Botstein et al., Gene 8: 17, 1979), *HIS3* (Struhl *et al*., ibid.) or POT1 (Kawasaki and Bell, EP 171,142). Other suitable selectable markers include the CAT gene, which confers chloramphenicol resistance on yeast cells. Preferred promoters for use in yeast include promoters from yeast glycolytic genes (Hitzeman et al., J Biol. Chem. 225: 12073-12080, 1980; Alber and Kawasaki, J. Mol. Appl. Genet. 1: 419-434, 1982; Kawasaki, U.S. Pat. No. 4,599,311) or alcohol dehydrogenase genes (Young et al., in Genetic Engineering of Microorganisms for Chemicals, Hollaender et al., (eds.), p. 355, Plenum, N.Y., 1982; Ammerer, Meth. Enzymol. 101: 192-201, 1983). In this regard, particularly preferred promoters are the TPI1 promoter (Kawasaki, U.S. Pat. No. 4,599,311) and the ADH2-4^{C} (see U.S. Patent 6,291,212 promoter (Russell et al., Nature 304: 652-654, 1983). The expression units may also include a transcriptional terminator. A preferred transcriptional terminator is the *TPI1* terminator (Alber and Kawasaki, ibid.). Other preferred vectors and preferred components such as promoters and terminators of a yeast expression system are disclosed in European Patents EP 0258067, EP 0286424, EP0317254, EP 0387319, EP 0386222, EP 0424117, EP 0431880, and EP 1002095; European Patent Publications EP 0828759, EP 0764209, EP 0749478, and EP 0889949; PCT Publication WO 00/44772 and WO 94/04687; and U.S. Patents 5,739,007; 5,637,504; 5,302,697; 5,260,202; 5,667,986; 5,728,553; 5,783,423; 5,965,386; 6150,133; 6,379,924; and 5,714,377.

In addition to yeast, modified fusion proteins can be expressed in filamentous fungi, for example, strains of the fungi *Aspergillus.* Examples of useful promoters include those derived from *Aspergillus nidulans* glycolytic genes, such as the *adh3* promoter (McKnight et al., EMBO J. 4: 2093-2099, 1985) and the tpiA promoter. An example of a suitable terminator is the *adh3* terminator (McKnight *et al*., ibid.). The expression units utilizing such components may be cloned into vectors that are capable of insertion into the chromosomal DNA of *Aspergillus*, for example.

Mammalian expression vectors will include a promoter capable of directing the transcription of the modified Tf fusion protein. Preferred promoters include viral promoters and cellular promoters. Preferred viral promoters include the major late promoter from adenovirus 2 (Kaufman and Sharp, Mol. Cell. Biol. 2: 1304-13199, 1982) and the SV40 promoter (Subramani et al., Mol. Cell. Biol. 1: 854-864, 1981). Preferred cellular promoters include the mouse metallothionein 1 promoter (Palmiter et al., Science 222: 809-814, 1983) and a mouse Vκ (see U.S. Patent 6,291,212) promoter (Grant et al., Nuc. Acids Res. 15: 5496, 1987). A particularly preferred promoter is a mouse V_{H} (see U.S. Patent 6,291,212) promoter (Loh *et al*., ibid.). Such expression vectors may also contain a set of RNA splice sites located downstream from the promoter and upstream from the DNA sequence encoding the transferrin fusion protein. Preferred RNA splice sites may be obtained from adenovirus and/or immunoglobulin genes.

Also contained in the expression vectors is a polyadenylation signal located downstream of the coding sequence of interest. Polyadenylation signals include the early or late polyadenylation signals from SV40 (Kaufman and Sharp, ibid.), the polyadenylation signal from the adenovirus 5 E1B region and the human growth hormone gene terminator (DeNoto et al., Nucl. Acid Res. 9: 3719-3730, 1981). A particularly preferred polyadenylation signal is the V_{H} (see U.S. Patent 6,291,212) gene terminator (Loh *et al*., ibid.). The expression vectors may include a noncoding viral leader sequence, such as the adenovirus 2 tripartite leader, located between the promoter and the RNA splice sites. Preferred vectors may also include enhancer sequences, such as the SV40 enhancer and the mouse µ (see U.S. Patent 6,291,212) enhancer (Gillies, Cell 33: 717-728, 1983). Expression vectors may also include sequences encoding the adenovirus VA RNAs.

### Transformation

Techniques for transforming fungi are well known in the literature, and have been described, for instance, by Beggs (ibid.), Hinnen et al. (Proc. Natl. Acad. Sci. USA 75: 1929-1933, 1978), Yelton et al., (Proc. Natl. Acad. Sci. USA 81: 1740-1747, 1984), and Russell (Nature 301: 167-169, 1983). Other techniques for introducing cloned DNA sequences into fungal cells, such as electroporation (Becker and Guarente, Methods in Enzymol. 194: 182-187, 1991) may be used. The genotype of the host cell will generally contain a genetic defect that is complemented by the selectable marker present on the expression vector. Choice of a particular host and selectable marker is well within the level of ordinary skill in the art.

Cloned DNA sequences comprising modified Tf fusion proteins may be introduced into cultured mammalian cells by, for example, calcium phosphate-mediated transfection (Wigler et al., Cell 14: 725, 1978; Corsaro and Pearson, Somatic Cell Genetics 7: 603, 1981; Graham and Van der Eb, Virology 52: 456, 1973.) Other techniques for introducing cloned DNA sequences into mammalian cells, such as electroporation (Neumann et al., EMBO J. 1: 841-845, 1982), or lipofection may also be used. In order to identify cells that have integrated the cloned DNA, a selectable marker is generally introduced into the cells along with the gene or cDNA of interest. Preferred selectable markers for use in cultured mammalian cells include genes that confer resistance to drugs, such as neomycin, hygromycin, and methotrexate. The selectable marker may be an amplifiable selectable marker. A preferred amplifiable selectable marker is the DHFR gene. A particularly preferred amplifiable marker is the DHFR' (see U.S. Patent 6,291,212) cDNA (Simonsen and Levinson, Proc. Natl. Acad. Sci. USA 80: 2495-2499, 1983). Selectable markers are reviewed by Thilly (Mammalian Cell Technology, Butterworth Publishers, Stoneham, Mass.) and the choice of selectable markers is well within the level of ordinary skill in the art.

### Host Cells

The present application also describes a cell, preferably a yeast cell transformed to express a modified transferrin fusion protein. In addition to the transformed host cells themselves, the application also describes a culture of those cells, preferably a monoclonal (clonally homogeneous) culture, or a culture derived from a monoclonal culture, in a nutrient medium. If the polypeptide is secreted, the medium will contain the polypeptide, with the cells, or without the cells if they have been filtered or centrifuged away.
Host cells for use in practicing the present invention include eukaryotic cells, and in some cases prokaryotic cells, capable of being transformed or transfected with exogenous DNA and grown in culture, such as cultured mammalian, insect, fungal, plant and bacterial cells.

Fungal cells, including species of yeast (*e*.*g*., *Saccharomyces* spp., *Schizosaccharomyces* spp., *Pichia* spp.) may be used as host cells within the present invention. Examples of fungi including yeasts as hosts for expressing the, transferrin fusion protein are *Pichia* (some species of which were formerly classified as *Hansenula*), *Saccharomyces, Kluyveromyces, Aspergillus, Candida, Torulopsis, Torulaspora, Schizosaccharomyces, Citeromyces, Pachysolen, Zygosaccharomyces, Debaromyces, Trichoderma, Cephalosporium, Humicola, Mucor, Neurospora, Yarrowia, Metschunikowia, Rhodosporidium, Leucosporidium, Botryoascus, Sporidiobolus, Endomycopsis,* and the like. Examples of *Saccharomyces* spp. are *S. cerevisiae, S. italicus and S. rouxii.* Examples of *Kluyveromyces* spp. are *K. fragilis, K. lactis and K. marxianus.* A suitable *Torulaspora* species is *T. delbrueckii.* Examples of *Pichia* spp. are *P. angusta* (formerly *H. polymorpha*), *P. anomala* (formerly *H. anomala*) and *P. pastoris*.

Particularly useful host cells to produce the Tf fusion proteins are the methylotrophic *Pichia pastoris* (Steinlein et al. (1995) Protein Express. Purif. 6:619-624). *Pichia pastoris* has been developed to be an outstanding host for the production of foreign proteins since its alcohol oxidase promoter was isolated and cloned; its transformation was first reported in 1985. *P. pastoris* can utilize methanol as a carbon source in the absence of glucose. The *P. pastoris* expression system can use the methanol-induced alcohol oxidase (AOX1) promoter, which controls the gene that codes for the expression of alcohol oxidase, the enzyme which catalyzes the first step in the metabolism of methanol. This promoter has been characterized and incorporated into a series of *P. pastoris* expression vectors. Since the proteins produced in *P. pastoris* are typically folded correctly and secreted into the medium, the fermentation of genetically engineered *P. pastoris* provides an excellent alternative to E. coli expression systems. A number of proteins have been produced using this system, including tetanus toxin fragment, Bordatella pertussis pertactin, human serum albumin and lysozyme.

Strains of the yeast *Saccharomyces cerevisiae* are another preferred host. In a preferred embodiment, a yeast cell, or more specifically, a *Saccharomyces cerevisiae* host cell that contains a genetic deficiency in a gene required for asparagine-linked glycosylation of glycoproteins is used. *S. cerevisiae* host cells having such defects may be prepared using standard techniques of mutation and selection, although many available yeast strains have been modified to prevent or reduce glycosylation or hypermannosylation. Ballou et al. (J. Biol. Chem. 255: 5986-5991, 1980) have described the isolation of mannoprotein biosynthesis mutants that are defective in genes which affect asparagine-linked glycosylation. Gentzsch and Tanner (Glycobiology 7:481-486, 1997) have described a family of at least six genes (PMT1-6) encoding enzymes responsible for the first step in O-glycosylation of proteins in yeast. Mutants defective in one or more of these genes show reduced O-linked glycosylation and/or altered specificity of O-glycosylation.

To optimize production of the heterologous proteins, it is also preferred that the host strain carries a mutation, such as the *S. cerevisiae* pep4 mutation (Jones, Genetics 85: 23-33, 1977), which results in reduced proteolytic activity. Host strains containing mutations in other protease encoding regions are particularly useful to produce large quantities of the Tf fusion proteins.

Host cells containing DNA constructs are grown in an appropriate growth medium. As used herein, the term "appropriate growth medium" means a medium containing nutrients required for the growth of cells. Nutrients required for cell growth may include a carbon source, a nitrogen source, essential amino acids, vitamins, minerals and growth factors. The growth medium will generally select for cells containing the DNA construct by, for example, drug selection or deficiency in an essential nutrient which is complemented by the selectable marker on the DNA construct or co-transfected with the DNA construct. Yeast cells, for example, are preferably grown in a chemically defined medium, comprising a carbon source, *e*.*g*. sucrose, a non-amino acid nitrogen source, inorganic salts, vitamins and essential amino acid supplements. The pH of the medium is preferably maintained at a pH greater than 2 and less than 8, preferably at pH 5.5-6.5. Methods for maintaining a stable pH include buffering and constant pH control. Preferred buffering agents include succinic acid and Bis-Tris (Sigma Chemical Co., St. Louis, Mo.). Yeast cells having a defect in a gene required for asparagine-linked glycosylation are preferably grown in a medium containing an osmotic stabilizer. A preferred osmotic stabilizer is sorbitol supplemented into the medium at a concentration between 0.1 M and 1.5 M., preferably at 0.5 M or 1.0 M.

Cultured mammalian cells are generally grown in commercially available serum-containing or serum-free media. Selection of a medium appropriate for the particular cell line used is within the level of ordinary skill in the art. Transfected mammalian cells are allowed to grow for a period of time, typically 1-2 days, to begin expressing the DNA sequence(s) of interest. Drug selection is then applied to select for growth of cells that are expressing the selectable marker in a stable fashion. For cells that have been transfected with an amplifiable selectable marker the drug concentration may be increased in a stepwise manner to select for increased copy number of the cloned sequences, thereby increasing expression levels.

Baculovirus/insect cell expression systems may also be used to produce the modified Tf fusion proteins. The BacPAK™ Baculovirus Expression System (BD Biosciences (Clontech)) expresses recombinant proteins at high levels in insect host cells. The target gene is inserted into a transfer vector, which is cotransfected into insect host cells with the linearized BacPAK6 viral DNA. The BacPAK6 DNA is missing an essential portion of the baculovirus genome. When the DNA recombines with the vector, the essential element is restored and the target gene is transferred to the baculovirus genome. Following recombination, a few viral plaques are picked and purified, and the recombinant phenotype is verified. The newly isolated recombinant virus can then be amplified and used to infect insect cell cultures to produce large amounts of the desired protein.

Tf fusion proteins may also be produced using transgenic plants and animals. For example, sheep and goats can make the therapeutic protein in their milk. Or tobacco plants can include the protein in their leaves. Both transgenic plant and animal production of proteins comprises adding a new gene coding the fusion protein into the genome of the organism. Not only can the transgenic organism produce a new protein, but it can also pass this ability onto its offspring.

### Secretory Signal Sequences

The terms "secretory signal sequence" or "signal sequence" or "secretion leader sequence" are used interchangeably and are described, for example in U.S. Pat. 6,291,212 and U.S. Pat 5,547,871. Secretory signal sequences or signal sequences or secretion leader sequences encode secretory peptides. A secretory peptide is an amino acid sequence that acts to direct the secretion of a mature polypeptide or protein from a cell. Secretory peptides are generally characterized by a core of hydrophobic amino acids and are typically (but not exclusively) found at the amino termini of newly synthesized proteins. Very often the secretory peptide is cleaved from the mature protein during secretion. Secretory peptides may contain processing sites that allow cleavage of the signal peptide from the mature protein as it passes through the secretory pathway. Processing sites may be encoded within the signal peptide or may be added to the signal peptide by, for example, *in vitro* mutagenesis.

Secretory peptides may be used to direct the secretion of modified Tf fusion proteins. One such secretory peptide that may be used in combination with other secretory peptides is the alpha mating factor leader sequence. Secretory signal sequences or signal sequences or secretion leader sequences are required for a complex series of post-translational processing steps which result in secretion of a protein. If an intact signal sequence is present, the protein being expressed enters the lumen of the rough endoplasmic reticulum and is then transported through the Golgi apparatus to secretory vesicles and is finally transported out of the cell. Generally, the signal sequence immediately follows the initiation codon and encodes a signal peptide at the amino-terminal end of the protein to be secreted. In most cases, the signal sequence is cleaved off by a specific protease, called a signal peptidase. Preferred signal sequences improve the processing and export efficiency of recombinant protein expression using viral, mammalian or yeast expression vectors. In some cases, the native Tf signal sequence may be used to express and secrete fusion proteins.

### Linkers

The Tf moiety and therapeutic protein moiety(s) of the modified transferrin fusion proteins can be fused directly or using a linker peptide of various lengths to provide greater physical separation and allow more spatial mobility between the fused proteins and thus maximize the accessibility of the therapeutic protein portion, for instance, for binding to its cognate receptor. The linker peptide may consist of amino acids that are flexible or more rigid. For example, a linker such as but not limited to a poly-glycine stretch. The linker can be less than about 50, 40, 30, 20, or 10 amino acid residues. The linker can be covalently linked to and between the transferrin protein or portion thereof and the therapeutic protein.

### Detection of Tf Fusion Proteins

Assays for detection of biologically active modified transferrin-therapeutic protein fusions may include Western transfer, protein blot or colony filter as well as activity based assays that detect the fused therapeutic protein. A Western transfer filter may be prepared using the method described by Towbin et al. (Proc. Natl. Acad. Sci. USA 76: 4350-4354, 1979). Briefly, samples are electrophoresed in a sodium dodecylsulfate polyacrylamide gel. The proteins in the gel are electrophoretically transferred to nitrocellulose paper. Protein blot filters may be prepared by filtering supernatant samples or concentrates through nitrocellulose filters using, for example, a Minifold (Schleicher & Schuell, Keene, N.H.). Colony filters may be prepared by growing colonies on a nitrocellulose filter that has been laid across an appropriate growth medium. In this method, a solid medium is preferred. The cells are allowed to grow on the filters for at least 12 hours. The cells are removed from the filters by washing with an appropriate buffer that does not remove the proteins bound to the filters. A preferred buffer comprises 25 mM Tris-base, 19 mM glycine, pH 8.3, 20% methanol.

Fusion proteins may also be detected by assaying for the activity of the therapeutic protein moiety. Such assays are readily available, including but not limited to, those assays described in Table 1 from PCT International Publication No. WO 03/020746. Specifically, transferrin fusion proteins may be assayed for functional activity (*e.g.*, biological activity or therapeutic activity) using the assay referenced in the "Exemplary Activity Assay" column of Table 1. Additionally, one of skill in the art may routinely assay fragments of a therapeutic protein corresponding to a therapeutic protein portion of a fusion protein, for activity using assays referenced in its corresponding row of Table 1. Further, one of skill in the art may routinely assay fragments of a modified transferrin protein for activity using assays known in the art.

For example, in one embodiment where one is assaying for the ability of a transferrin fusion protein to bind or compete with a therapeutic protein for binding to an anti-therapeutic polypeptide antibody and/or anti-transferrin antibody, various immunoassays known in the art can be used, including but not limited to, competitive and non-competitive assay systems using techniques such as radioimmunoassays, ELISA (enzyme linked immunosorbent assay), sandwich immunoassays, immunoradiometric assays, gel diffusion precipitation reactions, immunodiffusion assays, *in situ* immunoassays (using colloidal gold, enzyme or radioisotope labels, for example), western blots, precipitation reactions, agglutination assays (*e*.*g*., gel agglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay.

In a further embodiment, where a binding partner (*e*.*g*., a receptor or a ligand) of a therapeutic protein is identified, binding to that binding partner by a transferrin fusion protein containing that therapeutic protein as the therapeutic protein portion of the fusion can be assayed, *e*.*g*., by means well-known in the art, such as, for example, reducing and non-reducing gel chromatography, protein affinity chromatography, and affinity blotting. Other methods will be known to the skilled artisan.

### Isolation/Purification of Modified Transferrin Fusion Proteins

Secreted, biologically active, modified transferrin fusion proteins may be isolated from the medium of host cells grown under conditions that allow the secretion of the biologically active fusion proteins. The cell material is removed from the culture medium, and the biologically active fusion proteins are isolated using isolation techniques known in the art. Suitable isolation techniques include precipitation and fractionation by a variety of chromatographic methods, including gel filtration, ion exchange chromatography and affinity chromatography.

A particularly preferred purification method is affinity chromatography on an iron binding or metal chelating column or an immunoaffinity chromatography using an antibody directed against the transferrin or therapeutic protein or peptide portion of the polypeptide fusion. The antibody is preferably immobilized or attached to a solid support or substrate. A particularly preferred substrate is CNBr-activated Sepharose (Pharmacia LKB Technologies, Inc., Piscataway, N.J.). By this method, the medium is combined with the antibody/substrate under conditions that will allow binding to occur. The complex may be washed to remove unbound material, and the transferrin fusion protein is released or eluted through the use of conditions unfavorable to complex formation. Particularly useful methods of elution include changes in pH, wherein the immobilized antibody has a high affinity for the ligand at a first pH and a reduced affinity at a second (higher or lower) pH; changes in concentration of certain chaotropic agents; or through the use of detergents.

### Labeled Modified Transferrin Fusion Proteins

Transferrin fusion proteins may also be labeled with a radioisotope or other imaging agent and used for *in vivo* diagnostic purposes. Preferred radioisotope imaging agents include iodine-125 and technetium-99, with technetium-99 being particularly preferred. Methods for producing protein-isotope conjugates are well known in the art, and are described by, for example, Eckelman et al. (U.S. Pat. No. 4,652,440), Parker et al. (WO 87/05030) and Wilber et al. (EP 203,764): Alternatively, the transferrin fusion proteins may be bound to spin label enhancers and used for magnetic resonance (MR) imaging. Suitable spin label enhancers include stable, sterically hindered, free radical compounds such as nitroxides. Methods for labeling ligands for MR imaging are disclosed by, for example, Coffman et al. (U.S. Pat. No. 4,656,026). For administration, the labeled transferrin fusion proteins are combined with a pharmaceutically acceptable carrier or diluent, such as sterile saline or sterile water. Administration is preferably by bolus injection, preferably intravenously.

### Production of Fusion Proteins

The present application further describes methods for producing a modified fusion protein using nucleic acid molecules herein described. In general terms, the production of a recombinant form of a protein typically involves the following steps.

A nucleic acid molecule is first obtained that encodes a transferrin fusion protein of the invention. The nucleic acid molecule is then preferably placed in operable linkage with suitable control sequences, as described above, to form an expression unit containing the protein open reading frame. The expression unit is used to transform a suitable host and the transformed host is cultured under conditions that allow the production of the recombinant protein. Optionally the recombinant protein is isolated from the medium or from the cells; recovery and purification of the protein may not be necessary in some instances where some impurities may be tolerated.

Each of the foregoing steps can be accomplished in a variety of ways. For example, the construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier and are otherwise known to persons skilled in the art. Suitable restriction sites can, if not normally available, be added to the ends of the coding sequence so as to provide an excisable gene to insert into these vectors. A skilled artisan can readily adapt any host/expression system known in the art for use with the nucleic acid molecules of the invention to produce a desired recombinant protein.

As discussed above, any expression system may be used, including yeast, bacterial, animal, plant, eukaryotic and prokaryotic systems. In some embodiments, yeast, mammalian cell culture and transgenic animal or plant production systems are preferred. In other embodiments, yeast systems that have been modified to reduce native yeast glycosylation, hyper-glycosylation or proteolytic activity may be used.

### Therapeutic Molecules

Any therapeutic molecule may be used as the fusion partner to Tf according to the methods and compositions of the present invention. As used herein, a therapeutic molecule is typically a protein or peptide capable of exerting a beneficial biological effect *in vitro* or *in vivo* and includes proteins or peptides that exert a beneficial effect in relation to normal homeostasis, physiology or a disease state. Therapeutic molecules do not include fusion partners commonly used as markers or protein purification aids, such as bacterial galactosidases (see for example, U.S. Patent 5, 986, 067 and Aldred et al. (1984) Biochem. Biophys. Res. Commun. 122: 960-965). For instance, a beneficial effect as related to a disease state includes any effect that is advantageous to the treated subject, including disease prevention, disease stabilization, the lessening or alleviation of disease symptoms or a modulation, alleviation or cure of the underlying defect to produce an effect beneficial to the treated subject.

A modified transferrin fusion protein includes at least a fragment or variant of a therapeutic protein and at least a fragment or variant of modified serum transferrin, which are associated with one another, preferably by genetic fusion.

In one embodiment, the transferrin fusion protein includes a modified transferrin molecule linked to a neuropharmaceutical agent. In another embodiment, the modified transferrin fusion protein includes transferrin at the carboxyl terminus linked to a neuropharmaceutical agent at the amino terminus. In an alternate embodiment, the modified transferrin fusion protein includes transferrin at the amino terminus linked to a neuropharmaceutical agent at the carboxy terminus. In specific embodiments, the neuropharmaceutical agent is either nerve growth factor or ciliary neurotrophic factor.

In further embodiments, a modified transferrin fusion protein may contain at least a fragment or variant of a therapeutic protein, and/or at least a fragment or variant of an antibody. In a further embodiment, the transferrin fusion proteins can contain peptide fragments or peptide variants of proteins or antibodies wherein the variant or fragment retains at least one biological or therapeutic activity. The transferrin fusion proteins can contain therapeutic proteins that can be peptide fragments or peptide variants at least about 4, at least 5, at least 6, at least 7, at least 8, at least 9, at least 10, at least 11, at least 12, at least 13, at least 14, at least 15, at least 20, at least 25, at least 30, at least 35, or at least about 40, at least about 50, at least about 55, at least about 60 or at least about 70 or more amino acids in length fused to the N and/or C termini, inserted within, or inserted into a loop of a modified transferrin.

In another embodiment, the modified transferrin fusion molecules contain a therapeutic protein portion that can be fragments of a therapeutic protein that include the full length protein as well as polypeptides having one or more residues deleted from the amino terminus of the amino acid sequence.

In another embodiment, the modified transferrin fusion molecules contain a therapeutic protein portion that can be fragments of a therapeutic protein that include the full length protein as well as polypeptides having one or more residues deleted from the carboxy terminus of the amino acid sequence.

In another embodiment, the modified transferrin fusion molecules contain a therapeutic protein portion that can have one or more amino acids deleted from both the amino and the carboxy termini.

In another embodiment, the modified transferrin fusion molecules contain a therapeutic protein portion that is at least about 80%, 85%, 90%, 95%, 96%, 97%, 98% or 99% identical to a reference therapeutic protein set forth herein, or fragments thereof. In further embodiments, the transferrin fusion molecules contain a therapeutic protein portion that is at least about 80%, 85%, 90%, 95%, 96%, 97%, 98% or 99% identical to reference polypeptides having the amino acid sequence of N- and C-terminal deletions as described above.

In another embodiment, the modified transferrin fusion molecules contain the therapeutic protein portion that is at least about 80%, 85%, 90%, 95%, 96%, 97%, 98%, 99% or 100%, identical to, for example, the native or wild-type amino acid sequence of a therapeutic protein. Fragments, of these polypeptides are also provided.

The therapeutic proteins corresponding to a therapeutic protein portion of a modified transferrin fusion protein, such as cell surface and secretory proteins, can be modified by the attachment of one or more oligosaccharide groups. The modification referred to as glycosylation can significantly affect the physical properties of proteins and can be important in protein stability, secretion, and localization. Glycosylation occurs at specific locations along the polypeptide backbone. There are usually two major types of glycosylation: glycosylation characterized by O-linked oligosaccharides, which are attached to serine or threonine residues; and glycosylation characterized by N-linked oligosaccharides, which are attached to asparagine residues in an Asn-X-Ser/Thr sequence, where X can be an amino acid except proline. Variables such as protein structure and cell type influence the number and nature of the carbohydrate units within the chains at different glycosylation sites. Glycosylation isomers are also common at the same site within a given cell type. For example, several types of human interferon are glycosylated.

Therapeutic proteins corresponding to a therapeutic protein portion of a transferrin fusion protein of the invention, as well as analogs and variants thereof, may be modified so that glycosylation at one or more sites is altered as a result of manipulation(s) of their nucleic acid sequence by the host cell in which they are expressed, or due to other conditions of their expression. For example, glycosylation isomers may be produced by abolishing or introducing glycosylation sites, *e*.*g*., by substitution or deletion of amino acid residues, such as substitution of glutamine for asparagine, or unglycosylated recombinant proteins may be produced by expressing the proteins in host cells that will not glycosylate them, *e*.*g*. in glycosylation-deficient yeast. These approaches are known in the art.

Therapeutic proteins and their nucleic acid sequences are well known in the art and available in public databases such as Chemical Abstracts Services Databases (*e.g.*, the CAS Registry), GenBank, and GenSeq.

In other embodiments, the transferrin fusion proteins are capable of a therapeutic activity and/or biologic activity, corresponding to the therapeutic activity and/or biologic activity of the therapeutic protein listed in the corresponding row of Table 1 from PCT International Publication No. WO 03/020746, and elsewhere in this application. (See, *e*.*g*., the "Biological Activity" and "Therapeutic Protein X" columns of Table 1) In further embodiments, the therapeutically active protein portions of the transferrin fusion proteins are fragments or variants of the reference sequences cited herein.

Even if deletion of one or more amino acids from the N-terminus of a protein results in modification or loss of one or more biological functions of the therapeutic protein portion, other therapeutic activities and/or functional activities (*e*.*g*., biological activities, ability to multimerize, ability to bind a ligand) may still be retained. For example, the ability of polypeptides with N-terminal deletions to induce and/or bind to antibodies which recognize the complete or mature forms of the polypeptides generally will be retained with less than the majority of the residues of the complete polypeptide removed from the N-terminus. Whether a particular polypeptide lacking N-terminal residues of a complete polypeptide retains such immunologic activities can be assayed by routine methods described herein and otherwise known in the art. It is not unlikely that a mutant with a large number of deleted N-terminal amino acid residues may retain some biological or immunogenic activities. In fact, peptides composed of as few as six amino acid residues may often evoke an immune response.

Also as mentioned above, even if deletion of one or more amino acids from the N-terminus or C-terminus of a therapeutic protein results in modification or loss of one or more biological functions of the protein, other functional activities (*e*.*g*., biological activities, ability to multimerize, ability to bind a ligand) and/or therapeutic activities may still be retained. For example the ability of polypeptides with C-terminal deletions to induce and/or bind to antibodies which recognize the complete or mature forms of the polypeptide generally will be retained when less than the majority of the residues of the complete or mature polypeptide are removed from the C-terminus. Whether a particular polypeptide lacking the N-terminal and/or, C-terminal residues of a reference polypeptide retains therapeutic activity can readily be determined by routine methods described herein and/or otherwise known in the art.

Peptide fragments of the therapeutic proteins can be fragments comprising, or alternatively, consisting of, an amino acid sequence that displays a therapeutic activity and/or functional activity (*e*.*g*. biological activity) of the polypeptide sequence of the therapeutic protein of which the amino acid sequence is a fragment.

The peptide fragments of the therapeutic protein may comprise only the N- and C-termini of the protein, *i*.*e*., the central portion of the therapeutic protein has been deleted. Alternatively, the peptide fragments may comprise non-adjacent and/or adjacent portions of the central part of the therapeutic protein.

Other polypeptide fragments are biologically active fragments. Biologically active fragments are those exhibiting activity similar, but not necessarily identical, to an activity of a therapeutic protein used in the present invention. The biological activity of the fragments may include an improved desired activity, or a decreased undesirable activity.

Generally, variants of proteins are overall very similar, and, in many regions, identical to the amino acid sequence of the therapeutic protein corresponding to a therapeutic protein portion of a transferrin fusion protein of the invention. Nucleic acids encoding these variants are also encompassed by the invention.

Further therapeutic polypeptides that may be used in the invention are polypeptides encoded by polynucleotides which hybridize to the complement of a nucleic acid molecule encoding an amino acid sequence of a therapeutic protein under stringent hybridization conditions which are known to those of skill in the art. (see, for example, Ausubel, F.M. et al., eds., 1989 Current Protocols in Molecular Biology, Green Publishing Associates, Inc., and John Wiley & Sons Inc., New. York). Polynucleotides encoding these polypeptides are also encompassed by the invention.

By a polypeptide-having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, or substituted with another amino acid. These alterations of the reference sequence may occur at the amino- or carboxy-terminal positions of the reference amino acid sequence or anywhere between those terminal positions, interspersed either individually among residues in the reference sequence, or in one or more contiguous groups within the reference sequence.

As a practical matter, whether any particular polypeptide is at least about 80%, 85%, 90%, 95%, 96%, 97%, 98% or 99% identical to, for instance, the amino acid sequence of a transferrin fusion protein of the invention or a fragment thereof (such, as the therapeutic protein portion of the transferrin fusion protein or the transferrin portion of the transferrin fusion protein), can be determined conventionally using known computer programs. A preferred method for determining the best overall match between a query sequence (a sequence of the present invention) and a subject sequence, also referred to as a global sequence alignment, can be determined using the FASTDB computer program based on the algorithm of Brufiag et al. (Comp. App. Biosci 245 (1990)).

The polynucleotide variants may contain alterations in the coding regions, non-coding regions, or both. Polynucleotide variants containing alterations which produce silent substitutions, additions, or deletions, but do not alter the properties or activities of the encoded polypeptide may be used to produce modified Tf fusion proteins. Nucleotide variants produced by silent substitutions due to the degeneracy of the genetic code can be utilized. Moreover, polypeptide variants in which less than about 50, less than 40, less than 30, less than 20, less than 10, or 5-50, 5-25, 5-10, 1-5, or 1-2 amino acids are substituted, deleted, or added in any combination can also be utilized. Polynucleotide variants can be produced for a variety of reasons, *e*.*g*., to optimize codon expression for a particular host (change codons in the human mRNA to those preferred by a host, such as, yeast or *E. coli* as described above).

In other embodiments, the therapeutic protein moiety has conservative substitutions compared to the wild-type sequence. By "conservative substitutions" is intended swaps within groups such as replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly. Guidance concerning how to make phenotypically silent amino acid substitutions is provided, for example, in Bowie et al., "Deciphering the Message in Protein Sequences: Tolerance to Amino Acid Substitutions," Science 247:1306-1310 (1990). Fusion polypeptides may comprise, or alternatively, consist of, fragments or variants of the amino acid sequence of a therapeutic protein described herein and/or serum transferrin, and/ modified transferrin protein, wherein the fragments or variants have 1-5, 5-10, 5-25, 5-50, 10-50 or 50-150 amino acid residue additions, substitutions, and/or deletions when compared to the reference amino acid sequence. In further embodiments, the amino acid substitutions are conservative. Nucleic acids encoding these polypeptides are also described herein.

The modified fusion proteins can be composed of aminoacids joined to each other by peptide bonds or modified peptide bonds and may contain amino acids other than the 20 gene-encoded amino acids. The polypeptides may be modified by either natural processes, such as post-translational processing, or by chemical modification techniques which are well known in the art. Such modifications are well described in basic texts and in more detailed monographs, as well as in a voluminous research literature.

Modifications can occur anywhere in a polypeptide, including the peptide backbone, the amino acid side-chains and the amino or carboxy termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, glycosylation, GPI anchor formation, hydroxylation, iodination, methylation, myristylation, oxidation, pegylation, proteolytic processing, phosphorylation, prenylation, racemization, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York(1993); POST-TRANSLATIONAL COVALENT MODIFICATION OF PROTEINS, B. C. Johnson, Ed., Academic Press, New' York, pgs. 1-12 (1983); Seifter et al. (1990) Meth. Enzymol. 182:626-646; Rattan et al., Ann. N.Y. Acad. Sci. 663:48-62.

Therapeutic molecules that may be fused to or inserted into Tf include, but are not limited to, hormones, matrix proteins, immunosuppressants, bronchodilators, cardiovascular agents, enzymes, CNS agents, neurotransmitters, receptor proteins or peptides, growth hormones, growth factors, antiviral peptides, fusogenic inhibitor peptides, cytokines, lymphokines, monokines, interleukins, colony stimulating factors, differentiation factors, angiogenic factors, receptor ligands, cancer-associated proteins, antineoplastics, viral peptides, antibiotic peptides, blood proteins, antagonist proteins, transcription factors, anti-angiogenic factors, antagonist proteins or peptides, receptor antagonists, antibodies, single chain antibodies and cell adhesion molecules. Different therapeutic molecules may be combined into a single fusion protein to produce a bi or multi-functional therapeutic molecule. Different molecules may also be used in combination to produce a fusion protein with a therapeutic entity and a targeting entity.

Cytokines are soluble proteins released by cells of the immune system, which act nonenzymatically through specific receptors to regulate immune responses. Cytokines resemble hormones in that they act at low concentrations bound with high affinity to a specific receptor. The term "cytokine" is used herein to describe naturally occurring or recombinant proteins, analogs thereof, and fragments thereof which elicit a specific biological response in a cell which has a receptor for that cytokine. Cytokines preferably include interleukins such as interleukin-2 (IL-2) (GenBank Acc. No. S77834), IL-3 (GenBank Acc. No. M14743), IL-4 (GenBank Acc. No. M23442), IL-5 (GenBank Acc. No. J03478), IL-6 (GenBank Acc. No. M14584), IL-7 (GenBank Acc. No. NM_000880), IL-10 (GenBank Acc. No. NM_000572), IL-12 (GenBank Acc. No.AF180562 and GenBank Acc. No. AF180563), IL-13 (GenBank Acc. No. U10307), IL-14 (GenBank Acc. No. XM_170924), IL-15 (GenBank Acc. No. X91233), IL-16 (GenBank Acc. No. NM_004513), IL-17 (GenBank Acc. No. NM_002190) and IL-18 (GenBank Acc. No. NM_001562), hematopoietic factors such as granulocyte-macrophage colony stimulating factor (GM-CSF) (GenBank Acc. No. X03021), granulocyte colony stimulating factor (G-CSF) (GenBank Acc. No. X03656), platelet activating factor (GenBank Acc. No. NM_000437) and erythropoeitin (GenBank Acc. No. X02158), tumor necrosis factors (TNF) such as TNFα (GenBank Acc. No. X02910), lymphokines such as lymphotoxin- α (GenBank Acc. No. X02911), lymphotoxin-β (GenBank Acc. No. L11016), leukoregulin, macrophage migration inhibitory factor (GenBank Acc. No. M25639), and neuroleukin (GenBank Acc. No. K03515), regulators of metabolic processes such as leptin (GenBank Acc. No. U43415), interferons such as interferon α (IFNα) (GenBank Acc. No. M54886), IFNβ (GenBank Acc. No. V00534), IFNγ (GenBank Acc. No. J00219), IFNo (GenBank Acc. No. NM_002177), thrombospondin 1 (THBS1) (GenBank Acc. No. NM_003246), THBS2 (GenBank Acc. No. L12350), THBS3 (GenBank Acc. No. L38969), THBS4 (GenBank Acc. No. NM_003248), and chemokines. Preferably, the modified transferrin-cytokine fusion protein of the present invention displays cytokine biological activity.

The term "hormone" is used herein to describe any one of a number of biologically active substances that are produced by certain cells or tissues and that cause specific biological changes or activities to occur in another cell or tissue located elsewhere in the body. Hormones preferably include proinsulin (GenBank Acc. No. V00565), insulin (GenBank Acc. No. NM_000207), growth hormone 1 (GenBank Acc. No. V00520), growth hormone 2 (GenBank Acc. No. F006060), growth hormone release factor (GenBank Acc. No. NM_021081), insulin-like growth factor I (GenBank Acc. No. M27544), insulin-like growth factor II (GenBank Acc. No. NM_000612), insulin-like growth factor binding protein 1 (IGFBP-1) (GenBank Acc. No. M59316), IGFBP-2 (GenBank Acc. No. X16302), IGFBP-3 (GenBank Acc. No. NM_000598), IGFBP-4 (GenBank Acc. No. Y12508), IGFBP-5 (GenBank Acc. No. M65062), IGFBP-6 (GenBank Acc. No. NM_002178), IGFBP-7 (GenBank Acc. No. NM_001553), chorionic gonadotropin β chain (GenBank Acc. No. NM_033142), chorionic gonadotropin α chain (GenBank Acc. No. NM_000735), luteinizing hormone β (GenBank Acc. No. X00264), follicle-stimulating hormone β (GenBank Acc. No. NM_000510), thyroid-stimulating hormone β (GenBank Acc. No. NM_000549), prolactin (GenBank Acc. No. NM_000948), pro-opiomelanocortin (GenBank Acc. No. V01510), corticotropin (ACTH), β-lipotropin, α-melanocyte stimulating hormone (α-MSH), γ-lipotropin, β -MSH, β -endorphin, and corticotropin-like intermediate lobe peptide (CLIP).

The term "hormone" also includes Glucagon-Like Peptide-1 (GLP-1) which is a gastrointestinal hormone that regulates insulin secretion belonging to the so-called enteroinsular axis. The amino acid sequence of GLP-1(7-36) and GLP-1(7-37) is (SEQ ID NO: 34): wherein X is NH₂ for GLP-1(7-36) and X is Gly for GLP-1(7-37).

The term "growth factor" is used herein to describe any protein or peptide that binds to a receptor to stimulate cell proliferation. Growth factors preferably include platelet-derived growth factor-α (PDGF-α) (GenBank Acc. No. X03795), PDGF-β (GenBank Acc. No. X02811), hormones, epidermal growth factor (EGF) (GenBank Acc. No. NM_001963), fibroblast growth factors such as fibroblast growth factor 1 (FGF1) (GenBank Acc. No. NM_000800), FGF2 (GenBank Acc. No. NM_002006), FGF3 (GenBank Acc. No. NM_005247), FGF4 (GenBank Acc. No. NM_002007), FGF5 (GenBank Acc. No. M37825), FGF6 (GenBank Acc. No. X57075), FGF7 (GenBank Acc. No. NM_002009), FGF8 (GenBank Acc. No. AH006649), FGF9 (GenBank Acc. No. NM_002010), FGF10 (GenBank Acc. No. AB002097), FGF11 (GenBank Acc. No. NM_004112), FGF12 (GenBank Acc. No. NM_021032), FGF13 (GenBank Acc. No. NM_004114), FGF14 (GenBank Acc. No. NM_004115), FGF16 (GenBank Acc. No. AB009391), FGF17 (GenBank Acc. No. NM_003867), FGF18 (GenBank Acc. No. AF075292), FGF19 (GenBank Acc. No. NM_005117), FGF20 (GenBank Acc. No. NM_019851), FGF21 (GenBank Acc. No. NM_019113), FGF22 (GenBank Acc. No. NM_020637), and FGF23 (GenBank Acc. No. NM_020638), angiogenin (GenBank Acc. No. M11567), brain-derived neurotrophic factor (GenBank Acc. No. M61176), ciliary neurotrophic growth factor (GenBank Acc. No. X60542), transforming growth factor-α (TGF-α) (GenBank Acc. No. X70340), TGF-β (GenBank Acc. No. X02812), nerve growth factor-α (NGF-α) (GenBank Acc. No. NM_010915), NGF-β (GenBank Acc. No. X52599), tissue inhibitor of metalloproteinase 1 (TIMP1) (GenBank Acc. No. NM_003254), TIMP2 (GenBank Acc. No. NM_003255), TIMP3 (GenBank Acc. No. U02571), TIMP4 (GenBank Acc. No. U76456) and macrophage stimulating 1 (GenBank Acc. No. L11924).

The term "matrix protein" is used herein to describe proteins or peptides that are normally found in the extracellular matrix. These proteins may be functionally important for strength, filtration, or adhesion. Matrix proteins preferably include collagens such as collagen I (GenBank Acc. No. Z74615), collagen II (GenBank Acc. No. X16711), collagen III (GenBank Acc. No. X14420), collagen IV (GenBank Acc. No. NM_001845), collagen V (GenBank Acc. No. NM_000393), collagen VI (GenBank Acc. No. NM_058175), collagen VII (GenBank Acc. No. L02870), collagen VIII (GenBank Acc. No. NM_001850), collagen IX (GenBank Acc. No. X54412), collagen X (GenBank Acc. No. X60382), collagen XI (GenBank Acc. No. J04177), and collagen XII (GenBank Acc. No. U73778), laminin proteins such as LAMA2 (GenBank Acc. No. NM_000426), LAMA3 (GenBank Acc. No. L34155), LAMA4 (GenBank Acc. No. NM_002290), LAMB1 (GenBank Acc. No. NM_002291), LAMB3 (GenBank Acc. No. L25541), LAMC1 (GenBank Acc. No. NM_002293), nidogen (GenBank Acc. No. NM_002508), α-tectorin (GenBank Acc. No. NM_005422), β-tectorin (GenBank Acc. No. NM_058222), and fibronectin (GenBank Acc. No. X02761).

The term "blood proteins" are traditionally defined as those sourced from plasma, many now commonly produced by recombinant means, and include, but are not limited to native serum proteins, derivatives, fragments and mutants or variants thereof, blood clotting factors, derivatives, mutants, variants and fragments (including factors VII, VIII, IX, X), protease inhibitors (antithrombin III, alpha-1 antitrypsin), urokinase-type plasminogen activator, immunoglobulins, von Willebrand factor and von Willebrand mutants, fibronectin, fibrinogen, thrombin and hemoglobin.

The term "enzyme" is used herein to describe any protein or proteinaceous substance which catalyzes a specific reaction without itself being permanently altered or destroyed. Enzymes preferably include coagulation factors such as F2 (GenBank Acc. No. XM_170688), F7 (GenBank Acc. No. XM_027508), F8 (GenBank Acc. No. XM_013124), F9 (GenBank Acc. No. NM_000133), F10 (GenBank Acc. No. AF503510) and others, matrix metalloproteinases such as matrix metalloproteinase I (GenBank Acc. No. MMP1) (GenBank Acc. No. NM_002421), MMP2 (GenBank Acc. No. NM_004530), MMP3 (GenBank Acc. No. NM_002422), MMP7 (GenBank Acc. No. NM_002423), MMP8 (GenBank Acc. No. NM_002424), MMP9 (GenBank Acc. No. NM_004994), MMP10 (GenBank Acc. No. NM_002425), MMP12 (GenBank Acc. No. NM_002426), MMP13 (GenBank Acc. No. X75308), MMP20 (GenBank Acc. No. NM_004771), adenosine deaminase (GenBank Acc. No. NM_000022), mitogen activated protein kinases such as MAPK3 (GenBank Acc. No. XM_055766), MAP2K2 (GenBank Acc. No. N_030662), MAP2K1 (GenBank Acc. No. NM_002755), MAP2K4 (GenBank Acc. No. NM_003010), MAP2K7 (AF013588), and MAPK12 (NM_002969), kinases such as JNKK1 (GenBank Acc. No. U17743), JNKK2 (GenBank Acc. No. AF014401), JAK1 (M64174), JAK2 (NM_004972), and JAK3 (NM_000215), and phosphatases such as PPM1A (GenBank Acc. No. NM_021003) and PPM1D (GenBank Acc. No. NM_003620).

The term "transcription factors" is used herein to describe any protein or peptide involved in the transcription of protein-coding genes. Transcription factors may include Sp1, Sp2 (GenBank Acc. No. NM_003110), Sp3 (GenBank Acc. No. AY070137), Sp4 (GenBank Acc. No. NM_003112) NFYB (GenBank Acc. No. NM_006166), Hap2 (GenBank Acc. No. M59079), GATA-1 (GenBank Acc. No. NM_002049), GATA-2 (GenBank Acc. No. NM_002050), GATA-3 (GenBank Acc. No. X55122), GATA-4 (GenBank Acc. No. L34357), GATA-5, GATA-6 (GenBank Acc. No. NM_005257), FOG2 (NM_012082), Eryfl (GenBank Acc. No. X17254), TRPS1 (GenBank Acc. No. NM_014112), NF-E2 (GenBank Acc. No. NM_006163), NF-E3, NF-E4, TFCP2 (GenBank Acc. No. NM_005653), Oct-1 (GenBank Acc. No. X13403), homeobox proteins such as HOXB2 (GenBank Acc. No. NM_002145), HOX2H (GenBank Acc. No. X16665), hairless homolog (GenBank Acc. No. NM_005144), mothers against decapentaplegic proteins such as MADH1 (GenBank Acc. No. NM_005900), MADH2 (GenBank Acc. No. NM_005901), MADH3 (GenBank Acc. No. NM_005902), MADH4 (GenBank Acc. No. NM_005359), MADH5 (GenBank Acc. No. AF009678), MADH6 (GenBank Acc. No. NM_005585), MADH7 (GenBank Acc. No. NM_005904), MADH9 (GenBank Acc. No. NM_005905), and signal transducer and activator of transcription proteins such as STAT1 (GenBank Acc. No. XM_010893), STAT2 (GenBank Acc. No. NM_005419), STAT3 (GenBank Acc. No. AJ012463), STAT4 (GenBank Acc. No. NM_003151), STAT5 (GenBank Acc. No. L41142), and STAT6 (GenBank Acc. No. NM_003153).

In yet another embodiment of the invention, the therapeutic molecule is a non-human or non-mammalian protein. For example, HIV gp120, HIV Tat, surface proteins of other viruses such as hepatitis, herpes, influenza, adenovirus and RSV, other HIV components, parasitic surface proteins such as malarial antigens, and bacterial surface proteins are preferred. These non-human proteins may be used, for example, as antigens, or because they have useful activities. For example, the therapeutic molecule may be streptokinase, staphylokinase, asparaginase, or other proteins with useful enzymatic activities.

In an alternative embodiment, the therapeutic molecule is a ligand-binding protein with biological activity. Such ligand-binding proteins may, for example, (1) block receptor-ligand interactions at the cell surface; or (2) neutralize the biological activity of a molecule in the fluid phase of the blood, thereby preventing it from reaching its cellular target. In some embodiments, the modified transferrin fusion proteins include a modified transferrin molecule fused to a ligand-binding domain of a receptor selected from the group consisting of, but not limited to, a low density lipoprotein (LDL) receptor, an acetylated LDL receptor, a tumor necrosis factor α receptor, a transforming growth factor β receptor, a cytokine receptor, an immunoglobulin Fc receptor, a hormone receptor, a glucose receptor, a glycolipid receptor, and a glycosaminoglycan receptor. In other embodiments, ligand-binding proteins include CD2 (M14362), CD3G (NM_000073), CD3D (NM_000732), CD3E (NM_000733), CD3Z (J04132), CD28 (NM_006139), CD4 (GenBank Acc. No. NM_000616), CD1A (GenBank Acc. No. M28825), CD1B (GenBank Acc. No. NM_001764), CD1C (GenBank Acc. No. NM_001765), CD1D (GenBank Acc. No. NM_001766), CD80 (GenBank Acc. No. NM_005191), GNB3 (GenBank Acc. No. AF501884), CTLA-4 (GenBank Acc. No. NM_005214), intercellular adhesion molecules such as ICAM-1 (NM_000201), ICAM-2 (NM_000873), and ICAM-3 (NM_002162), tumor necrosis factor receptors such as TNFRSF1A (GenBank Acc. No. X55313), TNFR1SFB (GenBank Acc. No. NM_001066), TNFRSF9 (GenBank Acc. No. NM_001561), TNFRSF10B (GenBank Acc. No. NM_003842), TNFRSF11B (GenBank Acc. No. NM_002546), and TNFRSF13B (GenBank Acc. No. NM_006573), and interleukin receptors such as IL2RA (GenBank Acc. No. NM_000417), IL2RG (GenBank Acc. No. NM_000206), IL4R (GenBank Acc. No. AF421855), IL7R (GenBank. Acc. No. NM_002185), IL9R (GenBank Acc. No. XM_015989), and IL13R (GenBank Acc. No. X95302). Preferably, the Tf-ligand-binding protein fusion of the present invention displays the biological activity of the ligand-binding protein.

The term "cancer-associated proteins" is used herein to describe proteins or polypeptides whose expression is associated with cancer or the maintenance of controlled cell growth, such as proteins encoded by tumor suppressor genes or oncogenes. Cancer-associated proteins may include p16 (GenBank Acc. No. AH005371), p53 (GenBank Acc. No. NM_000546), p63 (GenBank Acc. No. NM_003722), p73 (GenBank Acc. No. NM_005427), BRCA1 (GenBank Acc. No. U14680), BRCA2 (GenBank Acc. No. NM_000059), CTBP interacting protein (GenBank Acc. No. U72066), DMBT1 (GenBank Acc. No. NM_004406), HRAS (GenBank Acc. No. NM_005343), NCYM (GenBank Acc. No. NM_006316), FGR (GenBank Acc. No. NM_005248), myb (GenBank Acc. No. AF104863), rafl (GenBank Acc. No. NM_002880), erbB2 (GenBank Acc. No. NM_004448), VAV (GenBank Acc. No. X16316), c-fos (V GenBank Acc. No. 01512), c-fes (GenBank Acc. No. X52192), c-jun (GenBank Acc. No. NM_002228), MAS1 (GenBank Acc. No. M13150), pim-1 (GenBank Acc. No. M16750), TIF1 (GenBank Acc. No. NM_003852), c-fms (GenBank Acc. No. X03663), EGFR (GenBank Acc. No. NM_005228), erbA (GenBank Acc. No. X04707), c-src tyrosine kinase (GenBank Acc. No. XM_044659), c-abl (GenBank Acc. No. M14752), N-ras (GenBank Acc. No. X02751), K-ras (GenBank Acc. No. M54968), jun-B (GenBank Acc. No. M29039), c-myc (GenBank Acc. No. AH001511), RB1 (GenBank Acc. No. M28419), DCC (GenBank Acc. No. X76132), APC (GenBank Acc. No. NM_000038), NF1 (GenBank Acc. No. M89914), NF2 (GenBank Acc. No. Y18000), and bcl-2 (GenBank Acc. No. M13994).

"Fusogenic inhibitor peptides" is used herein to describe peptides that show antiviral activity, anti-membrane fusion capability, and/or an ability to modulate intracellular processes, for instance, those involving coiled-coil peptide structures. Antiviral activity includes, but is not limited to, the inhibition of HIV-1, HIV-2, RSV, SIV, EBV, measles, virus, influenza virus, or CMV transmission to uninfected cells. Additionally, the antifusogenic capability, antiviral activity or intracellular modulatory activity of the peptides merely requires the presence of the peptides and specifically does not require the stimulation of a host immune response directed against such peptides. Antifusogenic refers to a peptide's ability to inhibit or reduce the level of membrane fusion events between two or more moieties relative to the level of membrane fusion which occurs between said moieties in the absence of the peptide. The moieties may be, for example, cell membranes or viral structures, such as viral envelopes or pili. The term "antiviral peptide", as used herein, refers to the peptide's ability to inhibit viral infection of cells or some viral activity required for productive viral infection and/or viral pathogenesis, via, for example, cell-cell fusion or free virus infection. Such infection may involve membrane fusion, as occurs in the case of enveloped viruses, or some other fusion event involving a viral structure and a cellular structure. Fusogenic inhibitor peptides and antiviral peptides often have amino acid sequences that are derived from greater than one viral protein (*e.g.*, an HIV-1, HIV-2, RSV, and SIV-derived polypeptide).

Examples of fusogenic inhibitor peptides and antiviral peptides can be found in WO 94/2820, WO 96/19495, WO 96/40191, WO 01/64013 and US patents 6,333,395, 6,258,782, 6,228,983, 6,133,418, 6,093,794, 6,068,973, 6,060,065, 6,054,265, 6,020,459, 6,017,536, 6,013,263, 5,464,933, 5,346,989, 5,603,933, 5,656,480, 5,759,517, 6,245,737; 6,326,004, and 6,348,568; all of which are herein incorporated by reference. In a preferred embodiment, antifusogenic peptides are selected from the group consisting of HIV T-20 (FWNWLSAWKDLELLEQENKEQQNQSEEILSHILSTY, SEQ ID NO: 4), HIV T-1249, RSV T786 (VYPSDEYDASISQVNEEINQALAYIRKADELLENV, SEQ ID NO: 5), RSV T1584 (AVSKVLHLEGEVNKIKSALLSTNKAVVSLSNGVSVLTSKVLDLKNYIDKQL, SEQ ID NO: 6) and RSV T112 (VFPSDEFDASISQVNEKINQSLAFIRESDELLHNV, SEQ ID NO: 7).

Examples of other types of peptides, include fragments of therapeutic proteins as described herein, in particular, fragments of human proteins that retain at least one activity of the parent molecule. Peptides that may be used to produce modified Tf fusion proteins of the invention also include mimetic peptides and peptides that exhibit a biological activity of a therapeutic protein but differ in sequence or three-dimensional structure from a full-length therapeutic protein. As a non-limited example, peptides include erythropoeitin mimetic peptides disclosed by Johnson et al. (2000) Nephrol. Dial. Transplant 15(9): 1274-7, Kuai et al. (2000) J. Pept. Res. 56(2):59-62, Barbone et al. (1999) Nephrol. Dial. Transplant. 14 Supp 2:80-4, Middleton et al. (1999) J. Biol. Chem. 274(20):14163-9, Johnson et al. (1998) Biochemistry 37(11):3699-710, Johnson et al. (1997) Chem. Biol. 12:939-50, Wrighton et al. (1997) Nat. Biotechnol. 15(12):1261-5, Livnah et al. (1996) Science 273:464-71, and Wrighton et al., (1996) Science 273:458-64.

Therapeutic molecules also include allergenic proteins and digested fragments thereof. These include pollen allergens from ragweed, rye, June grass, orchard grass, sweet vernal grass, red top grass, timothy grass, yellow dock, wheat, corn, sagebrush, blue grass, California annual grass, pigweed, Bermuda grass, Russian thistle, mountain cedar, oak, box elder, sycamore, maple, elm, *etc*., dust mites, bee venom, food allergens, animal dander, and other insect venoms.

Other therapeutic molecules include microbial vaccines which include viral, bacterial and protozoal vaccines and their various components such as surface antigens. These include vaccines which contain glycoproteins, proteins or peptides derived from these proteins. Such vaccines are prepared from *Staphylococcus aureus, Streptococcus pyogenes*, *Streptococcus pneumoniae, Neisseria meningitidis, Neisseria gonorrhoeae*, *Salmonella spp., Shigella spp., Escherichia coli, Klebsiella spp., Proteus spp., Vibrio cholera, Campylobacter pylori*, *Pseudomonas aeruginosa, Haemophilus influenzae, Bordetella pertussis*, *Mycobacterium tuberculosis*, *Legionella pneumophila*, *Tieponema pallidum*, chlamydia, tetanus toxoid, diphtheria toxoid, influenza viruses, adenoviruses, paramyxoviruses (mumps, measles), rubella viruses, polio viruses, hepatitis viruses, herpes viruses, rabies virus, HIV-1, HIV-2, RSV and papilloma viruses.

Preferred fusion molecules may contain anti-HIV viral peptides, anti-RSV peptides, human growth hormone, α and/or β interferons, erythropoietin (EPO), EPO like peptides, granulocyte-colony stimulating factor (GCSF), granulocyte-macrophage colony-stimulating factor (GMCSF), insulin, insulin-like growth factor (IGF), thrombopoeitin, peptides corresponding to the CDR of an antibody, Islet Neogenesis Associated Protein (INGAP), calcitonin, angiostatin, endostatin, interleukin-2, growth hormone releasing factor, human parathyroid hormone, anti-tumor necrosis factor (TNF) peptides, interleukin-1 (1L-1) receptor and/or single chain antibodies.

Fusion proteins used in libraries of the invention may also be prepared to include peptides or polypeptides derived from peptide libraries to screen for molecules with new or novel functions. Such peptide libraries may include those commercially or publicly available, *e.g.*, American Peptide Co. Inc., Cell Sciences Inc., Invitrogen Corporation, Phoenix Pharmaceuticals Inc., United States Biological, as well as those produced by available technologies, *e.g.*, bacteriophage and bacterial display libraries made using standard procedures.

In yet other embodiments of the invention, Tf fusion proteins may be prepared by using therapeutic protein moieties known in the art and exemplified by the peptides and proteins currently approved by the Food and Drug Administration (www.fda.gov/cber/efoi/approve.htm) as well as PCT Patent Publication Nos. WO 01/79258, WO 01/77137, WO 01/79442, WO 01/79443, WO 01/79444 and WO 01/79480.

Table 1 from PCT International Publication No. WO 03/020746 provides a non-exhaustive list of therapeutic proteins that correspond to a therapeutic protein portion of a modified transferrin fusion protein of the invention. The "Therapeutic Protein X" column discloses therapeutic protein molecules followed by parentheses containing scientific and brand names that comprise or alternatively consist of that therapeutic protein molecule or a fragment or variant thereof. "Therapeutic protein X" as used herein may refer either to an individual therapeutic protein molecule (as defined by the amino acid sequence obtainable from the CAS and Genbank accession numbers), or to the entire group of therapeutic proteins associated with a given therapeutic protein molecule disclosed in this column. The 'Exemplary Identifier' column provides Chemical Abstracts Services (CAS) Registry Numbers (published by the American Chemical Society) and/or Genbank Accession Numbers (*e*.*g*., Locus ID, NP - XXXXX (Reference Sequence Protein), and XP-XXXXX (Model Protein) identifiers available through the National Center for Biotechnology Information (NCBI) webpage (www.ncbi.nlm.nih.gov) that correspond to entries in the CAS Registry or Genbank database which contain an amino acid sequence of the protein molecule or of a fragment or variant of the therapeutic protein molecule. In addition GenSeq Accession numbers and/or journal publication citations are given to identify the exemplary amino acid sequence for some polypeptides.

The summary pages associated with each of these CAS and Genbank and GenSeq Accession Numbers as well as the cited journal publications are available (*e*.*g*., PubMed ID number (PMID)). The PCT/Patent Reference column provides U. S. Patent numbers, or PCT International Publication Numbers corresponding to patents and/or published patent- applications that describe the therapeutic protein molecule. The Biological Activity column describes biological activities associated with the therapeutic protein molecule. The Exemplary Activity Assay column provides references that describe assays which may be used to test the therapeutic and/or biological activity of a therapeutic protein or a transferrin fusion protein of the invention comprising a therapeutic protein X portion. These references are also herein incorporated by reference in their entirety. "The Preferred Indication Y" column describes disease, disorders, and/or conditions that may be treated, prevented, diagnosed, or ameliorated by therapeutic protein X or a transferrin fusion protein of the invention comprising a therapeutic protein X portion.

### Delivery of a Drug or Therapeutic Protein to the inside of a Cell and/or across the Blood Brain Barrier (BBB)

The modified transferrin fusion proteins may be used as a carrier to deliver a molecule or small molecule therapeutic complexed to the ferric ion of transferrin to the inside of a cell or across the blood brain barrier or other barriers including across the cell membrane of any cell type that naturally or engineered to express a Tf receptor. In these embodiments, the Tf fusion protein will typically be engineered or modified to inhibit, prevent or remove glycosylation to extend the serum half-life of the fusion protein and/or therapeutic protein portion. The addition of a targeting peptide or, for example, a single chain antibody is specifically contemplated to further target the Tf fusion protein to a particular cell type, *e*.*g*., a cancer cell.

In one embodiment, the iron-containing, anti-anemic drug, ferric-sorbitol-citrate complex is loaded onto a modified Tf fusion protein of the invention. Ferric-sorbitol-citrate (FSC) has been shown to inhibit proliferation of various murine cancer cells *in vitro* and cause tumor regression *in vivo*, while not having any effect on proliferation of non-malignant cells (Poljak-Blazi et al. (June 2000) Cancer Biotherapy and Radiopharmaceuticals (United States), 15/3:285-293).

In another embodiment, the antineoplastic drug Adriamycin® (doxorubicin) and/or the chemotherapeutic drug bleomycin, both of which are known to form complexes with ferric ion, is loaded onto a Tf fusion protein of the invention. In other embodiments, a salt of a drug, for instance, a citrate or carbonate salt, may be prepared and complexed with the ferric iron that is then bound to Tf. As tumor cells often display a higher turnover rate for iron; transferrin modified to carry at least one anti-tumor agent, may provide a means of increasing agent exposure or load to the tumor cells. (Demant, E.J, (1983) Eur. J. Biochem. 137/(1-2):113-118*;* Padbury et al. (1985) J. Biol. Chem. 260/13:7820-7823).

### Pharmaceutical Formulations and Treatment Methods

The modified fusion proteins may be administered to a patient in need thereof using standard administration protocols. For instance, the modified Tf fusion proteins can be provided alone, or in combination, or in sequential combination with other agents that modulate a particular pathological process. As used herein, two agents are said to be administered in combination when the two agents are administered simultaneously or are administered independently in a fashion such that the agents will act at the same or near the same time.

The agents can be administered via parenteral, subcutaneous, intravenous, intramuscular, intraperitoneal, transdermal and buccal routes. For example, an agent may be administered locally to a site of injury via microinfusion. Alternatively, or concurrently, administration may be noninvasive by either the oral, inhalation, nasal, or pulmonary route. The dosage administered will be dependent upon the age, health, and weight of the recipient, kind of concurrent treatment, if any, frequency of treatment, and the nature of the effect desired.

The present application also describes compositions containing one or more trans-bodies of the invention. While individual needs vary, determination of optimal ranges of effective amounts of each component is within the skill of the art. Typical dosages comprise about 1 pg/kg to about 100 mg/kg body weight. The preferred dosages for systemic administration comprise about 100 ng/kg to about 100 mg/kg body weight. The preferred dosages for direct administration to a target site via microinfusion comprise about 1 ng/kg to about 1 mg/kg body weight. When administered via direct injection or microinfusion, modified fusion proteins may be engineered to exhibit reduced or no binding of iron to prevent, in part, localized iron toxicity.

In addition to the pharmacologically active fusion protein, the compositions may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension and include, for example, sodium carboxymethyl cellulose, sorbitol and dextran. Optionally, the suspension may also contain stabilizers. Liposomes can also be used to encapsulate the agent for delivery into the cell.

The pharmaceutical formulation for systemic administration may be formulated for enteral, parenteral or topical administration. Indeed, all three types of formulations may be used simultaneously to achieve systemic administration of the active ingredient. Suitable formulations for oral administration include hard or soft gelatin capsules, pills, tablets, including coated tablets, elixirs, suspensions, syrups or inhalations and controlled release forms thereof.

In practicing the treatment methods the agents may be used alone or in combination, or in combination with other therapeutic or diagnostic agents. In certain preferred embodiments, the compounds described herein may be co-administered along with other compounds typically prescribed for these conditions according to generally accepted medical practice. The compounds can be utilized *in vivo*, ordinarily in mammals, such as humans, sheep, horses, cattle, pigs, dogs, cats, rats and mice, *ex vivo* or *in vitro*.

Modified fusion proteins may be used in the diagnosis, prognosis, prevention and/or treatment of diseases and/or disorders relating to diseases and disorders of the endocrine system, the nervous system, the immune system, respiratory system, cardiovascular system, reproductive system, digestive system, diseases and/or disorders relating to cell proliferation, and/or diseases or disorders relating to the blood.

In yet other embodiments, modified Tf fusion proteins may be used in the diagnosis, prognosis, prevention and/or treatment of diseases and/or disorders relating to diseases and disorders known to be associated with or treatable by therapeutic protein moieties as known in the art and exemplified by PCT Patent Publication Nos. WO 01/79258, WO 01/77137, WO 01/79442, WO 01/79443, WO 01/79444 and WO 01/79480. Accordingly, the present application describes a method of treating a disease or disorder listed in the "Preferred Indication Y" column of Table 1 of WO 03/020746 comprising administering to a patient in which such treatment, prevention or amelioration is desired a modified transferrin fusion protein that comprises a therapeutic protein portion corresponding to a therapeutic protein disclosed in the "Therapeutic Protein X" column of Table 1 in an amount effective to treat, prevent or ameliorate the disease or disorder.

In certain embodiments, a transferrin fusion protein may be used to diagnose and/or prognose diseases and/or disorders.

Modified transferrin fusion proteins and polynucleotides encoding transferrin fusion proteins may be useful in treating, preventing, diagnosing and/or prognosing diseases, disorders, and/or conditions of the immune system. Moreover, fusion proteins and/or polynucleotides encoding transferrin fusion proteins can be used as a marker or detector of a particular immune system disease or disorder.

In a preferred embodiment, fusion proteins and/or polynucleotides encoding modified transferrin fusion proteins could be used as an agent to boost immunoresponsiveness among immunodeficient individuals. In specific embodiments, fusion proteins and/or polynucleotides encoding transferrin fusion proteins could be used as an agent to boost immunoresponsiveness among B cell and/or T cell immunodeficient individuals.

The modified transferrin fusion proteins and/or polynucleotides encoding transferrin fusion proteins may be useful in treating, preventing, diagnosing, and/or prognosing autoimmune disorders. Many autoimmune disorders result from inappropriate recognition of self as foreign material by immune cells. This inappropriate recognition results in an immune response leading to the destruction of the host tissue. Therefore, the administration of fusion proteins and/or polynucleotides encoding transferrin fusion proteins, that can inhibit an immune response, particularly the proliferation, differentiation, or chemotaxis of T-cells, may be an effective therapy in preventing autoimmune disorders.

Modified transferrin fusion proteins and/or polynucleotides encoding transferrin fusion proteins may be useful in treating, preventing, prognosing, and/or diagnosing diseases, disorders, and/or conditions of hematopoietic cells. Transferrin fusion proteins and/or polynucleotides encoding transferrin fusion proteins could be used to increase differentiation and proliferation of hematopoietic cells, including the pluripotent stem cells, in an effort to treat or prevent those diseases, disorders, and/or conditions associated with a decrease in certain (or many) types hematopoietic cells, including but not limited to, leukopenia, neutropenia, anemia, and thrombocytopenia.

Alternatively, modified fusion proteins and/or polynucleotides encoding transferrin fusion proteins could be used to increase differentiation and proliferation of hematopoietic cells, including the pluripotent stem cells, in an effort to treat or prevent those diseases, disorders, and/or conditions associated with an increase in certain (or many) types of hematopoietic cells, including but not limited to, histiocytosis.

Allergic reactions and conditions, such as asthma (particularly allergic asthma) or other respiratory problems, may also be treated, prevented, diagnosed and/or prognosing and using modified fusion proteins and/or polynucleotides encoding transferrin fusion proteins. Moreover, these molecules can be used to treat, prevent, prognose, and/or diagnose anaphylaxis, hypersensitivity to an antigenic molecule, or blood group incompatibility.

Additionally, modified fusion proteins and/or polynucleotides encoding transferrin fusion proteins, may be used to treat, prevent, diagnose and/or prognose IgE-mediated allergic reactions. Such allergic reactions include, but are not limited to, asthma, rhinitis, and eczema. In specific embodiments, fusion proteins and/or polynucleotides encoding transferrin fusion proteins may be used to modulate IgE concentrations in vitro or in vivo.

Moreover, modified fusion proteins and/or polynucleotides encoding transferrin fusion proteins have uses in the diagnosis, prognosis, prevention, and/or treatment of inflammatory conditions. For example, since fusion proteins and/or polynucleotides encoding transferrin fusion proteins may inhibit the activation, proliferation, and/or differentiation of cells involved in an inflammatory response, these molecules can be used to prevent and/or treat chronic and acute inflammatory conditions. Such inflammatory conditions include, but are not limited to, for example, inflammation associated with infection (*e.g*., septic shock, sepsis, or systemic inflammatory response syndrome), ischemia-reperfusion injury, endotoxin lethality, complement-mediated hyperacute rejection, nephritis, cytokine or chemokine induced lung injury, inflammatory bowel disease, Crohn's disease, over production of cytokines (*e.g*., TNF or IL-1), respiratory disorders (*e.g*., asthma and allergy); gastrointestinal disorders (*e.g*., inflammatory bowel disease); cancers (*e.g*., gastric, ovarian, lung, bladder, liver, and breast); CNS disorders (*e*.*g*., multiple sclerosis; ischemic brain injury and/or stroke, traumatic brain injury; neurodegenerative disorders (*e*.*g*., Parkinson's disease and Alzheizmer's disease); AIDS-related dementia; and prion disease); cardiovascular disorders (*e.g*., atherosclerosis, myocarditis, cardiovascular disease, and cardiopulmonary bypass complications); as well as many additional diseases, conditions, and disorders that are characterized by inflammation (*e.g*., hepatitis, rheumatoid arthritis, gout, trauma, pancreatitis, sarcoidosis, dermatitis, renal ischemia-reperfusion injury, Grave's disease, systemic lupus erythematosus, diabetes mellitus, and allogenic transplant rejection).

Because inflammation is a fundamental defense mechanism, inflammatory disorders can affect virtually any tissue of the body. Accordingly, modified fusion proteins and/or polynucleotides encoding transferrin fusion proteins, have uses in the treatment of tissue-specific inflammatory disorders, including, but not limited to, adrenalitis, alveolitis, angiocholecystitis, appendicitis, balanitis, blepharitis, bronchitis, bursitis, carditis, cellulitis, cervicitis, cholecystitis, chorditis, colitis, conjunctivitis, cystitis, dermatitis, diverticulitis, encephalitis, endocarditis, esophagitis, eustachitis, fibrositis, folliculitis, gastritis, gastroenteritis, gingivitis, glossitis, hepatosplenitis, keratitis, labyrinthitis, laryngitis, lymphangitis, mastitis, media otitis, meningitis, metritis, mucitis, myocarditis, myosititis, myringitis, nephritis, neuritis, orchitis, osteochondritis, otitis, pericarditis, peritendonitis, peritonitis, pharyngitis, phlebitis, poliomyelitis, prostatititis, Pulpitis, retinitis, rhinitis, salpingitis, scleritis, sclerochoroiditis, scrotitis, sinusitis, spondylitis, steatitis, stomatitis, synovitis, syringitis, tendonitis, tonsillitis, urethritis, and vaginitis.

In specific embodiments, modified fusion proteins and/or polynucleotides encoding transferrin fusion proteins, are useful to diagnose, prognose, prevent, and/or treat organ transplant rejections and graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. Polypeptides, antibodies, or polynucleotides, and/or agonists or antagonists thereof, that inhibit an immune response, particularly the activation, proliferation, differentiation, or chemotaxis of T-cells, may be an effective therapy in preventing organ rejection or GVHD.

In another specific embodiment, modified transferrin fusion proteins and/or polynucleotides encoding transferrin fusion proteins are used as an adjuvant to enhance anti-viral immune responses. Anti-viral immune responses that may be enhanced using the compositions described herein as an adjuvant, include virus and virus associated diseases or symptoms described herein or otherwise known in the art. In specific embodiments, the compositions are used as an adjuvant to enhance an immune response to a virus, disease, or symptom selected from the group consisting of AIDS, meningitis, Dengue, EBV, and hepatitis (*e.g*., hepatitis B). In another specific embodiment, the compositions are used as an adjuvant to enhance an immune response to a virus, disease, or symptom selected from the group consisting of: HIV/AIDS, respiratory syncytial virus, Dengue, rotavirus, Japanese B encephalitis, influenza A and B, parainfluenza, measles, cytomegalovirus, rabies, Junin, Chikungunya, Rift Valley Fever, herpes simplex, and yellow fever.

Modified transferrin fusion proteins and/or polynucleotides encoding transferrin fusion proteins may also be used as an adjuvant to enhance anti-bacterial or anti-fungal immune responses. Anti-bacterial or anti-fungal immune responses that may be enhanced using the compositions described herein as an adjuvant include bacteria or fungus and bacteria or fungus associated diseases or symptoms described herein or otherwise known in the art. In specific embodiments, the compositions are used as an adjuvant to enhance an immune response to a bacterium or fungus disease, or symptom selected from the group consisting of tetanus, Diphtheria, botulism, meningitis type B, and candidiasis.

In another specific embodiment, the compositions are used as an adjuvant to enhance an immune response to a bacterium or fungus, disease, or symptom selected from the group consisting of *Vibrio cholerae*, *Mycobacterium leprae*, *Salmonella typhi*, *Salmonella paratyphi*, *Neisseria meningitidis*, *Streptococcus pneumoniae*, Group B *streptococcus*, *Shigella spp.,* Enterotoxigenic *Escherichia coli*, Enterohemorrhagic *E*. *coli*, *Borrelia burgdorferi*, and *Candida.*

Modified transferrin fusion proteins and/or polynucleotides encoding transferrin fusion proteins may also be used as an adjuvant to enhance anti-parasitic immune responses. Anti-parasitic immune responses that may be enhanced using the compositions as an adjuvant include parasite and parasite associated diseases or symptoms described herein or otherwise known in the art. The compositions described herein may also be used as an adjuvant to enhance an immune response to a parasite. The compositions described herein may also be used as an, adjuvant to enhance an immune response to Plasmodium (malaria) or Leishmania.

Modified transferrin fusion proteins and/or polynucleotides encoding transferrin fusion proteins may also be employed to treat infectious diseases including silicosis, sarcoidosis, and idiopathic pulmonary fibrosis; for example, by preventing the recruitment and activation of mononuclear phagocytes.

Modified transferrin fusion proteins and/or polynucleotides encoding transferrin fusion proteins may also be used as an antigen for the generation of antibodies to inhibit or enhance immune mediated responses against polypeptides of the invention.

Modified transferrin fusion proteins and/or polynucleotides encoding transferrin fusion proteins may be administered to an animal (*e.g*., mouse, rat, rabbit, hamster, guinea pig, pigs, micro-pig, chicken, camel, goat, horse, cow, sheep, dog, cat non-human primate, and human, most preferably human) to boost the immune system to produce increased quantities, of one or more antibodies (e.g., IgG, IgA, IgM, and IgE), to induce higher affinity antibody production and immunoglobulin class switching (*e.g*., IgG, IgA, IgM, and IgE), and/or to increase an immune response.

Modified transferrin fusion proteins and/or polynucleotides encoding transferrin fusion proteins may be used in one or more of the applications described herein, as they may apply to veterinary medicine.

Modified transferrin fusion proteins and/or polynucleotides encoding transferrin fusion proteins may be used as a means of blocking various aspects of immune responses to foreign agents or self. Examples of diseases or conditions in which blocking of certain aspects of immune responses may be desired include autoimmune disorders such as lupus, and arthritis, as well as immunoresponsiveness to skin allergies, inflammation, bowel disease, injury, and diseases/disorders associated with pathogens.

Modified transferrin fusion proteins and/or polynucleotides encoding transferrin fusion proteins may be used as a therapy for preventing the B cell proliferation and Ig secretion associated with autoimmune diseases such as idiopathic thrombocytopenic purpura, systemic lupus erythematosus and multiple sclerosis.

Modified transferrin fusion proteins or polynucleotides encoding transferrin fusion proteins may be used as an inhibitor of B and/or T cell activation in endothelial cells. This activity disrupts tissue architecture or cognate responses and is useful, for example in disrupting immune responses, and blocking sepsis.

Modified transferrin fusion proteins and/or polynucleotides encoding transferrin fusion proteins may be used as a therapy for chronic hypergammaglobulinemia evident in such diseases as monoclonal gammopathy of undetermined significance (MGUS), Waldenstrom's disease, related idiopathic monoclonal gammopathies, and plasmacytomas.

Modified transferrin fusion proteins and/or polynucleotides encoding transferrin fusion proteins may be employed for instance to inhibit polypeptide chemotaxis and activation of macrophages and their precursors, and of neutrophils, basophils, B lymphocytes and some T-cell subsets, *e.g*., activated and CD8 cytotoxic T cells and natural killer cells, in certain autoimmune and chronic inflammatory and infective diseases. Examples of autoimmune diseases are described herein and include multiple sclerosis, and insulin-dependent diabetes.

Modified transferrin fusion proteins and/or polynucleotides encoding transferrin fusion protein may also be employed for treating atherosclerosis, for example, by preventing monocyte infiltration in the artery wall.

Modified transferrin fusion proteins and/or-polynucleotides encoding transferrin fusion proteins may be employed to treat adult respiratory distress syndrome (ARDS).

Modified transferrin fusion proteins and/or polynucleotides encoding transferrin fusion proteins may be useful for stimulating wound and tissue repair, stimulating angiogenesis, and/or stimulating the repair of vascular or lymphatic diseases or disorders. Additionally, fusion proteins and/or polynucleotides encoding transferrin fusion proteins may be used to stimulate the regeneration of mucosal surfaces.

Modified transferrin fusion proteins and/or polynucleotides encoding transferrin fusion proteins may be used to diagnose, prognose, treat, and/or prevent a disorder characterized by primary or acquired immunodeficiency, deficient serum immunoglobulin production, recurrent infections, and/or immune system dysfunction. Moreover, modified fusion proteins and/or polynucleotides encoding transferrin fusion proteins may be used to treat or prevent infections of the joints, bones, skin, and/or parotid glands, blood-borne infections (*e.g*., sepsis, meningitis, septic arthritis, and/or osteomyelitis), autoimmune diseases (*e.g*., those disclosed herein), inflammatory disorders, and malignancies, and/or any disease or disorder or condition associated with these infections, diseases, disorders and/or malignancies) including, but not limited to, Common Variable Immunodeficiency (CVID), other primary immune deficiencies, HIV disease, Chronic Lymphocytic Leukemia (CLL), recurrent bronchitis, sinusitis, otitis media, conjunctivitis, pneumonia, hepatitis, meningitis, herpes zoster (*e.g*., severe herpes zoster), and/or pneumocystis carnii. Other diseases and disorders that may be prevented, diagnosed, prognosed, and/or treated with fusion proteins and/or polynucleotides encoding transferrin fusion proteins include, but are not limited to, HIV infection, HTLV-BLV infection, lymphopenia, phagocyte bactericidal dysfunction anemia, thrombocytopenia, and hemoglobinuria.

Modified transferrin fusion proteins and/or polynucleotides encoding transferrin fusion proteins may be used to diagnose, prognose, prevent, and/or treat cancers or neoplasms including immune cell or immune tissue-related cancers or neoplasms. Examples of cancers or neoplasms that may be prevented, diagnosed, or treated by fusion proteins and/or polynucleotides encoding transferrin fusion proteins include, but are not limited to, acute myelogenous leukemia, chronic myelogenous leukemia, Hodgkin's disease, non-Hodgkin's lymphoma, acute lymphocytic leukemia (ALL), chronic lymphocyte leukemia, plasmacytomas, multiple myeloma, Burkitt's lymphoma, EBV transformed diseases, and/or diseases and disorders described in the section entitled "Hyperproliferative Disorders" elsewhere herein.

Modified transferrin fusion proteins and/or polynucleotides encoding transferrin fusion proteins may be used as a therapy for decreasing cellular proliferation of Large B-cell Lymphomas.

The compositions described herein may be used as an agent to boost immunoresponsiveness among B cell immunodeficient individuals, such as, for example, an individual who has undergone a partial or complete splenectomy.

The modified transferrin fusion proteins and/or polynucleotides encoding transferrin fusion proteins may be used to modulate hemostatic (the stopping of bleeding) or thrombolytic (clot dissolving) activity. For example, by increasing hemostatic or thrombolytic activity, fusion proteins and/or polynucleotides encoding transferrin fusion proteins could be used to treat or prevent blood coagulation diseases, disorders, and/or conditions (*e.g*., afibrinogenemia, factor deficiencies, hemophilia), blood platelet diseases, disorders, and/or conditions (*e.g*. thrombocytopenia), or wounds resulting from trauma, surgery, or other causes. Alternatively, fusion proteins and/or polynucleotides encoding transferrin fusion proteins that can decrease hemostatic or thrombolytic activity could be used to inhibit or dissolve clotting. These molecules could be important in the treatment or prevention of heart attacks (infarction), strokes, or scarring.

The modified transferrin fusion proteins and/or polynucleotides encoding transferrin fusion proteins may be used to prevent diagnose, prognose, and/or treat thrombosis, arterial thrombosis, venous thrombosis, thromboembolism, pulmonary embolism, atherosclerosis, myocardial infarction, transient ischemic attack, unstable angina. The transferrin fusion proteins and/or polynucleotides encoding transferrin fusion proteins may be used for the prevention of occulsion of saphenous grafts, for reducing the risk of periprocedural thrombosis as might accompany angioplasty procedures, for reducing the risk of stroke in patients with atrial fibrillation including nonrheumatic atria fibrillation, for reducing the risk of embolism associated with mechanical heart valves and/or mitral valves disease. Other uses for the modified transferrin fusion proteins and/or polynucleotides encoding transferrin fusion proteins, include, but are not limited to, the prevention of occlusions in extracorporeal devices (*e*.*g*., intravascular canals, vascular access shunts in hemodialysis patients, hemodialysis machines, and cardiopulmonary bypass machines).

Modified transferrin fusion proteins and/or polynucleotides encoding transferrin fusion proteins may be used to prevent, diagnose, prognose, and/or treat diseases and disorders of the blood and/or blood forming organs associated with the tissue(s) in which the polypeptide is expressed.

The modified fusion proteins and/or polynucleotides encoding transferrin fusion proteins may be used to modulate hematopoietic activity (the formation of blood cells). For example, the transferrin fusion proteins and/or polynucleotides encoding transferrin fusion proteins may be used to increase the quantity of all or subsets of blood cells, such as, for example, erythrocytes, lymphocytes (B or T cells), myeloid cells (*e*.*g*., basophils, eosinophils, neutrophils, mast cells, macrophages) and platelets. The ability to decrease the quantity of blood cells or subsets of blood cells may be useful in the prevention, detection, diagnosis, and/or treatment of anemias and leukopenias described below. Alternatively, the modified transferrin fusion proteins and/or polynucleotides encoding transferrin fusion proteins may be used to decrease the quantity of all or subsets of blood cells, such as, for example, erythrocytes, lymphocytes (B or T cells), myeloid cells (*e*.*g*., basophils, eosinophils, neutrophils, mast cells, macrophages) and platelets. The ability to decrease the quantity of blood cells or subsets of blood cells may be useful in the prevention, detection, diagnosis, and/or treatment of leukocytoses, such as, for example eosinophilia. The modified fusion proteins and/or polynucleotides encoding transferrin fusion proteins may be used to prevent, treat, or diagnose blood dyscrasia.

Anemias are conditions in which the number of red blood cells or amount of hemoglobin (the protein that carries oxygen) in them is below normal. Anemia may be caused by excessive bleeding, decreased red blood cell production, or increased red blood cell destruction (hemolysis). The modified transferrin fusion proteins and/or polynucleotides encoding transferrin fusion proteins may be useful in treating, preventing, and/or diagnosing anemias. Anemias that may be treated prevented or diagnosed by the transferrin fusion proteins of the invention and/or polynucleotides encoding transferrin fusion proteins include iron deficiency anemia, hypochromic anemia, microcytic anemia, chlorosis, hereditary sideroblastic anemia, idiopathic acquired sideroblastic anemia, red cell aplasia, megaloblastic anemia (*e*.*g*., pernicious anemia, (vitamin B12 deficiency) and folic acid deficiency anemia), aplastic anemia, hemolytic anemias (*e*.*g*., autoimmunune hemolytic anemia, microangiopathic hemolytic anemia, and paroxysmal nocturnal hemoglobinuria). The modified transferrin fusion proteins and/or polynucleotides encoding transferrin fusion proteins may be useful in treating, preventing, and/or diagnosing anemias associated with diseases including but not limited to, anemias associated with systemic lupus erythematosus, cancers, lymphomas, chronic renal disease, and enlarged spleen. The transferrin fusion proteins and/or polynucleotides encoding transferrin fusion proteins may be useful in treating, preventing, and/or diagnosing anemia arising from drug treatments such as anemias associated with methyldopa, dapsone, and/or sulfa drugs. Additionally, modified fusion proteins and/or polynucleotides encoding transferrin fusion proteins may be useful in treating, preventing, and/or diagnosing anemias associated with abnormal red blood cell architecture including but not limited to, hereditary spherocytosis, hereditary elliptocytosis, glucose-6-phosphate dehydrogenase deficiency, and sickle cell anemia.

The modified transferrin fusion proteins and/or polynucleotides encoding transferrin fusion proteins may be useful in treating, preventing, and/or diagnosing hemoglobin abnormalities, (*e*.*g*., those associated with sickle cell anemia, hemoglobin C disease, hemoglobin S-C disease, and hemoglobin E disease). Additionally, the transferrin fusion proteins and/or polynucleotides encoding transferrin fusion proteins may be useful in diagnosing, preventing, and/or prognosing in treating thalassemias, including, but not limited to, major and minor forms of alpha-thalassemia and beta-thalassemia.

The modified transferrin fusion proteins and/or polynucleotides encoding transferrin fusion proteins may be useful in diagnosing, prognosing, preventing, and/or treating bleeding disorders including, but not limited to, thrombocytopenia (*e*.*g*., idiopathic thrombocytopenic purpura, and thrombotic thrombocytopenic purpura), Von Willebrand's disease, hereditary platelet disorders (*e*.*g*., storage pool disease such as Chediak-Higashi and Hermansky-Pudlak syndromes, thromboxane A2 dysfunction, thromboasthenia, and Bernard-Soulier syndrome), hemolyticuremic syndrome, hemophilias such as hemophilia A or Factor V11 deficiency and Christmas disease or Factor IX deficiency, Hereditary Hemorhhagic Telangiectsia, also known as Rendu-Osler-Webe syndrome, allergic purpura (Henoch Schonlein purpura) and disseminated intravascular coagulation.

the modified transferrin fusion proteins and/or polynucleotides encoding transferrin fusion proteins may be useful as an agent to increase cytokine production.

Fusion proteins, and/or polynucleotides encoding transferrin fusion proteins can be used to treat or detect hyperproliferative disorders, including neoplasms. Transferrin fusion proteins and/or polynucleotides encoding transferrin fusion proteins may inhibit the proliferation of the disorder through direct or indirect interactions. Alternatively, fusion proteins and/or polynucleotides encoding transferrin fusion proteins may cause proliferation of other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by modified fusion proteins and/or polynucleotides encoding transferrin fusion proteins include, but are not limited to neoplasms located in the colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvis, skin, soft tissue, spleen, thorax, and urogenital tract.

Similarly, other hyperproliferative disorders can also be treated or detected by modified fusion proteins and/or polynucleotides encoding transferrin fusion proteins. Examples of such hyperproliferative disorders include, but are not limited to Acute Childhood Lymphoblastic Leukemia; Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Disease, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma. Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Disease, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lympho proliferative Disorders, Macroglobulinemia, Male Breast Cancer, Malignant Mesothelioma, Malignant Thymoma, Medulloblastomia, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma During Pregnancy, Nonmelanoma Skin Cancer, Non-Small Cell Lung Cancer, Occult Primary Metastatic Squamous Neck Cancer, Oropharyngeal Cancer, Osteo/Malignant Fibrous Sarcoma,Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Purpura, Parathyroid, Cancer, Penile Cancer, Pheochromocytoma, Pituitary Tumor, Plasma Cell Neoplasm/Multiple Myeloma, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilm's Tumor, and any other hyperproliferative disease, besides neoplasia, located in an organ system listed above.

Modified transferrin fusion proteins and/or polynucleotides encoding transferrin fusion proteins may be used to diagnose, prognose, prevent, and/or treat premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth is consisting of hyperplasia, metaplasia, or most particularly, dysplasia has occurred (for review of such abnormal growth conditions, see Robbins. and Angell, 1976, Basic Pathology, 2d Ed. W. B. Saunders Co., Philadelphia, pp. 68-79).

Hyperplasia is a form of controlled cell proliferation, involving an increase in cell number in a tissue or organ, without significant alteration in structure or function. Hyperplastic disorders which can be diagnosed, prognosed, prevented, and/or treated with fusion proteins and/or polynucleotides encoding transferrin fusion proteins include, but are not limited to, angiofollicular mediastinal lymph node hyperplasia, angiolymphoid hyperplasia with eosinophilia, atypical melanocytic hyperplasia, basal cell hyperplasia, benign giant lymph node hyperplasia, cementum hyperplasia, congenital adrenal hyperplasia, congenital sebaceous hyperplasia, cystic hyperplasia, cystic hyperplasia of the breast, denture hyperplasia, ductal hyperplasia, endometrial hyperplasia, fibromuscular hyperplasia, foca epithelial hyperplasia, gingival hyperplasia, inflammatory fibrous hyperplasia, inflammatory papillary hyperplasia, intravascular papillary endothelial hyperplasia, nodular hyperplasia of prostate, nodular regenerative hyperplasia, pseudoepitheliomatous hyperplasia, senile sebaceous hyperplasia, and verrucous hyperplasia.

Modified transferrin fusion proteins and/or polynucleotides encoding transferrin fusion proteins conjugated to a toxin or a radio-active isotope, as described herein, may be used to treat cancers and neoplasms, including, but not limited to, those described herein. Transferrin fusion proteins and/or polynucleotides encoding transferrin fusion proteins conjugated to a toxin or a radioactive isotope, as described herein, may also be used to treat acute myelogenous leukemia.

Additionally, modified fusion proteins and/or polynucleotides encoding transferrin fusion proteins may affect apoptosis, and therefore, would be useful in treating a number of diseases associated with increased cell survival or the inhibition of apoptosis. For example, diseases associated with increased cell survival or the inhibition of apoptosis that could be diagnosed, prognosed, prevented, and/or treated by polynucleotides, polypeptides, and/or agonists or antagonists as described herein, include cancers (such as follicular- lymphomas, carcinomas with p53 mutations, and hormone-dependent tumors, including, but not limited to, colon cancer, cardiac tumors, pancreatic cancer, melanoma, retinoblastoma, glioblastoma, lung cancer, intestinal cancer, testicular cancer, stomach cancer, neuroblastoma, myxoma, myoma, lymphoma, endothelioma, osteoblastoma, osteoclastoma, osteosarcoma, chondrosarcoma, adenoma, breast cancer, prostrate cancer, Kaposi's sarcoma and ovarian cancer); autoimmune disorders such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) and viral infections (such as herpes viruses, pox viruses and adenoviruses), inflammation, graft v. host disease, acute graft rejection, and chronic graft rejection.

Modified fusion proteins and/or polynucleotides encoding transferrin fusion proteins may be used to inhibit growth, progression, and/or metastasis of cancers, in particular those listed above.

Additional diseases or conditions associated with increased cell survival that could be diagnosed, prognosed, prevented, and/or treated by modified fusion proteins and/or polynucleotides encoding transferrin fusion proteins include but are not limited to, progression and/or metastases of malignancies and related disorders such as leukemia (including acute leukemia (*e*.*g*., acute lymphocytic leukemia, acute myelocytic leukemia (including myeloblastic, promyelocytic, mylomonocytic, monocytic, and erythroleukemia)) and chronic leukemia (*e*.*g*., chronic myelocytic (granulocytic) leukemia and chronic lymphocytic leukemia)), polycythemia vera, lymphomas (*e*.*g*., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, fiposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendrogliomia, menangioma, melanoma, neuroblastoma, and retinoblastoma.

Diseases associated with increased apoptosis that could be diagnosed, prognosed, prevented, and/or treated by modified fusion proteins and/or polynucleotides encoding transferrin fusion proteins, include AIDS; neurodegenerative disorders (such as Alzheimer's disease, Parkinson's disease, amyotrophic lateral sclerosis, retinitis pigmentosa, cerebral degeneration and brain tumor or prion associated disease); autoimmune disorders (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) myelodysplastic syndromes (such as aplastic anemia), graft Y host disease, ischemic injury (such as that caused by myocardial infarction, stroke and repercussion injury), liver injury (*e*.*g*., hepatitis related liver injury, ischemia/reperfusion injury, cholestosis (bile duct injury) and liver cancer); toxin-induced liver disease (such as that caused by alcohol), septic shock, cachexia and anorexia.

polynucleotides encoding modified transferrin fusion proteins can also be used to inhibit aberrant cellular division, by gene therapy, and/or protein fusions or fragments thereof.

The polynucleotides described herein may be delivered directly to cell proliferative disorderly disease sites in internal organs, body cavities, and the like by use of imaging devices used to guide an injecting needle directly to the disease site. The polynucleotides may also be administered to disease sites at the time of surgical intervention.

By cell proliferative disease is meant any human or animal disease or disorder, affecting any one or any combination of organs, cavities, or body parts, which is characterized by single or multiple local abnormal proliferations of cells, groups of cells, or tissues, whether benign or malignant.

Any amount of the polynucleotides may be administered as long as it has a biologically inhibiting effect on the proliferation of the treated cells.

Moreover, it is possible to administer more than one of the polynucleotides simultaneously to the same site. By "biologically inhibiting" is meant partial or total growth inhibition as well as decreases in the rate of proliferation or growth of the cells.

Modified transferrin fusion proteins and/or polynucleotides encoding transferrin fusion proteins are useful in inhibiting the metastasis of proliferative cells or tissues. Inhibition may occur as a direct result of administering these transferrin fusion proteins and/or polynucleotides, or indirectly, such as activating the expression of proteins known to inhibit metastasis, for example alpha, integrins, (See, *e*.*g*., Curr. Top. Mirobiol. Immunol. 1998; 231:1 41). Such therapeutic affects of the present invention may be achieved either alone, or in combination with small molecule drugs or adjuvants.

The present application also describes a method of delivering compositions containing the transferrin fusion proteins and/or polynucleotides encoding transferrin fusion proteins to targeted cells expressing a polypeptide bound by, that binds to, or associates with a modified transferrin fusion protein. Transferrin fusion proteins may be associated with heterologous polypeptides, heterologous nucleic acids, toxins, or prodrugs via hydrophobic, hydrophilic, ionic and/or covalent interactions.

Kidney diseases which can be diagnosed, prognosed, prevented, and/or treated with compositions described herein include, but are not limited to, acute kidney failure, chronic kidney failure, atheroembolic renal failure, end-stage renal disease, inflammatory diseases of the kidney (*e*.*g*., acute glomerulonephritis, post infectious glomerulonephritis, rapidly progressive glomerulonephritis, nephritic syndrome, membranous glomerulonephritis, familial nephritic syndrome, membrane proliferative glomerulonephritis and mesangial proliferative glomerulonephritis, chronic glomerulonephritis, acute tubulo-interstitial nephritis, chronic tubulointerstitial nephritis, acute post-streptococcal glomerulonephritis(PSGN), pyelonephritis, lupus nephritis, chronic nephritis, interstitial nephritis, and post streptococcal glomerulonephritis), blood vessel disorders of the kidneys (*e*.*g*., kidney infarction, atheroembolic kidney disease, cortical necrosis, malignant nephrosclerosis, renal vein thrombosis, renal under perfusion, renal retinopathy, renal ischemia-reperfusion, renal artery embolism and renal artery stenosis), and kidney disorders resulting form urinary tract disease (*e*.*g*., pyelonephritis, hydronephrosis, urolithiasis (renal lithiasis, nephrolithiasis), reflux nephropathy, urinary tract infections, urinary retention, and acute or chronic unilateral obstructive uropathy). In addition, compositions described herein can be used to diagnose, prognose, prevent, and/or treat metabolic and congenital disorders of the kidney (*e*.*g*., uremia, renal amyloidosis, renal osteodystrophy, renal tubular acidosis, renal glycosuria, nephrogenic diabetes insipidus, cystinuria, Fanconi's syndrome, renal fibrocystic osteosis (renal rickets), Hartnup disease, Bartter's syndrome, Liddle's syndrome, polycystic kidney disease, medullary cystic disease, medullary sponge kidney, Alport's syndrome, nail-patella syndrome, congenital nephritic syndrome, CRUSH syndrome, horseshoe kidney, diabetic nephropathy, nephrogenic diabetes insipidus, analgesic nephropathy, kidney stones, and membranous nephropathy), and autoimmune disorders of the kidney (*e*.*g*., systemic lupus erythematosus (SLE), Good pasture syndrome, IgA nephropathy, and ICFM mesangial proliferative glomerulonephritis).

Compositions described herein can also be used to diagnose, prognose, prevent, and/or treat sclerotic or lecrotic disorders of the kidney (*e*.*g*., glomerulosclerosis, diabetic nephropathy, focal Fsegmental glomerulo sclerosis (FSGS), narcotizing glomerulonephritis, and renal papillary necrosis), cancers of the kidney (*e*.*g*., nephroma, hypernephroma, nephroblastoma, renal cell cancer, transitional cell cancer, renal adenocarcinoma, squamous cell cancer, and Wilm's tumor), and electrolyte imbalances (*e*.*g*., nephrocalcinosis, pyuria, edema, hydronephritis, proteinuria, hyponatremia, hypernatremia, hypokalemia, hyperkalemia, hypocalcemia, hypercalcemia, hypophosphatemia, and hyperphosphatemia).

Compositions described herein may be administered using any method known in the art, including, but not limited to, direct needle injection at the delivery site, intravenous injection, topical administration, catheter infusion, biolistic injectors, particle accelerators, gel foam sponge depots, other commercially available depot materials, osmotic pumps, oral or suppositorial solid pharmaceutical formulations, decanting or topical applications during surgery, aerosol delivery. Such methods are known in the art. Compositions described herein may be administered as part of a Therapeutic, described in more detail below.

Modified transferrin fusion proteins and/or polynucleotides encoding transferrin fusion proteins, may be used to treat, prevent, diagnose, and/or prognose cardiovascular disorders, including, but not limited to, peripheral artery disease, such as limb ischemia.

Cardiovascular disorders, includes, but is not limited to, cardiovascular abnormalities, such as arterio arterial fistula, arteriovenous fistula, cerebral arteriovenous malformations, congenital heart defects, pulmonary atresia, and Scimitar Syndrome.

Congenital heart defects include, but are not limited to, aortic coarctation, cortriatriatum, coronary vessel anomalies, crisscross heart, dextrocardia, patent ductus arteriosus, Ebstein's anomaly, Eisenmenger complex, hypoplastic left heart syndrome, levocardia, tetralogy of fallot, transposition of great vessels, double outlet right ventricle, tricuspidatresia, persistent truncus arteriosus, and heart septal defects, such as aortopulmonary septald defect, endocardial cushion defects, Lutembacher's Syndrome, trilogy of Fallot, ventricular heart septal defects.

Cardiovascular disorders also include, but are not limited to, heart disease, such arrhythmias, carcinoid heart disease, high cardiac output, low cardiac output, cardiactamponade, endocarditis (including bacteria), heart aneurysm, cardiac arrest, congestive heart failure, congestive cardiomyopathy, paroxysmal dyspnea, cardiac edema, heart hypertrophy, congestive cardiomyopathy left ventricular hypertrophy, right ventricularhypertrophy, post-infarction heart rupture, ventricular septal rupture, heart valve diseases myocardial diseases, myocardial ischemia, pericardial effusion, pericarditis (including constrictive and tuberculous), pricumopericardium, post pericardiotomy syndrome, pulmonary heart disease, rheumatic heart disease, ventricular dysfunction, hyperemia, cardiovascular pregnancy complications, Scimitar Syndrome, cardiovascular syphilis, and cardiovascular tuberculosis.

Arrhythmias include, but are not limited to, sinus arrhythmia, atrial fibrillation, atrial flutter, bradycardia, extrasystole, Adams-Stokes Syndrome, bundle-branch block, sinoatrial block, long QT syndrome, parasystole, Lown-Ganong-Levine Syndrome, Mahaim-type pre-excitation syndrome, Wolff-Parkinson-White syndrome, sick sinus syndrome, itachycardias, and ventricular fibrillation. Tachycardias include paroxysmal tachycardia, suprayentriculai tachycardia, accelerated idioventricular rhythm, atrioventricular nodal reentry tachyeardia, ectopic atrial tachycardia, ectopic junctional tachycardia, sinoattial nodalreentry tachycardia, sinus tachycardia, Torsades de Pointes, and ventricular tachycardia.

Heart valve diseases include, but are not limited to, aortic valve insufficiency aortic valve stenosis, heart murmurs, aortic valve prolapse, neutral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis.

Myocardial diseases include, but are not limited to, alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, endomyocardial fibrosis, Kearns Syndrome, myocardial reperfusion injury, and myocarditis.

Myocardial ischemias include, but are not limited to, coronary disease, such as angina pectoris, coronary aneurysm, coronary arteriosclerosis, coronary thrombosis, coronary vasospasm, myocardial infarction, and myocardial stunning.

Cardiovascular diseases also include vascular diseases such as aneurysms, angiodysplasia, angiomatosis, bacillary angiomiatosis, Hippel-Lindau Disease, Klippel Trenaunay Weber Syndrome, Sturge Weber Syndrome, angioneurotic edema, aortic diseases, Takayasu's Arthritis, aortitis, Leriche's Syndrome, arterial occlusive diseases, arthritis, enarteritis, polyarteritis nodosa, cerebrovascular disorders, diabetic angiopathies, diabetic retinopathy, embolisms, thrombosis, erythromelalgia, hemorrhoids, hepatic veno-occlusive disease, hypertension, hypotension, ischemia, peripheral vascular diseases, phlebitis, pulmonary veno-occlusive disease, Raynaud's disease, CREST syndrome, retinal vein occlusion, Scimitar syndrome, superior vena cava syndrome, telangiectasia, ataxia telangiectasia, hereditary hemorrhagic telangiectasia, varicocele, varicose veins, varicoseulcer, vasculitis, and venous insufficiency.

Cerebrovascular disorders include, but are not limited to, cardio artery diseases but includes respiratory disorders. Transferrin fusion proteins of the invention and/or polynucleotides encoding transferrin fusion proteins of the invention may be used to treat, prevent, diagnose, and/or prognose diseases and/or disorders of the respiratory system.

Diseases and disorders of the respiratory system include, but are not limited to, nasalvestibulitis, nonallergic rhinitis (*e*.*g*., acute rhinitis, chronic rhinitis, atrophic rhinitis, vasomotor rhinitis), nasal polyps, and sinusitis, juvenile angiofibromas, cancer of the nose and juvenile papillomas, vocal cord polyps, nodules (singer's nodules), contact ulcers, vocal cord paralysis, laryngoceles, pharyngitis (*e*.*g*., viral and bacterial), tonsillitis, tonsillar cellulitis, parapharyngeal abscess, laryngitis, laryngoceles, and throat cancers (*e*.*g*., cancer of the nasopharynx, tonsil cancer, larynx cancer), lung cancer (*e*.*g*., squamous cell carcinoma, small cell (oat cell) carcinoma, large cell carcinoma, and adenocarcinoma), allergic disorders (eosinophilic pneumonia, hypersensitivity pneumonitis (*e*.*g*., extrinsicallergic alveolitis, allergic interstitial pneumonitis, organic dust pneumoconiosis, allergic bronchopulmoniary aspergillosis, asthma, Wegener's granulomatosis (granulomatous vasculitis), Goodpasture's syndrome), pneumonia (*e*.*g*., bacterial pneumonia (*e*.*g*., Streptococcus pneumoniae (pneumoncoccal pneumonia), Staphylococcus aureus (staphylococeal pneumonia), Gram negative bacteria pneumonia (caused by, *e*.*g*., *Klebsiella* and *Pseudomonas* spp.), Mycoplasma pneumoniae pneumonia, Hemophilus influenza pneumonia, Legionella pneumophila (Legionnaires' disease), and *Chlamydia psittaci* (Psittacosis)), and viral pneumonia (*e*.*g*., influenza, chickenpox (varicella).

Additional diseases and disorders of the respiratory system include, but are not limited to bronchiolitis, polio (poliomyelitis), croup, respiratory syncytial viral infection, mumps, erythema infectiosum (fifth disease), roseola infantum, progressive rubellapanencephalitis, German measles, and subacute sclerosing panencephalitis), fungal pneumonia (*e*.*g*., Histoplasmosis, Coccidioidomycosis, Blastomycosis, fungal infections in people with severely suppressed immune systems (*e*.*g*., cryptococcosis, caused by *Cryptococcus neoformans*; aspergillosis, caused by *Aspergillus* spp.) candidiasis, caused by Candida; and mucormycosis)), *Pneumocystis carinii* (pneumocystis pneumonia), atypicalpneumonias (*e*.*g*., *Mycoplasma* and *Chlamydia* spp.), opportunistic infection pneumonia, nosocomial pneumonia, chemical pneumonitis, and aspiration pneumonia, pleural disorders (*e*.*g*., pleurisy, pleural effusion, and pneumothorax (*e*.*g*., simple spontaneous pneumothorax, complicated spontaneous pneumothorax, tension pneumothorax)),obstructive airway diseases (*e*.*g*., asthma,. chronic obstructive pulmonary disease (COPID),emphysema, chronic or acute bronchitis), occupational lung diseases (*e*.*g*., silicosis, blacklung (coal workers' pneumoconiosis, asbestosis, berylliosis, occupational asthma, and byssinosis), Infiltrative Lung Disease (*e*.*g*., pulmonary fibrosis (*e*.*g*., usual interstitial pneumonia), idiopathic pulmonary fibrosis, desquamative interstitial pneumonia, lymphoid interstitial pneumonia, histiocytosis (*e*.*g*., Letterer-Siwe disease, Hand-Schüller-Christian disease, eosinophilic granuloma), idiopathic pulmonary hemosiderosis, sarcoidosis and pulmonary, alveolar proteinosis), Acute respiratory distress syndrome (also called, *e*.*g*., adult respiratory distress syndrome), edema, pulmonary embolism, bronchitis (*e*.*g*., viral, bacterial), bronchiectasis, atelectasis, lung abscess (caused by, *e*.*g*., Staphylococcus aureus or Legionella pneumophila), and cystic fibrosis.

Cancers which may be treated with modified fusion proteins and/or polynucleotides encoding transferrin fusion proteins include, but are not limited to solid tumors, including prostate, lung, breast, ovarian, stomach, pancreas, larynx, esophagus, liver, parotid, biliary tract, colon, rectum, cervix, uterus, endometrium, kidney, bladder, thyroid cancer; primary tumors and metastases; melanomas; glioblastoma; Kaposi's sarcoma; leiomyosarcoma; non- small cell lung cancer; colorectal cancer; advanced malignancies; and blood bom tumors such as leukemia. For example, fusion proteins and/or polynucleotides encoding transferrin fusion proteins may be delivered topically, in order to treat cancers such as skin cancer, head and neck tumors, breast tumors, and Kaposi's sarcoma.

Modified transferrin fusion proteins and/or polynucleotides encoding transferrin fusion proteins may be useful, in treating other disorders, besides cancers, which involve angiogenesis. These disorders include, but are not limited to: benign tumors, for example hemangiomas, acoustic neuromas, neurofibromas, trachomas, and pyogenicgranulomas; artheroscleric plaques; ocular angiogenic diseases, for example, diabetic retinopathy, retinopathy of prematurity, macular degeneration, corneal graft rejection, neovascular glaucoma, retrolental fibroplasia, rubeosis, retinoblastoma, uvietis and Pterygiaab normal blood vessel growth) of the eye; rheumatoid arthritis; psoriasis; delayed wound healing; endometriosis; vasculogenesis; granulations; hypertrophic scars (keloids); nonunion fractures; scleroderma; trachoma; vascular adhesions; myocardial angiogenesis; coronary collaterals; cerebral collaterals; arteriovenous malformations; ischemic limb angiogenesis; Osler-Webber Syndrome; plaque neovascularization; telangiectasia; hemophiliac joints; angiofibroima; fibromuscular dysplasia; wound granulation; Crohn's disease; and atherosclerosis.

Additionally, disorders which can be treated with modified fusion proteins and/or polynucleotides encoding transferrin fusion proteins include, but are not limited to, hemangioma, arthritis, psoriasis, angiofibroma, atherosclerotic plaques, delayed wound healing, granulations, hemophilic joints hypertrophic scars, nonunion fractures, Osler-Weber syndrome, pyogenic granuloma, scleroderma, trachoma; and vascular adhesions.

Moreover, disorders and/or states, which can be treated, prevented, diagnosed, and/or prognosed with the modified transferrin fusion proteins and/or polynucleotides encoding transferrin fusion proteins include, but are not limited to, solid tumors, blood born tumors such as leukemia, tumor metastasis, Kaposi's sarcoma, benign tumors, for example hemangiomas, acoustic neuromas, neurofibromas, trachomas, and pyogenic granulomas, rheumatoid arthritis, psoriasis, ocularangiogenic diseases, for example, diabetic retinopathy, retinopathy of prematurity, macular degeneration, corneal graft rejection, neovascular glaucoma, retrolental fibroplasia, rubeosis, refinoblastoma, and uvietis, delayed wound healing, endometriosis, vasculogenesis, granulations, hypertrophic scars (keloids, nonunion fractures, scleroderma, trachoma, vascular adhesions, myocardial angiogenesis, coronary collaterals, cerebral collaterals, arteriovenous malformations, ischemic limb angiogenesis, Osler-Webber Syndrome, plaque neovascularization, telangiectasia, hemophiliac joints, angiofibroma fibromuscular dysplasia, wound granulation, Crohn's disease, atherosclerosis, birth control agent by preventing vascularization required for embryo, implantation controlling menstruation, diseases that have angiogenesis as a pathologic consequence such as cat scratch disease (Rochele nunalia quintosa), ulcers (Helicobacter pylori), Bartonellosis and baculary angiomatosis.

In one aspect of the birth control method, an amount of the compound sufficient to block embryo implantation is administered before or after intercourse and fertilization have occurred, thus providing an effective method of birth control, possibly a "morning after" method. Modified transferrin fusion proteins and/or polynucleotides encoding transferrin fusion proteins may also be used in controlling menstruation or administered as either a peritoneal lavage fluid or for peritoneal implantation in the treatment of endometriosis.

Modified transferrin fusion proteins and/or polynucleotides encoding transferrin fusion proteins may be utilized in a wide variety of surgical procedures.

Diseases associated with increased cell survival or the inhibition of apoptosis that could be treated, prevented, diagnosed, and/or prognosed using modified fusion proteins and/or polynucleotides encoring transferrin fusion proteins, include cancers (such as follicular lymphomas, carcinomas with mutations, and hormone-dependent tumors, including, but not limited to colon cancer, cardiac tumors, pancreatic cancer, melanoma, retinoblastoma, glioblastoma, lung cancer, intestinal cancer, testicular cancer, stomach cancer, neuroblastoma, myxoma, myoma, lymphoma, endothelioma, osteoblastoma, osteoclastoma, osteosarcoma, chondrosarcoma, adenoma, breast cancer, prostate cancer, Kaposi's sarcoma and ovarian cancer); autoimmune disorders (such as, multiple sclerosis, Sjogren's syndrome, Hashimoto's thyroiditis, biliary cirrhosis, Behcet's disease, Crohn's disease, polymyositis, systemic lupus thematosus and immune-related ryglomerulonephritis and rheumatoid arthritis) and viral infections (such as herpes viruses, pox viruses and adenoviruses), inflammation, graft v. host disease, acute graft rejection, and chronic graft rejection.

Modified fusion proteins and/or polynucleotides encoding transferrin fusion proteins may be used to inhibit growth, progression, and/or metasis of cancers, in particular those listed above.

Additional diseases or conditions associated with increased cell survival that could be treated or detected by modified fusion proteins and/or polynucleotides encoding, transferrin fusion proteins include, but are not limited to, progression, and/or metastases of malignances and related disorders such as leukemia (including acute leukemia (*e*.*g*., acute lymphocytic leukemia, acute myelocytic leukemia (including myeloblastic, promyelocytic, myelomonocytic, monocytic, and erythroleukemia)) and chronic leukemia (*e*.*g*., chronic myelocytic (granulocytic) leukemia and chroniclymphocytic leukemia)), polycytemia vera, lymphomas (*e*.*g*., Hodgkin's disease and non-Hodgkin's disease), multiple myeloma, Waldenstrom's macroglobulinemia, heavy chain disease, and solid tumors including, but not limited to, Sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basa cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, Jung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma neuroblastoma, and retinoblastoma.

Diseases associated with increased apoptosis that could be treated, prevented, diagnosed, and/or prognosed using modified fusion proteins and/or polynucleotides encoding transferrin fusion proteins, include, but are not limited to, AIDS; neurodegenerative disorders (such as Alzheimer's disease, Parkinson's disease, Amyotrophic lateral sclerosis, Retinitis pigmentosa, Cerebellar degeneration and brain tumor or prion associated disease); autoimmune disorders (such as, multiple sclerosis, Sjogren's syndrome, Hashimoto's thyroiditis, biliary cimhosis, Behcet's disease, Crohn's' disease, polymyositis, systemic lupus erythematosus and immune-related glomerulonephritis and rheumatoid arthritis) Myelodysplastic syndromes (such as aplastic anemia), graft v. host disease, ischemic injury (such as that caused, by myocardial. infarction, stroke and reperfusion injury), liver injury (*e*.*g*., hepatitis related liver injury, ischemia/reperfusion injury, cholestosis (bile duct injury) and liver cancer); toxin-induced liver disease (such as that caused by alcohol), septic shock, cachexia and anorexia.

In addition, modified fusion proteins and/or polynucleotides encoding transferrin fusion proteins could be, used to treat or prevent the onset of diabetes mellitus. In patients with newly diagnosed Types I and II diabetes, where some islet cell function remains, fusion proteins and/or polynucleotides encoding transferrin fusion proteins, could be used to maintain the islet function so as to alleviate, delay or prevent permanent manifestation of the disease. Also, fusion proteins and/or polynucleotides encoding transferrin fusion proteins could be used as an auxiliary in islet cell transplantation to improve or promote islet cell function.

The modified transferrin fusion proteins and/or polynucleotides encoding transferrin fusion proteins may be used for the diagnosis and/or treatment of diseases, disorders, damage or injury of the brain and/or nervous system. Nervous system disorders that can be treated with the compositions described herein (*e*.*g*., fusion proteins and/or polynucleotides encoding transferrin fusion proteins), limited to nervous systems include, but are not limited injuries, and diseases or disorders which result in either a disconnection of axons, a diminution or degeneration of neurons, or demyelination. Nervous system lesions which may be treated in a patient (including human and non-human mammalian patients), include but are not limited to, the following lesions of either the central (including spinal cord, brain) or peripheral nervous systems: (1) ischemic lesions, in which a lack of oxygen in a portion of the nervous system results in neuronal injury or death, including cerebral infarction or ischemia, or spinal cord infarction or ischemia; (2) traumatic lesions, including lesions caused by physical injury or associated with surgery, for example, lesions which sever a portion of the nervous system, or compression injuries; (3) malignant lesions, in which a portion of the nervous system is destroyed or injured by malignant tissue which is either a nervous system associated malignancy or a malignancy derived from nervous system tissue; (4) infectious lesions in which a portion of the nervous system is destroyed or injured as a result of infection, for example, by an abscess or associated with infection by human immunodeficiency virus, herpes zoster, or herpes simplex virus or with Lyme disease, tuberculosis, or syphilis; (5) degenerative lesions, in which a portion of the nervous system is destroyed or injured as a result of a degenerative process including but not limited to, degeneration associated with Parkinson's disease, Alzheimer's disease, Huntington's chorea, or amyotrophic lateral sclerosis (ALS); (6) lesions associated with nutritional diseases or disorders, in which a portion of the nervous system is destroyed or injured by a nutritional disorder or disorder of metabolism including, but not limited to vitamin B 12 deficiency, folic acid deficiency, Wernicke disease, tobacco-alcohol amblyopic, Marchiafava-Blanami disease (primary degeneration of the corpus callosum), and alcoholic cerebral degeneration; (7) neurological lesions associated with systemic diseases including, but not limited to diabetes (diabetic neuropathy, Bell's palsy), systemic lupus erythematosus, carcinoma, or sarcoidoisis; (8) lesions caused by toxic substances including alcohol, lead, or particular, neurotoxins; and (9) demyelinated lesions in which a portion of the nervous system is destroyed or injured by a demyelinating disease including, but not limited to, multiple sclerosis, human immunodeficiency virus-associated myelopathy, transverse myelopathy or various etiologies, progressive multifocal leukoencephalopathy, and central pontine myelinolysis.

The modified transferrin fusion proteins and/or polynucleotides encoding transferrin fusion proteins may be used to protect neural cells from the damaging effects of hypoxia. The modified transferrin fusion proteins and/or polynucleotides encoding transferrin fusion proteins may also be used to protect neural cells from the damaging effects of cerebral hypoxia.

Motor neuron disorders that may be treated include, but are not limited to, disorders such as infarction, infection, exposure to toxin, trauma, surgical damage, degenerative disease or malignancy that may affect motor neurons as well as other components of the nervous system, as well as disorders that selectively affect neurons such as amyotrophic lateral sclerosis, and including, but not limited to, progressive spinal muscular atrophy, progressive bulbar palsy, primary lateral sclerosis, infantile and juvenile muscular atrophy, progressive bulbar paralysis of childhood (Fazio-Londe syndrome), poliomyelitis and the post polio syndrome, and Hereditary Motor sensory Neuropathy (Charcot-Marie-Tooth Disease).

Further, modified fusion proteins and/or polynucleotides encoding transferrin fusion proteins may play a role in neuronal survival; synapse formation; conductance; neural differentiation, etc. Thus, compositions described herein (including fusion proteins and/or polynucleotides encoding transferrin fusion proteins) may be used to diagnose and/or treat or prevent diseases or disorders associated with these roles, including, but not limited to, learning and/or cognition disorders. The compositions may also be useful in the treatment or prevention of neurodegenerative disease states and/or behavioral disorders. Such neurodegenerative disease states and/or behavioral disorders include, but are not limited to, Alzheimer's Disease, Parkinson's Disease, Huntington's Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception.

Examples of neurologic diseases which can be treated or detected with modified fusion proteins and/or polynucleotides encoding transferrin fusion proteins include, brain diseases, such as metabolic brain diseases which includes phenylketonuria such as maternal phenylketonuria, pyruvate carboxylase deficiency, pyruvate dehydrogenase complex deficiency, Wernicke's Encephalopathy, brain edema, brain neoplasms such as cerebellar neoplasms which include infratentorial neoplasms, cerebral ventricle neoplasms such as choroid plexus neoplasms, hypothalamic neoplasms, supratentorial neoplasms, canavan disease, cerebellar diseases such as cerebellar ataxia which include spinocerebellar degeneration such as ataxia telangiectasia, cerebellar dyssynergia, Friederich's Ataxia, Machado-Joseph Disease, olivopontocerebellar atrophy, cerebellar neoplasms such as infratentorial neoplasms, diffuse cerebral sclerosis such as encephalitis periaxialis, globoid cell leukodystrophy, metachromatic leukodystrophy and subacute sclerosing panencephalitis.

Additional neurologic diseases which can be treated or detected with modified fusion proteins and/or polynucleotides encoding transferrin fusion proteins include cerebrovascular disorders (such as carotid artery diseases which include carotid artery thrombosis, carotid stenosis and Moyamoya Disease), cerebral amyloid angiopathy, cerebral aneurysm, cerebral anoxia, cerebral arteriosclerosis, cerebral arteriovenous malformations, cerebral artery diseases, cerebral embolism and thrombosis such as carotid artery thrombosis, sinus thrombosis and Wallenberg's Syndrome, cerebral hemorrhage such as epidermal hematoma, subdural hematoma and subarachnoid hemorrhage, cerebral infarction, cerebral ischemia such as transient cerebral ischemia, Subclavian Steal Syndrome and vertebrobasilar insufficiency, vascular dementia such as multi-infarct dementia, periventricular leukomalacia, vascular headache such as cluster headache and migraine.

Additional neurologic diseases which can be treated or detected with modified fusion proteins and/or polynucleotides encoding transferrin fusion proteins include dementia such as AIDS Dementia Complex, presenile dementia such as Alzheimer's Disease and Creutzfeldt-Jakob Syndrome, senile dementia such as Alzheimer's Disease and progressive supranuclear palsy, vascular dementia such as multi-infarct dementia, encephalitis which include encephalitis periaxialis, viral encephalitis such as epidemicencephalitis, Japanese Encephalitis, St. Louis Encephalitis, tick-borne encephalitis and West Nile Fever, acute disseminated encephalomyelitis, meningoencephalitis such as uveomeningoencephalitic syndrome, Postencephalitic Parkinson Disease and subacute sclerosing panencephalitis, encephalomalacia such as periventricular leukomalacia, epilepsy such as generalized epilepsy, which includes infantile spasms, absence epilepsy, myoclonic epilepsy which includes MERRF Syndrome, tonic-clonic epilepsy, partial epilepsy such as complex partial epilepsy, frontal lobe epilepsy and temporal lobe epilepsy, post-traumatic epilepsy, status epilepticus such as Epilepsia Partialis Continua, and Hallervorden-Spatz Syndrome.

Additional neurologic diseases which can be treated or detected with modified fusion proteins and/or polynucleotides encoding transferrin fusion proteins include hydrocephalus such as Dandy-Walker Syndrome and normal pressure hydrocephalus, hypothalamic diseases such as hypothalamic neoplasms, cerebral malaria, narcolepsy which includes cataplexy, bulbar poliomyelitis, cerebripseudo tumor, Rett Syndrome, Reye's Syndrome, thalamic diseases, cerebral toxoplasmosis, intracranialtuberculoma and Zellweger Syndrome, central nervous system infections such as AIDS, Dementia Complex, Brain Abscess, subdural empyema, encephalomyelitis such as Equine Encephalomyelitis, Venezuelan Equine Encephalomyelitis, Necrotizing Hemorrhabaic Encephalomyelitis, Visna, and cerebral malaria.

Additional neurologic diseases which can be treated or detected with modified fusion proteins and/or polynucleotides encoding transferrin fusion proteins include meningitis such as arachnoiditis, aseptic meningitis such as viral meningitis which includes lymphocytic chronic meningitis, Bacterial meningitis which includes Haemophilus Meningitis, Listeria Meningitis, Meningococcal Meningitis such as Waterhouse-Fridericlisen Syndrome, Pneumococcal Meningitis and meningeal tuberculosis, fungal meningitis such as Cryptococcal Meningitis, subdural effusion, meningencephalitis, myelitis such as transverse myelitis, neurosyphilis such as tabes dorsalis, poliomyelitis which includes bulbar poliomyelitis and post poliomyelitis syndrome, prion diseases (such as Creutzfeldt-Jakob Syndrome, Bovine Spongiform Encephalopathy, Gerstmann-Straussler Syndrome, Kuru, Scrapie), and cerebral toxoplasmosis.

Additional neurologic diseases which can be treated or detected with modified fusion proteins and/or polynucleotides encoding transferrin fusion proteins include central nervous system neoplasms such as brain neoplasms that include cerebellameoplasms such as infratentorial neoplasms, cerebral ventricle neoplasms such as choroids plexus neoplasms, hypothalamic neoplasms and supratentorial neoplasms, meningealneoplasms, spinal cord neoplasms which include epidural neoplasms, demyelinating diseases such as Canavan Diseases, diffuse cerebral sculleries which include sadrenoleukodystrophy, encephalitis periaxialis, globoid cell leukodystrophy, diffuse cerebral sclerosis such as metachromatic leukodystrophy, allergic encephalomyelitis, necrotizing hemorrhagic encephalomyelitis, progressive multifocal leukoencephalopathy, in multiple sclerosis, central pontine myelinolysis, transverse myelitis, neuromyelitis optica, Scrapie, Swayback, Chronic Fatigue Syndrome, Visna, High Pressure Nervous Syndrome, Meningism, spinal cord diseases such as amyotonia congenita, amyotrophic lateral-sclerosis, spinal muscular atrophy such as Werdnig-Hoffmann Disease, spinal cord compression, spinal cord neoplasms such as epidural neoplasms, syringomyelia., Tabes Dorsalis, Stiff-Man Syndrome, mental retardation such as Angelman Syndrome, Cri-du-Chat Syndrome, De Lange's Syndrome, Down Syndrome, Gangliosidoses such as gangliosidoses G(MI), Sandhoff Disease, Tay-Sachs Disease, Hartnup Disease, homocystinuria, Laurence-Moon- Bied Syndrome, Lesch-Nylian Syndrome, Maple Syrup Urine Disease, mucolipidosis such as fucosidosis, neuronal ceroid-lipofuscinosis, oculocerebrorenal syndrome, phenylketonuria such as maternal phenylketonuria, Prader-Willi Syndrome, Rett Syndrome, Rubinstein-Taybi Syndrome, Tuberous Sclerosis, WAGR Syndrome, nervous system abnormalities such as holoprosencephaly, neural tube defects such as anencephaly which includes hydrangencephaly, Arnold-Chairi Deformity, encephalocele, meningocele, meningomyelocele, spinal dysraphism such as Spina bifida cystica and spina bifida occulta.

Endocrine system and/or hormone imbalance and/or diseases encompass disorders of uterine motility including, but not limited to complications with pregnancy and labor (*e*.*g*., pre-term labor, post-term pregnancy, spontaneous abortion, and slow or stopped labor); and disorders and/or diseases of the menstrual cycle, (*e*.*g*., dysmenorrhea and endometriosis).

Endocrine system and/or hormone imbalance disorders and/or diseases include disorders and/or diseases of the pancreas, such as, for example, diabetes mellitus, diabetes insipidus, congenital pancreatic agenesis, pheochromocytoma islet cell tumor syndrome; disorders and/or diseases of the adrenal glands such as, for example, Addison's Disease, corticosteroid deficiency, virilizing disease, hirsutism, Cushing's Syndrome, hyperaldosterlonism, pheochromocytoma; disorders and/or diseases of the pituitary gland, such as, for example, hyperpituitarism, hypopituitarism, pituitary dwarfism, pituitary adenoma, panhypopituitarism, acromegaly, gigantism; disorders and/or diseases of the thyroid, including but not limited to, hyperthyroidism, hypothyroidism, Plummer's disease, Graves' disease (toxic diffuse goiter), toxic nodular goiter, thyroiditis (Hashimoto's thyroiditis, subacute granulomatous thyroiditis, and silent lymphocytic thyroiditis), Pendred's syndrome, myxedema, cretinism, thyrotoxicosis, thyroid hormone coupling defect, thymic aplasia, Hurthle cell tumors of the thyroid, thyroid cancer, thyroid carcinoma, Medullary thyroid carcinoma; disorders and/or diseases of the parathyroid, such as, for example, hyperparathyroidism, hypoparathyroidism; disorders and/or diseases of the hypothalamus.

In addition, endocrine system and/or hormone imbalance disorders and/or diseases may also include disorders and/or diseases of the testes or ovaries, including cancer. Other disorders and/or diseases of the testes or ovaries further include, for example, ovarian cancer, polycystic ovary syndrome, Klinefelter's syndrome, vanishing testes syndrome (bilateral anorchia), congenital absence of Leydig's cells, cryptorchidism, Noonan's syndrome, myotonic dystrophy, capillary haemangioma of the testis (benign), neoplasias of the testis and neotestis.

Moreover, endocrine system and/or hormone imbalance disorders and/or diseases may also include disorders and/or diseases such as, for example, polyglandular deficiency syndromes, pheochromocytoma, neuroblastoma, multiple Endocrine neoplasia, and disorders and/or cancers of endocrine tissues.

The modified transferrin fusion proteins and/or polynucleotides encoding transferrin fusion proteins may be used for the diagnosis, treatment, or prevention of diseases and/or disorders of the reproductive system. Reproductive system disorders that can be treated by the compositions of the invention, include, but are not limited to, reproductive system injuries, infections, neoplastic disorders, congenital defects, and diseases or disorders will result in infertility, complications with pregnancy, labor, or parturition, and postpartum difficulties.

Reproductive system disorders and/or diseases include diseases and/or disorders, of the testes, including testicular atrophy, testicular feminization, cryptorchism (unilateral and bilateral), anorchia, ectopic testis, epididymitis and orchitis (typically resulting from infections such as, for example, gonorrhea, mumps, tuberculosis, and syphilis), testiculartorsiori, vasitis nodosa, germ cell tumors (*e*.*g*., seminomas, embryonal cell carcinomas, teratocarcinomas, choriocarcinomas, yolk sac tumors, and teratomas), stromal tumors (*e*.*g*., Leydig cell tumors), hydrocele, hematocele, varicocele, spermatocele, inguinal hemia, and disorders of sperm production (*e*.*g*., immotile cilia syndrome, spermia, asthenozoospermia, azoospermia, oligospermia, and teratozoospermia).

Reproductive system disorders also include disorders of the prostate gland, such as acute non-bacterial prostatitis, chronic non-bacterial prostatitis, acute bacterial prostatitis, chronic bacterial prostatitis, postatodystonia, prostatosis, granulomatotis prostatitis, malacoplakia, benign prostatic hypertrophy or hyperplasia, and prostate neoplastic disorders, including adenocarcinomas, transitional cell carcinomas, ductal carcinomas, and squamous cell carcinomas.

Additionally, the compositions described herein may be useful in the diagnosis, treatment, and/or prevention of disorders or diseases of the penis and urethra, including inflammatory disorders, such as balanoposthitis, balanitis xerotica obliterans, phimosis, paraphmosis, syphilis, herpes simplex virus, gonorrhea, non-gonococcal urethritis, chlamydia, mycoplasma, trichomonas, HIV, AIDS, Reiter's syndrome, condyloma acuminatum, condyloma latum, and pearly penile papules, urethral abnormalities, such as hypospadias, epispadias, and phimosis, premalignant lesions, including Erythroplasia of Queyrat, Bowen's disease, Bowenoid paplosis, criant condyloma of Buscke-Lowenstein, and varrucous carcinoma; penile cancers, including squamous cell carcinomas, carcinoma in situ, verrucous carcinoma, and disseminated penile carcinoma; urethral neoplastic disorders, including penile urethial carcinoma, bulbomembranotis urethial carcinoma, and prostaticurethral carcinoma; and erectile disorders, such as priapism, Peyronie's disease, erectile dysfunction, and impotence.

Moreover, diseases and/or disorders of the vas deferens include vasculititis and CBAVD (congenital bilateral absence of the vas deferens); additionally, the transferrin fusion proteins of the invention and/or polynucleotides encoding transferrin fusion proteins of the invention may be used in the diagnosis, treatment, and/or prevention of diseases and/or disorders of the seminal vesicles, including hydatid disease, congenital chioride diarrhea, and polycystic kidney disease.

Other disorders and/or diseases of the male reproductive system include, for example, Klinefelters syndrome, Young's syndrome, premature ejaculation, diabetes mellitus, cystic fibrosis, Kartagener's syndrome, high fever, multiple sclerosis, and gynecomastia.

Further, the polynucleotides, modified fusion proteins and/or polynucleotides encoding transferrin fusion proteins may be used in the diagnosis treatment and/or prevention of diseases and/or disorders of the vagina and vulva, including bacterial vaginosis, candida vaginitis, herpes simplex virus, chancroid, granuloma inguinale, lymphogranuloma venereum, scabies, human papillomavirus, vaginal trauma, vulvartrauma, adenosis, chlamydia vaginitis, gonorrhea, trichomonas vaginitis, condylomaacuminatum, syphilis, molluscum contagiosum, atrophic vaginitis, Paaet's disease, lichensclerosus, lichen planus, vulvodynia, toxic shock syndrome, vaginismus, vulvovaginitis, vulvar vestibulitis, and neoplastic disorders, such as squamous cell hyperplasia, clear cell carcinoma, basal cell carcinoma, melanomas, cancer of Bartholin's gland, and vulvarintraepaelial neoplasia.

Disorders and/or diseases of the uterus include dysmenorrhea, retroverted uterus, endometriosis, fibroids, adenomyosis, anovulatory bleeding, amenorrhea, Cushiner's syndrome, hydatidiform moles, Asherman's syndrome, premature menopause, precocious puberty, uterine polyps, dysfunctional uterine bleeding (*e*.*g*., due to aberrant hormonal signals), and neoplastic disorders, such as adenocarcinomas, keiomyosarcomas, and sarcomas. Additionally, the transferrin fusion proteins and/or polynucleotides encoding transferrin fusion proteins may be useful as a marker or detector of, as well as, in the diagnosis, treatment, and/or prevention of congenital uterine abnormalities, such as bicomuate uterus, septate uterus, simple unicomuate uterus, unicomuate uterus with a noncavitary rudimentary horn, unicorriuate uterus with a non-communicating cavitary rudimentary horn, unicomuate uterus with a communicating cavitary horn, arcuate uterus, uterine didelfus, and T-shaped uterus.

Ovarian diseases and/or disorders include an ovulation, polycystic ovary syndrome (Stein-Leventhal syndrome), ovarian cysts, ovarian hypofunction, ovarian insensitivity to gonadotropins, ovarian over production of androgens, right ovarian vein syndrome, in amenorrhea, hirutism, and ovarian cancer (including, but not limited to, primary and secondary cancerous growth, Sertoli-Leydig tumors, endometriod carcinoma of the ovary, ovarian papillary serous adenocarcinoma, ovarian mucinous adenocarcinoma, and Ovarian Krukenberg tumors).

Cervical diseases and/or disorders include cervicitis, chronic cervicitis, mucopurulent cervicitis, and cervical dysplasia, cervical polyps, Nabothian cysts, cervical erosion; cervical incompetence, and cervical neoplasms (including, for example, cervical carcinoma, squamous metaplasia, squamous cell carcinoma, adenosquamous cell neoplasia, and columnar cell neoplasia).

Modified transferrin fusion proteins and/or polynucleotides encoding transferrin fusion proteins can be used to treat or detect infectious agents. For example, by increasing the immune response, particularly increasing the proliferation and differentiation of B and/or T cells, infectious diseases may be treated. The immune response may be increased by either enhancing an existing immune response, or by fusion proteins of the invention and/or initiating a new immune response. Alternatively, polynucleotides encoding transferrin fusion proteins may also directly inhibit infectious agent, without necessarily eliciting an immune response.

Viruses are one example of an infectious agent that can cause disease or symptoms that can be treated or detected by transferrin fusion proteins and/or polynucleotides encoding transferrin fusion proteins. Examples of viruses, include, but are not limited to the following DNA and RNA viruses and viral families: Arbovirus, Adenoviridae, Arenaviridae, Arterivirus, Bimaviridae, Bunyaviridae, Caliciviridae, Circoviridae, Coronaviridae, Dengue, EBV, HIV, Flaviviridae, Hepadnaviridae Hepatitis, Herpesviridae (such as, Cytomegalovirus, Herpes Simplex; Herpes Zoster), Mononegavirus (*e*.*g*., Paramyxoviridae, Morbillivirus, Rhabdoviridae), Orthomyxoviridae (*e*.*g*., Influenza A, Influenza B, and parainfluenza), Papilloma virus, Papovaviridae, Parvoviridae, Picomaviridae, Poxviridae (such as Smallpox or Vaccinia), Reoviridae (*e*.*g*., Rotavirus), Retroviridae (HTLV-I, HTLV-11, -Lentivirus), and Togaviridae (*e*.*g*., Rubivirus).

Similarly, bacterial and fungal agents that can cause disease or symptoms that can be treated or detected by transferrin fusion proteins and/or polynucleotides encoding transferrin fusion proteins include, but not limited to, the following Gram-negative and Gram-positive bacteria, bacterial families, and fungi: Actinomyces (*e*.*g*., *Norcardia*), Acinetobacter, Cryptococcus neoformans, Aspergillus, Bacillaceae (*e*.*g*., Bacillus anthrasis), *Bacteroides* (*e*.*g*., *Bacteroides fragilis*), *Blastomycosis, Bordetella, Borrelia (e*.*g*., *Borrelia burgdorferi), Brucella, Candidia, Campylobacter, Chlamydia, Clostridiuffi (e*.*g*., *Clostridium botulinum, Clostridium dificile, Clostridium perfringens, Clostridiumtetani), Coccidioides, Corynebacterium (e.g., Corynebacterium- diptheriae), Cryptococcus, Dermatocycoses, E. coli (e.g., Enterotoxigenic E. coli and Enterohemorrhagic E. coli), Enterobacter (e.g. Enterobacter aerogenes), Enterobacteriaceae (Klebsiella, Salmonella (e.g., Salmonella typhi, Salmonella enteritidis, Salmonella typhi), Serratia, Yersinia, Shigella), Erysipelothrix, Haemophilus (e.g., Haemophilus influenza type B), Helicobacter, Legionella (e. g., Legionella pneumophila), Leptospira, Listeria (e.g., Listeria monocytogenes), Mycoplasma, Mycobacterium (e.g., Mycobacterium leprae and Mycobacterium tuberculosis), Vibrio (e.g., Vibrio cholerae), Neisseriaceae (e.g., Neisseriagonorrhea, Neisseria meningitidis), Pasteurellaceae, Proteus, Pseudomonas (e.g., Pseudomionas aeruginosa), Rickettsiaceae, Spirochetes (e.g., Treponema. spp., Leptospiraspp., Borrielia spp.), Shigella spp., Staphylococcus (e.g., Staphylococcus aureus), Meningiococcus, Pneumococcus and Streptococcus (e.g., Streptococcus pneumoniae and* Groups A, B, and C *Streptococci),* and *Ureaplasmas.*

Moreover, parasitic agents causing disease or that can be treated, prevented, and/or diagnosed by fusion proteins and/or polynucleotides encoding transferrin fusion proteins include, but not limited to, the following families or class: Amebiasis, Babesiosis, Coccidiosis, Cryptosporidiosis, Dientamoebiasis, Dourine, Ectoparasitic, Giardias, Helminthiasis, Leishmaniasis, Schistisoma, Theileriasis, Toxoplasmosis, Trypanosomiasis, and Trichomonas and Sporozoans (*e*.*g*., Plasmodium vivax, Plasmodium falciparium, Plasmodium malariae and Plasmodium ovale).

Modified transferrin fusion proteins and/or polynucleotides encoding transferrin fusion proteins can be used to differentiate, proliferate, and attract cells, pleading to the regeneration of tissues. (See, Science 276:59-87 (1997)). The regeneration of tissues could be used to repair, replace, or protect tissue damaged by congenital defects, trauma (wounds, burns, incisions, or ulcers), age, disease (*e*.*g*. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (*e*.*g*., pancreas, liver, intestine, kidney, skin, endothelium), muscle (smooth, skeletal or cardiac), vasculature (including vascular and lymphatics), nervous, hematopoietic, and skeletal (bone, cartilage, tendon, and ligament) tissue. Preferably, regeneration occurs without or decreased scarring. Regeneration also may include angiogenesis.

Modified transferrin fusion proteins and/or polynucleotides encoding transferrin fusion proteins may be used to treat, prevent, diagnose, and/or prognose gastrointestinal disorders, including inflammatory diseases and/or conditions, infections, cancers (*e*.*g*., intestinal neoplasms (carcinoid tumor of the small intestine, non-Hodgkin's lymphoma of the small intestine, small bowel lymphoma), and ulcers, such as peptic ulcers.

Gastrointestinal disorders include dysphagia, odynophagia, inflammation of the esophagus, peptic esophagitis, gastric reflux, submucosal fibrosis and structuring, Mallory-Weiss lesions, lipomas, epidennal cancers, adeoncarcinomas, gastric retention disorders, gastroenteritis, gastric atrophy, gastric/stomach cancers, polyps of the stomach, autoimmune disorders such as pemicious anemia, pyloric stenosis, gastritis (bacterial, viral, eosinophilic, stress-induced, chronic erosive, atrophic, plasma cell, and Menetrier's), and peritoneal diseases (*e*.*g*., chylo perioneum, hemoperitoneum, mesenteric cyst, mesentericlymphadenitis, mesenteric vascular occlusion, panniculiti, neoplasms, peritonitis, pneumoperitoneum, bubphrenic abscess.

Gastrointestinal disorders also include disorders associated with the small intestine, such as malabsorption syndrome's, distension, irritable bowel syndrome, sugar intolerance, celiac disease, duodenal ulcers, duodenitis, tropical sprue, Whipple's disease, intestinal lymphangiectasia, Crohn's disease, appendicitis, obstructions of the ileum, Meckel's diverticulum, multiple diverticula, failure of complete rotation of the small and large intestine, lymphoma, and bacterial and parasitic diseases (such as Traveler's diarrhea, typhoid and paratyphoid, cholera, infection by Roundworms (*Ascariasis lumbricoides*), Hookworms (*Ancylostoma duodenale*), Threadworms (Enterobius vermicularis), Tapeworms *Taenia saginata, Echinococcus granulosus, Diphyllobothrium* spp. and *T. solium*).

Liver diseases and/or disorders include intrahepatic cholestasis (Alagille syndrome, biliary liver cirrhosis), fatty, liver (alcoholic fatty liver, Reye's syndrome), hepatic veiri, thrombosis, hepatolentricular degeneration, hepatomegaly, hepatopulmonary syndrome, hepatorenal, syndrome, portal hypertension (esophageal and gastric varices), liver abscess (amebic liver abscess), liver cirrhosis (alcoholic, biliary and experimental), alcoholic liver diseases (fatty liver, hepatitis, cirrhosis), parasitic (hepatic echinococcosis, fascioliasis, amebic liver abscess), jaundice (hemolytic, hepatocellular, and cholestatic), cholestasis, portal hypertension, liver, enlargement, ascites, hepatitis (alcoholic hepatitis, intra-familial hepatitis, chronic hepatitis (autoimmune, hepatitis B, hepatitis C, hepatitis D, drug induced), toxic hepatitis, viral human hepatitis (hepatitis A, hepatitis B, hepatitis C, hepatitis D, hepatitis E), Wilson's disease, granulomatous hepatitis, secondary biliary cirrhosis, hepaticencephalopathy, portal hypertension, varices, hepatic encepbalopathy, primary biliary hemangiomas, bilecirrhosis, primary sclerosing cholangitis, hepatocellular adenoma, stones, liver failure (hepatic encephalopathy, acute liver failure), and liver neoplasms (ancriomyolipoma, calcified liver metastases, cystic liver metastases, epithelial tumors, fibro lamellar hepatocarcinoma, focal nodular hyperplasia, hepatic adenoma, hepatobiliarycystadenoma, hepatoblastoma, hepatocellular carcinoma, hepatoma, liver cancer, liver hemangioendothelioma, mesenchymal hamartoma, mesenchymal tumors of liver, nodular regenerative hyperplasia, benign liver tumors (Hepatic cysts, Simple cysts, Polycystic liver disease, Hepatobiliary cystadenoma, Choledochal cysts, Mesenchymal tumors, Mesenchymal hamartoma, Infantile hemangioendothelioma, Hemangioma, Peliosis hepatis, Lipomas, Inflammatory pseudo tumor, Miscellaneous Epithelial tumors, Bile duct epithelium (Bile duct hamartoma, Bile duct adenoma), Hepatocyte (Adenoma, Focal nodular hyperplasia, Nodular regenerative hyperplasia), malignant liver tumors (hepatocellular, hepatoblastoma, hepatocellular carcinoma, cholangiocellular, cholangiocarcinoma, cystadenocarcinoma, tumors of blood vessels, anaiosarcoma, Karposi's sarcoma, hemangioendothelioma, other tumors, embryonal sarcorria, fibrosarcoma, rhabdomyosarcoma, carcinosarcoma, teratoma, carcinoid, squamous carcinoma, primarylymphorria)), peliosis hepatis, erythrohepatic porphyria, hepatic porphyria (acute interirtittentporphyria, porphyria cutanea tarda), Zelli Neger syndrome).

Pancreatic diseases and/or disorders include acute pancreatitis, chronic pancreatitis (acute necrotizing pancreatitis, alcoholic pancreatitis), neoplasms (adenocarcinoma of the pancreas, cystadenocarcinoma, insulinoma, gastrinoma, and glucacronoma, cysticcitmeoplasms, islet-cell tumors, pancreoblastoma), and other pancreatic diseases (*e*.*g*., cysticfibrosis, cyst (pancreatic pseudocyst, pancreatic fistula, insufficiency)).

Gallbladder diseases include gallstones (cholelithiasis and choledocholithiasis), postcholeeystectomy syndrome, diverticulosis of the gallbladder, acute cholecystitis, chronic cholecystitis, bile duct tumors, and mucocele.

Diseases and/or disorders of the large intestine include antibiotic-associated colitis, diverticulitis, ulcerative colitis, acquired megacolon, abscesses, fungal and bacterial infections, anorectal disorders (*e*.*g*., fissures, hemorrhoids), colonic diseases (colitis, colonic neoplastris, colon cancer, adenomatous colon polyps (*e*.*g*., villous adenoma), coloncarcinoma, colorectal cancer, colonic diverticulitis, colonic diverticulosis, megacolon, Hirschsprung disease, toxic inegacolon, sigmoid diseases proctocolitis, sigmoinneoplasmsj, constipation, Crohn's disease, diarrhea (infantile diarrhea, dysentery), duodenal diseases (duodenal neoplasins, duodenal obstruction, duodenal ulcer, duodenitis), enteritis (enterocolitis), HIV enteropathy, leal diseases (leal neoplasins, ileitis), immunoproliferative small intestinal disease, inflammatory bowel disease (ulcerative colitis, Crohn's disease), intestinal atresia, parasitic diseases (anisakiasis, balantidiasis, blastocystis infections, cryptosporidiosis, dientamoebiasis, amebic dysentery, giardiasis), intestinal fistula (rectal fistula), intestinal neoplasms (cecal neoplasms, colonic neoplasms, duodenalpneoplasms, leal neoplasms, intestinal polyps, jejunal neoplasins, rectal neoplasms), intestinal obstruction (afferent loop syndrome, duodenal obstruction, impacted feces, intestinal pseudo obstruction cecal volvulus, intussusception), intestinal perforation, intestinal polyps (colonic polyps, gardner syndrome, peutz-jeghers syndrome), jejunal diseases Oejunal neoplasms), mal absorption syndromes (blind loop syndrome, celiac disease, lactose intolerance, short bowl syndrome, tropical sprue, whipple's disease), mesenteric vascular occlusion, pneumatosis cystoides intestinalis, protein losing enteropathies (intestinal lymphagiectasis), rectal diseases (anus diseases, fecal incontinence, hemorrhoids, proctitis, rectal fistula, rectal prolapse, rectocele), peptic ulcer (duodenalulcer, peptic esophagitis, hemorrhage, perforation, stomach ulcer, Zollinger-Ellison syndrome), postgastrectomy syndromes (dumping syndrome), stomach diseases (*e*.*g*., achlorhydria, duodenogastric reflux (bile reflux), gastric antral vascular ectasia, gastricfistula, gastric outlet obstruction, gastritis (atrophic or hypertrophic), gastroparesis, stomach dilatation, stomach diverticulum, stomach neoplasms (gastric cancer, gastric polyps, gastric adenocarcinoma, hyperplastic gastric polyp), stomach rupture, stomach ulcer, stomach volvulus), tuberculosis, visceroptosis, vomiting (*e*.*g*., hematemesis, hyperemesisgravidarum, postoperative nausea- and vomiting) and hemorrhagic colitis.

Further diseases and/or disorders of the gastrointestinal system include biliary tract diseases, such as, gastroschisis, fistula (*e*.*g*., biliary fistula, esophageal fistula, gastricfistula, intestinal fistula, pancreatic fistula), neoplasms (*e*.*g*., biliary tract neoplasins, esophageal neoplasms, such as adenocarcinoma of the esophagus, esophageal squamous cell carcinoma, gastrointestinal neoplasms, pancreatic neoplasins, such as adenocarcinoma of the pancreas, mucinous cystic neoplasm of the pancreas, pancreatic eystic neoplasms, pancreatoblastoma, and peritoneal neoplasms), esophageal disease (*e*.*g*., bullous diseases, candidiasis, glycoaenie acanthosis, ulceration, barrett esophagus varices, atresia, cyst, diverticulum. (*e*.*g*., Zenker's diverticulum), fistula (*e*.*g*., tracheoesophageal fistula), motility disorders (*e*.*g*., CREST syndrome, deglutition disorders, achalasia, spasm, gastroesophageal reflux), neoplasms, perforation (*e*.*g*., Boerhaave syndrome, Mallory-Weiss syndrome), stenosis, esophagitis, diaphragmatic hernia (*e*.*g*., hiatal hernia); gastrointestinal diseases, such as, gastroenteritis (*e*.*g*., cholera morbus, norwalk virus infection), hemorrhage (*e*.*g*., hematemesis, melena, peptic ulcer hemorrhage), stomach neoplasms (gastric cancer, gastric polyps, gastric adenocarcinoma, stomach cancer)), hernia (*e*.*g*., congenital diaphragmatic hernia, femoral hernia, inguinal hernia, obturator hernia, umbilical hernia, ventral hernia), and intestinal diseases (*e*.*g*., cecal diseases (appendicitis, cecal neoplasms)).

Modified transferrin fusion proteins and/or polynucleotides encoding transferrin fusion proteins may have chemotaxis activity. A chemotaxic molecule attracts or mobilizes cells (*e*.*g*., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

Modified transferrin fusion proteins and/or polynucleotides encoding transferrin fusion proteins may increase chemotaxic activity of particular cells. These chemotactic molecules can then be used to treat inflammation, infection, hyperproliferative disorders, or any immune system disorder by increasing the number of cells targeted to a particular location in the body.

### Oral Pharmaceutical Compositions and Delivery Methods

Tf fusion proteins including but not limited to modified Tf fusion proteins, may be formulated for oral delivery. Methods of preparing Tf fusion proteins and methods of administering Tf fusion proteins are discussed in PCT Patent Application PCT/US03/ , entitled, "Oral Delivery of Modified Transferrin Fusion Proteins," filed August 28, 2003.

In particular, certain fusion proteins that are used to treat certain classes of diseases or medical conditions may be particularly amenable for oral formulation and delivery. Such classes of diseases or conditions include, but are not limited to, acute, chronic and recurrent diseases. Chronic or recurrent diseases include, but are not limited to, viral disease or infections, cancer, ametabolic diseases, obesity, autoimmune diseases, inflammatory diseases, allergy, graft-vs.-host disease, systemic microbial infection, anemia, cardiovascular disease, psychosis, genetic diseases, neurodegenerative diseases, disorders of hematopoietic cells, diseases of the endocrine system or reproductive systems, gastrointestinal diseases. Examples of these classes of disease include diabetes, multiple sclerosis, asthma, HCV or HIV infections, hypertension, hypercholesterolemia, arterial sclerosis, arthritis, and Alzheimer's disease. In many chronic diseases, oral formulations of Tf fusion proteins and methods of administration are particularly useful because they allow long-term patient care and therapy via home oral administration without reliance on injectable treatment or drug protocols.

Oral formulations and delivery methods comprising Tf fusion proteins take advantage of, in part, transferrin receptor mediated transcytosis across the gastrointestinal (GI) epithelium. The Tf receptor is found at a very high density in the human GI epithelium, transferrin is highly resistant to tryptic and chymotryptic digestion and Tf chemical conjugates have been used successfully to deliver proteins and peptides across the GI epithelium (Xia et al., (2000) J. Pharmacol. Experiment. Therap., 295:594-600; Xia et al. (2001) Pharmaceutical Res., 18(2):191-195; and Shah et al. (1996) J. Pharmaceutical Sci., 85(12):1306-1311). Once transported across the GI epithelium, Tf fusion proteins exhibit extended half-life in serum, that is, the therapeutic protein or peptide(s) attached or inserted into Tf exhibit an extended *in vivo* circulatory half-life compared to the protein or peptide in its non-fused state.

Oral formulations of Tf fusion proteins may be prepared so that they are suitable for transport to the GI epithelium and protection of the Tf fusion protein component and other active components in the stomach. Such formulations may include carrier and dispersant components and may be in any suitable form, including aerosols (for oral or pulmonary delivery), syrups, tablets, including chewable tablets, capsules, troches, lozenges, cachets or pellets granulates and powders. Also contemplated for use herein are other oral solid dosage forms, which are described generally in Remington's Pharmaceutical Sciences, 18th Ed. 1990 (Mack Publishing Co. Easton Pa. 18042) at Chapter 89.

Many of the oral formulations may also contain inert ingredients which allow for protection against the stomach environment, and release of the biologically active material in the intestine. Such formulations, or enteric coatings, are well known in the art. In a particularly preferred embodiment, oral pharmaceutical compositions of the invention are formulated in buffered liquid form which is then encapsulated into soft or hard-coated gelatin capsules which are then coated with an appropriate enteric coating. For the oral pharmaceutical compositions of the invention, the location of release may be anywhere in the GI system, including the small intestine (the duodenum, the jejunum, or the ileum), or the large intestine.

Pharmaceutically acceptable carriers or dispersants include, but are not limited to, aqueous buffers, sucrose, lactose, starch, fatty oils, fatty acid esters, polysaccharides, monoglycerides, triglycerides, phospholipid emulsifiers, non-ionic emulsifiers and refined colloidal clays. Pharmaceutical compositions may also be formulated for oral delivery using polyester microspheres, zein microspheres, proteinoid microspheres, polycyanoacrylate microspheres, and lipid-based systems (see, for example, DiBase and Morrel, Oral Delivery of Microencapsulated Proteins, "in Protein Delivery:Physical Systems, Sanders and Hendren (eds.), pages 255-288 (Plenum Press 1997)). In other embodiments, oral compositions are formulated to slowly release the active ingredients, including the Tf fusion proteins of the invention, in the GI system using known delayed release formulations.

Tf fusion proteins of the invention for oral delivery are capable of binding the Tf receptor found in the GI epithelium. To facilitate this binding and receptor mediated transport, Tf fusion proteins of the invention are typically produced with iron and in some instances carbonate, bound to the Tf moiety. Processes and methods to load the Tf moiety of the fusion protein compositions of the invention with iron and carbonate are known in the art

In some pharmaceutical formulations, the Tf moiety of the Tf fusion protein may be modified to increase the affinity or avidity of the Tf moiety to iron. Such methods are known in the art. For instance, mutagenesis can be used to produce mutant transferrin moieties that bind iron more avidly than natural transferrin. In human serum transferrin, the amino acids which are ligands for metal ion chelation include, but are not limited to N lobe amino acids Asp63, Tyr 95, Tyr188, Lys206, His207 and His249; and C lobe amino acids Asp392, Tyr426, Tyr517 and His584 (the number beside the amino acid indicates the position of the amino acid residue in the primary amino acid sequence where the valine of the mature protein is designated position 1). See U.S. Patent 5,986,067. In one embodiment, the Lys206 and His207 residues within the N lobe are replaced with Gln and Glu; respectively.

In some pharmaceutical formulations, the Tf fusion protein is engineered to contain a cleavage site between the therapeutic protein or peptide and the Tf moiety. Such cleavable sites or linkers are known in the art.

Pharmaceutical compositions and methods may include the addition of a transcytosis enhancer to facilitate transfer of the fusion protein across the GI epithelium. Such enhancers are known in the art. See Xia et al., (2000) J. Pharmacol. Experiment. Therap., 295:594-600; and Xia et al. (2001) Pharmaceutical Res., 18(2):191-195.

oral pharmaceutical formulations may include Tf fusion proteins comprising a modified Tf moiety exhibiting reduced or no glycosylation fused at the N terminal end to an insulin or GLP-1 protein or peptide as described above. Such pharmaceutical compositions may be used to treat glucose imbalance disorders such as diabetes by oral administration of the pharmaceutical composition comprising an effective dose of fusion protein.

The effective dose of fusion protein may be measured in a numbers of ways, including dosages calculated to alleviate symptoms associated with a specific disease state in a patient, such as the symptoms of diabetes. In other formulations, dosages are calculated to comprise an effective amount of fusion protein to induce a detectable change in blood glucose levels in the patient. Such detectable changes in blood glucose may include a decrease in blood glucose levels of between about 1% and 90%, or between about 5% and about 80%. These decreases in blood glucose levels will be dependent on the disease condition being treated and pharmaceutical compositions or methods of administration may be modified to achieve the desired result for each patient. In other instances, the pharmaceutical compositions are formulated and methods of administration modified to detect an increase in the activity level of the therapeutic protein or peptide in the patient, for instance, detectable increases in the activities of insulin or GLP-1. Such formulations and methods may deliver between about 1 pg to about 100 mg /kg body weight of fusion protein, about 100 ng to about 100 µg/kg body weight of fusion protein, about 100 µg/ to about 100 mg/kg body weight of fusion protein, about 1 µg to about 1 g of fusion protein, about 10 µg to about 100 mg of fusion protein or about 10 mg to about 50 mg of fusion protein. Formulations may also be calculated using a unit measurement of therapeutic protein activity, such as about 5 to about 500 units of human insulin or about 10 to about 100 units of human insulin. The measurements by weight or activity can be calculated using known standards for each therapeutic protein or peptide fused to Tf.

Also described herein are methods of orally administering the pharmaceutical compositions. Such methods may include, but are not limited to, steps of orally administering the compositions by the patient or a caregiver. Such administration steps may include administration on intervals such as once or twice per day depending on the Tf fusion protein, disease or patient condition or individual patient. Such methods also include the administration of various dosages of the individual Tf fusion protein. For instance, the initial dosage of a pharmaceutical composition may be at a higher level to induce a desired effect, such as reduction in blood glucose levels. Subsequent dosages may then be decreased once a desired effect is achieved. These changes or modifications to administration protocols may be done by the attending physician or health care worker. In some instances, the changes in the administration protocol may be done by the individual patient, such as when a patient is monitoring blood glucose levels and administering a mTf-insulin or mTF-GLP-1 oral composition.

Also described herein are methods of producing oral compositions or medicant compositions comprising formulating a Tf fusion protein into an orally administerable form. The application also describes methods of producing compositions or medicant compositions comprising formulating a Tf fusion protein into a form suitable for oral administration.

### Transgenic Animals

The production of transgenic non-human animals that contain a modified transferrin fusion construct with increased serum half-life increased serum stability or increased bioavailability of the instant invention is contemplated. Lactoferrin may be used as the Tf portion of the fusion protein so that the fusion protein is produced and secreted in milk.

The successful production of transgenic, non-human animals has been described in a number of patents and publications, such as, for example U.S. Patent 6,291,740 (issued September 18, 2001); U.S. Patent 6,281,408 (issued August 28, 2001); and U.S. Patent 6,271,436 (issued August 7, 2001).

The ability to alter the genetic make-up of animals, such as domesticated mammals including cows, pigs, goats, horses, cattle, and sheep, allows a number of commercial applications. These applications include the production of animals which express large quantities of exogenous proteins in an easily harvested form (*e*.*g*., expression into the milk or blood), the production of animals with increased weight gain, feed efficiency, carcass composition, milk production or content, disease resistance and resistance to infection by specific microorganisms and the production of animals having enhanced growth rates or reproductive performance. Animals which contain exogenous DNA sequences in their genome are referred to as transgenic animals.

The most widely used method for the production of transgenic animals is the microinjection of DNA into the pronuclei of fertilized embryos (Wall et al., J. Cell. Biochem. 49:113 [1992]). Other methods for the production of transgenic animals include the infection of embryos with retroviruses or with retroviral vectors. Infection of both pre- and post-implantation mouse embryos with either wild-type or recombinant retroviruses has been reported (Janenich, Proc. Natl. Acad. Sci. USA 73:1260 [1976]; Janenich et al., Cell 24:519 [1981]; Stuhlmann et al., Proc. Natl. Acad. Sci. USA 81:7151 [1984]; Jahner et al., Proc. Natl. Acad Sci. USA 82:6927 [1985]; Van der Putten et al., Proc. Natl. Acad Sci. USA 82:6148-6152 [1985]; Stewart et al., EMBO J. 6:383-388 [1987]).

An alternative means for infecting embryos with retroviruses is the injection of virus or virus-producing cells into the blastocoele of mouse embryos (Jahner, D. et al., Nature 298:623 [1982]). The introduction of transgenes into the germline of mice has been reported using intrauterine retroviral infection of the midgestation mouse embryo (Jahner *et al*., supra [1982]). Inflection of bovine and ovine embryos with retroviruses or retroviral vectors to create transgenic animals has been reported. These protocols involve the micro-injection of retroviral particles or growth arrested (*i*.*e*., mitomycin C-treated) cells which shed retroviral particles into the perivitelline space of fertilized eggs or early embryos (PCT International Application WO 90/08832 [1990]; and Haskell and Bowen, Mol. Reprod. Dev., 40:386 [1995]. PCT International Application WO 90/08832 describes the injection of wild-type feline leukemia virus B into the perivitelline space of sheep embryos at the 2 to 8 cell stage. Fetuses derived from injected embryos were shown to contain multiple sites of integration.

U.S. Patent 6,291,740 (issued September 18, 2001) describes the production of transgenic animals by the introduction of exogenous DNA into pre-maturation oocytes and mature, unfertilized oocytes (*i*.*e*., pre-fertilization oocytes) using retroviral vectors which transduce dividing cells (*e*.*g*., vectors derived from murine leukemia virus [MLV]). This patent also describes methods and compositions for cytomegalovirus promoter-driven, as well as mouse mammary tumor LTR expression of various recombinant proteins.

U.S. Patent 6,281,408 (issued August 28, 2001) describes methods for producing transgenic animals using embryonic stem cells. Briefly, the embryonic stem cells are used in a mixed cell co-culture with a morula to generate transgenic animals. Foreign genetic material is introduced into the embryonic stem cells prior to co-culturing by, for example, electroporation, microinjection or retroviral delivery. ES cells transfected in this manner are selected for integrations of the gene via a selection marker such as neomycin.

U.S. Patent 6,271,436 (issued August 7, 2001) describes the production of transgenic animals using methods including isolation of primordial germ cells, culturing these cells to produce primordial germ cell-derived cell lines, transforming both the primordial germ cells and the cultured cell lines, and using these transformed cells and cell lines to generate transgenic animals. The efficiency at which transgenic animals are generated is greatly increased, thereby allowing the use of homologous recombination in producing transgenic non-rodent animal species.

### Gene Therapy

The use of modified transferrin fusion constructs for gene therapy wherein a modified transferrin protein or transferrin domain is joined to a therapeutic protein or peptide is contemplated. The modified transferrin fusion constructs with increased serum half-life or serum stability are ideally suited to gene therapy treatments.

The successful use of gene therapy to express a soluble fusion protein has been described. Briefly, gene therapy via injection of an adenovirus vector containing a gene encoding a soluble fusion protein consisting of cytotoxic lymphocyte antigen 4 (CTLA4) and the Fc portion of human immunoglubulin G1 was recently shown in Ijima et al. (Human Gene Therapy (United States) 12/9:1063-77,2001). In this application of gene therapy, a murine model of type II collagen-induced arthritis was successfully treated via intraarticular injection of the vector.

Gene therapy is also described in a number of U.S. patents including U.S. Pat. 6,225,290 (issued May 1, 2001); U.S. Pat. 6,187,305 (issued February 13, 2001); and U.S. Pat. 6,140,111 (issued October 31, 2000).

U.S. Patent 6,225,290 provides methods and constructs whereby intestinal epithelial cells of a mammalian subject are genetically altered to operatively incorporate a gene which expresses a protein which has a desired therapeutic effect. Intestinal cell transformation is accomplished by administration of a formulation composed primarily of naked DNA, and the DNA may be administered orally. Oral or other intragastrointestinal routes of administration provide a simple method of administration, while the use of naked nucleic acid avoids the complications associated with use of viral vectors to accomplish gene therapy. The expressed protein is secreted directly into the gastrointestinal tract and/or blood stream to obtain therapeutic blood levels of the protein thereby treating the patient in need of the protein. The transformed intestinal epithelial cells provide short or long term therapeutic cures for diseases associated with a deficiency in a particular protein or which are amenable to treatment by overexpression of a protein.

U.S. Pat. 6,187,305 provides methods of gene or DNA targeting in cells of vertebrate, particularly mammalian, origin. Briefly, DNA is introduced into primary or secondary cells of vertebrate origin through homologous recombination or targeting of the DNA, which is introduced into genomic DNA of the primary or secondary cells at a preselected site.

U.S. Pat. 6,140,111 (issued October 31, 2000) describes retroviral gene therapy vectors. The disclosed retroviral vectors include an insertion site for genes of interest and are capable of expressing high levels of the protein derived from the genes of interest in a wide variety of transfected cell types. Also disclosed are retroviral vectors lacking a selectable marker, thus rendering them suitable for human gene therapy in the treatment of a variety of disease states without the co-expression of a marker product, such as an antibiotic. These retroviral vectors are especially suited for use in certain packaging cell lines. The ability of retroviral vectors to insert into the genome of mammalian cells has made them particularly promising candidates for use in the genetic therapy of genetic diseases in humans and animals. Genetic therapy typically involves (1) adding new genetic material to patient cells *in vivo*, or (2) removing patient cells from the body, adding new genetic material to the cells and reintroducing them into the body, *i*.*e*., *in vitro* gene therapy. Discussions of how to perform gene therapy in a variety of cells using retroviral vectors can be found, for example, in U.S. Pat. Nos. 4,868,116, issued Sep. 19, 1989, and 4,980,286, issued Dec. 25, 1990 (epithelial cells), WO89/07136 published Aug. 10, 1989 (hepatocyte cells) , EP 378,576 published Jul. 25, 1990 (fibroblast cells), and WO89/05345 published Jun. 15,1989 and WO/90/06997, published Jun. 28, 1990 (endothelial cells).

### Peptide Libraries

An increasingly important aspect of biopharmaceutical drug development and molecular biology is the identification of peptide structures, including the primary amino acid sequences, of peptides or peptidomimetics that interact with biological macromolecules. One method of identifying peptides that possess a desired structure or functional property, such as binding to a predetermined biological macromolecule (*e*.*g*., a receptor), involves the screening of a large library of peptides for individual library members which possess the desired structure or functional property conferred by the amino acid sequence of the peptide.

Screening of combinatorial libraries for potential drugs or therapeutically relevant target antigens is a rapidly growing and important field. Peptide libraries are an important subset of these libraries. However, in order to express and subsequently screen functional peptides in cells, the peptides need to be expressed in sufficient quantities to overcome catabolic mechanisms such as proteolysis. The peptides may also be conformationally stabilized relative to linear peptides to allow a higher binding affinity for their cellular targets. In addition, measuring the expression level of these peptides can be difficult: for example, it may be generally difficult to follow the expression of peptides in specific cells, to ascertain whether any particular cell is expressing a member of the library.

To overcome these problems, U.S. Patent 6,562,617 discloses fusion proteins comprising scaffold proteins, including variants, and random peptides that are fused in such a manner that the structure of the scaffold is not significantly perturbed and the peptide is metabolically and conformationally stabilized. This allows the creation of a peptide library that is easily monitored, both for its presence within cells and its quantity. Thus, the peptides within or fused to a scaffold protein are displayed on or at the surface of the scaffold, therefore being accessible for interaction with potential functional targets.

The present invention is directed to fusions of transferrin (Tf) proteins, including variants, and random peptides that are fused in such a manner that the structure of the peptide is metabolically and conformationally stabilized. The present invention provides Tf as the scaffold protein for generating peptide libraries.

### Scaffold Protein

The present invention provides peptide phage libraries containing Tf fusion proteins which comprise Tf and one or more peptides fused to it wherein the Tf peptide has reduced affinity for a transferrin receptor (TfR). Tf acts as the scaffold protein to stabilize and increase the half-life of the peptides.

By "scaffold protein", "scaffold polypeptide", "scaffold" or grammatical equivalents thereof, herein is meant a protein to which amino acid sequences, such as random peptides, can.be fused. The peptides are exogenous to the scaffold; that is, they are not usually present in the protein. Upon fusion, the scaffold protein usually allows the display of the random peptides in a way that they are accessible to other molecules. Scaffold proteins fall into several classes, including, reporter proteins (which includes detectable proteins, survival proteins and indirectly detectable proteins), and structural proteins.

By "reporter protein" or grammatical equivalents herein is meant a protein that by its presence in or on a cell or when secreted in the media allow the cell to be distinguished from a cell that does not contain the reporter protein. The cell usually comprises a reporter gene that encodes the reporter protein. Reporter genes fall into several classes, as outlined above, including, but not limited to, detection genes, indirectly detectable genes, and survival genes.

The scaffold protein could be a detectable protein. A "detectable protein" or "detection protein" (encoded by a detectable or detection gene) is a protein that can be used as a direct label; that is, the protein is detectable (and preferably, a cell comprising the detectable protein is detectable) without further manipulations or constructs. One embodiment of screening utilizes cell sorting (for example via FACS) to detect scaffold (and thus peptide library) expression. Thus, the protein product of the reporter gene itself can serve to distinguish cells that are expressing the detectable gene. Suitable detectable genes include those encoding autofluorescent proteins.

Reporter proteins are those that allow cells containing the reporter proteins to be distinguished from those that do not. Structural proteins allow the peptides to have different structural biases and are often desirable because different protein or other functional targets may require peptides of different specific structures to interact tightly with their surface or crevice binding sites. Thus, different libraries, each with a different structural bias, may be utilized to maximize the chances of having high affinity members for a variety of different targets. Thus, for example, random peptide libraries with a helical bias or extended structure bias may be made through fusion to the N- terminus and/ or C-terminus of the scaffold Tf proteins. Similarly, random peptide libraries with a coiled coil bias may be made via fusion to the N- and/or C-terminus of the scaffold Tf proteins. Extended conformations of the random library may be made using insertions between dimerizing scaffold Tf proteins. Other embodiments utilize loop formations via insertion into loops in scaffold Tf proteins; amino acid residues within the respective loop structures may be replaced by the random peptide library or the random peptide library may be inserted in between two amino acid residues located within a loop structure.

Accordingly, one may choose Tf scaffolds with the desired structure for generating a library. For example, the scaffold could contain only the N domain of Tf. Also, the present invention includes Tf fused to a reporter protein or to a detectable protein. Alternatively Tf could be tagged for detection.

A scaffold protein such as Tf or the gene encoding it may be wild type or variants thereof. These variants fall into one or more of three classes: substitutional, insertional or deletional variants. These variants ordinarily are prepared by site specific mutagenesis of nucleotides in the DNA encoding the scaffold protein, using cassette or PCR mutagenesis or other techniques well known in the art, to produce DNA encoding the variant, and thereafter expressing the DNA in recombinant cell culture as outlined herein. However, variant protein fragments having up to about 100-150 residues may be prepared by in vitro synthesis using established techniques. Amino acid sequence variants are characterized by the predetermined nature of the variation, a feature that sets them apart from naturally occurring allelic or interspecies variation of the scaffold protein amino acid sequence. The variants typically exhibit the same qualitative biological activity as the naturally occurring analogue, although variants can also be selected which have modified characteristics as will be more fully outlined below.

As discussed earlier, for generating Tf fusion proteins, the peptide may be fused to the N-terminus or C-terminus, or it may be inserted into the Tf scaffold. The peptide may be added to the Tf scaffold or may substitute or replace a portion, such as a loop or part of a loop of the Tf scaffold.

In one aspect of the invention, a random peptide is fused to a Tf scaffold, to form a fusion protein. The fusion protein could also include additional components, including, but not limited to, fusion partners and linkers.

### Generating Peptide Libraries

The isolation of ligands that bind biological targets is fundamental to discovering new therapeutics. The ability to synthesize DNA chemically has made possible the construction of extremely large collections of nucleic acid and peptide sequences as potential ligands, Recently developed methods allow efficient screening of libraries for desired binding activities (see Pluckthun and Ge, 1991, Angew. Chem. Int Ed. Engl. 30:296-298).

Generally, random peptide libraries could be generated to identify either peptides that bind to target molecules of interest or gene products that modify peptides or RNA in a desired fashion. The peptides are produced from libraries of random peptide expression vectors that encode peptides attached to a scaffold protein. A method of affinity enrichment allows a very large library of peptides to be screened and the vector carrying the desired peptide(s) to be selected. The nucleic acid can then be isolated from the vector and sequenced, to deduce the amino acid sequence of the desired peptide. Using these methods, one can identify a peptide as having a desired binding affinity for a molecule. The peptide can then be synthesized in bulk by conventional means.

Random peptide libraries can be used to identify peptides with affinity for a wide variety of target molecules, such as receptors, small molecules, macromolecules, and the like. Examples of peptides include but are not limitated to growth factors, hormones, enzyme substrates, interferons, interleukins, intracellular and intercellular messengers, lectins, cellular adhesion molecules, and the like. The present invention also uses the random peptide libraries to identify analogs and mimetics of these peptides.

U.S. 5,270,170 discloses a random peptide library constructed by transforming host cells with a collection of recombinant vectors that encode a fusion protein comprised of a DNA binding protein and a random peptide and also encode a binding site for the DNA binding protein to be used to screen for novel ligands.

Several approaches to generating and screening large libraries of random or pseudorandom peptide sequences suitable for screening, selection, and identification of desired individual library members have been proposed in the art.

In general, the simplest way to create a large number of diverse sequences involves oligonucleotide synthesis. For example, a random oligonucleotide of length 24 encodes all possible peptides of length 8, a number that exceeds ten billion. Libraries typically range in size from at least several thousand to about one hundred million individual species. Such libraries might involve all possible peptides of length 6, or might involve subsets of libraries composed of longer sequences.

Libraries may also be generated from natural DNA sequences such as mRNA or genomic DNA. Typically such libraries would be biased toward native proteins and protein fragments. Thus, these libraries may contain a significant fraction of sequences that encode polypeptides that interact with native proteins in the cell. When such fragments are inserted into the scaffold, they may fold into a conformation that resembles a domain from the cognate native protein from which they are derived (Bartel P. L., Roecklein J. A., et al. Nat Genet January 1996; 12(1):72-77).

Using known recombinant DNA techniques (see generally, Sambrook et al., 1989, Molecular Cloning, A Laboratory Manual, 2d ed., Cold Spring Harbor Laboratory Press, Cold Spring Harbor, N.Y., 1989, incorporated herein by reference), one can synthesize an oligonucleotide that, inter alia, removes unwanted restriction sites and adds desired ones, reconstructs the correct portions of any sequences that have been removed, inserts the spacer, conserved, or framework residues, if any, and corrects the translation frame (if necessary) to produce an active fusion protein comprised of a scaffold protein and random peptide. The central portion of the oligonucleotide will generally contain one or more random peptide coding sequences (variable region domain) and spacer or framework residues. The sequences are ultimately expressed as peptides (with or without spacer or framework residues) fused to or in the scaffold protein.

The variable region domain of the oligonucleotide encodes a key feature of the library: the random peptide. The size of the library will vary according to the number of variable codons, and hence the size of the peptides, that are desired. Generally, the library will be at least 10⁶ to 10⁸ or more members, although smaller libraries may be quite useful in some circumstances. To generate the collection of oligonucleotides that forms a series of codons encoding a random collection of amino acids and that is ultimately cloned into the vector, a codon motif is used, such as (NNK)ₓ, where N may be A, C, G, or T (nominally equimolar), K is G or T (nominally equimolar), and x is typically up to about 5, 6, 7, or 8 or more, thereby producing libraries of penta-, hexa-, hepta-, and octa-peptides or more. The third position may also be G or C, designated "S". Thus, NNK or NNS (i) codes for all the amino acids, (ii) codes for only one stop codon, and (iii) reduces the range of codon bias from 6:1 to 3:1. There are 32 possible codons resulting from the NNK motif: 1 for each of 12 amino acids, 2 for each of 5 amino acids, 3 for each of 3 amino acids, and only one of the three stop codons. With longer peptides, the size of the library that is generated can become a constraint in the cloning process, but the larger libraries can be sampled.

An exemplified codon motif (NNK)ₓ produces 32 codons, one for each of 12 amino acids, two for each of five amino acids, three for each of three amino acids and one (amber) stop codon. Although this motif produces a codon distribution as equitable as available with standard methods of oligonucleotide synthesis, it results in a bias against peptides containing one-codon residues. For example, a complete collection of hexacodons contains one sequence encoding each peptide made up of only one-codon amino acids, but contains 729 (3⁶) sequences encoding each peptide with three-codon amino acids.

An alternate approach that minimizes the bias against one-codon residues involves the synthesis of 20 activated trinucleotides, each representing the codon for one of the 20 genetically encoded amino acids. These trinucleotides are synthesized by conventional means, removed from the support with the base and 5-OH-protecting groups intact, and activated by the addition of 3'-O-phosphoramidite (and phosphate protection with beta-cyanoethyl groups) by the method used for the activation of mononucleosides, as generally described in McBride and Caruthers, 1983, Tetr. Letters 22:245, which is incorporated herein by reference.

Degenerate "oligocodons" are prepared using these trimers as building blocks. The trimers are mixed at the desired molar ratios and installed in the synthesizer. The ratios will usually be approximately equimolar, but may be a controlled unequal ratio to obtain the over- to under-representation of certain amino acids coded for by the degenerate oligonucleotide collection. The condensation of the trimers to form the oligocodons is done essentially as described for conventional synthesis employing activated mononucleosides as building blocks. See generally, Atkinson and Smith, 1984, Oligonucleotide Synthesis (M. J. Gait, ed.), pp. 35-82. This procedure generates a population of oligonucleotides for cloning that is capable of encoding an equal distribution (or a controlled unequal distribution) of the possible peptide sequences. This approach may be especially useful in generating longer peptide sequences, because the range of bias produced by the (NNK)ₓ motif increases by three-fold with each additional amino acid residue.

When the codon motif is (NNK)ₓ, as defined above, and when x equals 8, there are 2.6 X10¹⁰ possible octapeptides. A library containing most of the octapeptides may be produced, but a sampling of the octapeptides may be more conveniently constructed by making only a subset library using about 0.1%, and up to as much as 1%, 5%, or 10%, of the possible sequences, which subset of recombinant vectors is then screened. As the library size increases, smaller percentages are acceptable. If desired, to extend the diversity of a subset library the recovered vector subset may be subjected to mutagenesis and then subjected to subsequent rounds of screening. This mutagenesis step may be accomplished in two general ways: the variable region of the recovered phage may be mutagenized or additional variable amino acids may be added to the regions adjoining the initial variable sequences.

Examples of other types of random peptide libraries that have been constructed include the following: (NNK)₁₀, TGT(NNK)ₙTGT, (NNK)₂TGT(NNK)₁₄TGT(NNK)₂, TGT(NNK)₉, (NNK)₂TGT(NNK)₁₈.

### Transferrin Peptide Libraries

The present invention provides transferrin (Tf) peptide phage libraries containing a plurality of fusion proteins each comprising a transferrin protein or polypeptide fused to a peptide, wherein the Tf peptide has reduced affinity for a transferrin receptor (TfR). The transferrin protein or polypeptide acts as a scaffold in the peptide library. The transferrin scaffold could be a native transferrin protein or a modified transferrin (mTf) protein or polypeptide. For example, the Tf scaffold could be a lactoferrin or the Tf protein could exhibit reduced glycosylation. The Tf scaffold could comprise at least one amino acid substitution, deletion or addition in any region such as a glycosylation site, iron or receptor binding region, or other areas such as the hinge region.

In one embodiment, the present invention provides a system that utilizes the N domain of Transferrin, M13 and pIII. Other possible combinations are the entire transferrin sequence or derivatives thereof, double N domain (NN) Tf, alternative phage vectors *e*.*g*. T7 or coat proteins *e*.*g*. pVIII on M13.

As discussed earlier for generating Tf fusion proteins, various portions of Tf and mTf could be joined with therapeutic peptides. For example, the Tf scaffold could comprise a portion of the N domain of a Tf protein, a bridging peptide and a portion of the C domain of a Tf peptide. Preferably, the transferrin scaffold comprises or consists of the N domain of a Tf protein. The transferrin scaffold could also comprise a portion or subdomain of the N domain of a Tf protein. Alternatively, the transferrin scaffold comprises or consists of the C domain of a Tf protein. The transferrin scaffold could also comprise a portion of the C domain of a Tf protein.

The peptides in the library could be of various sizes and sequences. For example, the peptides could contain about 6 or more amino acids, about 4 to 30 amino acids, about 6 to 25 amino acids, about 8 to 20 amino acids, about 10 to 18 amino acids, and about 12 to 16 amino acids. Additionally, the peptides could comprise about 6, 9, 12, 16, or 19 amino acids. In some embodiments, the peptides are about 6 amino acids, the size of an epitope.

The peptides could be fused to the C-terminal end of the Tf scaffold or the N-terminal end of the Tf scaffold. The peptides could also be inserted into a loop of a Tf peptide. The peptides could also replace a portion of the Tf peptide. The peptides could also replace or be inserted into one or more of the loops of the Tf scaffold. The present disclosure provides detailed descriptions for making desirable Tf fusion proteins. For instance, peptides could be fused to the Tf scaffold in the same manner as described earlier for Tf fusion proteins.

The peptides could be fused to the Tf scaffold directly or through one or more linkers or spacers. The purpose of the linker or spacer is to allow the peptide to retain its active conformation for binding to its target. The linker or spacer may comprise polyglycine residues for flexibilty or polyproline residues for rigiditiy or a combination of both. The linker or spacer may also include Cys residues for added stability by the formation of disulfide bridges. The linker or spacer may also include hydrophilic residues such as Thr, His, Asn, Gln, Arg, Glu, Asp, Met, Lys, *etc.* The linker or spacer may also comprise hydrophobic residues such as Phe, Leu, Ile, Gly, Val, Ala, *etc*. The number of residues in the linker or spacer will vary depending on the fusion protein, and/or the random peptides

There are many advantages to using the Tf as the scaffold protein in a peptide library. First of all, Tf is an endogenous protein and can be used directly for therapeutic purposes compared to other systems such as standard phage display which utilizes a completely foreign protein which cannot be used therapeutically because it will cause an immunogenic reaction. In a phage display library, the Tf protein can be inserted into the phage protein with a peptide component, and in some instances, a Tf protein can replace the phage protein scaffold. Second, peptides have a short *in vivo* half-life and are not useful therapeutically. Since Tf and mTf have long circulating half-lives, peptides fused to Tf have extended half-life. The peptides in the Tf peptide libraries can have significant *in vivo* half-life due to the presence of the Tf backbone. Third, peptides are often insoluble in aqueous solutions, while Tf is very soluble, even at high concentrations. The peptides in the Tf peptide libraries are soluble because they may contribute little to the overall physical properties of the fusion protein. Thus, Tf is a perfect scaffold for insoluble peptides in aqueous solutions. Fourth, Tf can be made in microbial systems such as yeast which allows for large scale production and screening of large peptide libraries. Fifth, generally peptide libraries are made with the peptides localized in a specific three dimensional configuration, unlike the Tf peptide libraries of the present invention. Since various regions of Tf can be used to localize peptide libraries such as the N and C termini and the exposed loops within the protein, one can construct libraries with free moving peptides at the N or C terminus and structurally constrained peptides engineered within transferrin. Sixth, the transferrin scaffold allows for multiple copies of single peptides sequences or multiple different peptides to be engineered on the same Tf molecule. In fact, the Tf scaffold could contain other peptide moities that facilitates screening, identification, or production of the peptide.

There exists a number of libraries for producing and screening peptides. Numerous peptides with binding affinity to specific ligands have been identified using these libraries. However, the final product identified from these libraries is usually a short peptide with limited therapeutic use. Also, in some libraries, such as a standard phage display library, the peptide functions in the context of the phage protein, but the peptide may be inactive when excised, or even when re-inserted into a new scaffold protein. On the other hand, using the Tf peptide library of the present invention, one can identify a peptide on the Tf or mTf which binds the desired molecules or has the desired property, wherein the peptide moiety is already contained in the ultimate therapeutic protein, *i*.*e*., the Tf fusion protein. Additionally, the production of the identified peptide can be scaled up for therapeutic purposes. If the Tf scaffold is a portion of Tf, such as the N domain of Tf, the selected peptide may be inserted into analogous site in full length Tf to provide a derivative of full length Tf with binding properties conferred by the inserted peptide.

The present invention provides Tf and mTf constructs for generating various Tf or mTf peptide libraries. The Tf and mTf constructs contain various restriction sites for addition, insertion, or substitution of random peptides. It is within the skill of the artisan to generate random peptide constructs that could be added to or inserted into the Tf and mTf constructs in the proper orientation.

The library can then be screened for the peptides with the desired affinity to a specific molecule. Once the clone encoding the peptide is identified, it is grown to a large scale and purified for *in vivo* and/or *in vitro* studies.

### Vectors

The present invention preferably employs an expression vector capable of producing high levels of the peptide or protein fragment displayed on a Tf scaffold. The choice of promoter used to drive expression of the Tf scaffold depends on the assay used for screening. In general, strong promoters are preferred, because they will facilitate higher expression levels of library sequences in the chosen host cells. Such promoters may be derived from housekeeping genes that are expressed at high levels in most or all cell types in the organism, or from viruses. Numerous such cis regulatory sequences are known in the art, suitable for driving expression in mammalian cells, insect cells, plant cells, fungi or bacteria (Ausubel *et al*., 1996). For example, in eukaryotes the promoter for beta actin is useful (Qin Z., Kruger-Krasagakes S. et al., J. Exp. Med. 178:355-360); in plants the Cauliflower Mosaic Virus 35S promoter (Goddijn O. J., Pennings E. J., et al., Transgenic Res. 1995 4:315-323). In mammalian cells, the cytomegalovirus (CMV) promoter is commonly used; and in general, a promoter that drives high level expression of, *e*.*g*., a housekeeping or viral gene can be identified with relative ease using current molecular genetic methods.

In a peptide library, the recombinant vectors are constructed so that the random peptide is expressed as a fusion product; the peptide is fused to a scaffold protein. The peptide may also be inserted into the Tf scaffold.

DNA sequences generated as synthetic oligonucleotides or as cDNA or genomic DNA can be inserted into appropriate expression vectors in a variety of ways. Such methods for vector and insert preparation, ligation, and transformation are known in the art (Ausubel *et al*., supra). In general, it is necessary to produce a vector that has an appropriate restriction site for inserting foreign DNA into the scaffold gene, to produce a linear vector such that the site is available for ligation, to mix the vector and library insert DNAs together under suitable reaction conditions, to permit the ligation to proceed for sufficient time, and to introduce the ligated material into a suitable host such as, *e*.*g*., *E. coli* such that individual clones (preferably a few million) can be selected for further experiments.

### Spacer and Linkers

The Tf-random peptide fusion protein in a peptide library may be connected by a spacer or a linker. The spacer or linker serves to place the two molecules of the fusion protein in a preferred configuration. The spacer or linker residues may be somewhat flexible, comprising polyglycine, for example, to provide the diversity domains of the library with the ability to interact with sites in a large binding site relatively unconstrained by attachment to the Tf scaffold. Rigid spacers, such as, *e*.*g*., polyproline, may also be inserted separately or in combination with other spacers, including glycine residues. The variable domains can be close to one another with a spacer serving to orient the one variable domain with respect to the other, such as by employing a turn between the two sequences, as might be provided by a spacer of the sequence Gly--Pro--Gly, for example. To add stability to such a turn, it may be desirable or necessary to add Cys residues at either or both ends of each variable region. The Cys residues would then form disulfide bridges to hold the variable regions together in a loop, and in this fashion may also serve to mimic a cyclic peptide. Of course, those skilled in the art will appreciate that various other types of covalent linkages for cyclization may also be accomplished.

The spacer residues described above can also be encoded on either or both ends of the variable nucleotide region. For instance, a cyclic peptide coding sequence can be made without an intervening spacer by having a Cys codon on both ends of the random peptide coding sequence. As above, flexible spacers, *e*.*g*., polyglycine, may facilitate interaction of the random peptide with the selected targets. Alternatively, rigid spacers may allow the peptide to be presented as if on the end of a rigid arm, where the number of residues, *e*.*g*., Pro, determines not only the length of the arm but also the direction for the arm in which the peptide is oriented. Hydrophilic spacers, made up of charged and/or uncharged hydrophilic amino acids, (*e*.*g*., Thr, His, Asn, Gln, Arg, Glu, Asp, Met, Lys, *etc*.), or hydrophobic spacers made up of hydrophobic amino acids (*e*.*g*., Phe, Leu, Ile, Gly, Val, Ala, etc.) may be used to present the peptides to binding sites with a variety of local environments.

For example, one can construct a random peptide library that encodes a DNA binding protein, such as the lac repressor or a cysteine depleted lac repressor, a random peptide of formula NNK₅ (sequences up to and including NNK₁₀ or NNK₁₅ could also be used) fused to Tf, and a peptide ligand of known specificity. One would then screen the library for improved binding of the peptide ligand to the receptor specific for the ligand using the method of the present invention; fusion proteins that exhibit improved specificity would be isolated together with the vector that encodes them, and the vector would be sequenced to determine the structure of the spacer responsible for the improved binding.

### Screening of Peptide Library

The number of possible target molecules for which peptide ligands may be identified by screening peptide libraries is virtually unlimited. For example, the target molecule may be an antibody (or a binding portion thereof). The antigen to which the antibody binds may be known and perhaps even sequenced, in which case the invention may be used to map epitopes of the antigen. If the antigen is unknown, such as with certain autoimmune diseases, for example, sera, fluids, tissue, or cell from patients with the disease can be used in the present screening method to identify peptides, and consequently the antigen, that elicits the autoimmune response. Once a peptide has been identified, that peptide can serve as, or provide the basis for, the development of a vaccine, a therapeutic agent, a diagnostic reagent, *etc*.

Screening may be performed by using one of the methods well known to the practitioner in the art, such as phage-display, selectively infective phage, to screen for binding, assay systems for enzymatic activity or protein stability. Polypeptides and peptides having the desired property can be identified by sequencing of the corresponding nucleic acid sequence or by amino acid sequencing or mass spectrometry. In the case of subsequent optimization, the nucleic acid sequences encoding the initially selected polypeptides and peptides can optionally be used without sequencing. Optimization is performed by repeating the replacement of sub-sequences by different sequences, preferably by random sequences, and the screening step one or more times.

A Tf peptide library is especially useful in screening for peptides that bind to a target molecule of interest. As an example, a screening method may comprise the steps of (a) lysing the cells transformed with the Tf peptide library under conditions such that the fusion protein remains bound to the vector that encodes the fusion protein; (b) contacting the Tf fusion proteins of the peptide library with a receptor under conditions conducive to specific peptide--receptor binding; and (c) isolating the vector that encodes a peptide that binds to said receptor. By repetition of the affinity selection process one or more times, the vectors that encode the peptides of interest may be enriched. By increased stringency of the selection, peptides of increasingly higher affinity can be identified. If the presence of cytoplasmic or periplasmic proteins interferes with binding of fusion protein to target molecule, then partial purification of fusion protein-plasmid complexes by gel filtration, affinity, or other purification methods can be used to prevent such interference.

Once a Tf peptide library is constructed, host cells are transformed with the library vectors. The successful transformants are typically selected by growth in a selective medium or under selective conditions, *e*.*g*., an appropriate antibiotic, such as ampicillin or others depending on the vector used. This selection may be done on solid or in liquid growth medium. For growth of bacterial cells on solid medium, the cells are grown at a high density (about. 10⁸ to 10⁹ transformants per m²) on a large surface of, for example, L-agar containing the selective antibiotic to form essentially a confluent lawn. For growth in liquid culture, cells may be grown in L-broth (with antibiotic selection) through about 10 or more doublings. Growth in liquid culture may be more convenient because of the size of the libraries, while growth on solid media likely provides less chance of bias during the amplification process.

At some point during the growth of the transformants, the fusion protein will be expressed. The cells containing a library are lysed, and the complexes are partially purified away from cell debris. Following cell lysis, one should avoid cross reaction between unbound fusion proteins of one cell with heterologous DNA molecules of another cell.

After cell lysis, in a process called panning, plasmid-peptide complexes that bind specifically to immobilized receptors are separated from nonbinding complexes, which are washed away. Bulk DNA can be included during the lysis and panning steps to compete for non-specific binding sites and to lower the background of non-receptor-specific binding to the immobilized receptor. A variety of washing procedures can be used to enrich for retention of molecules with desired affinity ranges. For affinity enrichment of desired clones, from about 10² to 10⁶ library equivalents (a library equivalent is one of each recombinant; 10⁴ equivalents of a library of 10⁹ members is 10¹³ vectors), but typically 10³ to 10⁴ library equivalents, are incubated with a receptor (or portion thereof) for which a peptide ligand is desired. The receptor is in one of several forms appropriate for affinity enrichment schemes. In one example the receptor is immobilized on a surface or particle, and the library is then panned on the immobilized receptor generally according to the procedure described below.

The screening process involves reacting the Tf peptide library with the target of interest to establish a baseline binding level against which the binding activities of subsequent peptide libraries are compared. Binding may be determined by a variety of well known assay means, *e*.*g*., by ELISA, competition binding assays when the target's native binding partner is known, sandwich assays, radioreceptor assays using a radioactive ligand whose binding is blocked by the peptide library, *etc*. The nature of the assay is not critical so long as it is sufficiently sensitive to detect small quantities of peptide binding to or competing for binding to the target. The assay conditions may be varied to take into account optimal binding conditions for different binding substances of interest or other biological activities. Thus, the pH, temperature, salt concentration, volume and duration of binding, *etc.* may all be varied to achieve binding of peptide to target under conditions which resemble those of the environment of interest.

Once it is determined that the Tf peptide library possesses a peptide or peptides which bind to the target of interest, the iterative methods of the invention can be used to identify the sequence of the peptide(s) in the mixture. The amino acids are divided into groups, conveniently three groups of approximately even number in size. For example, the proportion of the first group (designated "α") is decreased, the proportion of the second group (designated "β") is increased, and the proportion of the third group (designated "γ") unchanged. When the concentration of a group is changed, it may be decreased to the point of being completely omitted, or may be increased to two or three times the molar concentration of the other group(s). The effect of changing the concentration of amino acids in a particular group for each position in the peptide is then determined, and the contribution of those amino acids in that group determined for that position in the peptide. Based on these determinations, the process is repeated using subsets of the contributing groups at each position, until ultimately the sequence of one or more peptides in the mixture which bind to the ligand is determined.

### Diversifying a Selected Random Peptide

Once a peptide ligand of interest has been identified, a variety of techniques can be used to diversify a Tf peptide library to construct ligands with improved properties. In one approach, the positive vectors (those identified in an early round of panning) are sequenced to determine the identity of the active peptides. Oligonucleotides are then synthesized based on these peptide sequences, employing all bases at each step at concentrations designed to produce slight variations of the primary oligonucleotide sequences. This mixture of (slightly) degenerate oligonucleotides is then cloned into the peptide library expression vector. This method produces systematic, controlled variations of the starting peptide sequences but requires, however, that individual positive vectors be sequenced before mutagenesis. This method is useful for expanding the diversity of small numbers of recovered vectors.

Another technique for diversifying a selected peptide involves the subtle misincorporation of nucleotide changes in the coding sequence for the peptide through the use of the polymerase chain reaction (PCR) under low fidelity conditions. A protocol described in Leung et al., 1989, Technique 1:11-15, utilizes altered ratios of nucleotides and the addition of manganese ions to produce a 2% mutation frequency.

Yet another approach for diversifying a selected random peptide vector involves the mutagenesis of a pool, or subset, of recovered vectors. Recombinant host cells transformed with vectors recovered from panning are pooled and isolated. The vector DNA is mutagenized by treating the cells with, *e*.*g*., nitrous acid, formic acid, hydrazine, or by use of a mutator strain. These treatments produce a variety of mutations in the vector DNA. The segment containing the sequence encoding the variable peptide can optionally be isolated by cutting with restriction endonuclease(s) specific for sites flanking the variable region and then recloned into undamaged vector DNA. Alternatively, the mutagenized vectors can be used without recloning of the mutagenized random peptide coding sequence.

In the second general approach for diversifying a set of peptide ligands, that of adding additional amino acids to a peptide or peptides found to be active, a variety of methods are available. In one, the sequences of peptides selected in early panning are determined individually and new oligonucleotides, incorporating all or part of the determined sequence and an adjoining degenerate sequence, are synthesized. These are then cloned to produce a secondary Tf library.

In another approach that adds a second variable region to a pool of random peptide expression vectors, a restriction site is installed next to the primary variable region. Preferably, the enzyme should cut outside of its recognition sequence, such as *Bsp*MI, which cuts leaving a four base 5' overhang, four bases to the 3' side of the recognition site. Thus, the recognition site may be placed four bases from the primary degenerate region. To insert a second variable region, a degenerately synthesized oligonucleotide is then ligated into this site to produce a second variable region juxtaposed to the primary variable region. This secondary library is then amplified and screened as before.

While in some instances it may be appropriate to synthesize peptides having contiguous variable regions to bind certain receptors, in other cases it may be desirable to provide peptides having two or more regions of diversity separated by spacer residues. For example, the variable regions may be separated by spacers that allow the diversity domains of the peptides to be presented to the receptor in different ways. The distance between variable regions may be as little as one residue or as many as five to ten to up to about 100 residues. For example, for probing a large binding site, one may construct variable regions separated by a spacer containing 20 to 30 amino acids. The number of spacer residues, when present, will preferably be at least two to three or more but usually will be less than eight to ten. An oligonucleotide library having variable domains separated by spacers can be represented by the formula: (NNK)_{y} --(abc)ₙ --(NNK)_{z}, where N and K are as defined previously (note that S as defined previously may be substituted for K); y+z is equal to about 5, 6, 7, 8, or more; a, b and c represent the same or different nucleotides comprising a codon encoding spacer amino acids; and n is up to about 20 to 30 codons or more. Alternatively, a second variable region may be inserted in an adjacent exposed loop of the Tf scaffold. Furthermore, a second variable region may be inserted in a non-adjacent surface-exposed loop of the Tf-scaffold. Other additional variable regions may be inserted in the adjacent exposed loop of the Tf scaffold or the non-adjacent surface-exposed loop of the Tf scaffold.

Unless modified during or after synthesis by the translation machinery, recombinant Tf peptide libraries consist of sequences of the 20 normal L-amino acids. While the available structural diversity for such a library is large, additional diversity can be introduced by a variety of means, such as chemical modifications of the amino acids. For example, as one source of added diversity a C-terminal Tf peptide library of the invention can be subjected to carboxy terminal amidation. Carboxy terminal amidation is necessary to the activity of many naturally occurring bioactive peptides. This modification occurs *in vivo* through cleavage of the N-C bond of a carboxy terminal Gly residue in a two-step reaction catalyzed by the enzymes peptidylglycine alpha-amidation monooxygenase (PAM) and hydroxyglycine aminotransferase (HGAT). See, Eipper et al., 1991, J. Biol. Chem. 266:7827-7833; Mizuno et al., 1986, Biochem. Biophys. Res. Comm. 137(3): 984-991; Murthy et al., 1986, J. Biol. Chem. 261(4): 1815-1822; Katopodis et al., 1990, Biochemistry 29:6115-6120; and Young and Tamburini, 1989, J. Am. Chem. Soc. 111:1933-1934, each of which are incorporated herein by reference.

Amidation can be performed by treatment with enzymes, such as PAM and HGAT, *in vivo* or *in vitro*, and under conditions conducive to maintaining the structural integrity of the fusion protein. In a random C-terminal Tf peptide library of the present invention, amidation will occur on a library subset, *i*.*e*., those peptides having a carboxy terminal Gly. A library of peptides designed for amidation can be constructed by introducing a Gly codon at the end of the variable region domain of the library. After amidation, an enriched library serves as a particularly efficient source of ligands for receptors that preferentially bind amidated peptides. Many of the C-terminus amidated bioactive peptides are processed from larger pro-hormones, where the amidated peptide is flanked at its C-terminus by the sequence --Gly--Lys--Arg--X ... (SEQ ID NO: 35) (where X is any amino acid). Oligonucleotides encoding the sequence --Gly--Lys--Arg--X--Stop can be placed at the 3' end of the variable oligonucleotide region. When expressed, the Gly--Lys--Arg--X (SEQ ID NO: 35) is removed by *in vivo* or *in vitro* enzymatic treatment, and the peptide library is carboxy terminal amidated.

Other modifications found in naturally occurring peptides and proteins can be introduced into the Tf libraries to provide additional diversity and to contribute to a desired biological activity. For example, the variable region library can be provided with codons that code for amino acid residues involved in phosphorylation, glycosylation, sulfation, isoprenylation (or the addition of other lipids), *etc.* Modifications not catalyzed by naturally occurring enzymes can be introduced by chemical means (under relatively mild conditions) or through the action of, *e*.*g*., catalytic antibodies and the like. In most cases, an efficient strategy for library construction involves specifying the enzyme (or chemical) substrate recognition site within or adjacent to the variable nucleotide region of the library so that most members of the library are modified. The substrate recognition site added can be simply a single residue (*e*.*g*., serine for phosphorylation) or a complex consensus sequence, as desired.

Conformational constraints, or scaffolding, can also be introduced into the structure of the peptide libraries. A number of motifs from known protein and peptide structures can be adapted for this purpose. The method involves introducing nucleotide sequences that code for conserved structural residues into or adjacent to the variable nucleotide region so as to contribute to the desired peptide structure. Positions nonessential to the structure are allowed to vary.

As an example, U.S. Patent 5,824,483 discloses combinatorial libraries of different-sequence peptide members. The libraries are comprised of stabilized, alpha-helical polypeptides having a similar tertiary structure but different amino acid residues at specific, "variable" positions in the sequence. The polypeptides are stabilized through coiled-coil interactions with other α-helical polypeptides and/or via intrahelical lactam bridges. Also disclosed are methods for using such libraries to screen for selected macromolecular ligands.

### Phage Display Libraries

A "phage-display library" is a protein expression library, for instance constructed in an M13-derived vector, that expresses a collection of cloned protein sequences in this case a Tf peptide library as fusions with a phage coat protein. Thus, in the present invention, the transferrin fusion proteins comprising peptides are expressed on the exterior of the phage particle. This allows, for instance, contact and binding between the peptide on the fusion protein and an immobilized target molecule. Those having ordinary skill in the art will recognize that phage clones expressing peptide binding proteins specific for a ligand or receptor can be substantially enriched by serial rounds of phage binding to the immobilized ligand, dissociation from the immobilized ligand and, amplification by growth in bacterial host cells.

Over recent years, many publications have reported the use of phage-display technology to produce and screen libraries of polypeptides for binding to a selected target. See, *e*.*g*., Cwirla et al., Proc. Natl. Acad. Sci. USA 87, 6378-6382 (1990); Devlin et al., Science 249, 404-406 (1990), Scott & Smith, Science 249, 386-388 (1990); Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phages displaying a polypeptide with affinity to a target bind to the target. These phages are enriched by affinity screening to the target. The identity of polypeptides displayed from these phage can be determined from their respective genomes. Using these methods a polypeptide identified as having a binding affinity for a desired target can then be synthesized in bulk by conventional means.

There are a number of types of phage display library, including the non-lytic phage display and the lytic phage display. The non-lytic phage display is derived from M13 bacterial filamentous phage and employs the pIII, pVIII, or pVI protein of the phage and is preferably fused with random peptides, custom-designed peptides or protein, and antibodies (single chain Fv Fusion to pIII). The Lytic phage display is based on λ, T7 or T4 phage.

As mentioned, application of efficient screening techniques to peptides requires the establishment of a physical or logical connection between each Tf peptide and the nucleic acid that encodes the Tf fusion peptide. After rounds of affinity enrichment, such a connection allows identification, usually by amplification and sequencing, of the genetic material encoding interesting peptides. The fusion phage approach of Parmley and Smith, 1988, Gene 73:305-318, can be used to screen proteins. Others have described phage based systems in which the peptide is fused to the pIII coat protein of filamentous phage (see Scott and Smith, 1990, Science 249:386-390; Devlin et al., 1990, Science 249:404-406; and Cwirla et al., 1990, Proc. Natl. Acad. Sci. USA 87:6378-6382).

In these latter publications, the authors describe expression of a peptide at the amino terminus of or internal to the pIII protein. The connection between peptide and the genetic material that encodes the peptide is established, because the fusion protein is part of the capsid enclosing the phage genomic DNA. Phage encoding peptide ligands for receptors of interest can be isolated from libraries of greater than 10⁸ peptides after several rounds of affinity enrichment followed by phage growth.

Generally, the display of a peptide sequence, antibody, or other protein is on the surface of a bacteriophage particle or cell. Each bacteriophage particle or cell serves as an individual library member displaying a single species of displayed peptide in addition to the natural bacteriophage or cell protein sequences. Each bacteriophage or cell contains the nucleotide sequence information encoding the particular displayed peptide sequence; thus, the displayed peptide sequence can be ascertained by nucleotide sequence determination of an isolated library member.

Tf peptide display methods include the presentation of a Tf scaffold fused with a peptide sequence on the surface of a filamentous bacteriophage, typically as a fusion with a bacteriophage coat protein. The bacteriophage library can be incubated with an immobilized, predetermined target molecule (*e*.*g*., a receptor or small molecule) so that bacteriophage particles which present a peptide sequence that binds to the immobilized macromolecule can be differentially partitioned from those that do not present peptide sequences that bind to the predetermined macromolecule. The bacteriophage particles (*i*.*e*., library members) which are bound to the immobilized macromolecule are then recovered and replicated to amplify the selected bacteriophage subpopulation for a subsequent round of affinity enrichment and phage replication. After several rounds of affinity enrichment and phage replication, the bacteriophage library members that are thus selected are isolated and the nucleotide sequence encoding the displayed peptide sequence is determined, thereby identifying the sequence(s) of peptides that bind to the predetermined macromolecule (*e*.*g*., receptor). Such methods are further described in PCT patent publication Nos. 91/17271, 91/18980, and 91/19818 and 93/08278.

Phage display technology has also been used to produce and screen libraries of heterodimeric proteins, such as Fab fragments and such systems can be used to produce and screen Tf peptides libraries of the invention. See *e*.*g*., Garrard et al., Bio/Tech 9, 1373-1377 (1991). Phage display libraries of Fab fragments are produced by expressing one of the component chains as a fusion with a coat protein, as for display of single-chain polypeptides. The partner antibody chain is expressed in the same cell from the same or a different replicon as the first chain, and assembly occurs within the cell. Thus, a phage-Fab fragment has one antibody chain fused to a phage coat protein so that it is displayed from the outersurface of the phage and the other antibody chain is complexed with the first chain. The present invention also provides Tf peptide phage display libraries comprising Tf scaffold fused to antibody fragments, such as CDRs.

U.S. Patent 6,555,310 discloses two related but self-sufficient improvements in conventional display methods. The first improvement provides methods of enriching conventional display libraries for members displaying more than one copy of a polypeptide prior to affinity screening of such libraries with a target of interest. These methods can achieve diverse populations in which the vast majority of members retaining full-length coding sequences encode polypeptides having specific affinity for the target. In a second aspect, the invention provides methods of subcloning nucleic acids encoding displayed polypeptides of enriched libraries from a display vector to an expression vector without the need for clonal isolation of individual members. These methods result in polyclonal libraries of antibodies and other polypeptides for use, *e*.*g*., as diagnostic or therapeutic reagents.

The surface expression Tf libraries of the invention may be screened for specific peptides which bind target molecules by standard affinity isolation procedures. Such methods include, for example, panning, affinity chromatography and solid phase blotting procedures. Panning as described by Parmley and Smith, Gene 73:305-318 (1988), which is incorporated herein by reference, is preferred because high titers of phage can be screened easily, quickly and in small volumes. Furthermore, this procedure can select minor peptide species within the population, which otherwise would have been undetectable, and amplified to substantially homogenous populations. The selected peptide sequences can be determined by sequencing the nucleic acid encoding such peptides after amplification of the phage population.

Random oligonucleotides synthesized by any of the methods described previously can also be expressed on the surface as a Tf fusion of filamentous bacteriophage, such as M13, for example, without the joining together of precursor oligonucleotides. A vector such as M13IX30 can be used. This vector exhibits all the functional features of the combined vector for surface expression of gVIII-peptide fusion proteins. The complete nucleotide sequence for M13IX30 is disclosed in U.S. Patent 6,258,350 which is incorporated by reference in its entirety.

M13IX30 contains a wild type gVIII for phage viability and a pseudo gVIII sequence for peptide fusions. The vector also contains in frame restriction sites for cloning random peptides. The cloning sites in this vector are Xho I, Stu I and Spe I. Oligonucleotides should therefore be synthesized with the appropriate complementary ends for annealing and ligation or insertional mutagenesis. Alternatively, the appropriate termini can be generated by PCR technology. Between the restriction sites and the pseudo gVIII sequence is an in-frame amber stop codon, again, ensuring complete viability of phage in constructing and manipulating the library. Expression and screening is performed as described above for the surface expression library of oligonucleotides generated from precursor portions.

### ScFv Libraries

The present invention provides phage display libraries comprising single-chain variable regions fused to Tf, mTf, and domains, subdomains and portions thereof. Recently, systems in which diverse peptide sequences are displayed on the surface of filamentous bacteriophage (Scott and Smith (1990) Science 249 : 386) have proven attractive for forming various combinations of antibody heavy chain variable regions and light chain variable regions (and the polynucleotide sequences encoding them) for *in vitro* selection and enrichment by binding to specific antigen. Polynucleotide sequences encoding heavy and light chain variable regions are linked to gene fragments that encode signals that direct them to the periplasmic space of *E. coli* and the resultant "antibodies" are displayed on the surface of bacteriophage, typically as fusions to bacteriophage coat proteins (*e*.*g*., pIII or pVIII). Variable region fragments of immunoglobulins (either Fv or Fab) can be displayed externally on phage capsids (phagebodies) and recombinant phage are selected for by binding to immobilized antigen.

Various embodiments of bacteriophage antibody display libraries and lambda phage expression libraries have been described (Kang et al. (1991) Proc. Natl. Acad. Sci. (U.S.A.) 88: 4363; Clackson et al. (1991) Nature 352: 624; McCafferty et al. (1990) Nature 348: 552; Burton et al. (1991) Proc. Natl. Acad. Sci. (U.S.A.) 88: 10134; Hoogenboom et al. (1991) Nucleic Acids Res. 19: 4133; Chang et al. (1991) J. Immunol. 147: 3610; Breitling et al. (1991) Gene 104: 147; Marks et al. (1991) J. Mol. Biol. 222: 581; Barbas et al. (1992) Proc. Natl. Acad. Sci. (U.S.A.) 89: 4457; Hawkins and Winter (1992) J. Immunol. 22: 867; Marks et al. (1992) Biotechnology 10: 779; Marks et al. (1992) J. Biol. Chem. 267: 16007; Lowman et al. (1991) Biochemistry 30: 10832; Lerner et al. (1992) Science 258: 1313).

One particularly advantageous approach has been the use of so-called single-chain fragment variable (ScFv) libraries (Marks et al. (1992) Biotechnology 10: 779; Winter G and Milstein C (1991) Nature 349: 293; Clackson *et al*. (1991) op.cit.; Harks et al. (1991) J. Mol. Biol. 222: 581; Chaudhary et al. (1990) Proc. Natl. Acad. Sci. (USA) 87: 1066; Chiswell et al. (1992) TIBTECH 10: 80; McCafferty *et al*. (1990) op.cit.; and Huston et al. (1988) Proc. Natl. Acad. Sci. (USA) 85:, 5879). Various embodiments of scFv libraries displayed on bacteriophage coat proteins have been described.

Beginning in 1988, single-chain analogues of Fv fragments and their fusion proteins have been reliably generated by antibody engineering methods. The first step generally involves obtaining the genes encoding V_{H} and V_{L} domains with desired binding properties; these V genes may be isolated from a specific hybridoma cell line, selected from a combinatorial V-gene library, or made by V gene synthesis. The single-chain Fv is formed by connecting the component V genes with an oligonucleotide that encodes an appropriately designed linker peptide, such as (Gly-Gly-Gly-Gly-Ser)₃ (SEQ ID NO: 36) or equivalent linker peptide(s). The linker bridges the C-terminus of the first V region and N-terminus of the second, ordered as either V_{H}-linker- V_{L} or V_{L}-linker-V_{H}. In principle, the ScFv binding site can faithfully replicate both the affinity and specificity of its parent antibody combining site.

The components from SCA can be fused to the N-, C- or N- and C- termini of transferrin or modified transferrin (V_{L}, V_{H} and/or one or more CDR regions). These fusions could also be carried out using different parts or domains of transferrin such as the N domain or C domain. The proteins could be fused directly or using a linker peptide of various length. It is also possible to fuse all or part of the active SCA within the scaffold of transferrin. In such instances the fusion protein is made by inserting the cDNA of the SCA within the cDNA of transferrin for production of the protein in cells.

In one embodiment, two V_{H} or two V_{L} regions could be attached to the two ends of or inserted into transferrin or modified transferrin. In another embodiment, one V_{H} and one V_{L} could be attached to or inserted in transferrin or modified transferrin. The variable regions could be connected to each other through a linker (L) and then fused to or inserted into transferrin. The linker is a molecule that is covalently linked to the variable domains for ease of attachment to or insertion into Tf. Together the linker and Tf provides enough spacing and flexibility between the two domains such that they are able to achieve a conformation in which they are capable of specifically binding the epitope to which the two domains, V_{H} and V_{L}, are directed. Additionally, transferrin can be modified so that the variable regions attached to the two termini can come close together. Examples of such modification include but are not limited to removal of C-terminal proline and/or the cystine loop close to the C-terminus of Tf to give more flexibility.

The present invention also contemplates multivalent Tf /scFv fusion proteins. Antibody variable regions having the order of V_{H} -L- V_{H} could be fused to one end of the transferrin and variable regions having the order V_{L}-L- V_{L} could be fused to the same transferrin at the other terminus. Other sequences of variable regions forming multivalent SCA are also contemplated by the present invention. Examples include, but are not limited to, V_{H} -L- V_{L} and V_{L} -L- V_{H} and those having more variable domains linked together. The variable regions and linkers could also be inserted into the transferrin molecule.

Alternatively, the multivalent antibody variable regions can be formed by inserting variable domains in the transferrin or modified transferrin molecule without using any nonnatural peptide linkers. In this way, the portions of the transferrin molecule act as linkers to provide spacing and flexibility between the variable domains.

As used herein, the term "trans-bodies" refers to transferrin with antibody activity. A trans-body comprises at least one antibody variable region and a transferrin molecule, modified transferrin molecule, or a fragment thereof. Trans-bodies may additionally comprise one or more antigenic peptides that are capable of inducing an immune response in a host. In one aspect of the invention, the variable regions binding the same antigen can be fused to the different termini of the same transferrin or modified transferrin molecule. In another aspect of the invention, variable regions that bind different antigens can be fused to the different termini of the same transferrin or modified transferrin molecule. Such trans-bodies can bridge two different antigens or bind and/or activate two different cells. Thus, the present invention provides chimeric antibody variable regions fused to transferrin or modified transferrin. Moreover, the variable regions can be inserted into a transferrin or modified transferrin molecule.

Thus, scFv fragments comprised of V_{H} and V_{L} domains are linked into a single polypeptide chain by a flexible linker peptide. After the scFv genes are assembled, they are cloned into a phagemid and expressed at the tip of the M13 phage (or similar filamentous bacteriophage) as fusion proteins with, for example, the bacteriophage pIII (gene 3) coat protein. Enriching for phage expressing an antibody of interest is accomplished by panning the recombinant phage displaying a population scFv for binding to a predetermined epitope (*e*.*g*., target antigen, receptor). As an example, in the present invention, the ScFv fragments may be fused to Tf and expressed at the tip of the M13 phage.

Various methods have been reported for increasing the combinatorial diversity of a scFv library to broaden the repertoire of binding species (idiotype spectrum). The use of PCR has permitted the variable regions to be rapidly cloned either from a specific hybridoma source or as a gene library from non-immunized cells, affording combinatorial diversity in the assortment of V_{H} and V_{L} cassettes which can be combined. Furthermore, the V_{H} and V_{L} cassettes can themselves be diversified, such as by random, pseudorandom, or directed mutagenesis. Typically, V_{H} and V_{L} cassettes are diversified in or near the complementarity-determining regions (CDRs), often the third CDR, CDR3. Enzymatic inverse PCR mutagenesis has been shown to be a simple and reliable method for constructing relatively large libraries of scFv site-directed mutants (Stemmer et al. (1993) Biotechniques 14: 256), as has error-prone PCR and chemical mutagenesis (Deng et al. (1994) J. Biol. Chem. 269: 9533). Riechmann et al. (1993) Biochemistry 32: 8848 showed semirational design of an antibody scFv fragment using site-directed randomization by degenerate oligonucleotide PCR and subsequent phage display of the resultant scFv mutants. Barbas et al. (1992, Proc. Natl. Acad. Sci. USA 89: 4457) attempted to circumvent the problem of limited repertoire sizes resulting from using biased variable region sequences by randomizing the sequence in a synthetic CDR region of a human tetanus toxoid-binding Fab.

U.S. Patent 5922545 discloses improved methods and novel compositions for identifying peptides and single-chain antibodies that bind to predetermined receptors or epitopes. Such peptides and antibodies are identified by improved and novel methods for affinity screening of polysomes displaying nascent peptides.

U.S. 6300064 relates to synthetic DNA sequences which encode one or more collections of homologous proteins/(poly)peptides, and methods for generating and applying libraries of these DNA sequences. In particular, the invention relates to the preparation of a library of human-derived antibody genes by the use of synthetic consensus sequences which cover the structural repertoire of antibodies encoded in the human genome. Furthermore, the invention relates to the use of a single consensus antibody gene as a universal framework for highly diverse antibody libraries.

In the present invention, antigen binding peptides or CDRs are fused to Tf and mTf to generate an improved antibody library. In particular, phage display technology may be used to generate large libraries of CDRs by exploiting the capability of bacteriophage to express and display Tf fusion protein molecules comprising functional CDRs on the bacteriophage's surface. In other embodiments, the library of CDRs may be prepared directly in modified Tf to create a library. Combinatorial libraries of antigen binding peptides have been generated in bacteriophage lambda expression systems which may be screened as bacteriophage plaques or as colonies of lysogens (Huse et al. (1989) Science 246: 1275; Caton and Koprowski (1990) Proc. Natl. Acad. Sci. (USA) 87: 6450; Mullinax et al. (1990) Proc. Natl. Acad. Sci. (USA) 87: 8095; Persson et al. (1991) Proc. Natl. Acad. Sci. (USA) 88: 2432). Bacteriophage antigen binding peptides display libraries and lambda phage expression libraries have been described (Kang et al. (1991) Proc. Natl. Acad. Sci. (USA) 88: 4363; Clackson et al. (1991) Nature 352: 624; McCafferty et al. (1990) Nature 348: 552; Burton et al. (1991) Proc. Natl. Acad. Sci. (USA) 88: 10134; Hoogenboom et al. (1991) Nucl. Acid Res. 19: 4133; Chang et al. (1991) J. Immunol. 147: 3610; Breitling et al. (1991) Gene 104: 147; Marks et al. (1991) J. Mol. Biol. 222: 581; Barbas et al. (1992) Proc. Natl. Acad. Sci. (USA) 89: 4457; Hawkins and Winter (1992) J. Immunol. 22: 867; Marks et al. (1992) Biotechnology 10: 779; Marks et al. (1992) J. Biol. Chem. 267: 16007; Lowman et al. (1991) Biochemistry 30: 10832; Lerner et al. (1992) Science 258: 1313). Also see review by Rader, C. and Barbas, C. F. (1997) "Phage display of combinatorial antibody libraries" Curr. Opin. Biotechnol. 8:503-508.

As part of this invention, transferrin or part of transferrin containing random peptides can be inserted into gene 3 of the phage instead of V_{L} or V_{H} fragments. In this manner the library can be screened for a transferrin protein which contains a peptide with a desired function.

In a similar manner, a library can express transferrin containing various inserted peptides instead of antibody fragments. This library is then screened for the peptide with the best binding activity for a particular target.

The diversity of the library of antibodies or peptides is preferably between about 10⁶ -10¹⁶, more preferably between about 10⁸ -10¹⁶, and most preferably between about 10¹⁰ - 10¹⁶.

### Examples of Peptide Libraries

Various peptide libraries have been generated for screening peptides. Some are commercially available. Using Tf as the scaffold protein, similar Tf peptide libraries could be generated using the present method.

Generally, two types of peptide libraries can serve as source of peptide epitopes: random peptide libraries (RPLs) and natural peptide libraries (NPLs). In RPLs, the phage-displayed peptides are encoded by synthetic random degenerate oligonucleotide inserts spliced into coat protein genes. In NPLs, the phage particles display fragments of natural proteins encoded by short DNA fragments of the genome of an organism of interest, *e*.*g*. a pathogen or an organism known to produce proteins or peptides with desirable properties including therapeutic properties.

Phage libraries based on well-characterized protein structures have been made. In these libraries, a single highly structured protein is selected and the amino acids in one portion of this parental protein are varied. Only the regions of the protein that are accessible to the surface are varied since it is these regions that are available for binding of target, while regions of the protein that are involved in maintaining its structure are not varied.

Human phage antibody libraries have also been generated. They contain genes encoding the heavy and light chain variable regions of the antibody producing cells of human donors which are displayed in the phage library as antibody fragments (Fabs). The library design includes the capability to rapidly produce and purify soluble Fabs.

Linear peptide phage libraries in which all amino acids, except cysteine, at each position in a 20-mer peptide are varied to create large libraries. These libraries have been used to identify novel linear peptides that may be used as therapeutics or as affinity ligands for the purification of therapeutic targets.

Peptide substrate phage libraries contain variations in the peptide sequence that serves as the recognition site for specific enzymes. These libraries have been used to identify novel peptides that are better substrates for specific enzymes.

Enzyme libraries that may be used to identify enzymes with made-to-order substrate specificities and enantioselectivities have been made. Industrially relevant enzymes are used as the parental protein, and the amino acids in relevant portions are varied to obtain enzyme variants with the desired function. For example, these phage display libraries can be used to create catalysts that perform the same class of reaction, but with different substrate and stereoselectivities.

U.S. Patent 6,573,098 discloses a method for DNA reassembly after random fragmentation, and its application to mutagenesis of nucleic acid sequences by *in vitro* or *in vivo* recombination. In particular, a method for the production of nucleic acid fragments or polynucleotides encoding mutant proteins is described. The invention also relates to a method of repeated cycles of mutagenesis, shuffling and selection which allow for the directed molecular evolution *in vitro* or *in vivo* of proteins.

### Brief Summary of Steps for Generating Tf Peptide Libraries in Bacteriophage

Peptide display is based primarily on the screening of peptides or proteins, such as single chain antibodies (SCA), for binding activity against a chosen target where the peptide or protein is inserted and displayed on the surface of bacteriophage, for example at the N-terminus of the bacteriophage M13 coat protein pIII.

In the case of peptides, an M13 phage library may be generated by splicing a randomized DNA sequence, coding for a randomized set of amino acids, 3' of the N-terminus of Tf which is fused to the bacteriophage pIII protein and immediately after the signal sequence cleavage site. These peptide sequences are typically in the range from 6, 8, 12, 15 or 20 amino acids in length. However, as the size of the peptide insert increases so does the potential complexity of the library. For example, a 6mer library using all 20 amino acids generates 10⁸ possible combinations; a 20mer library generates 10²⁶ possible combinations. In order to make the library representative at the screening stage, the initial library is usually amplified to give a five fold or greater duplication of all the possible sequences within the library.

Peptides can be linear or constrained, a constrained library is achieved by the addition of two cysteine residues within the peptide sequence such that a disulphide bond is formed within the peptide. Peptides can also be conserved at certain residues, particularly if the library is based on an existing sequence in which certain key residues are already determined.

The means of generating and screening phage libraries are well documented (see http://www.biosci.nussouri.edu/smithgp/PhageDisplayWebsite/PetrenkoSmithChemReview s.PDF). Briefly, a suitable host *E. coli* strain is infected with the library to generate phage particles. At the tip of the phage particle the product of gene III, pIII, is displayed along with the inserted peptide. These particles are then screened against a target and, as the DNA and peptide it encodes are inextricably linked in the phage particle, the amino acid sequence of the binding peptide(s) can be determined from the DNA sequence.

The same principle of screening large numbers of randomized sequences applies to both peptides and SCAs, the difference being that with SCA, the peptides are the hypervariable sequences in the CDR's of the heavy and light chains of the antibody.

For phage display using a protein such as a Tf, the methodology becomes a little more complicated than for a simple peptide as the size of the bacteriophage DNA exceeds that which can be packaged efficiently into the phage particle. This requires the use of a two vector system. The Tf protein-pIII fusion is carried on one vector, or phagemid, which is essentially a plasmid containing the Tf protein-pIII fusion DNA plus a functional phage packaging sequence. This vector cannot generate viable phage particles. To generate phage particles the host *E. coli* strain is infected with the second vector, or helper phage, which is a wild type M13 bacteriophage carrying a nonfunctional packaging signal. The helper phage provides all the genes required to make and assemble the proteins of a phage particle but is unable to package its DNA in to the phage particle. Only the phagemid DNA carries a functional packaging signal, and thus, it is only the phagemid DNA that is packaged into the phage particles.

On a single phage particle, there are five copies of the pIII protein. In a peptide display system all five copies typically carry the Tf peptide. This can result in the selection of peptides with lower affinity than might initially be indicated due to avidity effects. The protein display system, because the helper phage carries the gene for making native copies of the gene III protein, allows for the incorporation of usually only one or two copies of protein-pIII fusion. This has two effects, the first is better infectivity when the isolated phage requires propagation and the second, more importantly, is the selection of higher affinity peptides.

One can generate oligonucleotides which contain the same restriction sites at each end separated by a random set of nucleotides coding for the random peptides. The restriction sites are the same as the ones on the N terminus of Tf. The size of the peptide depends on the length of the random nucleotide region in these oligonucleotides.

The expression vector containing Tf is cut with the appropriate restriction enzymes and the oligonucleotides are inserted in that site. After ligation and transformation, the yeast can produce Tf with the added random peptides at its N-terminus.

Similar protocol could be used for attaching the peptides to the C-terminus or to the interior of Tf except that appropriate restriction sites at the 3' and 5' ends of the oligonucleotides should be used to allow for proper insertion.

The library can then be screened for peptides that have affinity for a specific molecule of interest. The screening can be done by the limiting dilution method similar to monoclonal antibody selection or by using replica blots of the culture plates which are probed with the labeled molecule of interest.

After selection, the selected clones are grown and the expressed Tf peptide is purified using affinity and/or ion exchange chromatography. The purified material can be used for *in vitro* and *in vivo* studies.

### Affinity Maturation

Affinity maturation refers to the increase in the affinity for the specific antigen of the antibodies produced during the course of a humoral immune response. It is particularly prominent in secondary and subsequent immunizations.

U.S. Patent 6,573,098 discloses a method for generating libraries of displayed polypeptides or displayed antibodies suitable for affmity interaction screening or phenotypic screening. The method comprises (1) obtaining a first plurality of selected library members comprising a displayed polypeptide or displayed antibody and an associated polynucleotide encoding said displayed polypeptide or displayed antibody, and obtaining said associated polynucleotides or copies thereof wherein said associated polynucleotides comprise a region of substantially identical sequence, optionally introducing mutations into said polynucleotides or copies, and (2) pooling and fragmenting, by nuclease digestion, partial extension PCR amplification, PCR stuttering, or other suitable fragmenting means, typically producing random fragments or fragment equivalents, said associated polynucleotides or copies to form fragments thereof under conditions suitable for PCR amplification, performing PCR amplification and optionally mutagenesis, and thereby homologously recombining said fragments to form a shuffled pool of recombined polynucleotides, whereby a substantial fraction (*e*.*g*., greater than 10 percent) of the recombined polynucleotides of said shuffled pool are not present in the first plurality of selected library members, said shuffled pool comprising a library of displayed polypeptides or displayed antibodies suitable for affinity interaction screening. Optionally, the method comprises the additional step of screening the library members of the shuffled pool to identify individual shuffled library members having the ability to bind or otherwise interact (*e*.*g*., such as catalytic antibodies) with a predetermined macromolecule, such as for example a proteinaceous receptor, peptide, oligosaccharide, virion, or other predetermined compound or structure. The displayed polypeptides, antibodies, peptidomimetic antibodies, and variable region sequences that are identified from such libraries can be used for therapeutic, diagnostic, research, and related purposes (*e*.*g*., catalysts, solutes for increasing osmolarity of an aqueous solution, and the like), and/or can be subjected to one or more additional cycles of shuffling and/or affinity selection. The method can be modified such that the step of selecting is for a phenotypic characteristic other than binding affinity for a predetermined molecule (*e*.*g*., for catalytic activity, stability, oxidation resistance, drug resistance, or detectable phenotype conferred on a host cell).

A preferred method for selection of a phage displaying a protein molecule with a desired specificity or affinity will often be elution from an affinity matrix with a ligand. Elution with increasing concentrations of ligand should elute phage displaying binding molecules of increasing affinity.

Another preferred method for selection according to affinity would be by binding to an affinity matrix containing low amounts of ligand. As a preferred strategy, a population of phage is bound to an affinity matrix which contains a low amount of ligand. Phages displaying high affinity and low affinity proteins compete for binding to the ligand on the matrix. Phage displaying high affinity protein is preferentially bound and low affinity protein is washed away. The high affinity protein is then recovered by elution with the ligand or by other procedures which elute the phage from the affinity matrix.

Any method of affinity maturation such as those described above may be used with the Tf scaffold libraries described herein. Accordingly, the present invention includes methods of using affinity maturation techniques to select peptides or other molecules for desired characteristics within the context of the Tf scaffold libraries herein described.

Without further description, it is believed that a person of ordinary skill in the art can, using the preceding description and the following illustrative examples, make and utilize the present invention and practice the claimed methods. For example, a skilled artisan would readily be able to determine the biological activity, both *in vitro* and *in vivo*, for the fusion protein constructs of the present invention as compared with the comparable activity of the therapeutic moiety in its unfused state. Similarly, a person skilled in the art could readily determine the serum half life and serum stability of constructs according to the present invention. The following working examples therefore, specifically point out the preferred embodiments of the present invention, and are not to be construed as limiting in any way the remainder of the disclosure.

### EXAMPLES

### Example 1

A fusion protein comprising modified Tf and an antifusogenic HIV-1 peptide (T-20) is made by fusing one or more copies of the nucleotide sequence encoding the peptide to the nucleotide sequence of mTf to produce a fusion protein with a peptide fused to the N- or C-terminus of Tf. Alternatively, the peptide may be fused internally into mTf.

In one embodiment, the Tf portion of the fusion protein is engineered not to allow glycosylation when produced in yeast. As discussed above, human transferrin has two N-linked glycosylation sites at N413 and N611; the N-linked glycosylation site comprises the sequence N-X-S/T. In one embodiment, N (Asn) is changed to Q (Gln); other changes are contemplated such as Asn to Ala or Ser or any other amino acid.

Specifically, the N413 and N611 codons are converted to GAT and GAC by oligonucleotide directed mutagenesis using the dut- and ung- method. See Kunkel et al. (1985) Proc. Natl. Acad. Sci. 82:488-492). The mutagenic oligonucleotides 5'-GCAGAAAACTACGATAAGAGCGATAAT-3' (SEQ ID NO: 9) and 5'-CTATTTGGAAGCGACGTAACTGACTGC-3' (SEQ ID NO: 10) are synthesized and used to mutagenize the N413 and N611 codons according to the methods of Funk et al. (U.S. Patent 5,986,067).

Receptor binding and/or iron or carbonate binding are then disrupted by mutating the following receptor binding residues and/or iron and/or carbonate ion binding residues:

| **Iron binding** | |
|---|---|
| N domain | C domain |
| Asp 63 (Asp 82 of SEQ ID NO: 2) | Asp 392 (Asp 411 of SEQ ID NO: 2) |
| Tyr 95 (Tyr 114 of SEQ ID NO: 2) | Tyr 426 (Tyr 445 of SEQ ID NO: 2) |
| Tyr 188 (Tyr 207 of SEQ ID NO: 2) | Tyr 514 or 517(Tyr 533 or Tyr 536 SEQ ID NO:2) |
| His 249 (His 268 of SEQ ID NO: 2) | His 585 (His 604 of SEQ ID NO: 2) |

| **Carbonate ion binding** | |
|---|---|
| N domain | C domain |
| Thr 120 (Thr 139 of SEQ ID NO: 2) | Thr 452 (Thr 471 of SEQ ID NO: 2) |
| Arg 124 (Arg 143 of SEQ ID NO: 2) | Arg 456 (Arg 475 of SEQ ID NO: 2) |
| Ala 126 (Ala 145 of SEQ ID NO: 2) | Ala 458 (Ala 477 of SEQ ID NO: 2) |
| Gly 127 (Gly 146 of SEQ ID NO: 2) | Gly 459 (Gly 478 of SEQ ID NO: 2) |

The production of mutants deficient in iron binding may be accomplished by numerous techniques. See U.S. Patent 5,986,067. A D63S substitution may be prepared using the method of Nelson, R. M. and Long, G. L. (1989) Anat. Biochem. 180:147-151. Briefly, an *Hpa*II/*Bam*HI fragment from the 5' end of the hTF/2N (human Tf with double N domain) coding sequence is subcloned into pUC18 and then used as a template for a two step PCR-based mutagenesis procedure. The fragment is then released from the double stranded form of the sequencing vector by digestion with *Xba*I and *BamH*I and then ligated to a *BamH*I/*Hind*III fragment from the original human Tf construct to produce a full length D63S-coding sequence.

For expression in *Pichia pastoris* the system from RCT/Invitrogen can be used. Three vectors are available for multicopy expression, pPIC9K, pPIC3.5K and pAO815. For this example the pPIC9K vector, which allows secretion into the growth medium, is used.

The modified transferrin sequence was cloned into the pPIC9K vector by altering the ends of the transferrin cDNA by overlapping PCR mutagenesis, this yielded the vector pREX0010. A number of restriction sites within the vector and coding sequence were removed or added to aid later cloning steps.

The sequence for the HIV anti-fusogenic peptide DP-178 is also known as T-20. This peptide is fused at the N- or C- termini of Transferrin, as the peptide may need freedom of movement to fulfill its function.

DP-178 sequence: YTSLIHSLIEESQNQQEKNEQELLELDKWASLWNWF (SEQ ID NO: 4)

When back translated in to DNA (using codons optimized for yeast) the following sequence was obtained (SEQ ID NOS: 13 and 14):

To insert the above sequence the vector pREX0010 with the modified transferrin cDNA, was digested with the restriction enzymes *Xba*I/*Kpn*I for insertion at the 5' end and *Sal*I/*Hind*III for insertion at the 3' end.

For the 5' insertion two overlapping oligos that form an *Xba*I overhang at the 5' end and a *Kpn*I overhang at the 3' end of the DP-178 sequence given above were synthesized. These oligos were then annealed together (see below) and ligated into the *Xba*I/*Kpn*I digested pREX0010 vector.

Insertion of the annealed oligos resulted in loss of the *Kpn*I site upon insertion. This resulted in the vector pREX0011.

For insertion at the C-terminus a similar approach was taken by the addition of a *Sal*I site at the 5' end and a *Hin*dIII at the 3' end.

Transformation, selection and expression were then performed as described in the Invitrogen *Pichia* Expression kit protocol booklet.

### Example 2

INGAP fusions are prepared using a reverse translated human INGAP amino acid sequence. The protein sequence from is as follows: sp|Q92778|PBCG_HUMAN Human INGAP (SwissProt): Reverse translated into DNA (codons optimized for yeast) gave the following (SEQ ID NO: 18 and 19).

The most likely point for cleavage of the leader sequence is C-terminal to the KK at the end of the underlined sequence above.

One methodology which may be used to generate constructs for the expression of INGAP fused to the N- or C-terminus of transferrin is to synthesize a series of overlapping oligos designed from the sequence given above (minus the underlined leader sequence). Annealing of these primers generate the mature INGAP cDNA. With different oligos designed for the 5' and 3' ends the annealed cDNA can be ligated into pREX0010 at the 5' or 3' end of the transferrin coding sequence.

The bracketed sequence is the peptide used to induce INGAP activity. Hence the sequence could be shortened to some point between the whole and this minimal sequence.

### N-terminal fusion.

For the N-terminus, the encoding nucleic acid would have an overhang which forms an *Xba*I site at the 5' end and an overhang compatible with a *Kpn*I site at the 3' end but which results in the destruction of the *Kpn*I site upon ligation.

Digestion of pREX0010 with *Xba*I and *Kpn*I and ligation of the above sequence yielded the vector pREX0013.

### C-terminal fusion.

For the C-terminus the 5' end would form a *Sal*I site and at the 3' end a stop codon plus a *Hin*dIII site.

Digestion of pREX0010 with *Sal*I and *Hin*dIII and ligation of the above sequence yielded the vector pREX0014.

Transformation, selection and expression were then performed as described in the Invitrogen *Pichia* Expression kit protocol booklet.

### Example 3

The peptide given below has been shown to mimic EPO activity by causing dimerization of the EPO receptor. The peptide, which is cyclic, has no homology to EPO. For activity the peptide has to act in concert with another peptide, *i*.*e*. as a dimer, such that two molecules of the receptor are brought in close enough proximity to form an active complex. As with many peptides the peptide dimer suffers from short half life and would benefit from the longevity that fusion to transferrin would give. In this example two peptides were engineered into the transferrin scaffold.

As detailed by Ali *et al*, a peptide can be successfully engineered into Transferrin between His289 and Gly290. The duplication inherent to the transferrin molecule, with the two domains mirroring each other, suggests that it may be possible to engineer a peptide into the analogous region of the C domain, between Glu625 and Thr626. For each insertion two overlapping mutagenic primers were synthesized (see below). Using pREX0010 as a template reactions were performed with each mutagenic primer and an external primer from the 5' or 3' of the Tf cDNA. The products from these two reactions were then mixed and a further reaction performed with the external primers to join the two products together. The His289-Gly290 insert PCR product was digested with *Xba*I and *Hpa*I for ligation into *Xba*I/*Hpa*I digested pREX0010. The resulting vector was then digested with *Hpa*I and *Sal*I for ligation of *Hpa*I/*Sal*I digested the Glu625-Thr626 insert PCR product.

These gave the plasmid pREX0015. Transformation, selection and expression were then performed as described in the Invitrogen *Pichia* Expression kit protocol booklet.

Alternative points for insertion of the EPO mimetic peptide(s), or any other peptide(s) are the two glycosylation sites on the C domain of Transferrin at N413 and N611. The advantage of this would be that insertion is achieved and glycosylation prevented, by disruption of the N-X-S/T sequence in one and the same event.

### Example 4

Fusion proteins between Tf and fusogenic inhibitor peptides against RSV are made by fusing the peptide sequences to the N- or C- terminal ends of Tf or by the insertion of the sequences into a loop of Tf, wherein the Tf is modified not to bind iron and/or is modified to prevent glycosylation. The RSV peptide may include: T786:
VYPSDEYDASISQVNEEINQALAYIRKADELLENV (SEQ ID NO: 5) and/or T1584:
AVSKVLHLEGEVNKIKSALLSTNKAVVSLSNGVSVLTSKVLDLKNYIDKQL (SEQ ID NO: 6).

The T786 peptide has a RK dipeptide which could act as a cleavage site for the yeast protease Kex2p. This would result in a truncated peptide. Accordingly, this peptide may be modified from RK to RE. Another version of the T786 peptide, T112 (VFPSDEFDASISQVNEKINQSLAFIRESDELLHNV, SEQ ID NO: 7), which is more potent than T786 has solubility problems in its unfused form. Accordingly, a version of T112 modified for the RK to RE is also made to produce a version of the peptide fused to Tf.

To produce the genetic constructs, the peptide sequences are backtranslated in DNA using codon bias for human, yeast or any other organism as appropriate.

### Example 5

Various cytokines can be fused to the N-, C- or N- and C- termini ofTf. These fusions can also be constructed using different parts or domains of modified transferrin such as the N domain or C domain. The proteins can be fused directly or using a linker peptide of various lengths. It is also possible to fuse all or part of the active cytokine within the scaffold of transferrin.

The cDNA for the cytokine of interest, such as EPO, can be isolated by a variety of means such as RT-PCR from mRNA, from cDNA libraries, by synthetically constructing the cDNA from overlapping oligonucleotides, by PCR or by other means known to the art, all using standard methods. The nucleotide sequences for all of these proteins are known and available, for instance, in U.S. Patents 4,703,008, 4,810,643 and 5,908,763 as well as in public databases such as GenBank. The cDNA can be tailored at the 5' and 3' ends to generate restriction sites, such that oligonucleotide linkers can be used, for cloning of the cDNA into a vector containing the cDNA for Transferrin. This can be at the N- or C-terminus, with or without the use of a spacer sequence, or by inserting the cDNA of the cytokine within the cDNA of Transferrin. The cytokine, *e.g.* EPO, and Tf cDNA are cloned into a vector from which the complete expression cassette is then excised and inserted into an expression vector to allow the expression of the fusion protein in yeast (or any other appropriate expression system). The fusion protein secreted from the yeast can then be collected and purified from the media and tested for its biological activity.

For expression in mammalian cell lines, a similar procedure is adopted except that the expression cassette used employs a mammalian promoter, leader sequence and terminator. This expression cassette is then excised and inserted into a plasmid suitable for the transfection of mammalian cell lines.

### Example 6

Various interferons can be fused to the N-, C- or N- and C- termini of transferrin or of modified transferrin. These fusions can be constructed using different parts or domains of transferrin such as the N domain or C domain. The proteins can be fused directly or using a linker peptide of various lengths. It is also possible to fuse all or part of the interferon within the scaffold of transferrin.

A specific example of an interferon that can be fused to Tf is interferon-β. The cDNA for the interferon of interest such as IFN β can be isolated by a variety of means such as RT-PCR from mRNA or cDNA, from cDNA libraries, by synthetically constructing the cDNA from overlapping oligonucleotides, by PCR or by other means known to the art, all using standard methods. The nucleotide sequences for interferons, such as IFNα, IFNβ, and IFNγ are known and available, for instance, in U.S. Patents 5,326,859 and 4,588,585, in EP 32 134, as well as in public databases such as GenBank. The cDNA can be tailored at the 5' and 3' ends to generate restriction sites, such that oligonucleotide linkers can be used to clone the cDNA into a vector containing the cDNA for modified transferrin. This can be at the N-, C- or N- and C-termini of the transferrin sequence, with or without the use of a spacer sequence. The IFN β (or other interferon) cDNA is cloned into a vector from which the complete expression cassette is then excised and inserted into an expression vector to allow the expression of the fusion protein in yeast. The fusion protein secreted from the yeast can then be collected and purified from the media and tested for its biological activity.

For expression in mammalian cell lines a similar procedure is adopted except that the expression cassette used employs a mammalian promoter, leader sequence and terminator. This expression cassette is then excised and inserted into a plasmid suitable for the transfection of mammalian cell lines. IFNs fused to transferrin have much longer half-life, thus, the therapeutic dosages of the fused proteins are much less than the IFNs. Therefore, the fused interferons are more efficacious with much less toxicity.

### Example 7

Various single chain antibodies (SCA) were originally invented to simplify antibody selection and production. However, they prove to be of limited or no therapeutic values due to their small size and short *in vivo* half-life. Addition of transferrin to SCA significantly increases the *in vivo* half-life of SCA.

SCA can be fused to the N-, C- or N- and C- termini of modified transferrin. These fusions could also be carried out using different parts or domains of transferrin such as the N domain or C domain. The proteins could be fused directly or using a linker peptide of various length. It is also possible to fuse all or part of the active SCA within the scaffold of transferrin. In such instances the fusion protein is made by inserting the cDNA of the SCA within the cDNA of transferrin for production of the protein in cells. A specific example of a SCA that can be fused to Transferrin is anti-TNF (tumor necrosis factor; Figs. 4A-4B). Anti-TNF has been used to treat various inflammatory and autoimmune diseases. TNF-SCA could be fused to the N- or C- terminus of modified transferrin in such manner that the coding N-terminus of TNF-SCA is directly attached to the C-terminal amino acid of Transferrin or the C-terminal amino acid of TNF-SCA is directly attached to the N-terminal amino acid of Transferrin. Alternatively, a peptide linker could be inserted to provide more separation between Transferrin and TNF-SCA and allow more spatial mobility to the two fused proteins. Several examples of TNF-SCA are shown in Figures 4A-4B.

Single chain antibodies are produced by several methods including but not limited to: selection from phage libraries, cloning of the variable region of a specific antibody by cloning the cDNA of the antibody and using the flanking constant regions as the primer to clone the variable region, or by synthesizing an oligonucleotide corresponding to the variable region of any specific antibody. The cDNA can be tailored at the 5' and 3' ends to generate restriction sites, such that oligonucleotide linkers can be used, for cloning of the cDNA into a vector containing the cDNA for transferrin. This can be at the N- or C-terminus or N- and C- termini with or without the use of a spacer sequence. The SCA molecule cDNA is cloned into a vector from which the complete expression cassette is then excised and inserted into an expression vector to allow the expression of the fusion protein in yeast. The fusion protein secreted from the yeast can then be collected and purified from the media and tested for its activity. For expression in mammalian cell lines a similar procedure is adopted except that the expression cassette used employs a mammalian promoter, leader sequence and terminator. This expression cassette is then excised and inserted into a plasmid suitable for the transfection of mammalian cell lines. The antibody produced in this manner can be purified from media and tested for its binding to its antigen using standard immunochemical methods.

### Example 8

CDRs are the variable regions of antibodies that interact with antigens. These usually consist of relatively short stretches of peptides. Antibodies normally have three CDRs in their heavy chains and three in their light chains. One or more CDRs of an antibody which can interact with the antigen can be fused to modified transferrin to confer antigen binding activity on transferrin molecule. The CDRs can be fused to the N-, C-, N- and C- termini or engineered into the interior scaffold of transferrin. Examples of the CDRs sequences from anti-TNF antibodies are shown in the TNF-SCA Figures 4A-4B. cDNAs corresponding to one or more CDRs can be fused with modified transferrin to confer TNF binding activity to transferrin.

### Example 9

Transferrin fusion technology can also be used to improve the therapeutic properties of peptides that are discovered in various systems such as phage display libraries and peptide libraries. Many of these peptides have biological activities without any homology to natural proteins or peptides. These peptides, due to their short *in vivo* half-lives, are good candidates for fusion to modified transferrin. Because of their small size they can be fused in variety of regions of the transferrin molecule. In addition to the N- and C- termini, they can be inserted in various regions within transferrin including but not limited to the cystine loops. In this manner the three-dimensional structure of the peptide within transferrin stays relatively rigid. More than one copy of each peptide and more than one peptide can be fused to modified transferrin. Moreover, the peptide sequence may be used to replace portion of transferrin to confer therapeutic activity to transferrin. Since most of these peptides are short, their cDNA can be synthesized with appropriate restriction sites for insertion into the modified transferrin cDNA. The cDNA could then be inserted in a vector containing the transferrin cDNA in such a manner that the peptide is expressed as part of transferrin or fused to transferrin molecule. Alternatively, PCR primers could be synthesized that contain the peptide of interest and appropriate section of transferrin. Using these primers amplification of transferrin cDNA results in the fusion of the peptide to the chosen site on transferrin. Examples of such peptides are the EPO mimetic peptides:
GGTYSCHFGPLTWVCKPQGG (SEQ ID NO: 11); DREGCRRGWVGQCKAWFN (SEQ ID NO: 12); and QRVEILEGRTECVLSNLRGRTRY (SEQ ID NO: 30), which have no homology with the natural EPO but have similar biological activities in that they activate the EPO receptor acting as agonists. These peptides also need to have specific conformation for their optimal activity. EPO mimetic peptides can be inserted in (or can replace) one or more cystine loops of transferrin. In this manner transferrin can acquire EPO activity. Other peptides that can be fused to transferrin are peptides with binding activity similar to antibodies. These peptides can bind to proteins with relatively high affmity and provide the same biological function as antibodies except their in vivo half-life is very short. Fusion of these peptides to transferrin could confer much longer half-life for these peptides without destroying their binding activities. These peptides could be fused to N- or C- terminus or both or within the transferrin molecule. The peptides can also replace part of transferrin. In addition more than one copy of a peptide or several different peptides could be attached to a single transferrin molecule. An example of such molecule is a peptide that can bind TNF. Attachment of this peptide to transferrin gives transferrin the ability to bind TNF and act similar to anti-TNF antibodies. In this manner antibody like molecules with much easier and economical manufacturing protocol could be made.

Also included in the present invention are enzyme inhibitory peptides fused to N- or C- terminus or both or within the transferrin molecule. The enzyme inhibitory peptides could be used in any manner including in pharmaceutical therapies or industrial processes.

### Example 10

Targeted Tf fusion proteins have a combination of two or more proteins or peptides fused to modified Tf to serve as a bifunctional molecule. In this case modified Tf is fused to one protein or peptide to have a new biological activity and to another protein or peptide to targeting. An example of such protein is a Tf that contains an inhibitory protein such as endostatin and a targeting peptide such as SCA or binding peptide which can recognize tumours. In this manner the inhibitory molecule is targeted to the tumour where it is needed. The cDNA for the protein of interest can be isolated from cDNA library or can be made synthetically using several overlapping oligonucleotide primers using standard molecular biology methods. The appropriate nucleotides can be engineered in the cDNA to form convenient restriction sites and also allow the attachment of the protein cDNA to transferrin cDNA similar to the method described for other fusions. Also a targeting protein or peptide cDNA such as single chain antibody or peptides, such as nuclear localization signals, that can direct proteins inside the cells can be fused to the other end or within transferrin. The protein of interest and the targeting peptide is cloned into a vector, which allows the fusion with transferrin cDNA. In this manner both proteins/peptides are fused to modified transferrin. The fused cDNA is then excised and is inserted into an expression vector to allow the expression of the fusion protein in yeast.

All the above procedures can be performed using standard methods in molecular biology. The fusion protein secreted from yeast can be collected and purified from the media and tested for its biological activity and its targeting activity using appropriate biochemical and biological tests. These proteins could also be made in other systems such as mammalian tissue culture using appropriate vector and transfection protocol.

### Example 11

The cDNA for an enzyme of interest can be isolated by a variety of means such as RT-PCR from mRNA, from cDNA libraries, by synthetically constructing the cDNA from overlapping oligonucleotides, by PCR or by other means known to the art, all using standard methods. The cDNA can be tailored at the 5' and 3' ends to generate restriction sites, such that oligonucleotide linkers can be used, for cloning of the cDNA into a vector containing the cDNA for modified transferrin. This can be at the N or C-terminus with or without the use of a spacer sequence. The enzyme cDNA is cloned into a vector such from which the complete expression cassette is then excised and inserted an expression vector to allow the expression of the fusion protein in yeast. The fusion protein secreted from the yeast can then be collected and purified from the media and tested for its biological activity. For expression in mammalian cell lines a similar procedure is adopted except that the expression cassette used employs a mammalian promoter, leader sequence and terminator. This expression cassette is then excised and inserted into a plasmid suitable for the transfection of mammalian cell lines.

### Example 12

Using phage display, peptides are isolated specific for a specific cell marker on the surface of, for example, a tumor cell. The peptide is then fused to the N-, C- or N- and C-termini of modified transferrin to target the fusion to that specific cell type. The transferrin fusion protein is then loaded with a metal ion which resembles iron in its transferrin binding properties, but which is cytotoxic, for example gallium or radioactive ions. By this mechanism the gallium or the radioactive ion is targeted to the cell type.

### Example 13

In one example, a system of peptide display for generating peptide sequences of the present invention utilizes the N-domain of transferrin incorporated into the pIII protein and the insertion of random peptides within the transferrin (Tf) scaffold. By their very nature these peptides are constrained and have the advantage of being selected in the environment, their position on the transferrin molecule, in which they will ultimately be used.

The first step is to clone the pIII gene from M13mp18 and engineer the N-domain of Transferrin into the N-terminus of pIII. Peptides are then inserted into exposed loops within the transferrin molecule.

### Construction of N-Domain Phage Display Library.

### Cloning of pIII.

The DNA sequence for pIII was PCR amplified using M13mp18 as a template for insertion into pUC18 under the control of the LacZ promoter. As pIII requires its own leader sequence for correct processing, the PCR product did not conveniently go into the multiple cloning site (MCS) of pUC18. The *Hin*dIII site of the MCS was used for ligation at the 3' end of pIII and allowed the construction of a double stop codon. However, for ligation at the 5' end a restriction site, *Nco*I, needed to be engineered around the start codon for the LacZ operon.

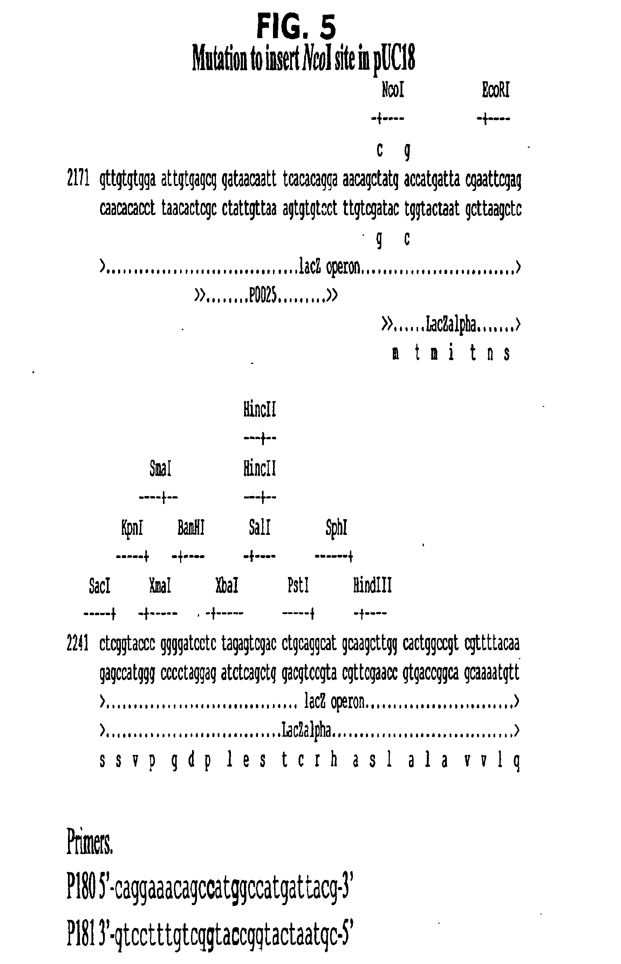

Utilizing the Stratagene QuickChange XL Site-Directed Mutagenesis Kit (Cat no. 20051) primers were designed for the mutations shown (Fig. 5). The product from this mutagenesis, pUC18 *Nco*I, was then digested *Nco*I/*Hin*dIII in preparation for the cloning of pIII.

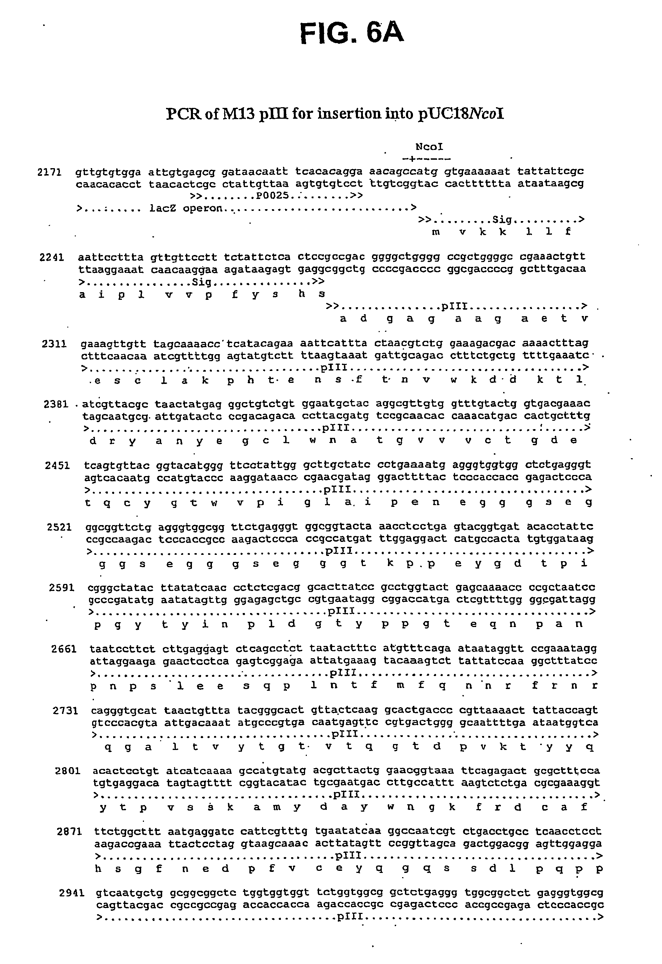

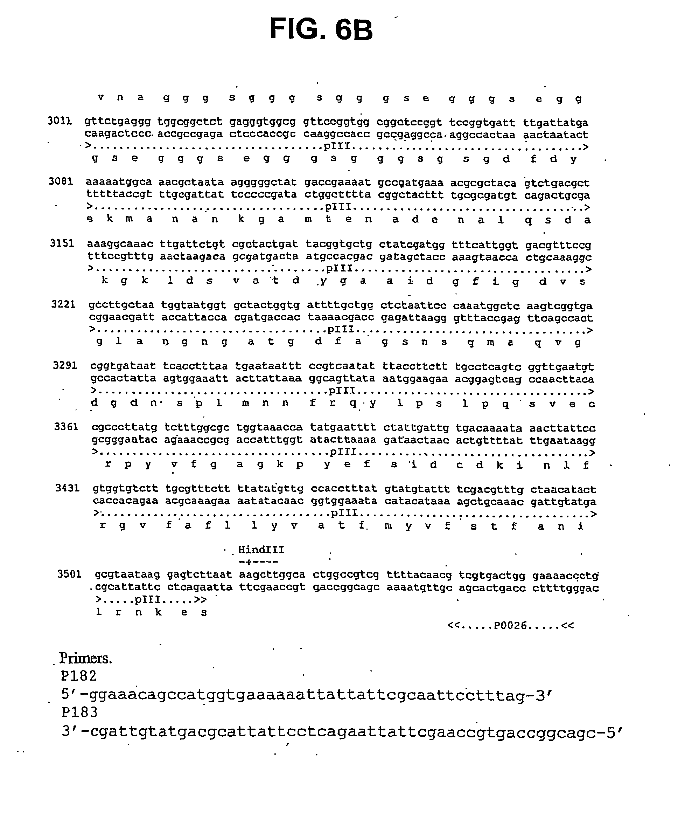

Primers were designed (Fig. 6) to introduce an *Nco*I site at the 5' of pIII and a double stop codon plus *Hin*dIII site at the 3' of pIII by PCR mutagenesis using M13mp18 as the template. These sites resulted in an in-frame insertion of pIII with the N terminal methionine of *Lac*Z and disruption of the normal *Lac*Z transcript when cloned into *Nco*I/*Hin*dIII digested pUC18*Nco*I as an *Nco*I/*Hin*dIII fragment. This gave the plasmid pUC18pIII.

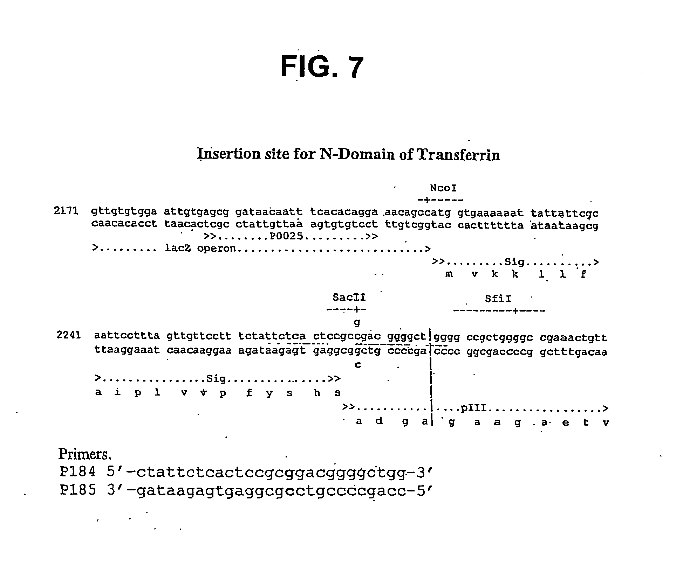

Primers were designed (Fig. 7) to introduce a *Sac*II site, using the Stratagene QuickChange XL Site-Directed Mutagenesis Kit (Cat no. 20051), at the junction between the pIII signal sequence and mature pIII resulting in the plasmid pUC18pIIISacII.

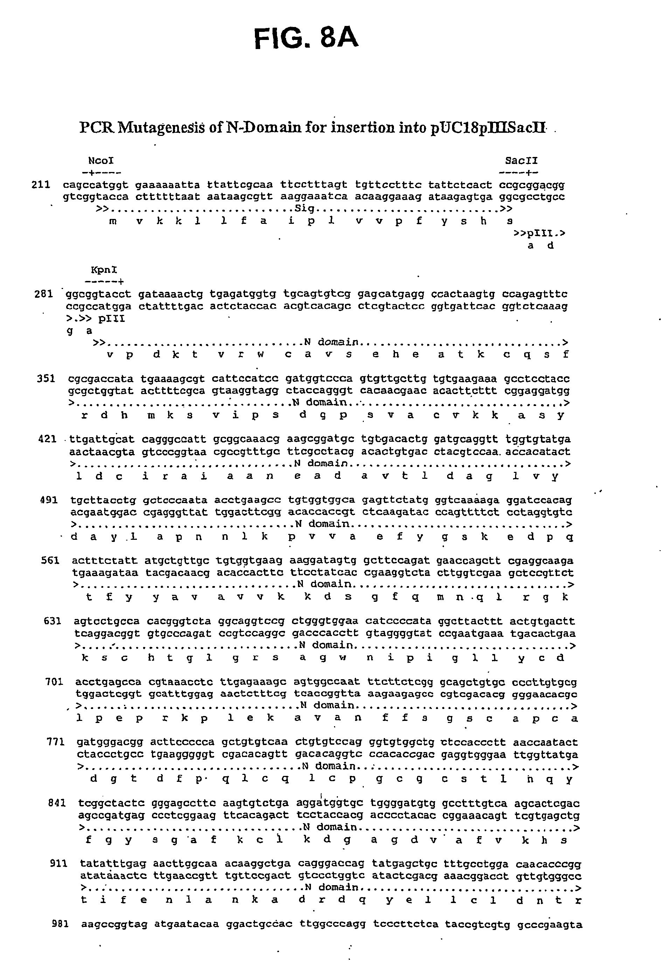

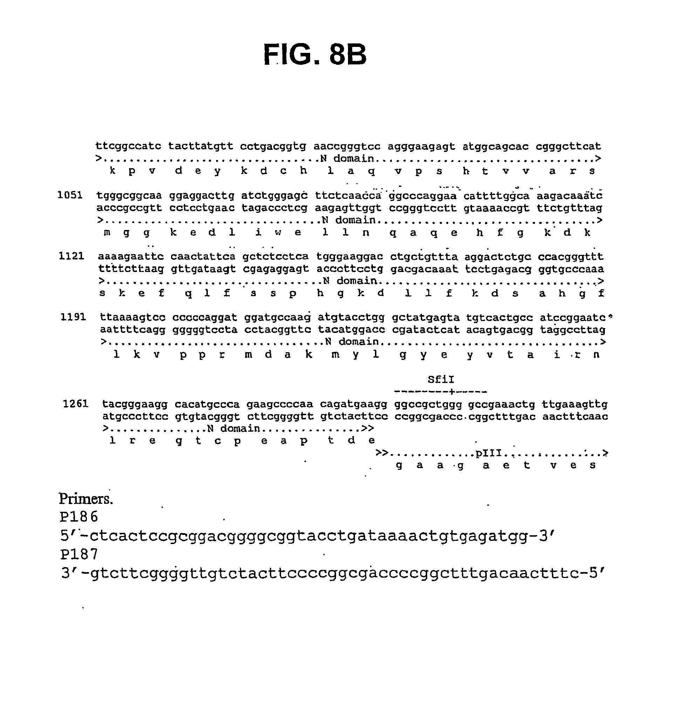

This allowed the N-domain of Transferrin to be modified by mutagenic PCR, with pREX0056 as the template, to introduce a 5' *Sac*II site and 3' *Sfi*I site (Fig. 8) for cloning into *Sac*II/*Sfi*I digested pUC18pIIISacII to give pUC18pIII Ndom.

### Insertion of Randomized Peptide Sequence

DNA encoding an eight amino acid randomized peptide sequence was spliced into the N domain of Transferrin between amino acids 288 and 289 for the purpose of generating a random peptide library presented on the surface of the N domain of Transferrin which can be screened against potential targets to isolate peptides that, in the context they are displayed, bind with high affinity. The methodology described can be used to splice DNA encoding a random peptide sequence(s) into other points on the surface of the Transferrin N domain. Due to the structural homology between the N and C domains of Transferrin, once a peptide has been isolated from the N domain library it can be duplicated in the equivalent position in the C domain to give multi-valency.

### Phage library

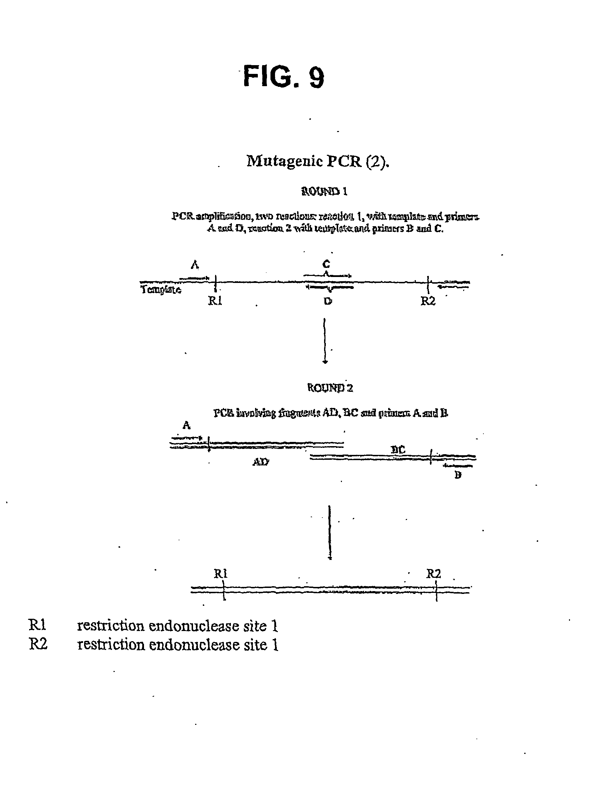

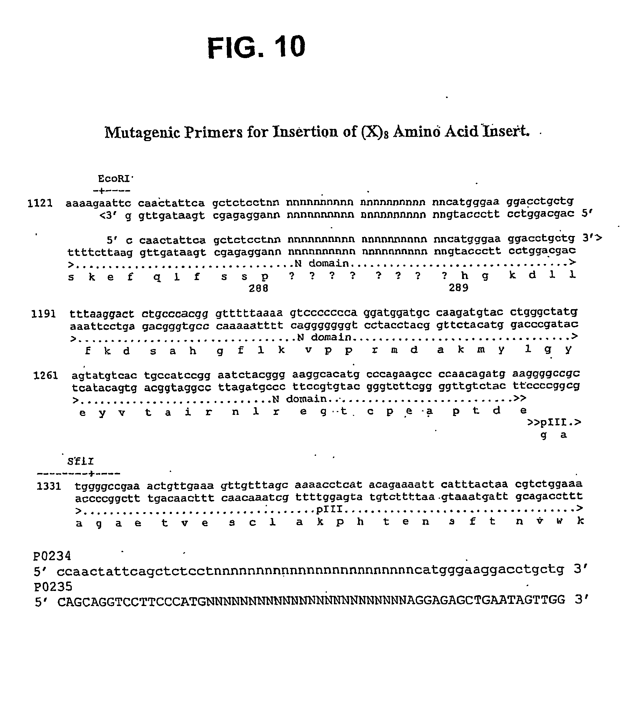

The randomized peptide sequence was generated by mutagenic PCR (Immunological Method Manual, Lefkovits, L. (Ed.) 1977 Academic Press) (Fig. 9). Two mutagenic oligonucleotide primers (C (P0234) & D (P0235)) were synthesized (Fig. 10). Using pUC18pIIINdom as the template and two outer primers (A & B) a DNA fragment was synthesized with the randomized sequence inserted. This fragment was digested with *Eco*RI/*Sfi*I and ligated into *Eco*RI/*Sfi*I digested pUC18pIIINdom. The ligated DNA was then transformed into library efficiency DH5α or other suitable *E. coli* strain. The library was checked by standard methods to determine library size and variability of insert sequence followed by amplification of the library for use in screening.

The library so constructed can be used in two ways. As is, the N domain-pIII fusion localizes to the cell membrane with the N domain orientated into the periplasmic space. By mild disruption of the outer membrane, whole cells can be screened against target antigen. The bacteria containing the phage display library can be plated on agar medium and allowed to grow and express the pIII protein. A replica filter paper from the plate is then probed with the target antigen. The bacterial colony which shows binding to the antigen can then be picked from the original plate and subcloned further. The sequence of the active peptide can be identified by isolating pIII-containing plasmid in the bacteria. Similar protocols can be used to isolate yeast containing peptides to a specific antigen. Since the yeast and bacterial colonies are localized, the protocol can be used for cell bound or secreted libraries.

By the addition of a functional packaging sequence into the pUC18pIIINdom vector the library can be used in the two vector system described earlier to generate phage particles which can be screened by various means as described in the literature.

### Yeast Library

Using the same set of mutagenic primers (P0234 and P0235) but a different set of outer primers and pREX0056 as the template, a PCR fragment with the randomized sequence is synthesized for construction of an N domain yeast display library. This is a secreted library or the expressed construct is anchored to the cell wall of an individual cell using a cell wall specific GPI anchor (Hamada et al., 1998, J. Biol. Chem. 273:26946-53) on a low copy number vector.

Although the present invention has been described in detail with reference to examples above, it is understood that various modifications can be made without departing from the spirit of the invention. Accordingly, the invention is limited only by the following claims.

## Claims

1. A phage library containing a plurality of fusion proteins, each comprising a first transferrin (Tf) polypeptide fused to at least one second peptide, wherein the Tf peptide has reduced affinity for a transferrin receptor (TfR).

2. A library of claim 1, wherein the Tf peptide comprises the N domain of a Tf protein.

3. A library of claim 1, wherein the Tf peptide consists of the N domain of a Tf protein.

4. A library of claim 1, wherein the Tf peptide comprises a portion of the N domain of a Tf protein.

5. A library of claim 1, wherein the Tf peptide exhibits reduced glycosylation.

6. A library of claim 1, wherein the second peptide is fused to the C-terminal end of the Tf peptide.

7. A library of claim 1, wherein the second peptide is fused to the N-terminal end of the Tf peptide.

8. A library of claim 1, wherein the second peptide is inserted within the Tf peptide.

9. A library of claim 8, wherein the second peptide is inserted in a surface exposed loop of the Tf peptide.

10. A library of claim 1, wherein the fusion proteins further comprise a flexible linker between the transferrin peptide and the random peptide.

11. A library of claim 1, wherein each of the second peptides in the library is different.

12. A library of claim 1, wherein the second peptide comprises at least one antibody complementarity-determining region (CDR).

13. A library of claim 12, wherein the second peptide comprises 3 different antibody CDRs.

14. A library of claim 1, wherein the second peptide comprises about 6 or more amino acids.

15. A library of claim 14, wherein the second peptide comprises about 6 to about 30 amino acids.

16. A library of claim 15, wherein the second peptide comprises about 6, 9, 12, 16, 19, 20, 25, or 30 amino acids.

17. A library of nucleic acid molecules encoding a library of claim 1.

18. A library of claim 1, wherein the library is a non-lytic phage display library.

19. A library of claim 1, wherein the library is a random peptide library or natural peptide library.

20. A library of claim 1, wherein the library is a structured peptide library, a protein library, a human antibody library, a linear peptide library, or an enzyme library.

21. A method of screening for a transferrin fusion protein having a particular activity comprising:
a) providing a phage library containing a plurality of transferrin fusion proteins, each comprising a first transferrin (Tf) peptide fused to at least one second peptide wherein the Tf peptide has reduced affinity for a transferrin receptor (TfR); and
b) screening the library to identify transferrin fusion proteins having the particular activity.

22. A method of claim 21, further comprising isolating at least one fusion protein identified in step (b).

23. A library of claim 1, wherein the fusion protein comprises a phage pIII protein.

24. A library of claim 1, wherein the Tf peptide comprises at least one amino acid substitution, deletion or addition at an amino acid residue corresponding to an amino acid of SEQ ID NO: 3 selected from the group consisting of Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458, and Gly 459.

25. A library of claim 1, wherein the Tf peptide does not bind TfR.

26. A library of claim 25, wherein the Tf peptide comprises at least one amino acid substitution, deletion or addition at an amino acid residue corresponding to an amino acid of SEQ ID NO: 3 selected from the group consisting of Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458, and Gly 459.

27. A library of claim 1, wherein the Tf peptide has reduced affinity for iron.

28. A library of claim 1, wherein the Tf peptide does not bind iron.

29. A library of claim 1, wherein the Tf protein comprises at least one mutation that prevents glycosylation.

30. A library of claim 29, wherein the mutation corresponds a mutation at amino acid N413 or amino acid N611.

31. A library of claim 1, wherein the Tf peptide has reduced affinity for bicarbonate.

32. A library of claim 1, wherein the Tf peptide does not bind bicarbonate.

33. A library of claim 1, wherein the Tf peptide is modified in one or more of the internal sites selected from the group comprising the iron binding site, the hinge site, the bicarbonate binding site, and the receptor binding site.

34. A library of claim 1, wherein the Tf peptide comprises a single N domain of a Tf protein.

## Patentansprüche

1. Phagenbibliothek, die eine Vielzahl an Fusionsproteinen enthält, wobei jedes ein erstes Transferrin- (Tf-) Polypeptid umfasst, das an zumindest ein zweites Peptid fusioniert ist, worin das Tf-Peptid eine verringerte Affinität für einen Transferrin-Rezeptor (TfR) aufweist.

2. Bibliothek nach Anspruch 1, worin das Tf-Peptid die N-Domäne eines Tf-Proteins umfasst.

3. Bibliothek nach Anspruch 1, worin das Tf-Peptid aus der N-Domäne eines Tf-Proteins besteht.

4. Bibliothek nach Anspruch 1, worin das Tf-Peptid einen Abschnitt der N-Domäne eines Tf-Proteins umfasst.

5. Bibliothek nach Anspruch 1, worin das Tf-Peptid eine reduzierte Glykosylierung zeigt.

6. Bibliothek nach Anspruch 1, worin das zweite Peptid an das C-terminale Ende des Tf-Peptids fusioniert ist.

7. Bibliothek nach Anspruch 1, worin das zweite Peptid an das N-terminale Ende des Tf-Peptids fusioniert ist.

8. Bibliothek nach Anspruch 1, worin das zweite Peptid in das Tf-Peptid insertiert ist.

9. Bibliothek nach Anspruch 8, worin das zweite Peptid in eine Oberflächenschleife des Tf-Peptids insertiert ist.

10. Bibliothek nach Anspruch 1, worin die Fusionsproteine weiters einen flexiblen Linker zwischen dem Transferrinpeptid und dem Zufallspeptid umfassen.

11. Bibliothek nach Anspruch 1, worin jedes der zweiten Peptide in der Bibliothek unterschiedlich ist.

12. Bibliothek nach Anspruch 1, worin das zweite Peptid zumindest eine komplementaritätsbestimmende Region (CDR) eines Antikörpers umfasst.

13. Bibliothek nach Anspruch 12, worin das zweite Peptid 3 unterschiedliche Antikörper-CDRs umfasst.

14. Bibliothek nach Anspruch 1, worin das zweite Peptid etwa 6 oder mehr Aminosäuren umfasst.

15. Bibliothek nach Anspruch 14, worin das zweite Peptid etwa 6 bis etwa 30 Aminosäuren umfasst.

16. Bibliothek nach Anspruch 15, worin das zweite Peptid etwa 6, 9, 12, 16, 19, 20, 25 oder 30 Aminosäuren umfasst.

17. Bibliothek aus Nucleinsäuremolekülen, die für eine Bibliothek nach Anspruch 1 kodieren.

18. Bibliothek nach Anspruch 1, worin die Bibliothek eine nicht-lytische Phagendisplay-Bibliothek ist.

19. Bibliothek nach Anspruch 1, worin die Bibliothek eine Zufallspeptid-Bibliothek oder eine natürliche Peptidbibliothek ist.

20. Bibliothek nach Anspruch 1, worin die Bibliothek eine strukturierte Peptidbibliothek, eine Proteinbibliothek, eine menschliche Antikörperbibliothek, eine lineare Peptidbibliothek oder eine Enzymbibliothek ist.

21. Verfahren zum Screenen auf ein Transferrin-Fusionsprotein mit einer bestimmten Aktivität, umfassend:
a) Bereitstellen einer Phagenbibliothek, die eine Vielzahl an Transferrin-Fusionsproteinen enthält, wobei jedes ein erstes Transferrin- (Tf-) Peptid umfasst, das an zumindest ein zweites Peptid fusioniert ist, worin das Tf-Peptid eine verringerte Affinität für einen Transferrinrezeptor (TfR) aufweist; und
b) Screenen der Bibliothek zur Identifizierung von Transferrin-Fusionsproteinen mit der bestimmten Aktivität.

22. Verfahren nach Anspruch 21, das weiters das Isolieren von zumindest einem Fusionsprotein, das in Schritt (b) identifiziert wurde, umfasst.

23. Bibliothek nach Anspruch 1, worin das Fusionsprotein ein Phage-plll-Protein umfasst.

24. Bibliothek nach Anspruch 1, worin das Tf-Peptid zumindest eine Aminosäuresubstitution, -deletion oder -addition an einem Aminosäurerest umfasst, der einer Aminosäure von Seq.-ID Nr. 3 entspricht, die aus der aus Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 und Gly 459 bestehenden Gruppe ausgewählt ist.

25. Bibliothek nach Anspruch 1, worin das Tf-Peptid TfR nicht bindet.

26. Bibliothek nach Anspruch 25, worin das Tf-Peptid zumindest eine Aminosäuresubstitution, -deletion oder -addition an einem Aminosäurerest umfasst, der einer Aminosäure von Seq.-ID Nr. 3 entspricht, die aus der aus Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 und Gly 459 bestehenden Gruppe ausgewählt ist.

27. Bibliothek nach Anspruch 1, worin das Tf-Peptid eine verringerte Affinität für Eisen aufweist.

28. Bibliothek nach Anspruch 1, worin das Tf-Peptid Eisen nicht bindet.

29. Bibliothek nach Anspruch 1, worin das Tf-Protein zumindest eine Mutation umfasst, die Glykosylierung verhindert.

30. Bibliothek nach Anspruch 29, worin die Mutation einer Mutation an Aminosäure N413 oder Aminosäure N611 entspricht.

31. Bibliothek nach Anspruch 1, worin das Tf-Peptid eine verringerte Affinität für Hydrogencarbonat aufweist.

32. Bibliothek nach Anspruch 1, worin das Tf-Peptid Hydrogencarbonat nicht bindet.

33. Bibliothek nach Anspruch 1, worin das Tf-Peptid in einem oder in mehreren der inneren Stellen modifiziert ist, die aus der Gruppe ausgewählt sind, welche die Eisenbindungsstelle, die Gelenksstelle, die Hydrogencarbonat-Bindungsstelle und die Rezeptorbindungsstelle umfasst.

34. Bibliothek nach Anspruch 1, worin das Tf-Peptid eine einzelne N-Domäne eines Tf-Proteins umfasst.

## Revendications

1. Banque phage contenant une pluralité de protéines de fusion, comprenant chacune un premier polypeptide de transférine (Tf) fusionné à au moins un deuxième peptide, où le peptide Tf a une affinité réduite pour un récepteur de transférine (TfR).

2. Banque selon la revendication 1, dans laquelle le peptide Tf comprend le domaine N d'une protéine Tf.

3. Banque selon la revendication 1, dans laquelle le peptide Tf est constitué du domaine N d'une protéine Tf.

4. Banque selon la revendication 1, dans laquelle le peptide Tf comprend une portion du domaine N d'une protéine Tf.

5. Banque selon la revendication 1, dans laquelle le peptide Tf présente une glycosylation réduite.

6. Banque selon la revendication 1, dans laquelle le deuxième peptide est fusionné à l'extrémité terminale C du peptide Tf.

7. Banque selon la revendication 1, dans laquelle le deuxième peptide est fusionné à l'extrémité terminale-N du peptide Tf.

8. Banque selon la revendication 1, dans laquelle le deuxième peptide est inséré dans le peptide Tf.

9. Banque selon la revendication 8, dans laquelle le deuxième peptide est inséré dans une boucle à surface exposée du peptide Tf.

10. Banque selon la revendication 1, dans laquelle les protéines de fusion comprennent en outre un lien flexible entre le peptide de transférine et le peptide non ordonné.

11. Banque selon la revendication 1, dans laquelle chacun des deuxièmes peptides dans la banque est différent.

12. Banque selon la revendication 1, dans laquelle le deuxième peptide comprend au moins une région de détermination de complémentarité d'anticorps (CDR).

13. Banque selon la revendication 12, dans laquelle le deuxième peptide comprend 3 CDRs d'anticorps différentes.

14. Banque selon la revendication 1, dans laquelle le deuxième peptide comprend environ 6 acides aminés ou plus.

15. Banque selon la revendication 14, dans laquelle le deuxième peptide comprend environ 6 à environ 30 acides aminés.

16. Banque selon la revendication 15, dans laquelle le deuxième peptide comprend environ 6, 9, 12, 16, 19, 20, 25 ou 30 acides aminés.

17. Banque de molécules d'acide nucléique codant pour une banque de la revendication 1.

18. Banque selon la revendication 1, où la banque est une banque d'affichage de phages non-virulents.

19. Banque selon la revendication 1, dans laquelle la banque est une banque de peptides non ordonnés ou une banque de peptides naturels.

20. Banque selon la revendication 1, dans laquelle la banque est une banque de peptides structurés, une banque de protéines, une banque d'anticorps humains, une banque de peptides linéaires ou une banque d'enzymes.

21. Procédé de criblage d'une protéine de fusion de transférine ayant une activité particulière comprenant:
a) réaliser une banque phage contenant une pluralité de protéines de fusion de transférine, chacune comprenant un premier peptide de transférine (Tf) fusionné à au moins un deuxième peptide, où le peptide Tf a une affinité réduite pour un récepteur de transférine (TfR); et
b) cribler la banque pour identifier des protéines de fusion de transférine ayant l'activité particulière.

22. Procédé selon la revendication 21, comprenant en outre l'isolation d'au moins une protéine de fusion identifiée à l'étape (b).

23. Banque selon la revendication 1, dans laquelle la protéine de fusion comprend une protéine pIII phage.

24. Banque selon la revendication 1, dans laquelle le peptide Tf comprend au moins une substitution, délétion ou addition d'acide aminé à un résidu d'acide aminé correspondant à un acide aminé de la SEQ ID NO: 3 sélectionné dans le groupe consistant en Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 et Gly 459.

25. Banque selon la revendication 1, dans laquelle le peptide Tf ne se lie pas à TfR.

26. Banque selon la revendication 25, dans laquelle le peptide Tf comprend au moins une substitution, délétion ou addition d'acide aminé à un résidu d'acide aminé correspondant à un acide aminé de la SEQ ID NO: 3 sélectionné dans le groupe consistant en Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 et Gly 459.

27. Banque selon la revendication 1, dans laquelle le peptide Tf a une affinité réduite pour le fer.

28. Banque selon la revendication 1, dans laquelle le peptide Tf ne se lie pas au fer.

29. Banque selon la revendication 1, dans laquelle la protéine Tf comprend au moins une mutation qui empêche la glycosylation.

30. Banque selon la revendication 29, dans laquelle la mutation correspond à une mutation à l'acide aminé N413 ou à l'acide aminé N611.

31. Banque selon la revendication 1, dans laquelle le peptide Tf a une affinité réduite pour le bicarbonate.

32. Banque selon la revendication 1, dans laquelle le peptide Tf ne se lie pas au bicarbonate.

33. Banque selon la revendication 1, dans laquelle le peptide Tf est modifié dans un ou plusieurs des sites internes sélectionnés dans le groupe comprenant le site de liaison du fer, le site d'articulation, le site de liaison du bicarbonate et le site de liaison du récepteur.

34. Banque selon la revendication 1, dans laquelle le peptide Tf comprend un domaine N unique d'une protéine Tf.
